# EUROPEAN PATENT SPECIFICATION

(11) **EP 3 169 693 B1**
(45) Date of publication and mention of the grant of the patent: **09.03.2022**
(21) Application number: 15822890.8
(22) Date of filing: 16.07.2015
(51) Int. Cl.: C07H 21/02

(54) **CHIMERIC POLYNUCLEOTIDES**
CHIMÄRE POLYNUKLEOTIDE
POLYNUCLÉOTIDES CHIMÉRIQUES

(30) Priority: 16.07.2014 US 201462025399 P; 03.09.2014 US 201462045359 P
(43) Date of publication of application: 24.05.2017
(73) Proprietor: ModernaTX, Inc., Cambridge, MA 02139 (US)
(72) Inventor: NELSON, Jennifer Ann, Brookline, MA 02446 (US); FRALEY, Andrew, Arlington, Massachusetts 02474 (US); RHODEN SMITH, Amy, Somerville, Massachusetts 02143 (US)
(74) Representative: Hoffmann Eitle
(86) International application number: PCT/US2015/040699
(87) International publication number: WO 2016/011226

(56) References cited:
- WO-A1-2013/090186
- WO-A1-2014/081507
- US-A- 5 034 506
- US-A- 5 637 459
- US-A1- 2002 164 635
- US-A1- 2007 037 770
- US-A1- 2013 046 083
- MASAYASU KUWAHARA ET AL: "Molecular evolution of functional nucleic acids with chemical modifications", MOLECULES: A JOURNAL OF SYNTHETIC ORGANIC AND NATURAL PRODUCT CHEMISTRY, M D P I AG, CH, vol. 15, no. 8, 9 August 2010 (2010-08-09) , pages 5423-5444, XP002733356, ISSN: 1420-3049, DOI: 10.3390/MOLECULES15085423
- KUMAR R ET AL: "Template-directed oligonucleotide strand ligation, covalent intramolecular DNA circularization and catenation using Click chemistry", JOURNAL OF THE AMERICAN CHEMICAL SOCIETY, AMERICAN CHEMICAL SOCIETY, US, vol. 129, no. 21, 30 May 2007 (2007-05-30) , pages 6859-6864, XP002640516, ISSN: 0002-7863, DOI: 10.1021/JA070273V [retrieved on 2007-05-09]
- JESSICA A. STEWART ET AL: "Effect of Azide Position on the Rate of Azido Glucose-Cyclooctyne Cycloaddition", JOURNAL OF CARBOHYDRATE CHEMISTRY, vol. 33, no. 7-8, 14 July 2014 (2014-07-14), pages 408-419, XP055451687, UK ISSN: 0732-8303, DOI: 10.1080/07328303.2014.931963
- OLESIAK ET AL.: 'THE SYNTHESIS OF DI- AND OLIG-NUCLEOTIDES CONTAINING A PHOSPHOrodithioate internucleotide linkage with one of the sulfur atoms in a 5'bridging position' ORGANIC & BIOMOLECULAR CHEMISTRY vol. 7, 2009, pages 2162 - 2169, XP055387414
- None

## Description

### REFERENCE TO RELATED APPLICATIONS

This application claims priority to U.S. Provisional Patent Application No. 62/025,399, filed July 16, 2014, entitled Chimeric Polynucleotides and U.S. Provisional Patent Application No. 62/045,359, filed September 3, 2014, entitled Chimeric Polynucleotides

### SEQUENCE LISTING

The present application is being filed along with a Sequence Listing in electronic format. The Sequence Listing is provided as a file entitled M137SL.txt created on July 16, 2015 which is 17,221 bytes in size.

### FIELD OF THE INVENTION

Described herein are compositions, methods, processes, kits and devices for the design, preparation, manufacture and/or formulation of chimeric polynucleotides.

### BACKGROUND OF THE INVENTION

In the early 1990's Bloom and colleagues successfully rescued vasopressin-deficient rats by injecting in vitro-transcribed vasopressin mRNA into the hypothalamus (Science 255: 996-998; 1992). However, the low levels of translation and the immunogenicity of the molecules hampered the development of mRNA as a therapeutic and efforts have since focused on alternative applications that could instead exploit these pitfalls, *i.e.* immunization with mRNAs coding for cancer antigens.

More recently, others have investigated the use of mRNA to deliver a construct encoding a polypeptide of interest and have shown that certain chemical modifications of mRNA molecules, particularly pseudouridine and 5-methyl-cytosine, have reduced immunostimulatory effect.

These studies are disclosed in, for example, Ribostem Limited in United Kingdom patent application serial number 0316089.2 filed on July 9, 2003 now abandoned, PCT application number PCT/GB2004/002981 filed on July 9, 2004 published as WO2005005622, United States patent application national phase entry serial number 10/563,897 filed on June 8, 2006 published as US20060247195 now abandoned, and European patent application national phase entry serial number EP2004743322 filed on July 9, 2004 published as EP1646714 now withdrawn; Novozymes, Inc. in PCT application number PCT/US2007/88060 filed on December 19, 2007 published as WO2008140615, United States patent application national phase entry serial number 12/520,072 filed on July 2, 2009 published as US20100028943 and European patent application national phase entry serial number EP2007874376 filed on July 7, 2009 published as EP2104739; University of Rochester in PCT application number PCT/US2006/46120 filed on December 4, 2006 published as WO2007064952 and United States patent application serial number 11/606,995 filed on December 1, 2006 published as US20070141030; BioNTech AG in European patent application serial number EP2007024312 filed December 14, 2007 now abandoned, PCT application number PCT/EP2008/01059 filed on December 12, 2008 published as WO2009077134, European patent application national phase entry serial number EP2008861423 filed on June 2, 2010 published as EP2240572, United States patent application national phase entry serial number 12/,735,060 filed November 24, 2010 published as US20110065103, German patent application serial number DE 10 2005 046 490 filed September 28, 2005, PCT application PCT/EP2006/0448 filed September 28, 2006 published as WO2007036366, national phase European patent EP1934345 published March, 21, 2012 and national phase US patent application serial number 11/992,638 filed August 14, 2009 published as 20100129877; Immune Disease Institute Inc. in United States patent application serial number 13/088,009 filed April 15, 2011 published as US20120046346 and PCT application PCT/US2011/32679 filed April 15, 2011 published as WO20110130624; Shire Human Genetic Therapeutics in United States patent application serial number 12/957,340 filed on November 20, 2010 published as US20110244026; Sequitur Inc. in PCT application PCT/US1998/019492 filed on September 18, 1998 published as WO1999014346; The Scripps Research Institute in PCT application number PCT/US2010/00567 filed on February 24, 2010 published as WO2010098861, and United States patent application national phase entry serial number 13/203,229 filed November 3, 2011 published as US20120053333; Ludwig-Maximillians University in PCT application number PCT/EP2010/004681 filed on July 30, 2010 published as WO2011012316; Cellscript Inc. in United States patent number 8,039,214 filed June 30, 2008 and granted October 18, 2011, United States patent application serial numbers 12/962,498 filed on December 7, 2010 published as US20110143436, 12/962,468 filed on December 7, 2010 published as US20110143397, 13/237,451 filed on September 20, 2011 published as US20120009649, and PCT applications PCT/US2010/59305 filed December 7, 2010 published as WO2011071931 and PCT/US2010/59317 filed on December 7, 2010 published as WO2011071936; The Trustees of the University of Pennsylvania in PCT application number PCT/US2006/32372 filed on August 21, 2006 published as WO2007024708, and United States patent application national phase entry serial number 11/990,646 filed on March 27, 2009 published as US20090286852; Curevac GMBH in German patent application serial numbers DE10 2001 027 283.9 filed June 5, 2001, DE10 2001 062 480.8 filed December 19, 2001, and DE 20 2006 051 516 filed October 31, 2006 all abandoned, European patent numbers EP1392341 granted March 30, 2005 and EP1458410 granted January 2, 2008, PCT application numbers PCT/EP2002/06180 filed June 5, 2002 published as WO2002098443, PCT/EP2002/14577 filed on December 19, 2002 published as WO2003051401, PCT/EP2007/09469 filed on December 31, 2007 published as WO2008052770, PCT/EP2008/03033 filed on April 16, 2008 published as WO2009127230, PCT/EP2006/004784 filed on May 19, 2005 published as WO2006122828, PCT/EP2008/00081 filed on January 9, 2007 published as WO2008083949, and United States patent application serial numbers 10/729,830 filed on December 5, 2003 published as US20050032730, 10/870,110 filed on June 18, 2004 published as US20050059624, 11/914,945 filed on July 7, 2008 published as US20080267873, 12/446,912 filed on October 27, 2009 published as US2010047261 now abandoned, 12/522,214 filed on January 4, 2010 published as US20100189729, 12/787,566 filed on May 26, 2010 published as US20110077287, 12/787,755 filed on May 26, 2010 published as US20100239608, 13/185,119 filed on July 18, 2011 published as US20110269950, and 13/106,548 filed on May 12, 2011 published as US20110311472.

Notwithstanding these reports which are limited to a selection of chemical modifications including pseudouridine and 5-methyl-cytosine where the modifications are uniformly present in the mRNA, there remains a need in the art for therapeutic modalities to address the myriad of barriers surrounding the efficacious modulation of intracellular translation and processing of nucleic acids encoding polypeptides including the barrier to selective incorporation of different chemical modifications in order to fine tune or tailor physiologic responses and outcomes.

US 5 637 459 describes methods for producing chimeric nucleic acid molecules with two or more functions. The methods involve the generation of a chimeric library in which chimeric molecules combine the functions of multiple parent libraries.

Kuwahara and Sugimoto (Molecules 15(8):5423-5444 (2010)) describes a template-free click-ligation reaction for the intramolecular circularization of a single stranded oligonucleotide.

Kumar et al. (Journal of the American Chemical Society 129(21):6859-6864 (2007)) describes a template-free click-ligation reaction for the intramolecular circularization of a single stranded oligonucleotide.

Stewart et al. (Journal of Carbohydrate, Chemistry 33(7):408-419 (2014)) describes factors that influence the reactivity of azides with cyclooctynes in the context of strain-promoted azide-alkyne cycloaddition reaction.

US 2002/016463 describes methods for designing chimeric polynucleotides by way of directed evolution. Particularly, the methods involve the use of an algorithm to identify and select splice points within polynucleotides that can serve as junctions for creating chimeric molecules.

WO 2013/090186 describes the generation of modified nucleic acids that exhibit a reduced innate immune response when introduced into a population of cells, as well as the use of such modified nucleic acids in acute care situations, including life-threatening situations such as traumatic injury, or bacterial or viral infections.

To date, no studies have been reported on positionally modified polynucleotides, e.g., those having selective incorporation of modifications. The present disclosure addresses this need by describing nucleic acid based compounds or chimeric polynucleotides (both coding and non-coding and combinations thereof) which have structural and/or chemical features that avoid one or more of the problems in the art, for example, features which are useful for optimizing nucleic acid-based therapeutics while retaining structural and functional integrity, overcoming the threshold of expression, improving expression rates, half-life and/or protein concentrations, optimizing protein localization, and avoiding deleterious bio-responses such as the immune response and/or degradation pathways. Each of these barriers may be reduced or eliminated using the disclosure.

In this regard, the present inventors have developed chimeric polynucleotides and methods of synthesizing these polynucleotides which allow for customized placement, position and percent load of chemical modifications, which improve, alter or optimize certain physicochemical and pharmaceutical properties of the polynucleotides.

### Summary of the invention

The invention provides a chimeric polynucleotide encoding a polypeptide, wherein said chimeric polynucleotide has a sequence comprising Formula II:

### [An]-L1-[Bo]

### Formula II

wherein each A and B independently comprises any nucleoside;
n and o are, independently 10 to 1000, wherein [An] or [Bo] independently comprise an mRNA, said mRNA comprising a 5' untranslated region (UTR), 3'UTR and a coding region; and

### L1 comprises the structure

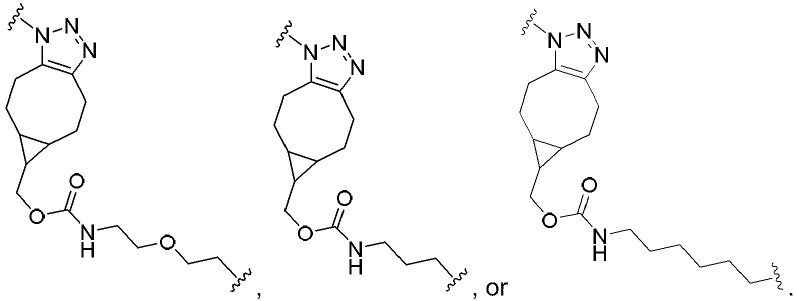

wherein L1 is attached to [An] at the 3' or 4' position of the sugar of one of the nucleosides and to [Bo] at the 5' or 6' position of the sugar of one of the nucleosides.

Further aspects of the invention are defined in the claims.

Described herein are compositions, methods, processes, kits and devices for the design, preparation, manufacture and/or formulation of chimeric polynucleotides. Herein, chimeric polynucleotides may take the form or function as modified mRNA molecules which encode a polypeptide of interest. In one case such chimeric polynucleotides are substantially non-coding.

Described herein are chimeric polynucleotides encoding a polypeptide, where the chimeric polynucleotide having a sequence or structure comprising Formula I,

5' [Aₙ]ₓ₋L1-[Bₒ]_{y-}L2-[Cₚ]_{z}-L3 3' **Formula I**

wherein:
each of A and B independently comprise a region of linked nucleosides;
C is an optional region of linked nucleosides;
at least one of regions A, B, or C is positionally modified, wherein said positionally modified region comprises at least two chemically modified nucleosides of one or more of the same nucleoside type of adenosine, thymidine, guanosine, cytidine, or uridine, and wherein at least two of the chemical modifications of nucleosides of the same type are different chemical modifications;
n, o and p are independently an integer between 15-1000;
x and y are independently 1-20;
z is 0-5;
L1 and L2 are independently optional linker moieties, said linker moieties being either nucleic acid based or non-nucleic acid based; and
L3 is an optional conjugate or an optional linker moiety, said linker moiety being either nucleic acid based or non-nucleic acid based.

Also described are methods of making and using the chimeric polynucleotides in research, diagnostics and therapeutics.

Also described herein is a chimeric polynucleotide encoding a polypeptide, wherein the polynucleotide has a sequence including Formula II:

[Aₙ]-L¹-[Bₒ] **Formula II**

wherein each A and B independently includes any nucleoside (e.g., a nucleotide);
n and o are, independently 10 to 10,000, e.g., 10 to 1000 or 10 to 2000; and
L¹ has the structure of Formula III: 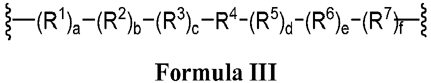
wherein a, b, c, d, e, and f are each, independently, 0 or 1;
each of R¹, R³, R⁵, and R⁷, is, independently, selected from optionally substituted C₁-C₆ alkylene, optionally substituted C₁-C₆ heteroalkylene, O, S, and NR⁸;
R² and R⁶ are each, independently, selected from carbonyl, thiocarbonyl, sulfonyl, or phosphoryl;
R⁴ is optionally substituted C₁-C₁₀ alkylene, optionally substituted C₂-C₁₀ alkenylene, optionally substituted C₂-C₁₀ alkynylene, optionally substituted C₂-C₉ heterocyclylene, optionally substituted C₆-C₁₂ arylene, optionally substituted C₂-C₁₀₀ polyethylene glycolene, or optionally substituted C₁-C₁₀ heteroalkylene, or a bond linking (R¹)ₐ-(R²)_{b}-(R³)_{c} to (R⁵)_{d}-(R⁶)ₑ-(R⁷)_{f}, wherein if a, b, c, d, e, and f are 0, R⁴ is not a bond; and
R⁸ is hydrogen, optionally substituted C₁-C₄ alkyl, optionally substituted C₂-C₄ alkenyl, optionally substituted C₂-C₄ alkynyl, optionally substituted C₂-C₆ heterocyclyl, optionally substituted C₆-C₁₂ aryl, or optionally substituted C₁-C₇ heteroalkyl;
wherein L¹ is attached to [Aₙ] and [Bₒ] at the sugar of one of the nucleosides (e.g., at the 3' position of a five-membered sugar ring or 4' position of a six membered sugar ring of a nucleoside of [Aₙ] and the 5' position of a five-membered sugar ring or 6' position of a six membered sugar ring of a nucleoside of [Bₒ] or at the 5' position of a five-membered sugar ring or 6' position of a six membered sugar ring of a nucleoside of [Aₙ] and the 3' position of a five-membered sugar ring or 4' position of a six membered sugar ring of a nucleoside of [Bₒ]).

In some cases, at least one of [Aₙ] and [Bₒ] includes the structure of Formula IV or Formula XVIII:

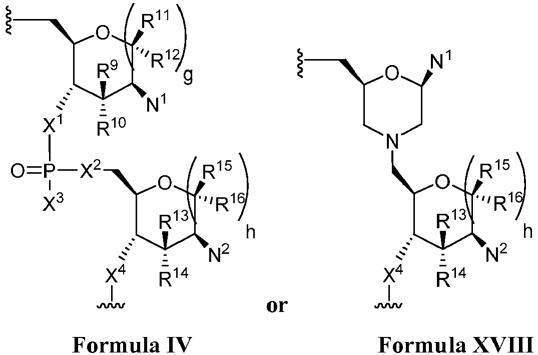

wherein each of N¹ and N² is independently a nucleobase;

each of R⁹, R¹⁰, R¹¹, R¹², R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

each of g and h is, independently, 0 or 1;

each X¹ and X⁴ is, independently, O, NH, or S;

each X² is independently O, NH, or S; and

each X³ is OH or SH, or a salt thereof.

In some cases, h is 0; R¹³ is H; and R¹⁴ is optionally substituted C₁-C₆ heteroalkyl.

In other cases, the optionally substituted C₁-C₆ heteroalkyl is methoxy.

In certain cases, X³ is SH.

Also described herein is a chimeric polynucleotide encoding a polypeptide, wherein the polynucleotide has a sequence including Formula II:

[Aₙ]-L¹-[Bₒ] **Formula II**

wherein each A and B independently includes any nucleoside (e.g., a nucleotide);

n and o are, independently 10 to 10,000, e.g., 10 to 1000 or 10 to 2000; and

L¹ is a bond or has the structure of Formula III:

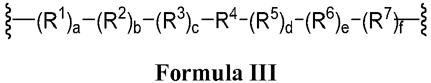

wherein a, b, c, d, e, and f are each, independently, 0 or 1;

each of R¹, R³, R⁵, and R⁷, is, independently, selected from optionally substituted C₁-C₆ alkylene, optionally substituted C₁-C₆ heteroalkylene, O, S, and NR⁸;

R² and R⁶ are each, independently, selected from carbonyl, thiocarbonyl, sulfonyl, or phosphoryl;

R⁴ is optionally substituted C₁-C₁₀ alkylene, optionally substituted C₂-C₁₀ alkenylene, optionally substituted C₂-C₁₀ alkynylene, optionally substituted C₂-C₉ heterocyclylene, optionally substituted C₆-C₁₂ arylene, optionally substituted C₂-C₁₀₀ polyethylene glycolene, or optionally substituted C₁-C₁₀ heteroalkylene, or a bond linking (R¹)ₐ-(R²)_{b}-(R³)_{c} to (R⁵)_{d}-(R⁶)ₑ-(R⁷)_{f}; and

R⁸ is hydrogen, optionally substituted C₁-C₄ alkyl, optionally substituted C₂-C₄ alkenyl, optionally substituted C₂-C₄ alkynyl, optionally substituted C₂-C₆ heterocyclyl, optionally substituted C₆-C₁₂ aryl, or optionally substituted C₁-C₇ heteroalkyl;

wherein L¹ is attached to [Aₙ] and [Bₒ] at the sugar of one of the nucleosides (e.g., at the 3' position of a five-membered sugar ring or 4' position of a six membered sugar ring of a nucleoside of [Aₙ] and the 5' position of a five-membered sugar ring or 6' position of a six membered sugar ring of a nucleoside of [Bₒ] or at the 5' position of a five-membered sugar ring or 6' position of a six membered sugar ring of a nucleoside of [Aₙ] and the 3' position of a five-membered sugar ring or 4' position of a six membered sugar ring of a nucleoside of [Bₒ]).

wherein at least one of [Aₙ] or [Bₒ] includes the structure of Formula IV or Formula XVIII:

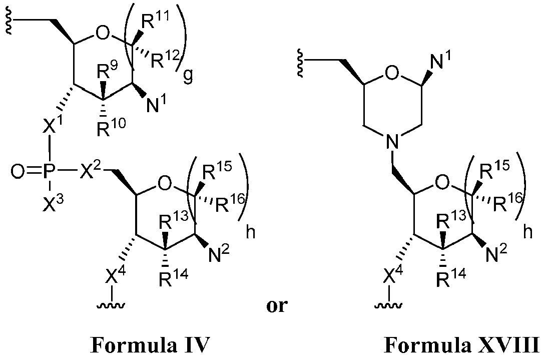

wherein each of N¹ and N² is independently a nucleobase;

each of R⁹, R¹⁰, R¹¹, R¹², R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

each of g and h is, independently, 0 or 1;

each X¹ and X⁴ is, independently, O, NH, or S; and

each X² is independently O, NH, or S; and

each X³ is OH or SH, or a salt thereof;

wherein, for Formula IV, at least one of X¹, X², or X⁴ is NH or S.

In some cases, X¹ is NH. In other cases, X⁴ is NH. In certain cases, X² is S.

In some cases, the polynucleotide includes: (a) a coding region; (b) a 5' UTR; and (c) a 3' UTR. In some cases, the polynucleotide further includes (d) at least one 5' cap structure. In other cases, the polynucleotide further includes (e) a poly-A tail.

In some cases, one of the coding region, the 5' UTR , the 3' UTR, the 5' cap structure, or the poly-A tail includes [Aₙ]-L¹-[Bₒ].

In other cases, one of the coding region, the 5' UTR, the 3' UTR, the 5' cap structure, or the poly-A tail includes [Aₙ] and another of the coding region, the 5' UTR, the 3' UTR, the 5' cap structure, or the poly-A tail includes [Bₒ].

In some cases, the 5' UTR includes at least one Kozak sequence.

In certain cases, the polynucleotide includes at least one modified nucleoside (e.g., a nucleoside of Table 2).

In some cases,

R⁴ is optionally substituted C₂₋₉ heterocyclylene, for example, the heterocycle may have the structure:

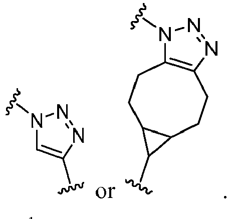

In some cases, L¹ includes the structure:

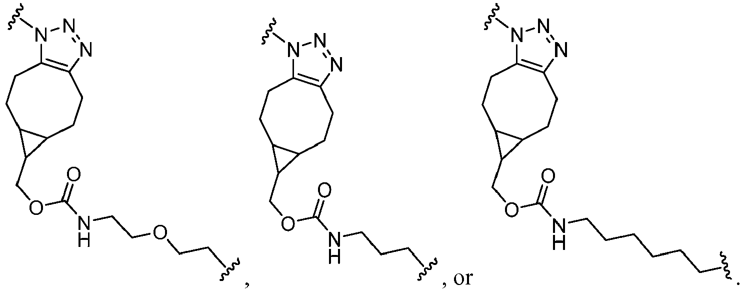

In certain cases, L¹ is attached to [Aₙ] at the 3' position of a five-membered sugar ring or 4' position of a six membered sugar ring of one of the nucleosides and to [Bₒ] at the 5' position of a five-membered sugar ring or 6' position of a six membered sugar ring of one of the nucleosides.

In some cases, the polynucleotide is circular.

In certain cases, the poly-A tail terminates in the structure of Formula XXI: 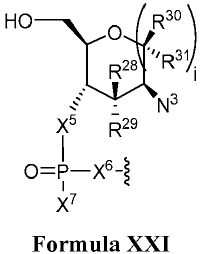 wherein N³ is a nucleobase
each of R²⁸, R²⁹, R³⁰, and R³¹ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;
i is 0 or 1;
X⁵ is O, NH, or S; and
X⁶ is O or S; and
X⁷ is OH or SH, or a salt thereof.

In some cases, the structure of Formula XXI is:

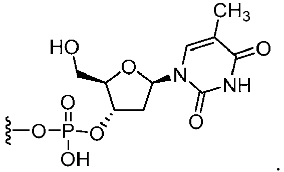

In other cases, the poly-A tail has 40 to 80 nucleosides (SEQ ID NO: 23).

In certain cases, the structure of Formula XXI is attached to two to four 2'-methoxy-adenosines and/or 2'-fluoro-adenosines.

In some cases, the poly-A tail terminates in the structure:

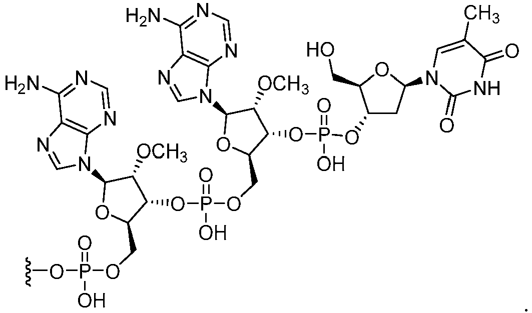

In other cases, the poly-A tail terminates in the structure:

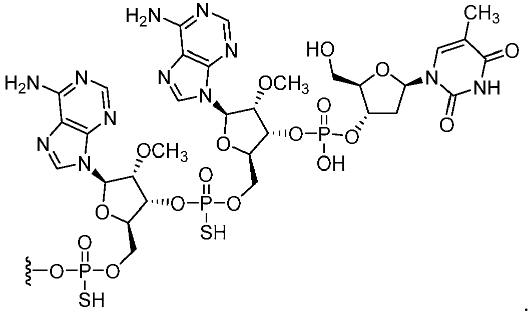

In certain cases, the poly-A tail includes the structure:

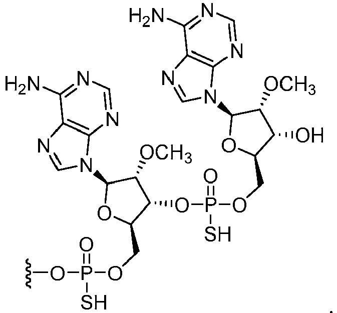

In another case, described herein is a method of producing a composition including a chimeric polynucleotide encoding a polypeptide, wherein the polynucleotide includes the structure of Formula Va or Vb:

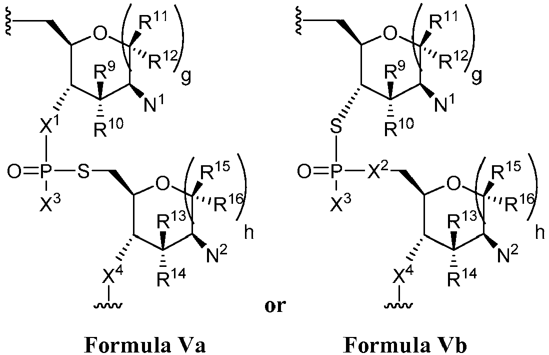

This method includes reacting (e.g., under alkylating conditions) a compound having the structure of Formula VIa or VIb: 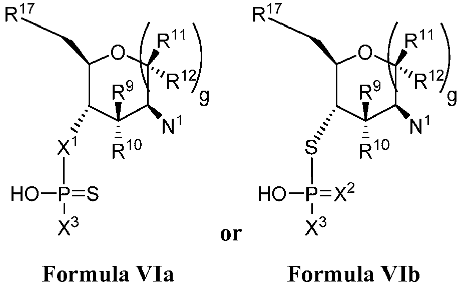 with a compound having the structure of Formula VII:

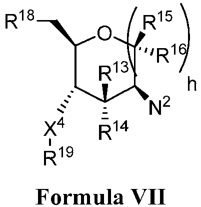

wherein each of N¹ and N² is, independently, a nucleobase;

each of R⁹, R¹⁰, R¹¹, R¹², R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

each of g and h is, independently, 0 or 1;

each X¹ and X⁴ is, independently, O, NH, or S;

each X² is O or S; and

each X³ is independently OH or SH, or a salt thereof;

each of R¹⁷ and R¹⁹ is, independently, a region of linked nucleosides; and

R¹⁸ is a halogen,

to produce a composition comprising a chimeric polynucleotide encoding a polypeptide, wherein the polynucleotide comprises the structure of Formula Va or Vb.

In another case, described herein is a method of producing a composition including a chimeric polynucleotide encoding a polypeptide, wherein the polynucleotide includes the structure of Formula VIIIa or VIIIb:

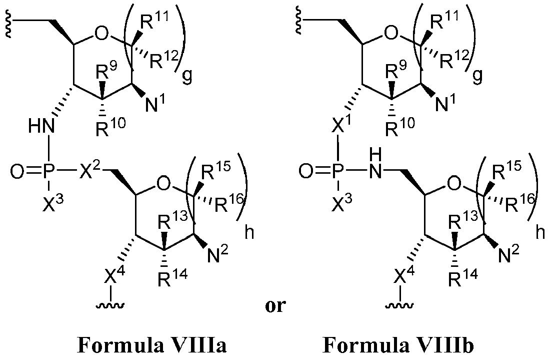

This method includes reacting (e.g., under Staudinger reaction conditions) a compound having the structure of Formula IXa or IXb: 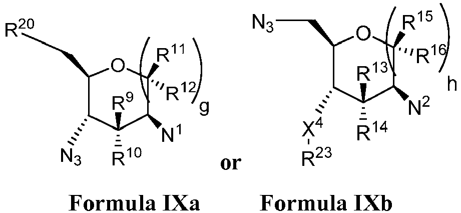 with a compound having the structure of Formula Xa or Xb:

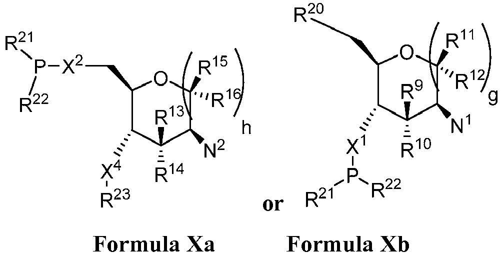

wherein each of N¹ and N² is, independently, a nucleobase;

each of R⁹, R¹⁰, R¹¹, R¹², R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

each of g and h is, independently, 0 or 1;

each X⁴ is, independently, O, NH, or S; and

each X¹ and X² is independently O or S;

each X³ is independently OH, SH, or a salt thereof;

each of R²⁰ and R²³ is, independently, a region of linked nucleosides; and

each of R²¹ and R²² is, independently, optionally substituted C₁-C₆ alkoxy;

to produce a composition comprising a chimeric polynucleotide encoding a polypeptide, wherein the polynucleotide comprises the structure of Formula VIIIa or VIIIb.

In another case, described herein is a method of producing a composition including a chimeric polynucleotide encoding a polypeptide, wherein the polynucleotide includes the structure of Formula XIa, XIb, XIIa, or XIIb:

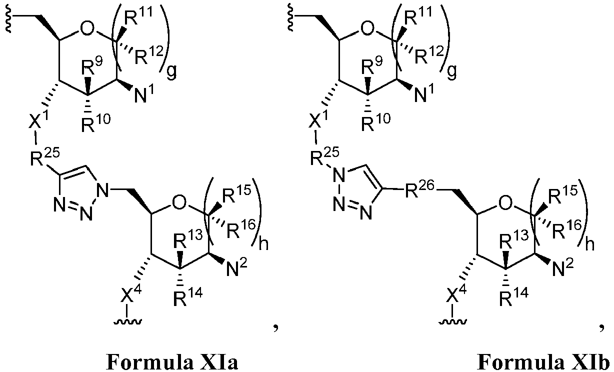

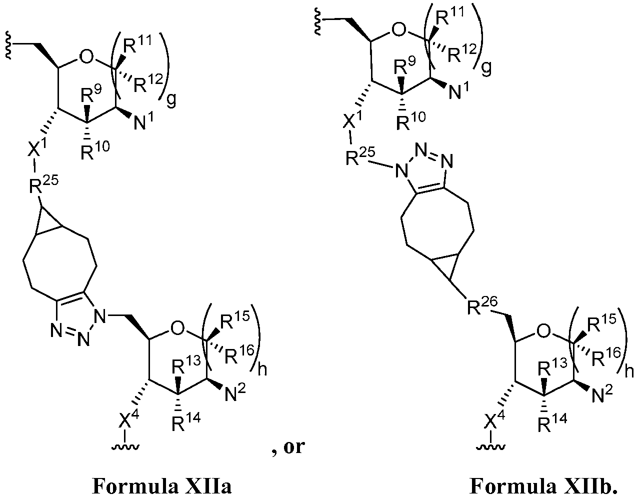

This method includes reacting (e.g., under [3+2] cylcoaddition conditions in the presence or absence of a copper source) a compound having the structure of Formula XIIIa, XIIIb, XIVa, or XIVb:

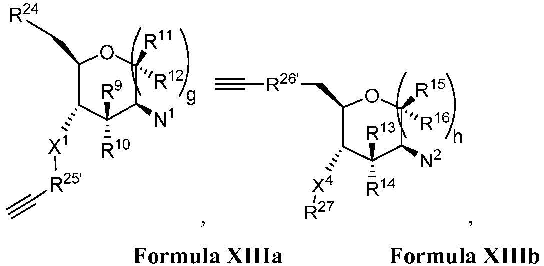

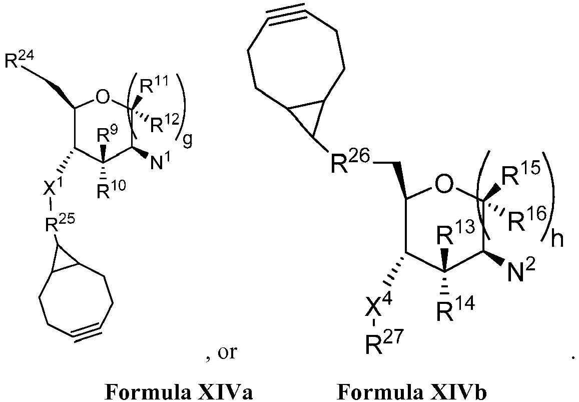

with a compound having the structure of Formula XVa or XVb:

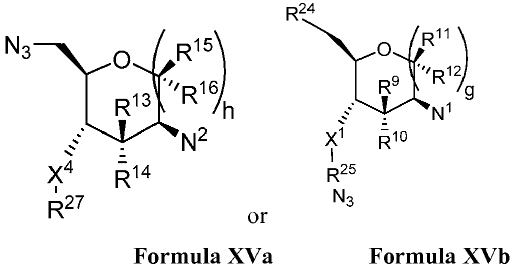

wherein each of N¹ and N² is, independently, a nucleobase;

each of R⁹, R¹⁰, R¹¹, R¹², R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

each of g and h is, independently, 0 or 1;

each X¹ and X⁴ is, independently, absent, O, NH, or S or a salt thereof;

each of R²⁴ and R²⁷ is, independently, a region of linked nucleosides; and

each of R²⁵, R^{25'}, R²⁶, and R^{26'} is absent or optionally substituted C₁-C₆ alkylene or optionally substituted C₁-C₆ heteroalkylene or R^{25'} or R^{26'} and the alkynyl group together form optionally substituted cycloalkynyl;

to produce a composition comprising a chimeric polynucleotide encoding a polypeptide, wherein the polynucleotide comprises the structure of Formula XIa, XIb, XIIa, or XIIb.

In another case, described herein is a method of producing a composition including a chimeric polynucleotide encoding a polypeptide, wherein the polynucleotide has a sequence including Formula II:

[Aₙ]-L¹-[Bₒ], **Formula II**

This method includes reacting (e.g., under [3+2] cycloaddition conditions in the presence or absence of a copper source) a compound having the structure of Formula XVI:

[Aₙ]-(R¹)ₐ-(R²)_{b}-(R³)_{c}-N₃ **Formula XVI**

with a compound having the structure of Formula XVII:

R²⁷-(R⁵)_{d}-(R⁶)ₑ-(R⁷)_{f}-[Bₒ] **Formula XVII**

wherein each A and B is independently any nucleoside;

n and o are, independently 10 to 10,000, e.g., 10 to 1000 or 10 to 2000; and

L¹ has the structure of Formula III:

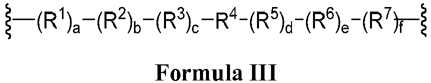

wherein a, b, c, d, e, and f are each, independently, 0 or 1;

R¹, R³, R⁵, and R⁷ each, independently, is selected from optionally substituted C₁-C₆ alkylene, optionally substituted C₁-C₆ heteroalkylene, O, S, and NR⁸;

R² and R⁶ are each, independently, selected from carbonyl, thiocarbonyl, sulfonyl, or phosphoryl;

R⁴ is an optionally substituted triazolene; and

R⁸ is hydrogen, optionally substituted C₁-C₄ alkyl, optionally substituted C₃-C₄ alkenyl, optionally substituted C₂-C₄ alkynyl, optionally substituted C₂-C₆ heterocyclyl, optionally substituted C₆-C₁₂ aryl, or optionally substituted C₁-C₇ heteroalkyl; and

R²⁷ is an optionally substituted C₂-C₃ alkynyl or an optionally substituted C₈-C₁₂ cycloalkynyl,

wherein L¹ is attached to [Aₙ] and [Bₒ] at the sugar of one of the nucleosides;

to produce a composition comprising a chimeric polynucleotide encoding a polypeptide, wherein the polynucleotide has a sequence comprising Formula II.

In some cases, the optionally substituted triazolene has the structure:

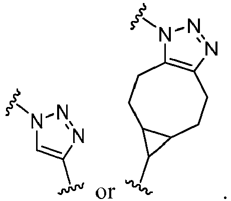

In another case, described herein is a method of producing a composition comprising a chimeric polynucleotide encoding a polypeptide, wherein the polynucleotide comprises the structure of Formula XVIII:

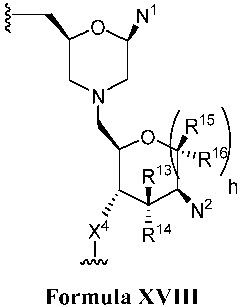

the method comprising reacting (e.g., under reductive amination conditions) a compound having the structure of Formula XIX:

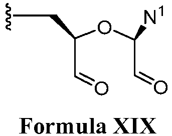

with a compound having the structure of Formula XX:

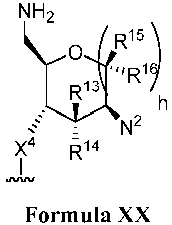

wherein each of N¹ and N² is, independently, a nucleobase;

each of R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

his 0 or 1; and

X⁴ is O, NH, or S;

to produce a composition comprising a chimeric polynucleotide encoding a polypeptide, wherein the polynucleotide comprises the structure of Formula XVIII.

In some cases, the method includes producing a compound of Formula XIX from a compound of Formula XXI:

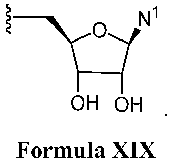

The details of various cases of the disclosure are set forth in the description below. Other features, objects, and advantages of the disclosure will be apparent from the description and the drawings, and from the claims.

### BRIEF DESCRIPTION OF THE DRAWINGS

The foregoing and other objects, features and advantages will be apparent from the following description of particular cases, as illustrated in the accompanying drawings in which like reference characters refer to the same parts throughout the different views. The drawings are not necessarily to scale, emphasis instead being placed upon illustrating the principles of various cases.

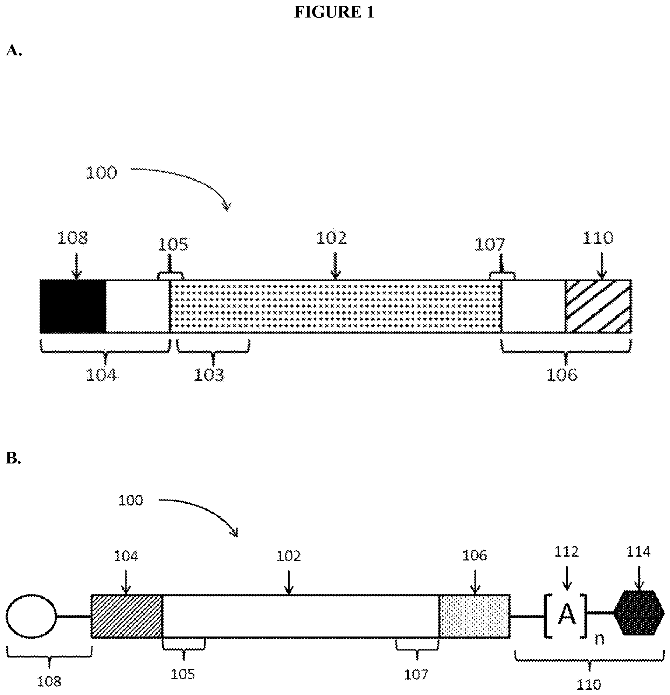

FIG. 1 is a schematic of a polynucleotide construct. Figure 1A is a schematic of a polynucleotide construct taught in commonly owned co-pending US

Patent Application 13/791,922 filed March 9, 2013. Figure 1B is a schematic of a linear polynucleotide construct.

FIG. 2 is a schematic of a series of chimeric polynucleotides of the present disclosure.

FIG. 3 is a schematic of a series of chimeric polynucleotides illustrating various patterns of positional modifications and showing regions analogous to those regions of an mRNA polynucleotide.

FIG. 4 is a schematic of a series of chimeric polynucleotides illustrating various patterns of positional modifications based on Formula I.

FIG. 5 is a is a schematic of a series of chimeric polynucleotides illustrating various patterns of positional modifications based on Formula I and further illustrating a blocked or structured 3' terminus.

FIG. 6 is a schematic of a circular construct of the present disclosure.

FIG. 7 is a schematic of a circular construct of the present disclosure.

FIG. 8 is a schematic of a circular construct of the present disclosure comprising at least one spacer region.

FIG. 9 is a schematic of a circular construct of the present disclosure comprising at least one sensor region.

FIG. 10 is a schematic of a circular construct of the present disclosure comprising at least one sensor region and a spacer region.

FIG. 11 is a schematic of a non-coding circular construct of the present disclosure.

FIG. 12 is a schematic of a non-coding circular construct of the present disclosure disclosure.

FIG. 13 is an image showing a capillary electrophoresis (CE) generated gel of RNA 1-3 before and after 3'-azido ddATP incorporation. Lane 1 is a ladder, lane 2 is RNA 1, lane 3 is RNA 1 after 3 '-azido ddATP incorporation, lane 4 is RNA 2, lane 5 is RNA 2 after 3 '-azido ddATP incorporation, lane 6 is RNA 3, and lane 7 is RNA 3 after 3 '-azido ddATP incorporation.

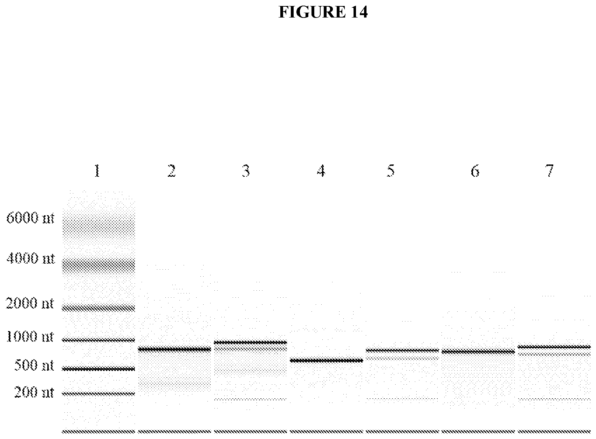

FIG. 14 is an image showing a CE generated gel of the formation of RNA-poly(A) tail conjugates. Lane 1 is a ladder, lane 2 is RNA 1 after 3 '-azido ddATP incorporation, lane 3 is 3'-azido RNA 1 after reaction with tail 1, lane 4 is RNA 2 after 3 '-azido ddATP incorporation, lane 5 is 3 '-azido RNA 2 after reaction with tail 1, lane 6 is RNA 3 after 3 '-azido ddATP incorporation, and lane 7 is 3 '-azido RNA 3 after reaction with tail 1. The RNA in lanes 2, 4, and 6 is a mixture of unmodified and 3 'azido RNA. In lanes 3, 5, and 7, three distinct bands going from shortest to longest are unreacted tail 1, a putative mix of unmodified RNA and unreacted 3'-azido RNA, and RNA-tail 1 conjugate.

FIG. 15 is an image showing a CE generated gel of DNA splint-templated conjugation of RNA 1 and tail 1 to give RNA 1-tail 1 conjugate and subsequent purification by oligo(T) Dynabeads. Lane 1 is a ladder, lane 2 is unmodified RNA 1, lane 3 is the DNA splint-templated reaction mixture after desalting by ultrafiltration, lane 4 is the reaction mixture after digestion with DNase, lane 5 is the fraction of the reaction mixture which did not bind to the oligo(T) Dynabeads, and lane 6 is the purified RNA 1-tail 1 conjugate after elution from the oligo(T) Dynabeads.

FIG. 16 is an image showing a CE generated gel of RNA 1-tail conjugates with tails 1-6 purified by oligo(T) Dynabeads. Lane 1 is a ladder, lane 2 is 3 '-azido RNA 1, lane 3 is RNA 1-tail 1 conjugate, lane 4 is RNA 1-tail 4 conjugate, lane 5 is RNA 1-tail 2 conjugate, lane 6 is RNA 1-tail 5 conjugate, lane 7 is RNA 1-tail 3 conjugate, lane 8 is RNA 1-tail 6 conjugate, lane 9 is 3 '-azido RNA 2, lane 10 is RNA 2-tail 1 conjugate, lane 11 is RNA 2-tail 4 conjugate, lane 12 is 3 '-azido RNA 3, lane 13 is RNA 3-tail 1 conjugate, and lane 14 is RNA 3-tail 4 conjugate.

FIG. 17 is an image showing PAGE analysis of capped TP oligos 1 and 2. Lane 1 is TP oligo 1, lane 2 is capped TP oligo 1, lane 3 is TP oligo 2, and lane 4 is capped TP oligo 2.

FIG. 18 is an image showing CE analysis of cap oligo-RNA 5 conjugates. Lane 1 is a ladder, lane 2 is 5'-azido RNA 5, lane 3 is uncapped TP oligo 1-RNA 5 conjugate, lane 4 is capped TP oligo 1-RNA 5 conjugate, lane 5 is uncapped TP oligo 2-RNA 5 conjugate, and lane 6 is capped TP oligo 1-RNA 5 conjugate. Lanes 7-11 are those aforementioned samples after the SPAAC reaction with 5'-BCN tail.

FIG. 19 is an image showing CE electropherograms of the mixture of RNAs after the SPAAC reaction of 3'-azido RNA 3 and tail 1 before and after treatment with poly(A) polymerase (PAP). In this instance, 3 '-azido ddATP incorporation was calculated to be 46%.

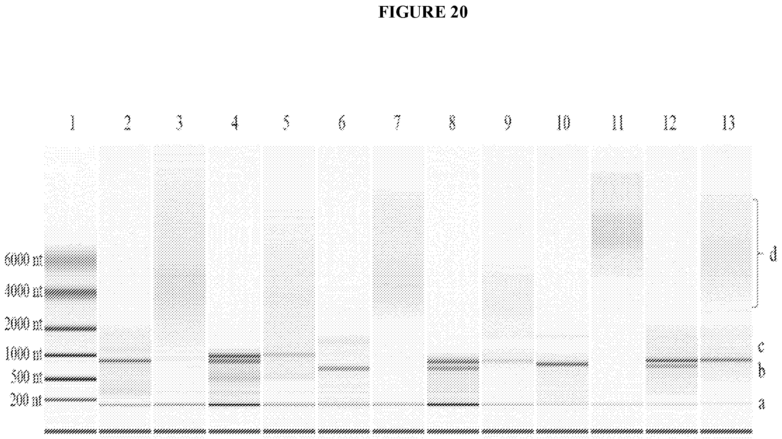

FIG. 20 is an image showing a CE generated gel of 3 '-azido ddATP incorporation into RNA 1 - 3 by treating SPAAC reactions with poly(A) polymerase. Lane 1 is a ladder. Lanes 2 and 3 are unmodified RNA 1 with tail 1 before and after treatment with PAP, respectively. Lanes 4 and 5 are 3 '-azido RNA 1 with tail 1 before and after treatment with PAP, respectively. Lanes 6 - 9 and lanes 10 - 13 are those same reactions with RNA 2 and RNA 3, respectively. Letters are for general designation of the RNA's in each lane, where a corresponds to unreacted tail 1, b corresponds to unconjugated RNA, c corresponds to RNA-tail 1 conjugates, and d corresponds to RNA that has an added poly(A) tail by reaction with PAP.

### DETAILED DESCRIPTION

It is of great interest in the fields of therapeutics, diagnostics, reagents and for biological assays to be able design, synthesize and deliver a nucleic acid, e.g., a ribonucleic acid (RNA) inside a cell, whether *in vitro, in vivo, in situ* or *ex vivo*, such as to effect physiologic outcomes which are beneficial to the cell, tissue or organ and ultimately to an organism. One beneficial outcome is to cause intracellular translation of the nucleic acid and production of an encoded polypeptide of interest. In like manner, non-coding RNA has become a focus of much study; and utilization of non-coding polynucleotides, alone and in conjunction with coding polynucleotides, could provide beneficial outcomes in therapeutic scenarios.

Described herein are compositions (including pharmaceutical compositions) and methods for the design, preparation, manufacture and/or formulation of polynucleotides, specifically chimeric polynucleotides.

Also described herein are systems, processes, devices and kits for the selection, design and/or utilization of the chimeric polynucleotides.

According to the present disclosure, chimeric polynucleotides are preferably modified in a manner as to avoid the deficiencies of other molecules of the art.

The use of modified polynucleotides encoding polypeptides (i.e., modified mRNA) in the fields of human disease, antibodies, viruses, veterinary applications and a variety of *in vivo* settings has been explored by the inventors and these studies are disclosed in for example, those listed in Table 6 of International Publication Nos. WO2013151666, WO2013151668, WO2013151663, WO2013151669, WO2013151670, WO2013151664, WO2013151665, WO2013151736; Tables 6 and 7 International Publication No. WO2013151672; Tables 6, 178 and 179 of International Publication No. WO2013151671; Tables 6, 185 and 186 of International Publication No WO2013151667.

Any of the foregoing may be synthesized as a chimeric polynucleotide and such cases are contemplated by the present disclosure.

Described herein, therefore, are chimeric polynucleotides which, due to their chimeric nature, have been designed to improve one or more of the stability and/or clearance in tissues, receptor uptake and/or kinetics, cellular access, engagement with translational machinery, mRNA half-life, translation efficiency, immune evasion, immune induction (for vaccines), protein production capacity, secretion efficiency (when applicable), accessibility to circulation, protein half-life and/or modulation of a cell's status, function and/or activity.

### I. Compositions of the Disclosure

Nucleic acid molecules are described, specifically polynucleotides which are chimeric and which, in some cases, encode one or more polypeptides of interest. The term "nucleic acid," in its broadest sense, includes any compound and/or substance that comprise a polymer of nucleotides. These polymers are often referred to as polynucleotides.

Exemplary nucleic acids or polynucleotides of the disclosure include, but are not limited to, ribonucleic acids (RNAs), deoxyribonucleic acids (DNAs), threose nucleic acids (TNAs), glycol nucleic acids (GNAs), peptide nucleic acids (PNAs), locked nucleic acids (LNAs, including LNA having a β- D-ribo configuration, α-LNA having an α-L-ribo configuration (a diastereomer of LNA), 2'-amino-LNA having a 2'-amino functionalization, and 2'-amino- α-LNA having a 2'-amino functionalization), ethylene nucleic acids (ENA), cyclohexenyl nucleic acids (CeNA) or hybrids or combinations thereof.

In preferred cases, the nucleic acid molecule is or functions as a messenger RNA (mRNA). As used herein, the term "messenger RNA" (mRNA) refers to any polynucleotide which encodes a polypeptide of interest and which is capable of being translated to produce the encoded polypeptide of interest *in vitro*, *in vivo*, *in situ* or *ex vivo.*

Traditionally, the basic components of an mRNA molecule include at least a coding region, a 5'UTR, a 3'UTR, a 5' cap and a poly-A tail. Figure 1 illustrates a representative polynucleotide **100** which may serve as a starting, parent or scaffold molecule for the design of chimeric polynucleotides which encode polypeptides.

According to FIG. **1A** and **1B****,** the polynucleotide **100** here contains a first region of linked nucleotides **102** that is flanked by a first flanking region **104** and a second flaking region **106.** The polynucleotide may encode at its 5' terminus one or more signal sequences in the signal sequence region **103.** The flanking region **104** may comprise a region of linked nucleotides comprising one or more complete or incomplete 5' UTRs sequences which may be completely codon optimized or partially codon optimized. The flanking region **104** may include at least one nucleic acid sequence including, but not limited to, miR sequences, TERZAK^{™} sequences and translation control sequences. The flanking region **104** may also comprise a 5' terminal cap **108.** The 5' terminal capping region **108** may include a cap such as a naturally occurring cap, a synthetic cap or an optimized cap. Non-limiting examples of optimized caps include the caps taught by Rhoads in US Patent No. US7074596 and International Patent Publication No. WO2008157668, WO2009149253 and WO2013103659.

The second flanking region **106** may comprise a region of linked nucleotides comprising one or more complete or incomplete 3' UTRs. The second flanking region **106** may be completely codon optimized or partially codon optimized. The flanking region **106 may** include at least one nucleic acid sequence including, but not limited to, miR sequences and translation control sequences. The flanking region **106** may also comprise a 3' tailing sequence **110.** The 3' tailing sequence **110** may include a synthetic tailing region **112** and/or a chain terminating nucleoside **114.** Non-liming examples of a synthetic tailing region include a polyA sequence, a polyC sequence, and a polyA-G quartet. Non-limiting examples of chain terminating nucleosides include 2'-O methyl, F and locked nucleic acids (LNA).

Bridging the 5' terminus of the first region **102** and the first flanking region **104** is a first operational region **105.** Traditionally this operational region comprises a Start codon. The operational region may alternatively comprise any translation initiation sequence or signal including a Start codon.

Bridging the 3' terminus of the first region **102** and the second flanking region **106** is a second operational region **107.** Traditionally this operational region comprises a Stop codon. The operational region may alternatively comprise any translation initiation sequence or signal including a Stop codon. Multiple serial stop codons may also be used.

Building on this wild type modular structure, the present disclosure expands the scope of functionality of traditional mRNA molecules as well as those produced via IVT in the art, by providing chimeric polynucleotides or RNA constructs which maintain a modular organization, but which comprise one or more structural and/or chemical modifications or alterations which impart useful properties to the polynucleotide. As such, the chimeric polynucleotides which are modified mRNA molecules of the present disclosure are termed "chimeric modified mRNA" or "chimeric mRNA."

### Chimeric Polynucleotide Architecture

A "chimera" according to the present disclosure is an entity having two or more incongruous or heterogeneous parts or regions. As used herein, "chimeric polynucleotides" or "chimeric polynucleotides" are those nucleic acid polymers having portions or regions which differ in size and/or chemical modification pattern, chemical modification position, chemical modification percent or chemical modification population and combinations of the foregoing. As used herein a "part" or "region" of a polynucleotide is defined as any portion of the polynucleotide which is less than the entire length of the polynucleotide.

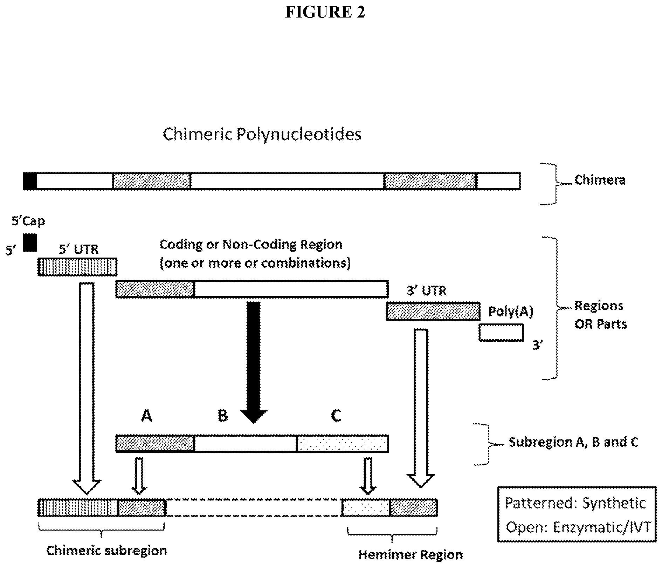

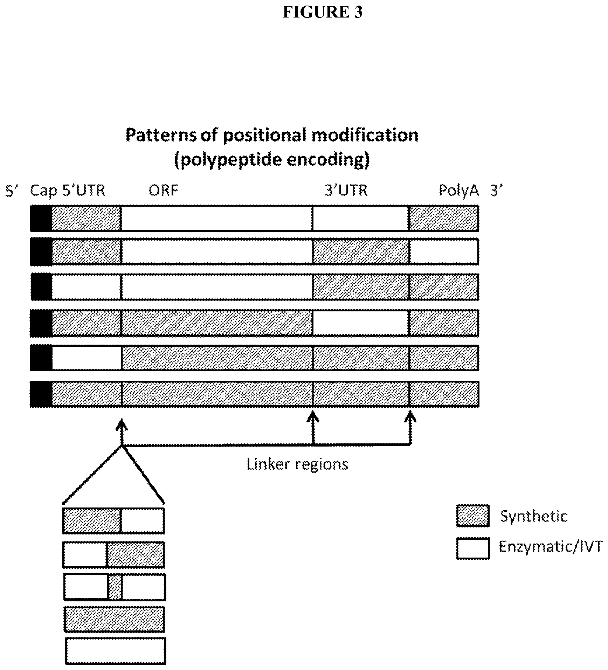

Examples of parts or regions, where the chimeric polynucleotide functions as an mRNA and encodes a polypeptide of interest include, but are not limited to, untranslated regions (UTRs, such as the 5' UTR or 3' UTR), coding regions, cap regions, polyA tail regions, start regions, stop regions, signal sequence regions, and combinations thereof. Figure 2 illustrates certain cases of the chimeric polynucleotides which may be used as mRNA. Figure 3 illustrates a schematic of a series of chimeric polynucleotides identifying various patterns of positional modifications and showing regions analogous to those regions of an mRNA polynucleotide. Regions or parts that join or lie between other regions may also be designed to have subregions. These are shown in the figure.

Chimeric polynucleotides may have a structure comprising Formula I.

5' [Aₙ]ₓ₋L1-[Bₒ]_{y-}L2-[Cₚ]_{z}-L3 3' **Formula I**

wherein:

each of A and B independently comprise a region of linked nucleosides;

C is an optional region of linked nucleosides;

at least one of regions A, B, or C is positionally modified, wherein the positionally modified region comprises at least two chemically modified nucleosides of one or more of the same nucleoside type of adenosine, thymidine, guanosine, cytidine, or uridine, and wherein at least two of the chemical modifications of nucleosides of the same type are different chemical modifications;

n, o and p are independently an integer between 15-1000;

x and y are independently 1-20;

z is 0-5;

L1 and L2 are independently optional linker moieties, the linker moieties being either nucleic acid based or non-nucleic acid based; and

L3 is an optional conjugate or an optional linker moiety, the linker moiety being either nucleic acid based or non-nucleic acid based.

In some cases the chimeric polynucleotide of Formula I encodes one or more peptides or polypeptides of interest. Such encoded molecules may be encoded across two or more regions.

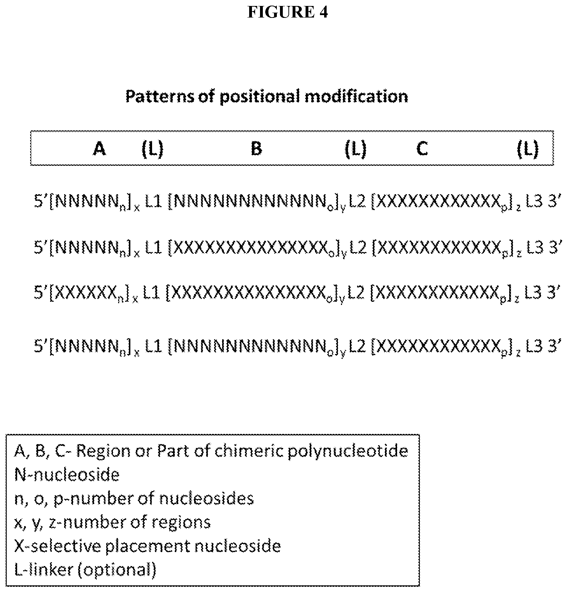

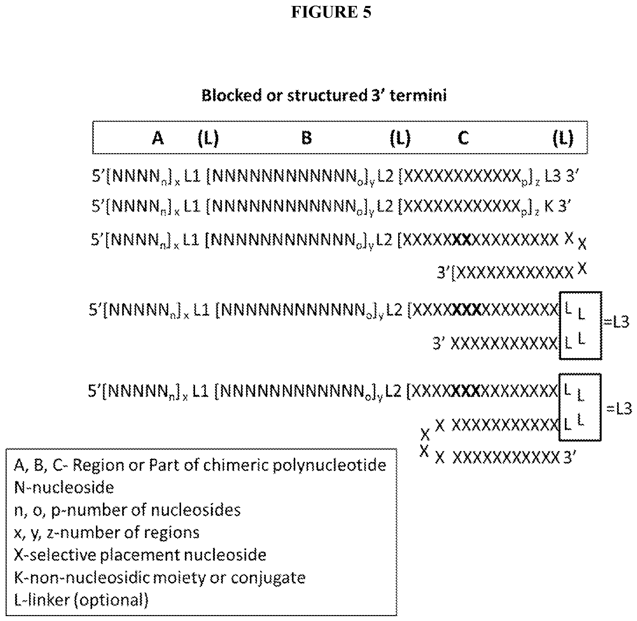

Figures 4 and 5 provide schematics of a series of chimeric polynucleotides illustrating various patterns of positional modifications based on Formula I as well as those having a blocked or structured 3' terminus.

Chimeric polynucleotides, including the parts or regions thereof, may be classified as hemimers, gapmers, wingmers, or blockmers.

As used herein, a "hemimer" is chimeric polynucleotide comprising a region or part which comprises half of one pattern, percent, position or population of a chemical modification(s) and half of a second pattern, percent, position or population of a chemical modification(s). Chimeric polynucleotides may also comprise hemimer subregions. In one case, a part or region is 50% of one and 50% of another.

In one case the entire chimeric polynucleotide can be 50% of one and 50% of the other. Any region or part of any chimeric polynucleotide may be a hemimer. Types of hemimers include pattern hemimers, population hemimers or position hemimers. By definition, hemimers are 50:50 percent hemimers.

As used herein, a "gapmer" is a chimeric polynucleotide having at least three parts or regions with a gap between the parts or regions. The "gap" can comprise a region of linked nucleosides or a single nucleoside which differs from the chimeric nature of the two parts or regions flanking it. The two parts or regions of a gapmer may be the same or different from each other.

As used herein, a "wingmer" is a chimeric polynucleotide having at least three parts or regions with a gap between the parts or regions. Unlike a gapmer, the two flanking parts or regions surrounding the gap in a wingmer are the same in degree or kind. Such similarity may be in the length of number of units of different modifications or in the number of modifications. The wings of a wingmer may be longer or shorter than the gap. The wing parts or regions may be 20, 30, 40, 50, 60 70, 80, 90 or 95% greater or shorter in length than the region which comprises the gap.

As used herein, a "blockmer" is a patterned polynucleotide where parts or regions are of equivalent size or number and type of modifications. Regions or subregions in a blockmer may be 50, 51, 52, 53, 54, 55, 56, 57, 58, 59, 60, 61 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 310, 320, 330, 340, 350, 360, 370, 380, 390, 400, 410, 420, 430, 440, 450, 460, 470, 480, 490 or 500, nucleosides long.

Chimeric polynucleotides, including the parts or regions thereof, of the present disclosure having a chemical modification pattern are referred to as "pattern chimeras." Pattern chimeras may also be referred to as blockmers. Pattern chimeras are those polynucleotides having a pattern of modifications within, across or among regions or parts.

Patterns of modifications within a part or region are those which start and stop within a defined region. Patterns of modifications across a part or region are those patterns which start in on part or region and end in another adjacent part or region. Patterns of modifications among parts or regions are those which begin and end in one part or region and are repeated in a different part or region, which is not necessarily adjacent to the first region or part.

The regions or subregions of pattern chimeras or blockmers may have simple alternating patterns such as ABAB[AB]n where each "A" and each "B" represent different chemical modifications (at least one of the base, sugar or backbone linker), different types of chemical modifications (e.g., naturally occurring and non-naturally occurring), different percentages of modifications or different populations of modifications. The pattern may repeat n number of times where n=3-300. Further, each A or B can represent from 1-2500 units (e.g., nucleosides) in the pattern. Patterns may also be alternating multiples such as AABBAABB[AABB]n (an alternating double multiple) or AAABBBAAABBB[AAABBB]n (an alternating triple multiple) pattern. The pattern may repeat n number of times where n=3-300.

Different patterns may also be mixed together to form a second order pattern. For example, a single alternating pattern may be combined with a triple alternating pattern to form a second order alternating pattern A'B'. One example would be [ABABAB][AAABBBAAABBB] [ABABAB][AAABBBAAABBB] [ABABAB][AAABBBAAABBB], where [ABABAB] is A' and [AAABBBAAABBB] is B'. In like fashion, these patterns may be repeated n number of times, where n=3-300.

Patterns may include three or more different modifications to form an ABCABC[ABC]n pattern. These three component patterns may also be multiples, such as AABBCCAABBCC[AABBCC]n and may be designed as combinations with other patterns such as ABCABCAABBCCABCABCAABBCC, and may be higher order patterns.

Regions or subregions of position, percent, and population modifications need not reflect an equal contribution from each modification type. They may form series such as "1-2-3-4", "1-2-4-8", where each integer represents the number of units of a particular modification type. Alternatively, they may be odd only, such as '1-3-3-1-3-1-5" or even only "2-4-2-4-6-4-8" or a mixture of both odd and even number of units such as "1-3-4-2-5-7-3-3-4".

Pattern chimeras may vary in their chemical modification by degree (such as those described above) or by kind (e.g., different modifications).

Chimeric polynucleotides, including the parts or regions thereof, of the present disclosure having at least one region with two or more different chemical modifications of two or more nucleoside members of the same nucleoside type (A, C, G, T, or U) are referred to as "positionally modified" chimeras. Positionally modified chimeras are also referred to herein as "selective placement" chimeras or "selective placement polynucleotides". As the name implies, selective placement refers to the design of polynucleotides which, unlike polynucleotides in the art where the modification to any A, C, G, T or U is the same by virtue of the method of synthesis, can have different modifications to the individual As, Cs, Gs, Ts or Us in a polynucleotide or region thereof. For example, in a positionally modified chimeric polynucleotide, there may be two or more different chemical modifications to any of the nucleoside types of As, Cs, Gs, Ts, or Us. There may also be combinations of two or more to any two or more of the same nucleoside type. For example, a positionally modified or selective placement chimeric polynucleotide may comprise 3 different modifications to the population of adenines in the molecule and also have 3 different modifications to the population of cytosines in the construct-all of which may have a unique, non-random, placement.

Chimeric polynucleotides, including the parts or regions thereof, of the present disclosure having a chemical modification percent are referred to as "percent chimeras." Percent chimeras may have regions or parts which comprise at least 1%, at least 2%, at least 5%, at least 8%, at least 10%, at least 20%, at least 30%, at least 40%, at least 50%, at least 60%, at least 70%, at least 80%, at least 90%, at least 95%, or at least 99% positional, pattern or population of modifications. Alternatively, the percent chimera may be completely modified as to modification position, pattern, or population. The percent of modification of a percent chimera may be split between naturally occurring and non-naturally occurring modifications.

Chimeric polynucleotides, including the parts or regions thereof, of the present disclosure having a chemical modification population are referred to as "population chimeras." A population chimera may comprise a region or part where nucleosides (their base, sugar or backbone linkage, or combination thereof) have a select population of modifications. Such modifications may be selected from functional populations such as modifications which induce, alter or modulate a phenotypic outcome. For example, a functional population may be a population or selection of chemical modifications which increase the level of a cytokine. Other functional populations may individually or collectively function to decrease the level of one or more cytokines. Use of a selection of these like-function modifications in a chimeric polynucleotide would therefore constitute a "functional population chimera." As used herein, a "functional population chimera" may be one whose unique functional feature is defined by the population of modifications as described above or the term may apply to the overall function of the chimeric polynucleotide itself. For example, as a whole the chimeric polynucleotide may function in a different or superior way as compared to an unmodified or non-chimeric polynucleotide.

It should be noted that polynucleotides which have a uniform chemical modification of all of any of the same nucleoside type or a population of modifications produced by mere downward titration of the same starting modification in all of any of the same nucleoside type, or a measured percent of a chemical modification of all any of the same nucleoside type but with random incorporation, such as where all uridines are replaced by a uridine analog, e.g., pseudouridine, are not considered chimeric. Likewise, polynucleotides having a uniform chemical modification of two, three, or four of the same nucleoside type throughout the entire polynucleotide (such as all uridines and all cytosines, etc. are modified in the same way) are not considered chimeric polynucleotides. One example of a polynucleotide which is not chimeric is the canonical pseudouridine/5-methyl cytosine modified polynucleotide of the prior art. These prior art polynucleotides are arrived at entirely via *in vitro* transcription (IVT) enzymatic synthesis; and due to the limitations of the synthesizing enzymes, they contain only one kind of modification at the occurrence of each of the same nucleoside type, i.e., adenosine (A), thymidine (T), guanosine (G), cytidine (C) or uridine (U), found in the polynucleotide.

The chimeric polynucleotides may be structurally modified or chemically modified. As used herein, a "structural" modification is one in which two or more linked nucleosides are inserted, deleted, duplicated, inverted or randomized in a chimeric polynucleotide without significant chemical modification to the nucleotides themselves. Because chemical bonds will necessarily be broken and reformed to effect a structural modification, structural modifications are of a chemical nature and hence are chemical modifications. However, structural modifications will result in a different sequence of nucleotides. For example, the polynucleotide "ATCG" may be chemically modified to "AT-5meC-G". The same polynucleotide may be structurally modified from "ATCG" to "ATCCCG". Here, the dinucleotide "CC" has been inserted, resulting in a structural modification to the polynucleotide.

In some cases, the chimeric polynucleotides may encode two or more proteins or peptides. Such proteins or peptides include the heavy and light chains of antibodies, an enzyme and its substrate, a label and its binding molecule, a second messenger and its enzyme or the components of multimeric proteins or complexes.

The regions or parts of the chimeric polynucleotides may be separated by a linker or spacer moiety. Such linkers or spaces may be nucleic acid based or non-nucleosidic.

In one case, the chimeric polynucleotides may include a sequence encoding a self-cleaving peptide. The self-cleaving peptide may be, but is not limited to, a 2A peptide. As a non-limiting example, the 2A peptide may have the protein sequence: GSGATNFSLLKQAGDVEENPGP (SEQ ID NO: 1), fragments or variants thereof. In one case, the 2A peptide cleaves between the last glycine and last proline. As another non-limiting example, the chimeric polynucleotides may include a polynucleotide sequence encoding the 2A peptide having the protein sequence GSGATNFSLLKQAGDVEENPGP (SEQ ID NO: 1) fragments or variants thereof.

One such polynucleotide sequence encoding the 2A peptide is GGAAGCGGAGCTACTAACTTCAGCCTGCTGAAGCAGGCTGGAGACGTGGA GGAGAACCCTGGACCT (SEQ ID NO: 2). The polynucleotide sequence may be modified or codon optimized by the methods described herein and/or are known in the art.

In one case, this sequence may be used to separate the coding region of two or more polypeptides of interest. As a non-limiting example, the sequence encoding the 2A peptide may be between a first coding region A and a second coding region B (A-2Apep-B). The presence of the 2A peptide would result in the cleavage of one long protein into protein A, protein B and the 2A peptide. Protein A and protein B may be the same or different polypeptides of interest. In another case, the 2A peptide may be used in the chimeric polynucleotides of the present disclosure to produce two, three, four, five, six, seven, eight, nine, ten or more proteins.

In some cases, the chimeric polynucleotides have a sequence comprising Formula II: [Aₙ]-L¹-[Bₒ] **Formula II**

wherein each A and B independently includes any nucleoside (e.g., a nucleotide);

n and o are, independently 10 to 10,000, e.g., 10 to 1000 or 10 to 2000; and

L¹ has the structure of Formula III:

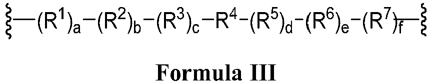

wherein a, b, c, d, e, and f are each, independently, 0 or 1;

each of R¹, R³, R⁵, and R⁷, is, independently, selected from optionally substituted C₁-C₆ alkylene, optionally substituted C₁-C₆ heteroalkylene, O, S, and NR⁸;

R² and R⁶ are each, independently, selected from carbonyl, thiocarbonyl, sulfonyl, or phosphoryl;

R⁴ is optionally substituted C₁-C₁₀ alkylene, optionally substituted C₂-C₁₀ alkenylene, optionally substituted C₂-C₁₀ alkynylene, optionally substituted C₂-C₉ heterocyclylene, optionally substituted C₆-C₁₂ arylene, optionally substituted C₂-C₁₀₀ polyethylene glycolene, or optionally substituted C₁-C₁₀ heteroalkylene, or a bond linking (R¹)ₐ-(R²)_{b}-(R³)_{c} to (R⁵)_{d}-(R⁶)ₑ-(R⁷)_{f}, wherein if a, b, c, d, e, and f are 0, R⁴ is not a bond; and

R⁸ is hydrogen, optionally substituted C₁-C₄ alkyl, optionally substituted C₂-C₄ alkenyl, optionally substituted C₂-C₄ alkynyl, optionally substituted C₂-C₆ heterocyclyl, optionally substituted C₆-C₁₂ aryl, or optionally substituted C₁-C₇ heteroalkyl;

wherein L¹ is attached to [Aₙ] and [Bₒ] at the sugar of one of the nucleosides (e.g., at the 3' position of a sugar of a nucleoside of [Aₙ] and the 5' position of a sugar of a nucleoside of [Bₒ] or at the 5' position of a sugar of a nucleoside of [Aₙ] and the 3' position of a sugar of a nucleoside of [Bₒ]).

In other cases,

the chimeric polynucleotides of the disclosure have a sequence comprising Formula II: [Aₙ]-L¹-[Bₒ] **Formula II**

wherein each A and B independently includes any nucleoside (e.g., a nucleotide);

n and o are, independently 10 to 10,000, e.g., 10 to 1000 or 10 to 2000; and

L¹ is a bond or has the structure of Formula III:

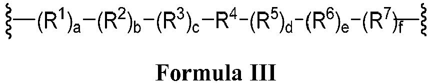

wherein a, b, c, d, e, and f are each, independently, 0 or 1;

each of R¹, R³, R⁵, and R⁷, is, independently, selected from optionally substituted C₁-C₆ alkylene, optionally substituted C₁-C₆ heteroalkylene, O, S, and NR⁸;

R² and R⁶ are each, independently, selected from carbonyl, thiocarbonyl, sulfonyl, or phosphoryl;

R⁴ is optionally substituted C₁-C₁₀ alkylene, optionally substituted C₂-C₁₀ alkenylene, optionally substituted C₂-C₁₀ alkynylene, optionally substituted C₂-C₉ heterocyclylene, optionally substituted C₆-C₁₂ arylene, optionally substituted C₂-C₁₀₀ polyethylene glycolene, or optionally substituted C₁-C₁₀ heteroalkylene, or a bond linking (R¹)ₐ-(R²)_{b}-(R³)_{c} to (R⁵)_{d}-(R⁶)ₑ-(R⁷)_{f}; and

R⁸ is hydrogen, optionally substituted C₁-C₄ alkyl, optionally substituted C₂-C₄ alkenyl, optionally substituted C₂-C₄ alkynyl, optionally substituted C₂-C₆ heterocyclyl, optionally substituted C₆-C₁₂ aryl, or optionally substituted C₁-C₇ heteroalkyl;

wherein L¹ is attached to [Aₙ] and [Bₒ] at the sugar of one of the nucleosides (e.g., at the 3' position of a sugar of a nucleoside of [Aₙ] and the 5' position of a sugar of a nucleoside of [Bₒ] or at the 5' position of a sugar of a nucleoside of [Aₙ] and the 3' position of a sugar of a nucleoside of [Bₒ]);

wherein at least one of [Aₙ] or [Bₒ] comprises the structure of Formula IV or Formula XVII:

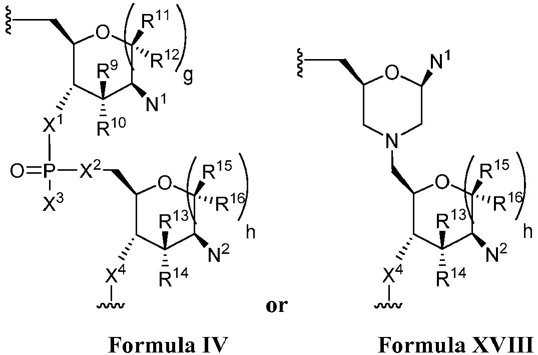

wherein each of N¹ and N² is independently a nucleobase;

each of R⁹, R¹⁰, R¹¹, R¹², R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

each of g and h is, independently, 0 or 1;

each X¹ and X⁴ is, independently, O, NH, or S; and

each X² is independently O, NH, or S; and

each X³ is OH or SH, or a salt thereof;

wherein, for Formula IV, at least one of X¹, X², or X⁴ is NH or S.

For example, in some cases, the chimeric polynucleotides of the disclosure include the structure: 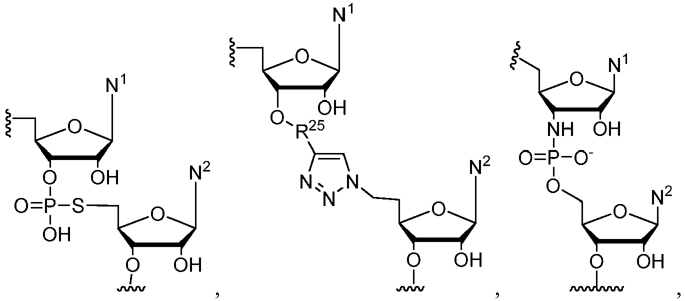 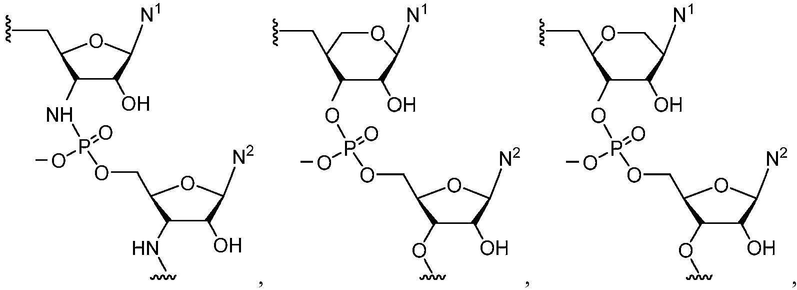 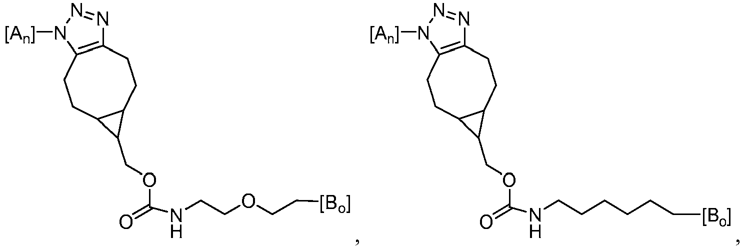 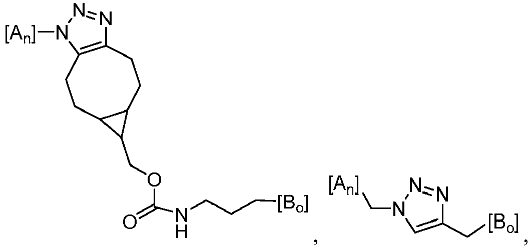 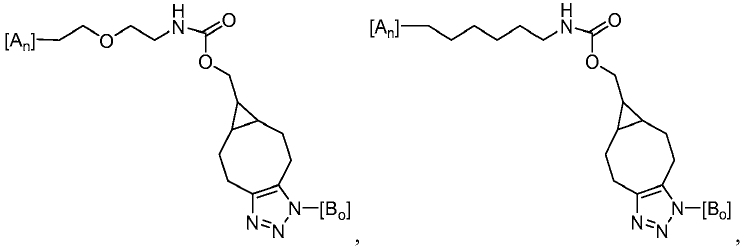 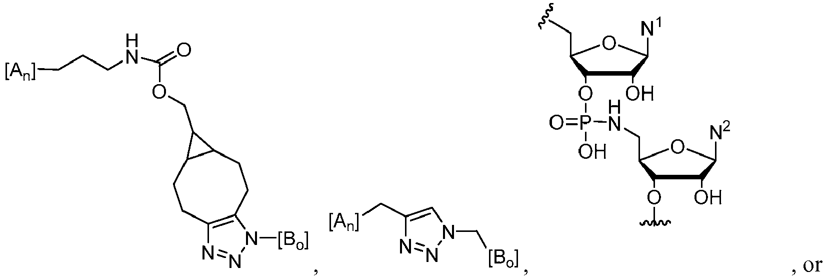 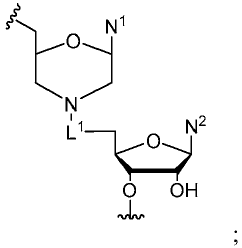 wherein R²⁵ is absent, optionally substituted C₁-C₆ alkylene, or optionally substituted C₁-C₆ heteroalkylene.

In some cases, the presence of a hydroxyl at the 2' position of the sugar allows for increased ribosomal recognition.

In certain cases, of the chimeric polynucleotides of the disclosure one of the coding region, the 5' UTR, the 3' UTR, the 5' cap structure, or the poly-A tail comprises [Aₙ]-L¹-[Bₒ].

In other cases, of the chimeric polynucleotides of the disclosure one of the coding region, the 5' UTR, the 3' UTR, the 5' cap structure, or the poly-A tail comprises [Aₙ] and another of the coding region, the 5' UTR, the 3' UTR, the 5' cap structure, or the poly-A tail comprises [Bₒ]. For example, in some cases, the poly A tail comprises one of [Aₙ] or [Bₒ] and the 3' UTR comprises the other. In other cases, the 5' cap structure comprises one of [Aₙ] or [Bₒ] and the 5' UTR comprises the other.

In some cases, the 5' UTR includes at least one Kozak sequence.

Notwithstanding the foregoing, the chimeric polynucleotides may comprise a region or part which is not positionally modified or not chimeric as defined herein.

For example, a region or part of a chimeric polynucleotide may be uniformly modified at one or more A, T, C, G, or U but according to the disclosure, the polynucleotides will not be uniformly modified throughout the entire region or part.

Regions or parts of chimeric polynucleotides may be from 15-1000 nucleosides in length and a polynucleotide may have from 2-100 different regions or patterns of regions as described herein.

In one case, chimeric polynucleotides encode one or more polypeptides of interest. In another case, the chimeric polynucleotides are substantially non-coding. In another case, the chimeric polynucleotides have both coding and non-coding regions and parts.

Figure 2 illustrates the design of certain chimeric polynucleotides of the present disclosure when based on the scaffold of the polynucleotide of Figure 1. Shown in the figure are the regions or parts of the chimeric polynucleotides where patterned regions represent those regions which are positionally modified and open regions illustrate regions which may or may not be modified but which are, when modified, uniformly modified. Chimeric polynucleotides may be completely positionally modified or partially positionally modified. They may also have subregions which may be of any pattern or design. Shown in the figure are a chimeric subregion and a hemimer subregion.

In one case, the shortest length of a region of the chimeric polynucleotide of the present disclosure encoding a peptide can be the length that is sufficient to encode for a dipeptide, a tripeptide, a tetrapeptide, a pentapeptide, a hexapeptide, a heptapeptide, an octapeptide, a nonapeptide, or a decapeptide. In another case, the length may be sufficient to encode a peptide of 2-30 amino acids, e.g. 5-30, 10-30, 2-25, 5-25, 10-25, or 10-20 amino acids. The length may be sufficient to encode for a peptide of at least 11, 12, 13, 14, 15, 17, 20, 25 or 30 amino acids, or a peptide that is no longer than 40 amino acids, e.g. no longer than 35, 30, 25, 20, 17, 15, 14, 13, 12, 11 or 10 amino acids. Examples of dipeptides that the polynucleotide sequences can encode or include, but are not limited to, carnosine and anserine.

In one case, the length of a region encoding the polypeptide of disclosure interest of the present disclosure is greater than about 30 nucleotides in length (e.g., at least or greater than about 35, 40, 45, 50, 55, 60, 70, 80, 90, 100, 120, 140, 160, 180, 200, 250, 300, 350, 400, 450, 500, 600, 700, 800, 900, 1,000, 1,100, 1,200, 1,300, 1,400, 1,500, 1,600, 1,700, 1,800, 1,900, 2,000, 2,500, and 3,000, 4,000, 5,000, 6,000, 7,000, 8,000, 9,000, 10,000, 20,000, 30,000, 40,000, 50,000, 60,000, 70,000, 80,000, 90,000 or up to and including 100,000 nucleotides). As used herein, such a region may be referred to as a "coding region" or "region encoding."

In some cases, the chimeric polynucleotide includes from about 30 to about 100,000 nucleotides (e.g., from 30 to 50, from 30 to 100, from 30 to 250, from 30 to 500, from 30 to 1,000, from 30 to 1,500, from 30 to 3,000, from 30 to 5,000, from 30 to 7,000, from 30 to 10,000, from 30 to 25,000, from 30 to 50,000, from 30 to 70,000, from 100 to 250, from 100 to 500, from 100 to 1,000, from 100 to 1,500, from 100 to 3,000, from 100 to 5,000, from 100 to 7,000, from 100 to 10,000, from 100 to 25,000, from 100 to 50,000, from 100 to 70,000, from 100 to 100,000, from 500 to 1,000, from 500 to 1,500, from 500 to 2,000, from 500 to 3,000, from 500 to 5,000, from 500 to 7,000, from 500 to 10,000, from 500 to 25,000, from 500 to 50,000, from 500 to 70,000, from 500 to 100,000, from 1,000 to 1,500, from 1,000 to 2,000, from 1,000 to 3,000, from 1,000 to 5,000, from 1,000 to 7,000, from 1,000 to 10,000, from 1,000 to 25,000, from 1,000 to 50,000, from 1,000 to 70,000, from 1,000 to 100,000, from 1,500 to 3,000, from 1,500 to 5,000, from 1,500 to 7,000, from 1,500 to 10,000, from 1,500 to 25,000, from 1,500 to 50,000, from 1,500 to 70,000, from 1,500 to 100,000, from 2,000 to 3,000, from 2,000 to 5,000, from 2,000 to 7,000, from 2,000 to 10,000, from 2,000 to 25,000, from 2,000 to 50,000, from 2,000 to 70,000, and from 2,000 to 100,000).

Regions or subregions of chimeric polynucleotides may also range independently from 15-1,000 nucleotides in length (e.g., greater than 30, 40, 45, 50, 55, 60, 70, 80, 90, 100, 120, 140, 160, 180, 200, 250, 300, 350, 400, 450, 500, 600, 700, 800, and 900 nucleotides or at least 30, 40, 45, 50, 55, 60, 70, 80, 90, 100, 120, 140, 160, 180, 200, 250, 300, 350, 400, 450, 500, 600, 700, 800, 900, and 1,000 nucleotides).

Regions or subregions of chimeric polynucleotides may range from absent to 500 nucleotides in length (e.g., at least 60, 70, 80, 90, 100, 110, 120, 130, 140, 150, 160, 170, 180, 190, 200, 250, 300, 350, 400, 450, or 500 nucleotides). Where the region is a polyA tail, the length may be determined in units of or as a function of polyA Binding Protein binding. In this case, the polyA tail is long enough to bind at least 4 monomers of PolyA Binding Protein. PolyA Binding Protein monomers bind to stretches of approximately 38 nucleotides. As such, it has been observed that polyA tails of about 80 nucleotides and 160 nucleotides are functional. The chimeric polynucleotides of the present disclosure which function as an mRNA need not comprise a polyA tail.

Chimeric polynucleotides which function as an mRNA may have a capping region. The capping region may comprise a single cap or a series of nucleotides forming the cap. In this case, the capping region may be from 1 to 10, e.g. 2-9, 3-8, 4-7, 1-5, 5-10, or at least 2, or 10 or fewer nucleotides in length. In some cases, the cap is absent.

### Circular Chimeric Polynucleotide Architecture

Described herein are chimeric polynucleotides which are circular or cyclic. As the name implies circular polynucleotides are circular in nature meaning that the termini are joined in some fashion, whether by ligation, covalent bond, common association with the same protein or other molecule or complex or by hybridization. Any of the circular polynucleotides as taught in in co-pending International Publication No. WO2015034925 may be made chimeric according to the present disclosure.

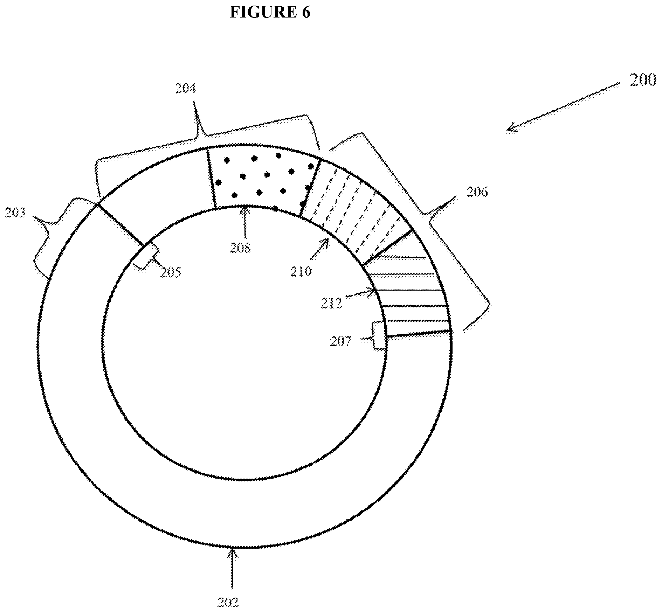

Chimeric polynucleotides may be designed according to the circular RNA construct scaffolds shown in Figures 6-12. Such polynucleotides are circular chimeric polynucleotides or circular constructs.

As used herein, "circular polynucleotides" or "circP" means a single stranded circular polynucleotide which acts substantially like, and has the properties of, an RNA. The term "circular" is also meant to encompass and secondary or tertiary configuration of the circP.

The circPs of the present disclosure which encode at least one polypeptide of interest are known as circular RNAs or circRNA. As used herein, "circular RNA" or "circRNA" means a circular polynucleotide that can encode at least one polypeptide of interest. The circPs of the present disclosure which comprise at least one sensor sequence and do not encode a polypeptide of interest are known as circular sponges or circSP. As used herein, "circular sponges," "circular polynucleotide sponges" or "circSP" means a circular polynucleotide which comprises at least one sensor sequence and does not encode a polypeptide of interest. As used herein, "sensor sequence" means a receptor or pseudo-receptor for endogenous nucleic acid binding molecules. Non-limiting examples of sensor sequences include, microRNA binding sites, microRNA seed sequences, microRNA binding sites without the seed sequence, transcription factor binding sites and artificial binding sites engineered to act as pseudo-receptors and portions and fragments thereof.

The circPs of the present disclosure which comprise at least one sensor sequence and encode at least one polypeptide of interest are known as circular RNA sponges or circRNA-SP. As used herein, "circular RNA sponges" or "circRNA-SP" means a circular polynucleotide which comprises at least one sensor sequence and at least one region encoding at least one polypeptide of interest.

Figure 6 shows a representative circular construct 200 of the present disclosure. As used herein, the term "circular construct" refers to a circular polynucleotide transcript which may act substantially similar to and have properties of a RNA molecule. In one case, the circular construct acts as an mRNA. If the circular construct encodes one or more polypeptides of interest (e.g., a circRNA or circRNA-SP) then the polynucleotide transcript retains sufficient structural and/or chemical features to allow the polypeptide of interest encoded therein to be translated. Circular constructs may be polynucleotides of the disclosure. When structurally or chemically modified, the construct may be referred to as a modified circP, circSP, circRNA or circRNA-SP.

Returning to FIG. **6****,** the circular construct **200** here contains a first region of linked nucleotides **202** that is flanked by a first flanking region **204** and a second flanking region **206.** As used herein, the "first region" may be referred to as a "coding region," a "non-coding region" or "region encoding" or simply the "first region." In one case, this first region may comprise nucleotides such as, but not limited to, encoding the polypeptide of interest and/or nucleotides encodes or comprises a sensor region. The polynucleotide may encode at its 5' terminus one or more signal peptide sequences in the signal sequence region **203.** The first flanking region **204** may comprise a region of linked nucleosides or portion thereof which may act similarly to an untranslated region (UTR) in an mRNA and/or DNA sequence. The first flanking region may also comprise a region of polarity **208.** The region of polarity **208** may include an IRES sequence or portion thereof. As a non-limiting example, when linearized this region may be split to have a first portion be on the 5' terminus of the first region **202** and second portion be on the 3' terminus of the first region **202.** The second flanking region **206** may comprise a tailing sequence region **210** and may comprise a region of linked nucleotides or portion thereof **212** which may act similarly to a UTR in an mRNA and/or DNA.

Bridging the 5' terminus of the first region **202** and the first flanking region **204** is a first operational region **205.** In one case, this operational region may comprise a start codon. The operational region may alternatively comprise any translation initiation sequence or signal including a start codon.

Bridging the 3' terminus of the first region **202** and the second flanking region **206** is a second operational region **207.** Traditionally this operational region comprises a stop codon. The operational region may alternatively comprise any translation initiation sequence or signal including a stop codon.

Multiple serial stop codons may also be used. In one case, the operation region may comprise two stop codons. The first stop codon may be "TGA" or "UGA" and the second stop codon may be selected from the group consisting of "TAA," "TGA," "TAG," "UAA," "UGA" or "UAG."

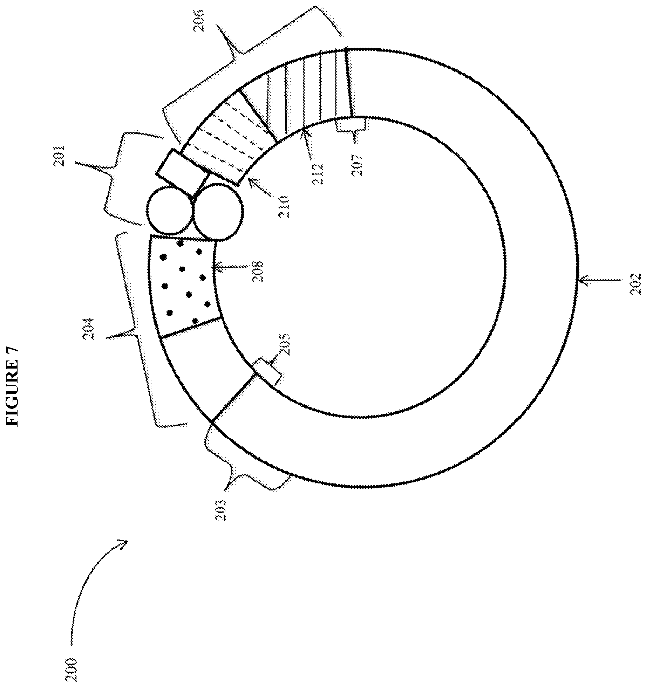

Turning to Figure **7****,** at least one non-nucleic acid moiety **201** may be used to prepare a circular polynucleotide **200** where the non-nucleic acid moiety **201** is used to bring the first flanking region **204** near the second flanking region **206.** Non-limiting examples of non-nucleic acid moieties which may be used
are described herein. The circular polynucleotides **200** may comprise more than one non-nucleic acid moiety wherein the additional non-nucleic acid moieties may be heterologous or homologous to the first non-nucleic acid moiety.

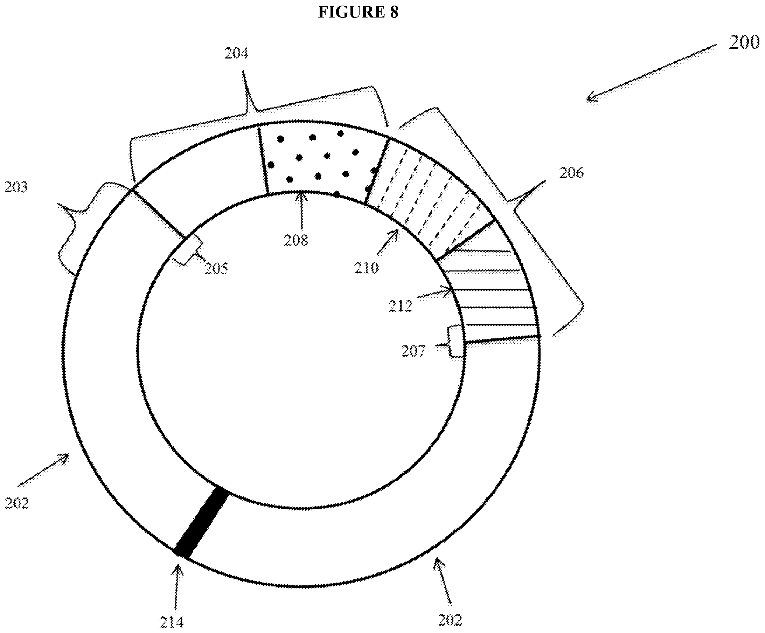

Turning to Figure **8****,** the first region of linked nucleosides **202** may comprise a spacer region **214.** This spacer region **214** may be used to separate the first region of linked nucleosides **202** so that the circular construct can include more than one open reading frame, non-coding region or an open reading frame and a non-coding region.

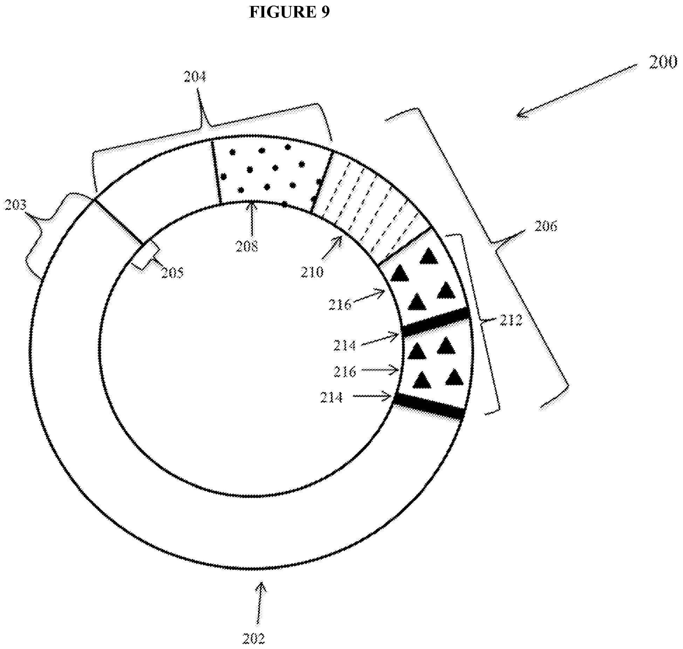

Turning to Figure **9****,** the second flanking region **206** may comprise one or more sensor regions **216** in the 3'UTR **212.** These sensor sequences as discussed herein operate as pseudo-receptors (or binding sites) for ligands of the local microenvironment of the circular construct or circular polynucleotide. For example, microRNA binding sites or miRNA seeds may be used as sensors such that they function as pseudoreceptors for any microRNAs present in the environment of the circular polynucleotide. As shown in Figure **9****,** the one or more sensor regions **216** may be separated by a spacer region **214.**

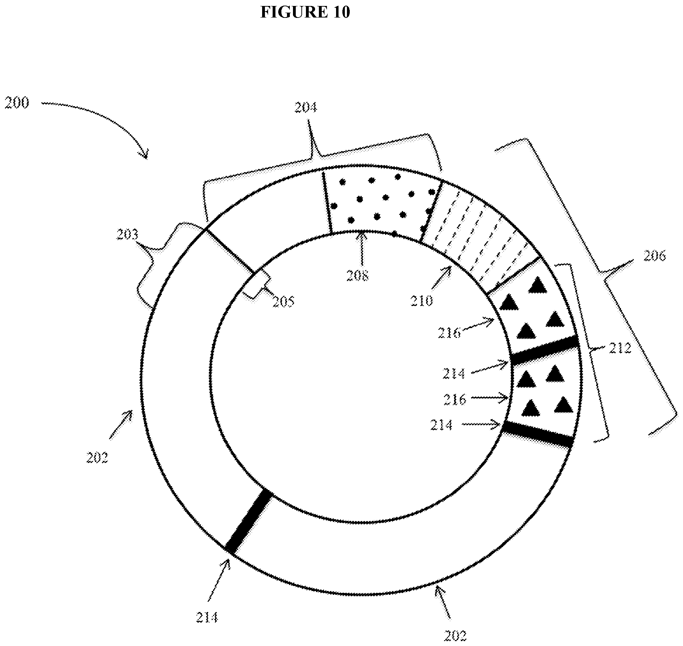

As shown in Figure **10****,** a circular construct **200,** which includes one or more sensor regions **216,** may also include a spacer region **214** in the first region of linked nucleosides **202.** As discussed above for Figure **7****,** this spacer region **214** may be used to separate the first region of linked nucleosides **202** so that the circular construct can include more than one open reading frame and/or more than one non-coding region.

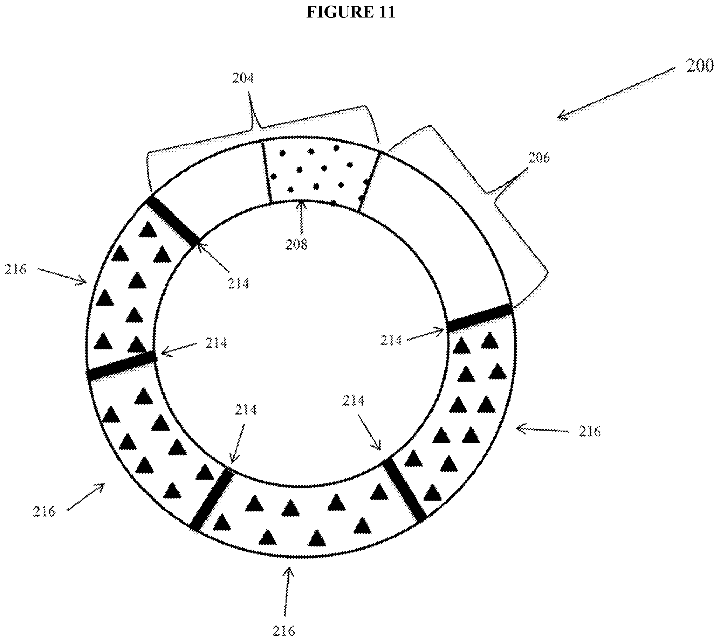

Turning to Figure **11****,** a circular construct **200** may be a non-coding construct known as a circSP comprising at least one non-coding region such as, but not limited to, a sensor region **216.** Each of the sensor regions **216** may include, but are not limited to, a miR sequence, a miR seed, a miR binding site and/or a miR sequence without the seed.

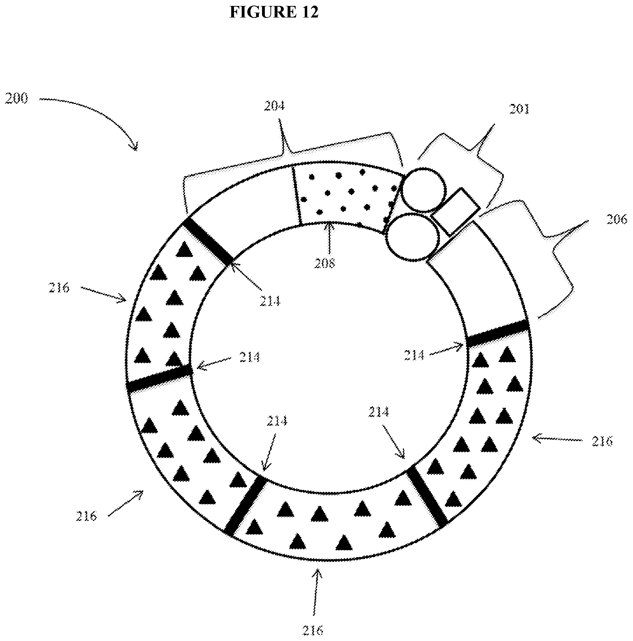

Turning to Figure **12****,** at least one non-nucleic acid moiety **201** may be used to prepare a circular polynucleotide **200** which is a non-coding construct. The circular polynucleotides **200** which is a non-coding construct may comprise more than one non-nucleic acid moiety wherein the additional non-nucleic acid moieties may be heterologous or homologous to the first non-nucleic acid moiety.

### Multimers of chimeric polynucleotides

Multiple distinct chimeric polynucleotides may be linked together through the 3'-end using nucleotides which are modified at the 3'-terminus. Chemical conjugation may be used to control the stoichiometry of delivery into cells. For example, the glyoxylate cycle enzymes, isocitrate lyase and malate synthase, may be supplied into cells at a 1:1 ratio to alter cellular fatty acid metabolism. This ratio may be controlled by chemically linking chimeric polynucleotides using a 3'-azido terminated nucleotide on one chimeric polynucleotides species and a C5-ethynyl or alkynyl-containing nucleotide on the opposite chimeric polynucleotide species. The modified nucleotide is added post-transcriptionally using terminal transferase (New England Biolabs, Ipswich, MA) according to the manufacturer's protocol. After the addition of the 3'-modified nucleotide, the two chimeric polynucleotides species may be combined in an aqueous solution, in the presence or absence of copper, to form a new covalent linkage via a click chemistry mechanism as described in the literature.

In another example, more than two polynucleotides may be linked together using a functionalized linker molecule. For example, a functionalized saccharide molecule may be chemically modified to contain multiple chemical reactive groups (SH-, NH₂-, N₃, etc...) to react with the cognate moiety on a 3'-functionalized mRNA molecule (i.e., a 3'-maleimide ester, 3'-NHS-ester, alkynyl). The number of reactive groups on the modified saccharide can be controlled in a stoichiometric fashion to directly control the stoichiometric ratio of conjugated chimeric polynucleotides.

### Conjugates and Combinations of Chimeric polynucleotides

In order to further enhance protein production, chimeric polynucleotides of the present disclosure can be designed to be conjugated to other polynucleotides, dyes, intercalating agents (*e.g.* acridines), cross-linkers (*e.g.* psoralene, mitomycin C), porphyrins (TPPC4, texaphyrin, Sapphyrin), polycyclic aromatic hydrocarbons (e.g., phenazine, dihydrophenazine), artificial endonucleases (e.g. EDTA), alkylating agents, phosphate, amino, mercapto, PEG (e.g., PEG-40K), MPEG, [MPEG]₂, polyamino, alkyl, substituted alkyl, radiolabeled markers, enzymes, haptens (e.g. biotin), transport/absorption facilitators (e.g., aspirin, vitamin E, folic acid), synthetic ribonucleases, proteins, e.g., glycoproteins, or peptides, e.g., molecules having a specific affinity for a co-ligand, or antibodies e.g., an antibody, that binds to a specified cell type such as a cancer cell, endothelial cell, or bone cell, hormones and hormone receptors, non-peptidic species, such as lipids, lectins, carbohydrates, vitamins, cofactors, or a drug.

Conjugation may result in increased stability and/or half-life and may be particularly useful in targeting the chimeric polynucleotides to specific sites in the cell, tissue or organism.

The chimeric polynucleotides may be administered with, conjugated to or further encode one or more of RNAi agents, siRNAs, shRNAs, miRNAs, miRNA binding sites, antisense RNAs, ribozymes, catalytic DNA, tRNA, RNAs that induce triple helix formation, aptamers or vectors, and the like.

### Bifunctional chimeric polynucleotides

Described herein are bifunctional polynucleotides (e.g., bifunctional chimeric polynucleotides). As the name implies, bifunctional polynucleotides are those having or capable of at least two functions. These molecules may also by convention be referred to as multi-functional.

The multiple functionalities of bifunctional polynucleotides may be encoded by the RNA (the function may not manifest until the encoded product is translated) or may be a property of the polynucleotide itself. It may be structural or chemical. Bifunctional modified polynucleotides may comprise a function that is covalently or electrostatically associated with the polynucleotides. Further, the two functions may be described in the context of a complex of a chimeric polynucleotide and another molecule.

Bifunctional polynucleotides may encode peptides which are anti-proliferative. These peptides may be linear, cyclic, constrained or random coil. They may function as aptamers, signaling molecules, ligands or mimics or mimetics thereof. Anti-proliferative peptides may, as translated, be from 3 to 50 amino acids in length. They may be 5-40, 10-30, or approximately 15 amino acids long. They may be single chain, multichain or branched and may form complexes, aggregates or any multi-unit structure once translated.

### Noncoding chimeric polynucleotides

Described herein are chimeric polynucleotides having sequences that are partially or substantially not translatable, e.g., having a noncoding region. Such noncoding region may be the "first region" of the chimeric polynucleotide. Alternatively, the noncoding region may be a region other than the first region. Such molecules are generally not translated, but can exert an effect on protein production by one or more of binding to and sequestering one or more translational machinery components such as a ribosomal protein or a transfer RNA (tRNA), thereby effectively reducing protein expression in the cell or modulating one or more pathways or cascades in a cell which in turn alters protein levels. The chimeric polynucleotide may contain or encode one or more long noncoding RNA (lncRNA, or lincRNA) or portion thereof, a small nucleolar RNA (sno-RNA), micro RNA (miRNA), small interfering RNA (siRNA) or Piwi-interacting RNA (piRNA). Examples of such IncRNA molecules and RNAi constructs designed to target such IncRNA any of which may be encoded in the chimeric polynucleotides are taught in

International Publication, WO2012/018881 A2.

### Polypeptides of interest

Chimeric polynucleotides may encode one or more peptides or polypeptides of interest. They may also affect the levels, signaling or function of one or more polypeptides. Polypeptides of interest, according to the present disclosure include any of those taught in, for example, those listed in Table 6 of International Publication Nos. WO2013151666, WO2013151668, WO2013151663, WO2013151669, WO2013151670, WO2013151664, WO2013151665, WO2013151736; Tables 6 and 7 International Publication No. WO2013151672; Tables 6, 178 and 179 of International Publication No. WO2013151671; Tables 6, 185 and 186 of International Publication No WO2013151667.

The chimeric polynucleotide may be designed to encode one or more polypeptides of interest or fragments thereof. Such polypeptide of interest may include, but is not limited to, whole polypeptides, a plurality of polypeptides or fragments of polypeptides, which independently may be encoded by one or more regions or parts or the whole of a chimeric polynucleotide. As used herein, the term "polypeptides of interest" refer to any polypeptide which is selected to be encoded within, or whose function is affected by, the chimeric polynucleotides of the present disclosure.

As used herein, "polypeptide" means a polymer of amino acid residues (natural or unnatural) linked together most often by peptide bonds. The term, as used herein, refers to proteins, polypeptides, and peptides of any size, structure, or function. In some instances the polypeptide encoded is smaller than about 50 amino acids and the polypeptide is then termed a peptide. If the polypeptide is a peptide, it will be at least about 2, 3, 4, or at least 5 amino acid residues long. Thus, polypeptides include gene products, naturally occurring polypeptides, synthetic polypeptides, homologs, orthologs, paralogs, fragments and other equivalents, variants, and analogs of the foregoing. A polypeptide may be a single molecule or may be a multimolecular complex such as a dimer, trimer or tetramer. They may also comprise single chain or multichain polypeptides such as antibodies or insulin and may be associated or linked. Most commonly disulfide linkages are found in multichain polypeptides. The term polypeptide may also apply to amino acid polymers in which one or more amino acid residues are an artificial chemical analogue of a corresponding naturally occurring amino acid.

The term "polypeptide variant" refers to molecules which differ in their amino acid sequence from a native or reference sequence. The amino acid sequence variants may possess substitutions, deletions, and/or insertions at certain positions within the amino acid sequence, as compared to a native or reference sequence. Ordinarily, variants will possess at least about 50% identity (homology) to a native or reference sequence, and preferably, they will be at least about 80%, more preferably at least about 90% identical (homologous) to a native or reference sequence.

In some cases "variant mimics" are described. As used herein, the term "variant mimic" is one which contains one or more amino acids which would mimic an activated sequence. For example, glutamate may serve as a mimic for phosphoro-threonine and/or phosphoro-serine. Alternatively, variant mimics may result in deactivation or in an inactivated product containing the mimic, e.g., phenylalanine may act as an inactivating substitution for tyrosine; or alanine may act as an inactivating substitution for serine.

"Homology" as it applies to amino acid sequences is defined as the percentage of residues in the candidate amino acid sequence that are identical with the residues in the amino acid sequence of a second sequence after aligning the sequences and introducing gaps, if necessary, to achieve the maximum percent homology. Methods and computer programs for the alignment are well known in the art. It is understood that homology depends on a calculation of percent identity but may differ in value due to gaps and penalties introduced in the calculation.

By "homologs" as it applies to polypeptide sequences means the corresponding sequence of other species having substantial identity to a second sequence of a second species.

"Analogs" is meant to include polypeptide variants which differ by one or more amino acid alterations, e.g., substitutions, additions or deletions of amino acid residues that still maintain one or more of the properties of the parent or starting polypeptide.

Described herein are several types of compositions which are polypeptide based including variants and derivatives. These include substitutional, insertional, deletion and covalent variants and derivatives. The term "derivative" is used synonymously with the term "variant" but generally refers to a molecule that has been modified and/or changed in any way relative to a reference molecule or starting molecule.

As such, chimeric polynucleotides encoding polypeptides containing substitutions, insertions and/or additions, deletions and covalent modifications with respect to reference sequences, in particular the polypeptide sequences disclosed herein, are included within the scope of this
disclosure. For example, sequence tags or amino acids, such as one or more lysines, can be added to the peptide sequences of the disclosure (e.g., at the N-terminal or C-terminal ends). Sequence tags can be used for peptide purification or localization. Lysines can be used to increase peptide solubility or to allow for biotinylation. Alternatively, amino acid residues located at the carboxy and amino terminal regions of the amino acid sequence of a peptide or protein may optionally be deleted providing for truncated sequences. Certain amino acids (e.g., C-terminal or N-terminal residues) may alternatively be deleted depending on the use of the sequence, as for example, expression of the sequence as part of a larger sequence which is soluble, or linked to a solid support.

"Substitutional variants" when referring to polypeptides are those that have at least one amino acid residue in a native or starting sequence removed and a different amino acid inserted in its place at the same position. The substitutions may be single, where only one amino acid in the molecule has been substituted, or they may be multiple, where two or more amino acids have been substituted in the same molecule.

As used herein the term "conservative amino acid substitution" refers to the substitution of an amino acid that is normally present in the sequence with a different amino acid of similar size, charge, or polarity. Examples of conservative substitutions include the substitution of a non-polar (hydrophobic) residue such as isoleucine, valine and leucine for another non-polar residue. Likewise, examples of conservative substitutions include the substitution of one polar (hydrophilic) residue for another such as between arginine and lysine, between glutamine and asparagine, and between glycine and serine. Additionally, the substitution of a basic residue such as lysine, arginine or histidine for another, or the substitution of one acidic residue such as aspartic acid or glutamic acid for another acidic residue are additional examples of conservative substitutions. Examples of non-conservative substitutions include the substitution of a non-polar (hydrophobic) amino acid residue such as isoleucine, valine, leucine, alanine, methionine for a polar (hydrophilic) residue such as cysteine, glutamine, glutamic acid or lysine and/or a polar residue for a non-polar residue.

"Insertional variants" when referring to polypeptides are those with one or more amino acids inserted immediately adjacent to an amino acid at a particular position in a native or starting sequence. "Immediately adjacent" to an amino acid means connected to either the alpha-carboxy or alpha-amino functional group of the amino acid.

"Deletional variants" when referring to polypeptides are those with one or more amino acids in the native or starting amino acid sequence removed. Ordinarily, deletional variants will have one or more amino acids deleted in a particular region of the molecule.

"Covalent derivatives" when referring to polypeptides include modifications of a native or starting protein with an organic proteinaceous or non-proteinaceous derivatizing agent, and/or post-translational modifications. Covalent modifications are traditionally introduced by reacting targeted amino acid residues of the protein with an organic derivatizing agent that is capable of reacting with selected side-chains or terminal residues, or by harnessing mechanisms of post-translational modifications that function in selected recombinant host cells. The resultant covalent derivatives are useful in programs directed at identifying residues important for biological activity, for immunoassays, or for the preparation of anti-protein antibodies for immunoaffinity purification of the recombinant glycoprotein. Such modifications are within the ordinary skill in the art and are performed without undue experimentation.

Certain post-translational modifications are the result of the action of recombinant host cells on the expressed polypeptide. Glutaminyl and asparaginyl residues are frequently post-translationally deamidated to the corresponding glutamyl and aspartyl residues. Alternatively, these residues are deamidated under mildly acidic conditions. Either form of these residues may be present in the polypeptides produced disclosure in accordance with the present disclosure.

Other post-translational modifications include hydroxylation of proline and lysine, phosphorylation of hydroxyl groups of seryl or threonyl residues, methylation of the alpha-amino groups of lysine, arginine, and histidine side chains (T. E. Creighton, Proteins: Structure and Molecular Properties, W.H. Freeman & Co., San Francisco, pp. 79-86 (1983)).

"Features" when referring to polypeptides are defined as distinct amino acid sequence-based components of a molecule. Features of the polypeptides encoded disclosure by the chimeric polynucleotides of the present disclosure include surface manifestations, local conformational shape, folds, loops, half-loops, domains, half-domains, sites, termini or any combination thereof.

As used herein when referring to polypeptides the term "surface manifestation" refers to a polypeptide based component of a protein appearing on an outermost surface.

As used herein when referring to polypeptides the term "local conformational shape" means a polypeptide based structural manifestation of a protein which is located within a definable space of the protein.

As used herein when referring to polypeptides the term "fold" refers to the resultant conformation of an amino acid sequence upon energy minimization. A fold may occur at the secondary or tertiary level of the folding process. Examples of secondary level folds include beta sheets and alpha helices. Examples of tertiary folds include domains and regions formed due to aggregation or separation of energetic forces. Regions formed in this way include hydrophobic and hydrophilic pockets, and the like.

As used herein the term "turn" as it relates to protein conformation means a bend which alters the direction of the backbone of a peptide or polypeptide and may involve one, two, three or more amino acid residues.

As used herein when referring to polypeptides the term "loop" refers to a structural feature of a polypeptide which may serve to reverse the direction of the backbone of a peptide or polypeptide. Where the loop is found in a polypeptide and only alters the direction of the backbone, it may comprise four or more amino acid residues. Oliva et al. have identified at least 5 classes of protein loops (J. Mol Biol 266 (4): 814-830; 1997). Loops may be open or closed. Closed loops or "cyclic" loops may comprise 2, 3, 4, 5, 6, 7, 8, 9, 10 or more amino acids between the bridging moieties. Such bridging moieties may comprise a cysteine-cysteine bridge (Cys-Cys) typical in polypeptides having disulfide bridges or alternatively bridging moieties may be non-protein based such as the dibromozylyl agents used herein.

As used herein when referring to polypeptides the term "half-loop" refers to a portion of an identified loop having at least half the number of amino acid resides as the loop from which it is derived. It is understood that loops may not always contain an even number of amino acid residues. Therefore, in those cases where a loop contains or is identified to comprise an odd number of amino acids, a half-loop of the odd-numbered loop will comprise the whole number portion or next whole number portion of the loop (number of amino acids of the loop/2+/-0.5 amino acids). For example, a loop identified as a 7 amino acid loop could produce half-loops of 3 amino acids or 4 amino acids (7/2=3.5+/-0.5 being 3 or 4).

As used herein when referring to polypeptides the term "domain" refers to a motif of a polypeptide having one or more identifiable structural or functional characteristics or properties (e.g., binding capacity, serving as a site for protein-protein interactions).

As used herein when referring to polypeptides the term "half-domain" means a portion of an identified domain having at least half the number of amino acid resides as the domain from which it is derived. It is understood that domains may not always contain an even number of amino acid residues. Therefore, in those cases where a domain contains or is identified to comprise an odd number of amino acids, a half-domain of the odd-numbered domain will comprise the whole number portion or next whole number portion of the domain (number of amino acids of the domain/2+/- 0.5 amino acids). For example, a domain identified as a 7 amino acid domain could produce half-domains of 3 amino acids or 4 amino acids (7/2=3.5+/-0.5 being 3 or 4). It is also understood that sub-domains may be identified within domains or half-domains, these subdomains possessing less than all of the structural or functional properties identified in the domains or half domains from which they were derived. It is also understood that the amino acids that comprise any of the domain types herein need not be contiguous along the backbone of the polypeptide (i.e., nonadjacent amino acids may fold structurally to produce a domain, half-domain or subdomain).

As used herein when referring to polypeptides the terms "site" as it pertains to amino acid based cases is used synonymously with "amino acid residue" and "amino acid side chain." A site represents a position within a peptide or polypeptide that may be modified, manipulated, altered, derivatized or varied within the polypeptide based molecules of the present disclosure.

As used herein the terms "termini" or "terminus" when referring to polypeptides refers to an extremity of a peptide or polypeptide. Such extremity is not limited only to the first or final site of the peptide or polypeptide but may include additional amino acids in the terminal regions. The polypeptide based molecules of the present disclosure may be characterized as having both an N-terminus (terminated by an amino acid with a free amino group (NH2)) and a C-terminus (terminated by an amino acid with a free carboxyl group (COOH)). Proteins of the disclosure are in some cases made up of multiple polypeptide chains brought together by disulfide bonds or by non-covalent forces (multimers, oligomers). These sorts of proteins will have multiple N- and C-termini. Alternatively, the termini of the polypeptides may be modified such that they begin or end, as the case may be, with a non-polypeptide based moiety such as an organic conjugate.

Once any of the features have been identified or defined as a desired component of a polypeptide to be encoded by the chimeric polynucleotide any of several manipulations and/or modifications of these features may be performed by moving, swapping, inverting, deleting, randomizing or duplicating. Furthermore, it is understood that manipulation of features may result in the same outcome as a modification to the molecules. For example, a manipulation which involved deleting a domain would result in the alteration of the length of a molecule just as modification of a nucleic acid to encode less than a full length molecule would.

Modifications and manipulations can be accomplished by methods known in the art such as, but not limited to, site directed mutagenesis or *a priori* incorporation during chemical synthesis. The resulting modified molecules may then be tested for activity using *in vitro* or *in vivo* assays such as those described herein or any other suitable screening assay known in the art.

The polypeptides may comprise a consensus sequence which is discovered through rounds of experimentation. As used herein a "consensus" sequence is a single sequence which represents a collective population of sequences allowing for variability at one or more sites.

As recognized by those skilled in the art, protein fragments, functional protein domains, and homologous proteins are also considered to be within the scope of polypeptides of interest of this disclosure. For example, described herein is any protein fragment (meaning a polypeptide sequence at least one amino acid residue shorter than a reference polypeptide sequence but otherwise identical) of a reference protein 10, 20, 30, 40, 50, 60, 70, 80, 90, 100 or greater than 100 amino acids in length. In another example, any protein that includes a stretch of about 20, about 30, about 40, about 50, or about 100 amino acids which are about 40%, about 50%, about 60%, about 70%, about 80%, about 90%, about 95%, or about 100% identical to any of the sequences described herein can be utilized in accordance with the disclosure. In certain cases, a polypeptide to be utilized in accordance with the disclosure includes 2, 3, 4, 5, 6, 7, 8, 9, 10, or more mutations as shown in any of the sequences described herein.

### Types of polypeptides of interest

The chimeric polynucleotides may be designed to encode polypeptides of interest selected from any of several target categories including, but not limited to, biologics, antibodies, vaccines, therapeutic proteins or peptides, cell penetrating peptides, secreted proteins, plasma membrane proteins, cytoplasmic or cytoskeletal proteins, intracellular membrane bound proteins, nuclear proteins, proteins associated with human disease, targeting moieties or those proteins encoded by the human genome for which no therapeutic indication has been identified but which nonetheless have utility in areas of research and discovery.

In one case chimeric polynucleotides may encode variant polypeptides which have a certain identity with a reference polypeptide sequence. As used herein, a "reference polypeptide sequence" refers to a starting polypeptide sequence. Reference sequences may be wild type sequences or any sequence to which reference is made in the design of another sequence. A "reference polypeptide sequence" may, e.g., be any one of those polypeptides disclosed in Table 6 of International Publication Nos. WO2013151666, WO2013151668, WO2013151663, WO2013151669, WO2013151670, WO2013151664, WO2013151665, WO2013151736; Tables 6 and 7 International Publication No. WO2013151672; Tables 6, 178 and 179 of International Publication No. WO2013151671; Tables 6, 185 and 186 of International Publication No WO2013151667;

Reference molecules (polypeptides or polynucleotides) may share a certain identity with the designed molecules (polypeptides or polynucleotides). The term "identity" as known in the art, refers to a relationship between the sequences of two or more peptides, polypeptides or polynucleotides, as determined by comparing the sequences. In the art, identity also means the degree of sequence relatedness between them as determined by the number of matches between strings of two or more amino acid residues or nucleosides. Identity measures the percent of identical matches between the smaller of two or more sequences with gap alignments (if any) addressed by a particular mathematical model or computer program (i.e., "algorithms"). Identity of related peptides can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988).

In some cases, the encoded polypeptide variant may have the same or a similar activity as the reference polypeptide. Alternatively, the variant may have an altered activity (e.g., increased or decreased) relative to a reference polypeptide. Generally, variants of a particular polynucleotide or polypeptide of the disclosure will have at least about 40%, 45%, 50%, 55%, 60%, 65%, 70%, 75%, 80%, 85%, 90%, 91%, 92%, 93%, 94%, 95%, 96%, 97%, 98%, 99% but less than 100% sequence identity to that particular reference polynucleotide or polypeptide as determined by sequence alignment programs and parameters described herein and known to those skilled in the art. Such tools for alignment include those of the BLAST suite (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402.) Other tools are described herein, specifically in the definition of "Identity."

Default parameters in the BLAST algorithm include, for example, an expect threshold of 10, Word size of 28, Match/Mismatch Scores 1, -2, Gap costs Linear. Any filter can be applied as well as a selection for species specific repeats, e.g., Homo sapiens.

### Biologics

The chimeric polynucleotides disclosed herein, may encode one or more biologics. As used herein, a "biologic" is a polypeptide-based molecule produced by the methods described herein and which may be used to treat, cure, mitigate, prevent, or diagnose a serious or life-threatening disease or medical condition. Biologics are described in co-pending International Publication No. WO2015034928, such as in paragraphs [000159] and [000160].

### Antibodies

The chimeric polynucleotides disclosed herein, may encode one or more antibodies or fragments thereof. The term "antibody" includes monoclonal antibodies (including full length antibodies which have an immunoglobulin Fc region), antibody compositions with polyepitopic specificity, multispecific antibodies (e.g., bispecific antibodies, diabodies, and single-chain molecules), as well as antibody fragments. Antibodies are described in co-pending International Publication No. WO2015034928, such as in paragraphs [000161] - [000167].

### Vaccines

The chimeric polynucleotides disclosed herein, may encode one or more vaccines. As used herein, a "vaccine" is a biological preparation that improves immunity to a particular disease or infectious agent.

One or more vaccines currently being marketed or in development may be encoded by the chimeric polynucleotides of the present disclosure. While not wishing to be bound by theory, it is believed that incorporation into the chimeric polynucleotides of the disclosure will result in improved therapeutic efficacy due at least in part to the specificity, purity and selectivity of the construct designs.

Vaccines encoded in the chimeric polynucleotides may be utilized to treat conditions or diseases in many therapeutic areas such as, but not limited to, cardiovascular, CNS, dermatology, endocrinology, oncology, immunology, respiratory, and anti-infective.

### Therapeutic proteins or peptides

The chimeric polynucleotides disclosed herein, may encode one or more validated or "in testing" therapeutic proteins or peptides.

One or more therapeutic proteins or peptides currently being marketed or in development may be encoded by the chimeric polynucleotides of the present disclosure. While not wishing to be bound by theory, it is believed that incorporation into the chimeric polynucleotides of the disclosure will result in improved therapeutic efficacy due at least in part to the specificity, purity and selectivity of the construct designs.

Therapeutic proteins and peptides encoded in the chimeric polynucleotides may be utilized to treat conditions or diseases in many therapeutic areas such as, but not limited to, blood, cardiovascular, CNS, poisoning (including antivenoms), dermatology, endocrinology, genetic, genitourinary, gastrointestinal, musculoskeletal, oncology, and immunology, respiratory, sensory and anti-infective.

### Cell-Penetrating Polypeptides

The chimeric polynucleotides disclosed herein, may encode one or more cell-penetrating polypeptides. As used herein, "cell-penetrating polypeptide" or CPP refers to a polypeptide which may facilitate the cellular uptake of molecules. A cell-penetrating polypeptide may contain one or more detectable labels. The polypeptides may be partially labeled or completely labeled throughout. The chimeric polynucleotides may encode the detectable label completely, partially or not at all. The cell-penetrating peptide may also include a signal sequence. As used herein, a "signal sequence" refers to a sequence of amino acid residues bound at the amino terminus of a nascent protein during protein translation. The signal sequence may be used to signal the secretion of the cell-penetrating polypeptide.

In one case, the chimeric polynucleotides may also encode a fusion protein. The fusion protein may be created by operably linking a charged protein to a therapeutic protein. As used herein, "operably linked" refers to the therapeutic protein and the charged protein being connected in such a way to permit the expression of the complex when introduced into the cell. As used herein, "charged protein" refers to a protein that carries a positive, negative or overall neutral electrical charge. Preferably, the therapeutic protein may be covalently linked to the charged protein in the formation of the fusion protein. The ratio of surface charge to total or surface amino acids may be approximately 0.1, 0.2, 0.3, 0.4, 0.5, 0.6, 0.7, 0.8 or 0.9.

The cell-penetrating polypeptide encoded by the chimeric polynucleotides may form a complex after being translated. The complex may comprise a charged protein linked, e.g. covalently linked, to the cell-penetrating polypeptide. "Therapeutic protein" refers to a protein that, when administered to a cell has a therapeutic, diagnostic, and/or prophylactic effect and/or elicits a desired biological and/or pharmacological effect.

In one case, the cell-penetrating polypeptide may comprise a first domain and a second domain. The first domain may comprise a supercharged polypeptide. The second domain may comprise a protein-binding partner. As used herein, "protein-binding partner" includes, but is not limited to, antibodies and functional fragments thereof, scaffold proteins, or peptides. The cell-penetrating polypeptide may further comprise an intracellular binding partner for the protein-binding partner. The cell-penetrating polypeptide may be capable of being secreted from a cell where the chimeric polynucleotides may be introduced. The cell-penetrating polypeptide may also be capable of penetrating the first cell.

In a further case, the cell-penetrating polypeptide is capable of penetrating a second cell. The second cell may be from the same area as the first cell, or it may be from a different area. The area may include, but is not limited to, tissues and organs. The second cell may also be proximal or distal to the first cell.

In one case, the chimeric polynucleotides may encode a cell-penetrating polypeptide which may comprise a protein-binding partner. The protein binding partner may include, but is not limited to, an antibody, a supercharged antibody or a functional fragment. The chimeric polynucleotides may be introduced into the cell where a cell-penetrating polypeptide comprising the protein-binding partner is introduced.

### Secreted proteins

Human and other eukaryotic cells are subdivided by membranes into many functionally distinct compartments. Each membrane-bounded compartment, or organelle, contains different proteins essential for the function of the organelle. The cell uses "sorting signals," which are amino acid motifs located within the protein, to target proteins to particular cellular organelles.

One type of sorting signal, called a signal sequence, a signal peptide, or a leader sequence, directs a class of proteins to an organelle called the endoplasmic reticulum (ER).

Proteins targeted to the ER by a signal sequence can be released into the extracellular space as a secreted protein. Similarly, proteins residing on the cell membrane can also be secreted into the extracellular space by proteolytic cleavage of a "linker" holding the protein to the membrane. While not wishing to be bound by theory, the molecules may be used to exploit the cellular trafficking described above. As such, in some cases of the present disclosure, chimeric polynucleotides are described to express a secreted protein. The secreted proteins may be selected from those described herein or those in US Patent Publication, 20100255574.

In one case, these may be used in the manufacture of large quantities of human gene products.

### Plasma membrane proteins

In some cases of the present disclosure, chimeric polynucleotides are described to express a protein of the plasma membrane.

### Cytoplasmic or cytoskeletal proteins

In some cases of the present disclosure, chimeric polynucleotides are described to express a cytoplasmic or cytoskeletal protein.

### Intracellular membrane bound proteins

In some cases of the present disclosure, chimeric polynucleotides are described to express an intracellular membrane bound protein.

### Nuclear proteins

In some cases of the present disclosure, chimeric polynucleotides are described to express a nuclear protein.

### Proteins associated with human disease

In some cases of the present disclosure, chimeric polynucleotides are described to express a protein associated with human disease.

### Miscellaneous proteins

In some cases of the present disclosure chimeric polynucleotides are described to express a protein with a presently unknown therapeutic function.

### Targeting Moieties

In some cases of the present disclosure, chimeric polynucleotides are described to express a targeting moiety. These include a protein-binding partner or a receptor on the surface of the cell, which functions to target the cell to a specific tissue space or to interact with a specific moiety, either *in vivo* or *in vitro.* Suitable protein-binding partners include, but are not limited to, antibodies and functional fragments thereof, scaffold proteins, or peptides. Additionally, chimeric polynucleotides can be employed to direct the synthesis and extracellular localization of lipids, carbohydrates, or other biological moieties or biomolecules.

### Polypeptide Libraries

In one case, the chimeric polynucleotides may be used to produce polypeptide libraries. These libraries may arise from the production of a population of chimeric polynucleotides, each containing various structural or chemical modification designs. A population of chimeric polynucleotides may comprise a plurality of encoded polypeptides, including but not limited to, an antibody or antibody fragment, protein binding partner, scaffold protein, and other polypeptides taught herein or known in the art. In one case, the chimeric polynucleotides may be suitable for direct introduction into a target cell or culture which in turn may synthesize the encoded polypeptides.

In certain cases, multiple variants of a protein, each with different amino acid modification(s), may be produced and tested to determine the best variant in terms of pharmacokinetics, stability, biocompatibility, and/or biological activity, or a biophysical property such as expression level. Such a library may contain 10, 10², 10³, 10⁴, 10⁵, 10⁶, 10⁷, 10⁸, 10⁹, or over 10⁹ possible variants (including, but not limited to, substitutions, deletions of one or more residues, and insertion of one or more residues).

### Anti-Microbial and Anti-viral Polypeptides

The chimeric polynucleotides may be designed to encode on or more antimicrobial peptides (AMP) or antiviral peptides (AVP). AMPs and AVPs have been isolated and described from a wide range of animals such as, but not limited to, microorganisms, invertebrates, plants, amphibians, birds, fish, and mammals (Wang et al., Nucleic Acids Res. 2009; 37 (Database issue):D933-7). Anti-microbial and anti-viral polypeptides are described in International Publication No. WO201315166. As a non-limiting example, anti-microbial polypeptides are described in paragraphs [000189] -[000199] of International Publication No. WO2013151666. As another non-limiting example, anti-viral polypeptides are described in paragraphs [000189] -[000195] and [000200] of International Publication No. WO201315166.

### Chimeric Polynucleotide Regions

In some cases, chimeric polynucleotides may be designed to comprise regions, subregions or parts which function in a similar manner as known regions or parts of other nucleic acid based molecules. Such regions include those mRNA regions discussed herein as well as noncoding regions. Noncoding regions may be at the level of a single nucleoside such as the case when the region is or incorporates one or more cytotoxic nucleosides.

### Cytotoxic Nucleosides

In one case, the chimeric polynucleotides may incorporate one or more cytotoxic nucleosides. Cytotoxic nucleosides are described in co-pending International Publication No. WO2015034928, such as in paragraphs [000194]-[000198].

### . Chimeric polynucleotides having Untranslated Regions (UTRs)

The chimeric polynucleotides may comprise one or more regions or parts which act or function as an untranslated region. Where chimeric polynucleotides are designed to encode a polypeptide of interest, they may comprise one or more of these untranslated regions.

By definition, wild type untranslated regions (UTRs) of a gene are transcribed but not translated. In mRNA, the 5'UTR starts at the transcription start site and continues to the start codon but does not include the start codon; whereas, the 3'UTR starts immediately following the stop codon and continues until the transcriptional termination signal. There is growing body of evidence about the regulatory roles played by the UTRs in terms of stability of the nucleic acid molecule and translation. The regulatory features of a UTR can be incorporated into the chimeric polynucleotides of the present disclosure to, among other things, enhance the stability of the molecule. The specific features can also be incorporated to ensure controlled down-regulation of the transcript in case they are misdirected to undesired organs sites.

### 5' UTR and Translation Initiation

Natural 5'UTRs bear features which play roles in translation initiation. They harbor signatures like Kozak sequences which are commonly known to be involved in the process by which the ribosome initiates translation of many genes. Kozak sequences have the consensus CCR(A/G)CCAUGG, where R is a purine (adenine or guanine) three bases upstream of the start codon (AUG), which is followed by another 'G'. 5'UTR also have been known to form secondary structures which are involved in elongation factor binding.

By engineering the features typically found in abundantly expressed genes of specific target organs, one can enhance the stability and protein production of the chimeric polynucleotides of the disclosure. For example, introduction of 5' UTR of liver-expressed mRNA, such as albumin, serum amyloid A, Apolipoprotein A/B/E, transferrin, alpha fetoprotein, erythropoietin, or Factor VIII, could be used to enhance expression of a nucleic acid molecule, such as a chimeric polynucleotides, in hepatic cell lines or liver. Likewise, use of 5' UTR from other tissue-specific mRNA to improve expression in that tissue is possible for muscle (MyoD, Myosin, Myoglobin, Myogenin, Herculin), for endothelial cells (Tie-1, CD36), for myeloid cells (C/EBP, AML1, G-CSF, GM-CSF, CD11b, MSR, Fr-1, i-NOS), for leukocytes (CD45, CD18), for adipose tissue (CD36, GLUT4, ACRP30, adiponectin) and for lung epithelial cells (SP-A/B/C/D). Untranslated regions useful in the design and manufacture of chimeric polynucleotides include, but are not limited, to those disclosed in co-pending, co-owned US Serial Number (USSN) 61/829372 (Attorney Docket Number M42).

Other non-UTR sequences may also be used as regions or subregions within the chimeric polynucleotides. For example, introns or portions of introns sequences may be incorporated into regions of the chimeric polynucleotides of the disclosure. Incorporation of intronic sequences may increase protein production as well as polynucleotide levels.

Combinations of features may be included in flanking regions and may be contained within other features. For example, the ORF may be flanked by a 5' UTR which may contain a strong Kozak translational initiation signal and/or a 3' UTR which may include an oligo(dT) sequence for templated addition of a poly-A tail. 5'UTR may comprise a first polynucleotide fragment and a second polynucleotide fragment from the same and/or different genes such as the 5'UTRs described in US Patent Application Publication No. 20100293625.

Co-pending, co-owned International Publication No. WO201416453 (Attorney Docket Number M42) provides a listing of exemplary UTRs which may be utilized in the chimeric polynucleotide of the present disclosure as flanking regions. Variants of 5' or 3' UTRs may be utilized wherein one or more nucleotides are added or removed to the termini, including A, T, C or G.

It should be understood that any UTR from any gene may be incorporated into the regions of the chimeric polynucleotide. Furthermore, multiple wild-type UTRs of any known gene may be utilized. Also described herein are artificial UTRs which are not variants of wild type regions. These UTRs or portions thereof may be placed in the same orientation as in the transcript from which they were selected or may be altered in orientation or location. Hence a 5' or 3' UTR may be inverted, shortened, lengthened, made chimeric with one or more other 5' UTRs or 3' UTRs. As used herein, the term "altered" as it relates to a UTR sequence, means that the UTR has been changed in some way in relation to a reference sequence. For example, a 3' or 5' UTR may be altered relative to a wild type or native UTR by the change in orientation or location as taught above or may be altered by the inclusion of additional nucleotides, deletion of nucleotides, swapping or transposition of nucleotides. Any of these changes producing an "altered" UTR (whether 3' or 5') comprise a variant UTR.

In one case, a double, triple or quadruple UTR such as a 5' or 3' UTR may be used. As used herein, a "double" UTR is one in which two copies of the same UTR are encoded either in series or substantially in series. For example, a double beta-globin 3' UTR may be used as described in US Patent publication 20100129877.

It is also disclosed herein to have patterned UTRs. As used herein "patterned UTRs" are those UTRs which reflect a repeating or alternating pattern, such as ABABAB or AABBAABBAABB or ABCABCABC or variants thereof repeated once, twice, or more than 3 times. In these patterns, each letter, A, B, or C represent a different UTR at the nucleotide level.

In one case, flanking regions are selected from a family of transcripts whose proteins share a common function, structure, feature of property. For example, polypeptides of interest may belong to a family of proteins which are expressed in a particular cell, tissue or at some time during development. The UTRs from any of these genes may be swapped for any other UTR of the same or different family of proteins to create a new chimeric polynucleotide. As used herein, a "family of proteins" is used in the broadest sense to refer to a group of two or more polypeptides of interest which share at least one function, structure, feature, localization, origin, or expression pattern.

The untranslated region may also include translation enhancer elements (TEE). As a non-limiting example, the TEE may include those described in US Application No. 20090226470.

### 3' UTR and the AU Rich Elements

Natural or wild type 3' UTRs are known to have stretches of Adenosines and Uridines embedded in them. These AU rich signatures are particularly prevalent in genes with high rates of turnover. Based on their sequence features and functional properties, the AU rich elements (AREs) can be separated into three classes (Chen et al, 1995): Class I AREs contain several dispersed copies of an AUUUA motif within U-rich regions. C-Myc and MyoD contain class I AREs. Class II AREs possess two or more overlapping UUAUUUA(U/A)(U/A) nonamers. Molecules containing this type of AREs include GM-CSF and TNF-a. Class III ARES are less well defined. These U rich regions do not contain an AUUUA motif. c-Jun and Myogenin are two well-studied examples of this class. Most proteins binding to the AREs are known to destabilize the messenger, whereas members of the ELAV family, most notably HuR, have been documented to increase the stability of mRNA. HuR binds to AREs of all the three classes. Engineering the HuR specific binding sites into the 3' UTR of nucleic acid molecules will lead to HuR binding and thus, stabilization of the message

### in vivo.

Introduction, removal or modification of 3' UTR AU rich elements (AREs) can be used to modulate the stability of chimeric polynucleotides of the disclosure. When engineering specific chimeric polynucleotides, one or more copies of an ARE can be introduced to make chimeric polynucleotides of the disclosure less stable and thereby curtail translation and decrease production of the resultant protein. Likewise, AREs can be identified and removed or mutated to increase the intracellular stability and thus increase translation and production of the resultant protein. Transfection experiments can be conducted in relevant cell lines, using chimeric polynucleotides of the disclosure and protein production can be assayed at various time points post-transfection. For example, cells can be transfected with different ARE-engineering molecules and by using an ELISA kit to the relevant protein and assaying protein produced at 6 hour, 12 hour, 24 hour, 48 hour, and 7 days post-transfection.

### microRNA Binding Sites

microRNAs (or miRNA) are 19-25 nucleotide long noncoding RNAs that bind to the 3'UTR of nucleic acid molecules and down-regulate gene expression either by reducing nucleic acid molecule stability or by inhibiting translation. The chimeric polynucleotides may comprise one or more microRNA target sequences, microRNA sequences, or microRNA seeds. Such sequences may correspond to any known microRNA such as those taught in US Publication US2005/0261218 and US Publication US2005/0059005.

A microRNA sequence comprises a "seed" region, i.e., a sequence in the region of positions 2-8 of the mature microRNA, which sequence has perfect Watson-Crick complementarity to the miRNA target sequence. A microRNA seed may comprise positions 2-8 or 2-7 of the mature microRNA. In some cases, a microRNA seed may comprise 7 nucleotides (e.g., nucleotides 2-8 of the mature microRNA), wherein the seed-complementary site in the corresponding miRNA target is flanked by an adenine (A) opposed to microRNA position 1. In some cases, a microRNA seed may comprise 6 nucleotides (e.g., nucleotides 2-7 of the mature microRNA), wherein the seed-complementary site in the corresponding miRNA target is flanked by an adenine (A) opposed to microRNA position 1. See for example, Grimson A, Farh KK, Johnston WK, Garrett-Engele P, Lim LP, Bartel DP; Mol Cell. 2007 Jul 6;27(1):91-105.

The bases of the microRNA seed have complete complementarity with the target sequence. By engineering microRNA target sequences into the chimeric polynucleotides (e.g., in a 3'UTR like region or other region) of the disclosure one can target the molecule for degradation or reduced translation, given the microRNA in question is available. This process will reduce the hazard of off target effects upon nucleic acid molecule delivery. Identification of microRNA, microRNA target regions, and their expression patterns and role in biology have been reported (Bonauer et al., Curr Drug Targets 2010 11:943-949; Anand and Cheresh Curr Opin Hematol 2011 18:171-176; Contreras and Rao Leukemia 2012 26:404-413 (2011 Dec 20. doi: 10.1038/leu.2011.356); Bartel Cell 2009 136:215-233; Landgraf et al, Cell, 2007 129:1401-1414).

For example, if the nucleic acid molecule is an mRNA and is not intended to be delivered to the liver but ends up there, then miR-122, a microRNA abundant in liver, can inhibit the expression of the gene of interest if one or multiple target sites of miR-122 are engineered into the 3' UTR region of the chimeric polynucleotides. Introduction of one or multiple binding sites for different microRNA can be engineered to further decrease the longevity, stability, and protein translation of a chimeric polynucleotides.

As used herein, the term "microRNA site" refers to a microRNA target site or a microRNA recognition site, or any nucleotide sequence to which a microRNA binds or associates. It should be understood that "binding" may follow traditional Watson-Crick hybridization rules or may reflect any stable association of the microRNA with the target sequence at or adjacent to the microRNA site.

Conversely, for the purposes of the chimeric polynucleotides of the present disclosure, microRNA binding sites can be engineered out of (i.e. removed from) sequences in which they occur, e.g., in order to increase protein expression in specific tissues. For example, miR-122 binding sites may be removed to improve protein expression in the liver. Regulation of expression in multiple tissues can be accomplished through introduction or removal or one or several microRNA binding sites.

Examples of tissues where microRNA are known to regulate mRNA, and thereby protein expression, include, but are not limited to, liver (miR-122), muscle (miR-133, miR-206, miR-208), endothelial cells (miR-17-92, miR-126), myeloid cells (miR-142-3p, miR-142-5p, miR-16, miR-21, miR-223, miR-24, miR-27), adipose tissue (let-7, miR-30c), heart (miR-1d, miR-149), kidney (miR-192, miR-194, miR-204), and lung epithelial cells (let-7, miR-133, miR-126). MicroRNA can also regulate complex biological processes such as angiogenesis (miR-132) (Anand and Cheresh Curr Opin Hematol 2011 18:171-176;

Expression profiles, microRNA and cell lines useful in the present disclosure include those taught in for example,U.S. Provisional Application Nos 61/857,436 (Attorney Docket Number M39) and 61/857,304 (Attorney Docket Number M37) each filed July 23, 2013

In the chimeric polynucleotides of the present disclosure, binding sites for microRNAs that are involved in such processes may be removed or introduced, in order to tailor the expression of the chimeric polynucleotides expression to biologically relevant cell types or to the context of relevant biological processes. A listing of microRNA, miR sequences and miR binding sites is listed in Table 9 of U.S. Provisional Application No. 61/753,661 filed January 17, 2013, in Table 9 of U.S. Provisional Application No. 61/754,159 filed January 18, 2013, and in Table 7 of U.S.

Provisional Application No. 61/758,921 filed January 31, 2013.

Examples of use of microRNA to drive tissue or disease-specific gene expression are listed (Getner and Naldini, Tissue Antigens. 2012, 80:393-403; In addition, microRNA seed sites can be incorporated into mRNA to decrease expression in certain cells which results in a biological improvement. An example of this is incorporation of miR-142 sites into a UGT1A1-expressing lentiviral vector. The presence of miR-142 seed sites reduced expression in hematopoietic cells, and as a consequence reduced expression in antigen-presentating cells, leading to the absence of an immune response against the virally expressed UGT1A1 (Schmitt et al., Gastroenterology 2010; 139:999-1007; Gonzalez-Asequinolaza et al. Gastroenterology 2010, 139:726-729; Incorporation of miR-142 sites into modified mRNA could not only reduce expression of the encoded protein in hematopoietic cells, but could also reduce or abolish immune responses to the mRNA-encoded protein. Incorporation of miR-142 seed sites (one or multiple) into mRNA would be important in the case of treatment of patients with complete protein deficiencies (UGT1A1 type I, LDLR-deficient patients, CRIM-negative Pompe patients, etc.) .

Lastly, through an understanding of the expression patterns of microRNA in different cell types, chimeric polynucleotides can be engineered for more targeted expression in specific cell types or only under specific biological conditions. Through introduction of tissue-specific microRNA binding sites, chimeric polynucleotides could be designed that would be optimal for protein expression in a tissue or in the context of a biological condition.

Transfection experiments can be conducted in relevant cell lines, using engineered chimeric polynucleotides and protein production can be assayed at various time points post-transfection. For example, cells can be transfected with different microRNA binding site-engineering chimeric polynucleotides and by using an ELISA kit to the relevant protein and assaying protein produced at 6 hour, 12 hour, 24 hour, 48 hour, 72 hour and 7 days post-transfection. *In vivo* experiments can also be conducted using microRNA-binding site-engineered molecules to examine changes in tissue-specific expression of formulated chimeric polynucleotides.

### Regions having a 5' Cap

The 5' cap structure of a natural mRNA is involved in nuclear export, increasing mRNA stability and binds the mRNA Cap Binding Protein (CBP), which is responsible for mRNA stability in the cell and translation competency through the association of CBP with poly(A) binding protein to form the mature cyclic mRNA species. The cap further assists the removal of 5' proximal introns removal during mRNA splicing.

Endogenous mRNA molecules may be 5'-end capped generating a 5'-ppp-5'-triphosphate linkage between a terminal guanosine cap residue and the 5'-terminal transcribed sense nucleotide of the mRNA molecule. This 5'-guanylate cap may then be methylated to generate an N7-methyl-guanylate residue. The ribose sugars of the terminal and/or anteterminal transcribed nucleotides of the 5' end of the mRNA may optionally also be 2'-O-methylated. 5'-decapping through hydrolysis and cleavage of the guanylate cap structure may target a nucleic acid molecule, such as an mRNA molecule, for degradation.

In some cases, chimeric polynucleotides may be designed to incorporate a cap moiety. Modifications to the chimeric polynucleotides may generate a non-hydrolyzable cap structure preventing decapping and thus increasing mRNA half-life. Because cap structure hydrolysis requires cleavage of 5'-ppp-5' phosphorodiester linkages, modified nucleotides may be used during the capping reaction. For example, a Vaccinia Capping Enzyme from New England Biolabs (Ipswich, MA) may be used with α-thio-guanosine nucleotides according to the manufacturer's instructions to create a phosphorothioate linkage in the 5'-ppp-5' cap. Additional modified guanosine nucleotides may be used such as α-methylphosphonate and seleno-phosphate nucleotides.

Additional modifications include, but are not limited to, 2'-O-methylation of the ribose sugars of 5'-terminal and/or 5'-anteterminal nucleotides of the chimeric polynucleotide (as mentioned above) on the 2'-hydroxyl group of the sugar ring. Multiple distinct 5'-cap structures can be used to generate the 5'-cap of a nucleic acid molecule, such as a chimeric polynucleotide which functions as an mRNA molecule.

Cap analogs, which herein are also referred to as synthetic cap analogs, chemical caps, chemical cap analogs, or structural or functional cap analogs, differ from natural (i.e. endogenous, wild-type or physiological) 5'-caps in their chemical structure, while retaining cap function. Cap analogs may be chemically (i.e. non-enzymatically) or enzymatically synthesized and/or linked to the chimeric polynucleotides of the disclosure.

For example, the Anti-Reverse Cap Analog (ARCA) cap contains two guanines linked by a 5'-5'-triphosphate group, wherein one guanine contains an N7 methyl group as well as a 3'-O-methyl group (i.e., N7,3'-O-dimethyl-guanosine-5'-triphosphate-5'-guanosine (m⁷G-3'mppp-G; which may equivalently be designated 3' O-Me-m7G(5')ppp(5')G). The 3'-O atom of the other, unmodified, guanine becomes linked to the 5'-terminal nucleotide of the capped chimeric polynucleotide. The N7- and 3'-O-methlyated guanine provides the terminal moiety of the capped chimeric polynucleotide.

Another exemplary cap is mCAP, which is similar to ARCA but has a 2'-O-methyl group on guanosine (i.e., N7,2'-O-dimethyl-guanosine-5'-triphosphate-5'-guanosine, m⁷Gm-ppp-G).

While cap analogs allow for the concomitant capping of a chimeric polynucleotide or a region thereof, in an in vitro transcription reaction, up to 20% of transcripts can remain uncapped. This, as well as the structural differences of a cap analog from an endogenous 5'-cap structures of nucleic acids produced by the endogenous, cellular transcription machinery, may lead to reduced translational competency and reduced cellular stability.

Chimeric polynucleotides may also be capped post-manufacture (whether IVT or chemical synthesis), using enzymes, in order to generate more authentic 5'-cap structures. As used herein, the phrase "more authentic" refers to a feature that closely mirrors or mimics, either structurally or functionally, an endogenous or wild type feature. That is, a "more authentic" feature is better representative of an endogenous, wild-type, natural or physiological cellular function and/or structure as compared to synthetic features or analogs, etc., of the prior art, or which outperforms the corresponding endogenous, wild-type, natural or physiological feature in one or more respects. Non-limiting examples of more authentic 5'cap structures of the present disclosure are those which, among other things, have enhanced binding of cap binding proteins, increased half-life, reduced susceptibility to 5' endonucleases and/or reduced 5'decapping, as compared to synthetic 5'cap structures known in the art (or to a wild-type, natural or physiological 5'cap structure). For example, recombinant Vaccinia Virus Capping Enzyme and recombinant 2'-O-methyltransferase enzyme can create a canonical 5'-5'-triphosphate linkage between the 5'-terminal nucleotide of a chimeric polynucleotide and a guanine cap nucleotide wherein the cap guanine contains an N7 methylation and the 5'-terminal nucleotide of the mRNA contains a 2'-O-methyl. Such a structure is termed the Cap1 structure. This cap results in a higher translational-competency and cellular stability and a reduced activation of cellular pro-inflammatory cytokines, as compared, e.g., to other 5'cap analog structures known in the art. Cap structures include, but are not limited to, 7mG(5')ppp(5')N,pN2p (cap 0), 7mG(5')ppp(5')NlmpNp (cap 1), and 7mG(5')-ppp(5')NlmpN2mp (cap 2).

Because the chimeric polynucleotides may be capped post-manufacture, and because this process is more efficient, nearly 100% of the chimeric polynucleotides may be capped. This is in contrast to ~80% when a cap analog is linked to a chimeric polynucleotide in the course of an in vitro transcription reaction.

5' terminal caps may include endogenous caps or cap analogs. A 5' terminal cap may comprise a guanine analog. Useful guanine analogs include, but are not limited to, inosine, N1-methyl-guanosine, 2'fluoro-guanosine, 7-deaza-guanosine, 8-oxo-guanosine, 2-amino-guanosine, LNA-guanosine, and 2-azido-guanosine.

### Viral Sequences

Additional viral sequences such as, but not limited to, the translation enhancer sequence of the barley yellow dwarf virus (BYDV-PAV), the Jaagsiekte sheep retrovirus (JSRV) and/or the Enzootic nasal tumor virus (See e.g., International Pub. No. WO2012129648; can be engineered and inserted in the chimeric polynucleotides of the disclosure and can stimulate the translation of the construct *in vitro* and *in vivo.* Transfection experiments can be conducted in relevant cell lines at and protein production can be assayed by ELISA at 12hr, 24hr, 48hr, 72 hr and day 7 post-transfection.

### IRES Sequences

Further, described herein are chimeric polynucleotides which may contain an internal ribosome entry site (IRES). First identified as a feature Picorna virus RNA, IRES plays an important role in initiating protein synthesis in absence of the 5' cap structure. An IRES may act as the sole ribosome binding site, or may serve as one of multiple ribosome binding sites of an mRNA. Chimeric polynucleotides containing more than one functional ribosome binding site may encode several peptides or polypeptides that are translated independently by the ribosomes ("multicistronic nucleic acid molecules"). When chimeric polynucleotides are described with an IRES, further optionally described is a second translatable region. Examples of IRES sequences that can be used according to the disclosure include without limitation, those from picornaviruses (e.g. FMDV), pest viruses (CFFV), polio viruses (PV), encephalomyocarditis viruses (ECMV), foot-and-mouth disease viruses (FMDV), hepatitis C viruses (HCV), classical swine fever viruses (CSFV), murine leukemia virus (MLV), simian immune deficiency viruses (SIV) or cricket paralysis viruses (CrPV).

### Poly-A tails

During RNA processing, a long chain of adenine nucleotides (poly-A tail) may be added to a polynucleotide such as an mRNA molecule in order to increase stability. Immediately after transcription, the 3' end of the transcript may be cleaved to free a 3' hydroxyl. Then poly-A polymerase adds a chain of adenine nucleotides to the RNA. The process, called polyadenylation, adds a poly-A tail that can be between, for example, approximately 100 and 250 residues long.

PolyA tails may also be added after the construct is exported from the nucleus.

Terminal groups on the poly A tail may be incorporated for stabilization. Chimeric polynucleotides may include des-3' hydroxyl tails. They may also include structural moieties or 2'-Omethyl modifications as taught by Junjie Li, et al. (Current Biology, Vol. 15, 1501-1507, August 23, 2005).

The chimeric polynucleotides may be designed to encode transcripts with alternative polyA tail structures including histone mRNA. According to Norbury, "Terminal uridylation has also been detected on human replication-dependent histone mRNAs. The turnover of these mRNAs is thought to be important for the prevention of potentially toxic histone accumulation following the completion or inhibition of chromosomal DNA replication. These mRNAs are distinguished by their lack of a 3' poly(A) tail, the function of which is instead assumed by a stable stem-loop structure and its cognate stem-loop binding protein (SLBP); the latter carries out the same functions as those of PABP on polyadenylated mRNAs" (Norbury, "Cytoplasmic RNA: a case of the tail wagging the dog," Nature Reviews Molecular Cell Biology; AOP, published online 29 August 2013; doi:10.1038/nrm3645)

Unique poly-A tail lengths provide certain advantages to the chimeric polynucleotides of the present disclosure.

Generally, the length of a poly-A tail, when present, is greater than 30 nucleotides in length. In another case, the poly-A tail is greater than 35 nucleotides in length (e.g., at least or greater than about 35, 40, 45, 50, 55, 60, 70, 80, 90, 100, 120, 140, 160, 180, 200, 250, 300, 350, 400, 450, 500, 600, 700, 800, 900, 1,000, 1,100, 1,200, 1,300, 1,400, 1,500, 1,600, 1,700, 1,800, 1,900, 2,000, 2,500, and 3,000 nucleotides). In some cases, the chimeric polynucleotide or region thereof includes from about 30 to about 3,000 nucleotides (e.g., from 30 to 50, from 30 to 100, from 30 to 250, from 30 to 500, from 30 to 750, from 30 to 1,000, from 30 to 1,500, from 30 to 2,000, from 30 to 2,500, from 50 to 100, from 50 to 250, from 50 to 500, from 50 to 750, from 50 to 1,000, from 50 to 1,500, from 50 to 2,000, from 50 to 2,500, from 50 to 3,000, from 100 to 500, from 100 to 750, from 100 to 1,000, from 100 to 1,500, from 100 to 2,000, from 100 to 2,500, from 100 to 3,000, from 500 to 750, from 500 to 1,000, from 500 to 1,500, from 500 to 2,000, from 500 to 2,500, from 500 to 3,000, from 1,000 to 1,500, from 1,000 to 2,000, from 1,000 to 2,500, from 1,000 to 3,000, from 1,500 to 2,000, from 1,500 to 2,500, from 1,500 to 3,000, from 2,000 to 3,000, from 2,000 to 2,500, and from 2,500 to 3,000).

In one case, the poly-A tail is designed relative to the length of the overall chimeric polynucleotides or the length of a particular region of the chimeric polynucleotide. This design may be based on the length of a coding region, the length of a particular feature or region or based on the length of the ultimate product expressed from the chimeric polynucleotides.

In this context the poly-A tail may be 10, 20, 30, 40, 50, 60, 70, 80, 90, or 100% greater in length than the chimeric polynucleotides or feature thereof. The poly-A tail may also be designed as a fraction of chimeric polynucleotides to which it belongs. In this context, the poly-A tail may be 10, 20, 30, 40, 50, 60, 70, 80, or 90% or more of the total length of the construct, a construct region or the total length of the construct minus the poly-A tail. Further, engineered binding sites and conjugation of chimeric polynucleotides for Poly-A binding protein may enhance expression.

Additionally, multiple distinct chimeric polynucleotides may be linked together via the PABP (Poly-A binding protein) through the 3'-end using modified nucleotides at the 3'-terminus of the poly-A tail. Transfection experiments can be conducted in relevant cell lines at and protein production can be assayed by ELISA at 12hr, 24hr, 48hr, 72 hr and day 7 post-transfection.

In one case, the chimeric polynucleotides of the present disclosure are designed to include a polyA-G Quartet region. The G-quartet is a cyclic hydrogen bonded array of four guanine nucleotides that can be formed by G-rich sequences in both DNA and RNA. In this case, the G-quartet is incorporated at the end of the poly-A tail. The resultant polynucleotide is assayed for stability, protein production and other parameters including half-life at various time points. It has been discovered that the polyA-G quartet results in protein production from an mRNA equivalent to at least 75% of that seen using a poly-A tail of 120 nucleotides alone (SEQ ID NO: 21).

### Start codon region

In some cases, chimeric polynucleotides may have regions that are analogous to or function like a start codon region.

In one case, translation of a chimeric polynucleotide may initiate on a codon which is not the start codon AUG. Translation of the chimeric polynucleotide may initiate on an alternative start codon such as, but not limited to, ACG, AGG, AAG, CTG/CUG, GTG/GUG, ATA/AUA, ATT/AUU, TTG/UUG (see Touriol et al. Biology of the Cell 95 (2003) 169-178 and Matsuda and Mauro PLoS ONE, 2010 5:11; As a non-limiting example, the translation of a chimeric polynucleotide begins on the alternative start codon ACG. As another non-limiting example, chimeric polynucleotide translation begins on the alternative start codon CTG/CUG. As yet another non-limiting example, the translation of a chimeric polynucleotide begins on the alternative start codon GTG/GUG.

Nucleotides flanking a codon that initiates translation such as, but not limited to, a start codon or an alternative start codon, are known to effect the translation efficiency, the length and/or the structure of the polynucleotide. (See e.g., Matsuda and Mauro PLoS ONE, 2010 5:11; Masking any of the nucleotides flanking a codon that initiates translation may be used to alter the position of translation initiation, translation efficiency, length and/or structure of a polynucleotide.

In one case, a masking agent may be used near the start codon or alternative start codon in order to mask or hide the codon to reduce the probability of translation initiation at the masked start codon or alternative start codon. Non-limiting examples of masking agents include antisense locked nucleic acids (LNA) polynucleotides and exon-junction complexes (EJCs) (See e.g., Matsuda and Mauro describing masking agents LNA polynucleotides and EJCs (PLoS ONE, 2010 5:11).

In another case, a masking agent may be used to mask a start codon of a chimeric polynucleotide in order to increase the likelihood that translation will initiate on an alternative start codon.

In one case, a masking agent may be used to mask a first start codon or alternative start codon in order to increase the chance that translation will initiate on a start codon or alternative start codon downstream to the masked start codon or alternative start codon.

In one case, a start codon or alternative start codon may be located within a perfect complement for a miR binding site. The perfect complement of a miR binding site may help control the translation, length and/or structure of the chimeric polynucleotide similar to a masking agent. As a non-limiting example, the start codon or alternative start codon may be located in the middle of a perfect complement for a miR-122 binding site. The start codon or alternative start codon may be located after the first nucleotide, second nucleotide, third nucleotide, fourth nucleotide, fifth nucleotide, sixth nucleotide, seventh nucleotide, eighth nucleotide, ninth nucleotide, tenth nucleotide, eleventh nucleotide, twelfth nucleotide, thirteenth nucleotide, fourteenth nucleotide, fifteenth nucleotide, sixteenth nucleotide, seventeenth nucleotide, eighteenth nucleotide, nineteenth nucleotide, twentieth nucleotide or twenty-first nucleotide.

In another case, the start codon of a chimeric polynucleotide may be removed from the chimeric polynucleotide sequence in order to have the translation of the chimeric polynucleotide begin on a codon which is not the start codon. Translation of the chimeric polynucleotide may begin on the codon following the removed start codon or on a downstream start codon or an alternative start codon. In a non-limiting example, the start codon ATG/AUG is removed as the first 3 nucleotides of the chimeric polynucleotide sequence in order to have translation initiate on a downstream start codon or alternative start codon. The chimeric polynucleotide sequence where the start codon was removed may further comprise at least one masking agent for the downstream start codon and/or alternative start codons in order to control or attempt to control the initiation of translation, the length of the chimeric polynucleotide and/or the structure of the chimeric polynucleotide.

### Stop Codon Region

In one case, the chimeric polynucleotides may include at least two stop codons before the 3' untranslated region (UTR). The stop codon may be selected from TGA, TAA and TAG. In one case, the chimeric polynucleotides include the stop codon TGA and one additional stop codon. In a further case the addition stop codon may be TAA. In another case, the chimeric polynucleotides may include three stop codons.

### Signal Sequences

The chimeric polynucleotides may also encode additional features which facilitate trafficking of the polypeptides to therapeutically relevant sites. One such feature which aids in protein trafficking is the signal sequence. As used herein, a "signal sequence" or "signal peptide" is a polynucleotide or polypeptide, respectively, which is from about 9 to 200 nucleotides (3-60 amino acids) in length which is incorporated at the 5' (or N-terminus) of the coding region or polypeptide encoded, respectively. Addition of these sequences result in trafficking of the encoded polypeptide to the endoplasmic reticulum through one or more secretory pathways. Some signal peptides are cleaved from the protein by signal peptidase after the proteins are transported.

Additional signal sequences which may be utilized in the present disclosure include those taught in, for example, databases such as those found at http://www.signalpeptide.de/ or http://proline.bic.nus.edu.sg/spdb/. Those described in US Patents 8,124,379; 7,413,875 and 7,385,034 are also within the scope of the disclosure.

### Protein Cleavage Signals and Sites

In one case, the polypeptides may include at least one protein cleavage signal containing at least one protein cleavage site. The protein cleavage site may be located at the N-terminus, the C-terminus, at any space between the N- and the C- termini such as, but not limited to, half-way between the N- and C-termini, between the N-terminus and the half way point, between the half way point and the C-terminus, and combinations thereof.

The polypeptides may include, but is not limited to, a proprotein convertase (or prohormone convertase), thrombin or Factor Xa protein cleavage signal. Proprotein convertases are a family of nine proteinases, comprising seven basic amino acid-specific subtilisin-like serine proteinases related to yeast kexin, known as prohormone convertase 1/3 (PC1/3), PC2, furin, PC4, PC5/6, paired basic amino-acid cleaving enzyme 4 (PACE4) and PC7, and two other subtilases that cleave at non-basic residues, called subtilisin kexin isozyme 1 (SKI-1) and proprotein convertase subtilisin kexin 9 (PCSK9).

In one case, the chimeric polynucleotides may be engineered such that the chimeric polynucleotide contains at least one encoded protein cleavage signal. The encoded protein cleavage signal may be located in any region including but not limited to before the start codon, after the start codon, before the coding region, within the coding region such as, but not limited to, half way in the coding region, between the start codon and the half way point, between the half way point and the stop codon, after the coding region, before the stop codon, between two stop codons, after the stop codon and combinations thereof.

In one case, the chimeric polynucleotides may include at least one encoded protein cleavage signal containing at least one protein cleavage site. The encoded protein cleavage signal may include, but is not limited to, a proprotein convertase (or prohormone convertase), thrombin and/or Factor Xa protein cleavage signal.

As a non-limiting example, U.S. Pat. No. 7,374,930 and U.S. Pub. No. 20090227660 use a furin cleavage site to cleave the N-terminal methionine of GLP-1 in the expression product from the Golgi apparatus of the cells. In one case, the polypeptides of the present disclosure include at least one protein cleavage signal and/or site with the proviso that the polypeptide is not GLP-1.

### Insertions and Substitutions

In one case, the 5'UTR of the chimeric polynucleotide may be replaced by the insertion of at least one region and/or string of nucleosides of the same base. The region and/or string of nucleotides may include, but is not limited to, at least 3, at least 4, at least 5, at least 6, at least 7 or at least 8 nucleotides and the nucleotides may be natural and/or unnatural. As a non-limiting example, the group of nucleotides may include 5-8 adenine, cytosine, thymine, a string of any of the other nucleotides disclosed herein and/or combinations thereof.

In one case, the 5'UTR of the chimeric polynucleotide may be replaced by the insertion of at least two regions and/or strings of nucleotides of two different bases such as, but not limited to, adenine, cytosine, thymine, and any of the other nucleotides disclosed herein and/or combinations thereof. For example, the 5'UTR may be replaced by inserting 5-8 adenine bases followed by the insertion of 5-8 cytosine bases. In another example, the 5'UTR may be replaced by inserting 5-8 cytosine bases followed by the insertion of 5-8 adenine bases.

In one case, the chimeric polynucleotide may include at least one substitution and/or insertion downstream of the transcription start site which may be recognized by an RNA polymerase. As a non-limiting example, at least one substitution and/or insertion may occur downstream the transcription start site by substituting at least one nucleic acid in the region just downstream of the transcription start site (such as, but not limited to, +1 to +6). Changes to region of nucleotides just downstream of the transcription start site may affect initiation rates, increase apparent nucleotide triphosphate (NTP) reaction constant values, and increase the dissociation of short transcripts from the transcription complex curing initial transcription (Brieba et al, Biochemistry (2002) 41: 5144-5149). The modification, substitution and/or insertion of at least one nucleoside may cause a silent mutation of the sequence or may cause a mutation in the amino acid sequence.

In one case, the chimeric polynucleotide may include the substitution of at least 1, at least 2, at least 3, at least 4, at least 5, at least 6, at least 7, at least 8, at least 9, at least 10, at least 11, at least 12 or at least 13 guanine bases downstream of the transcription start site.

In one case, the chimeric polynucleotide may include the substitution of at least 1, at least 2, at least 3, at least 4, at least 5 or at least 6 guanine bases in the region just downstream of the transcription start site. As a non-limiting example, if the nucleotides in the region are GGGAGA the guanine bases may be substituted by at least 1, at least 2, at least 3 or at least 4 adenine nucleotides. In another non-limiting example, if the nucleotides in the region are GGGAGA the guanine bases may be substituted by at least 1, at least 2, at least 3 or at least 4 cytosine bases. In another non-limiting example, if the nucleotides in the region are GGGAGA the guanine bases may be substituted by at least 1, at least 2, at least 3 or at least 4 thymine, and/or any of the nucleotides described herein.

In one case, the chimeric polynucleotide may include at least one substitution and/or insertion upstream of the start codon. For the purpose of clarity, one of skill in the art would appreciate that the start codon is the first codon of the protein coding region whereas the transcription start site is the site where transcription begins. The chimeric polynucleotide may include, but is not limited to, at least 1, at least 2, at least 3, at least 4, at least 5, at least 6, at least 7 or at least 8 substitutions and/or insertions of nucleotide bases. The nucleotide bases may be inserted or substituted at 1, at least 1, at least 2, at least 3, at least 4 or at least 5 locations upstream of the start codon. The nucleotides inserted and/or substituted may be the same base (e.g., all A or all C or all T or all G), two different bases (e.g., A and C, A and T, or C and T), three different bases (e.g., A, C and T or A, C and T) or at least four different bases. As a non-limiting example, the guanine base upstream of the coding region in the chimeric polynucleotide may be substituted with adenine, cytosine, thymine, or any of the nucleotides described herein. In another non-limiting example the substitution of guanine bases in the chimeric polynucleotide may be designed so as to leave one guanine base in the region downstream of the transcription start site and before the start codon (see Esvelt et al. Nature (2011) 472(7344):499-503; As a non-limiting example, at least 5 nucleotides may be inserted at 1 location downstream of the transcription start site but upstream of the start codon and the at least 5 nucleotides may be the same base type.

### Incorporating Post Transcriptional Control Modulators

In one case, the chimeric polynucleotides may include at least one post transcriptional control modulator. These post transcriptional control modulators may be, but are not limited to, small molecules, compounds and regulatory sequences. As a non-limiting example, post transcriptional control may be achieved using small molecules identified by PTC Therapeutics Inc. (South Plainfield, NJ) using their GEMS^{™} (Gene Expression Modulation by Small-Molecules) screening technology.

In one case, the chimeric polynucleotides may include at least one post transcriptional control modulator as described in International Patent Publication No. WO2013151666. Non-limiting examples of post transcriptional control modulators are described in paragraphs [000299] - [000304] of International Patent Publication No. WO2013151666.

### II. Design, Synthesis and Quantitation of chimeric polynucleotides

### Design-Codon Optimization

The chimeric polynucleotides, their regions or parts or subregions may be codon optimized. Codon optimization methods are known in the art and may be useful in efforts to achieve one or more of several goals. These goals include to match codon frequencies in target and host organisms to ensure proper folding, bias GC content to increase mRNA stability or reduce secondary structures, minimize tandem repeat codons or base runs that may impair gene construction or expression, customize transcriptional and translational control regions, insert or remove protein trafficking sequences, remove/add post translation modification sites in encoded protein (e.g. glycosylation sites), add, remove or shuffle protein domains, insert or delete restriction sites, modify ribosome binding sites and mRNA degradation sites, to adjust translational rates to allow the various domains of the protein to fold properly, or to reduce or eliminate problem secondary structures within the polynucleotide. Codon optimization tools, algorithms and services are known in the art, non-limiting examples include services from GeneArt (Life Technologies), DNA2.0 (Menlo Park CA) and/or proprietary methods. In one case, the ORF sequence is optimized using optimization algorithms. Codon options for each amino acid are given in Table 1.

**Table 1. Codon Options**

| **Amino Acid** | **Single Letter Code** | **Codon Options** |
|---|---|---|
| Isoleucine | I | ATT, ATC, ATA |
| Leucine | L | CTT, CTC, CTA, CTG, TTA, TTG |
| Valine | V | GTT, GTC, GTA, GTG |
| Phenylalanine | F | TTT, TTC |
| Methionine | M | ATG |
| Cysteine | C | TGT, TGC |
| Alanine | A | GCT, GCC, GCA, GCG |
| Glycine | G | GGT, GGC, GGA, GGG |
| Proline | P | CCT, CCC, CCA, CCG |
| Threonine | T | ACT, ACC, ACA, ACG |
| Serine | S | TCT, TCC, TCA, TCG, AGT, AGC |
| Tyrosine | Y | TAT, TAC |
| Tryptophan | W | TGG |
| Glutamine | Q | CAA, CAG |
| Asparagine | N | AAT, AAC |
| Histidine | H | CAT, CAC |
| Glutamic acid | E | GAA, GAG |
| Aspartic acid | D | GAT, GAC |
| Lysine | K | AAA, AAG |
| Arginine | R | CGT, CGC, CGA, CGG, AGA, AGG |
| Selenocysteine | Sec | UGA in mRNA in presence of Selenocysteine insertion element (SECIS) |
| Stop codons | Stop | TAA, TAG, TGA |

Features, which may be considered beneficial in some cases, may be encoded by regions of the chimeric polynucleotide and such regions may be upstream (5') or downstream (3') to a region which encodes a polypeptide. These regions may be incorporated into the chimeric polynucleotide before and/or after codon optimization of the protein encoding region or open reading frame (ORF). It is not required that a chimeric polynucleotide contain both a 5' and 3' flanking region. Examples of such features include, but are not limited to, untranslated regions (UTRs), Kozak sequences, an oligo(dT) sequence, and detectable tags and may include multiple cloning sites which may have XbaI recognition.

In some cases, a 5' UTR and/or a 3' UTR region may be described as flanking regions. Multiple 5' or 3' UTRs may be included in the flanking regions and may be the same or of different sequences. Any portion of the flanking regions, including none, may be codon optimized and any may independently contain one or more different structural or chemical modifications, before and/or after codon optimization.

After optimization (if desired), the chimeric polynucleotides components are reconstituted and transformed into a vector such as, but not limited to, plasmids, viruses, cosmids, and artificial chromosomes. For example, the optimized polynucleotide may be reconstituted and transformed into chemically competent *E*. *coli,* yeast, neurospora, maize, drosophila, etc. where high copy plasmid-like or chromosome structures occur by methods described herein.

Synthetic polynucleotides and their nucleic acid analogs play an important role in the research and studies of biochemical processes. Various enzyme-assisted and chemical-based methods have been developed to synthesize polynucleotides and nucleic acids.

### Enzymatic Methods

### In Vitro Transcription-enzymatic synthesis

cDNA encoding chimeric polynucleotides may be transcribed using an *in vitro* transcription (IVT) system. The system typically comprises a transcription buffer, nucleotide triphosphates (NTPs), an RNase inhibitor and a polymerase. The NTPs may be manufactured in house, may be selected from a supplier, or may be synthesized as described herein. The NTPs may be selected from, but are not limited to, those described herein including natural and unnatural (modified) NTPs. The polymerase may be selected from, but is not limited to, T7 RNA polymerase, T3 RNA polymerase and mutant polymerases such as, but not limited to, polymerases able to incorporate chimeric polynucleotides (e.g., modified nucleic acids).

### RNA Polymerases useful for synthesis

Any number of RNA polymerases or variants may be used in the synthesis of the chimeric polynucleotides of the present disclosure.

RNA polymerases may be modified by inserting or deleting amino acids of the RNA polymerase sequence. As a non-limiting example, the RNA polymerase may be modified to exhibit an increased ability to incorporate a 2'-modified nucleotide triphosphate compared to an unmodified RNA polymerase (see International Publication WO2008078180 and U.S. Patent 8,101,385;

Variants may be obtained by evolving an RNA polymerase, optimizing the RNA polymerase amino acid and/or nucleic acid sequence and/or by using other methods known in the art. As a non-limiting example, T7 RNA polymerase variants may be evolved using the continuous directed evolution system set out by Esvelt et al. (Nature (2011) 472(7344):499-503; where clones of T7 RNA polymerase may encode at least one mutation such as, but not limited to, lysine at position 93 substituted for threonine (K93T), I4M, A7T, E63V, V64D, A65E, D66Y, T76N, C125R, S128R, A136T, N165S, G175R, H176L, Y178H, F182L, L196F, G198V, D208Y, E222K, S228A, Q239R, T243N, G259D, M267I, G280C, H300R, D351A, A354S, E356D, L360P, A383V, Y385C, D388Y, S397R, M401T, N410S, K450R, P451T, G452V, E484A, H523L, H524N, G542V, E565K, K577E, K577M, N601S, S684Y, L699I, K713E, N748D, Q754R, E775K, A827V, D851N or L864F. As another non-limiting example, T7 RNA polymerase variants may encode at least mutation as described in U.S. Pub. Nos. 20100120024 and 20070117112. Variants of RNA polymerase may also include, but are not limited to, substitutional variants, conservative amino acid substitution, insertional variants, deletional variants and/or covalent derivatives.

In one case, the chimeric polynucleotide may be designed to be recognized by the wild type or variant RNA polymerases. In doing so, the chimeric polynucleotide may be modified to contain sites or regions of sequence changes from the wild type or parent chimeric polynucleotide.

Polynucleotide or nucleic acid synthesis reactions may be carried out by enzymatic methods utilizing polymerases. Polymerases catalyze the creation of phosphodiester bonds between nucleotides in a polynucleotide or nucleic acid chain. Currently known DNA polymerases can be divided into different families based on amino acid sequence comparison and crystal structure analysis. DNA polymerase I (pol I) or A polymerase family, including the Klenow fragments of *E. Coli, Bacillus* DNA polymerase I, *Thermus aquaticus* (Taq) DNA polymerases, and the T7 RNA and DNA polymerases, is among the best studied of these families. Another large family is DNA polymerase α (pol α) or B polymerase family, including all eukaryotic replicating DNA polymerases and polymerases from phages T4 and RB69. Although they employ similar catalytic mechanism, these families of polymerases differ in substrate specificity, substrate analog-incorporating efficiency, degree and rate for primer extension, mode of DNA synthesis, exonuclease activity, and sensitivity against inhibitors.

DNA polymerases are also selected based on the optimum reaction conditions they require, such as reaction temperature, pH, and template and primer concentrations. Sometimes a combination of more than one DNA polymerases is employed to achieve the desired DNA fragment size and synthesis efficiency. For example, Cheng et al. increase pH, add glycerol and dimethyl sulfoxide, decrease denaturation times, increase extension times, and utilize a secondary thermostable DNA polymerase that possesses a 3' to 5' exonuclease activity to effectively amplify long targets from cloned inserts and human genomic DNA. (Cheng et al., PNAS, Vol. 91, 5695-5699 (1994).

RNA polymerases from bacteriophage T3, T7, and SP6 have been widely used to prepare RNAs for biochemical and biophysical studies. RNA polymerases, capping enzymes, and poly-A polymerases are disclosed in the co-pending International Publication No. WO2014028429.

In one case, the RNA polymerase which may be used in the synthesis of the chimeric polynucleotides described herein is a Syn5 RNA polymerase (see Zhu et al. Nucleic Acids Research 2013).

The Syn5 RNA polymerase was recently characterized from marine cyanophage Syn5 by Zhu et al. where they also identified the promoter sequence (see Zhu et al. Nucleic Acids Research 2013).

Zhu et al. found that Syn5 RNA polymerase catalyzed RNA synthesis over a wider range of temperatures and salinity as compared to T7 RNA polymerase. Additionally, the requirement for the initiating nucleotide at the promoter was found to be less stringent for Syn5 RNA polymerase as compared to the T7 RNA polymerase making Syn5 RNA polymerase promising for RNA synthesis.

In one case, a Syn5 RNA polymerase may be used in the synthesis of the chimeric polynucleotides described herein. As a non-limiting example, a Syn5 RNA polymerase may be used in the synthesis of the chimeric polynucleotide requiring a precise 3'-termini.

In one case, a Syn5 promoter may be used in the synthesis of the chimeric polynucleotides. As a non-limiting example, the Syn5 promoter may be 5'-ATTGGGCACCCGTAAGGG-3' (SEQ ID NO: 3) as described by Zhu et al. (Nucleic Acids Research 2013).

In one case, a Syn5 RNA polymerase may be used in the synthesis of chimeric polynucleotides comprising at least one chemical modification described herein and/or known in the art. (see e.g., the incorporation of pseudo-UTP and 5Me-CTP described in Zhu et al. Nucleic Acids Research 2013,

In one case, the chimeric polynucleotides described herein may be synthesized using a Syn5 RNA polymerase which has been purified using modified and improved purification procedure described by Zhu et al. (Nucleic Acids Research 2013).

Various tools in genetic engineering are based on the enzymatic amplification of a target gene which acts as a template. For the study of sequences of individual genes or specific regions of interest and other research needs, it is necessary to generate multiple copies of a target gene from a small sample of polynucleotides or nucleic acids. Such methods may be applied in the manufacture of the chimeric polynucleotides.

Polymerase chain reaction (PCR) has wide applications in rapid amplification of a target gene, as well as genome mapping and sequencing. The key components for synthesizing DNA comprise target DNA molecules as a template, primers complementary to the ends of target DNA strands, deoxynucleoside triphosphates (dNTPs) as building blocks, and a DNA polymerase. As PCR progresses through denaturation, annealing and extension steps, the newly produced DNA molecules can act as a template for the next circle of replication, achieving exponentially amplification of the target DNA. PCR requires a cycle of heating and cooling for denaturation and annealing. Variations of the basic PCR include asymmetric PCR [Innis et al., PNAS, vol. 85, 9436-9440 (1988)], inverse PCR [Ochman et al., Genetics, vol. 120(3), 621-623, (1988)], reverse transcription PCR (RT-PCR) (Freeman et al., BioTechniques, vol. 26(1), 112-22, 124-5 (1999)).

In RT-PCR, a single stranded RNA is the desired target and is converted to a double stranded DNA first by reverse transcriptase.

A variety of isothermal *in vitro* nucleic acid amplification techniques have been developed as alternatives or complements of PCR. For example, strand displacement amplification (SDA) is based on the ability of a restriction enzyme to form a nick-(Walker et al., PNAS, vol. 89, 392-396 (1992)).

A restriction enzyme recognition sequence is inserted into an annealed primer sequence. Primers are extended by a DNA polymerase and dNTPs to form a duplex. Only one strand of the duplex is cleaved by the restriction enzyme. Each single strand chain is then available as a template for subsequent synthesis. SDA does not require the complicated temperature control cycle of PCR.

Nucleic acid sequence-based amplification (NASBA), also called transcription mediated amplification (TMA), is also an isothermal amplification method that utilizes a combination of DNA polymerase, reverse transcriptase, RNAse H, and T7 RNA polymerase- [Compton, Nature, vol. 350, 91-92 (1991)]

A target RNA is used as a template and a reverse transcriptase synthesizes its complementary DNA strand. RNAse H hydrolyzes the RNA template, making space for a DNA polymerase to synthesize a DNA strand complementary to the first DNA strand which is complementary to the RNA target, forming a DNA duplex. T7 RNA polymerase continuously generates complementary RNA strands of this DNA duplex. These RNA strands act as templates for new cycles of DNA synthesis, resulting in amplification of the target gene.

Rolling-circle amplification (RCA) amplifies a single stranded circular polynucleotide and involves numerous rounds of isothermal enzymatic synthesis where Φ29 DNA polymerase extends a primer by continuously progressing around the polynucleotide circle to replicate its sequence over and over again. Therefore, a linear copy of the circular template is achieved. A primer can then be annealed to this linear copy and its complementary chain can be synthesized [Lizardi et al., Nature Genetics, vol. 19, 225-232 (1998)].

A single stranded circular DNA can also serve as a template for RNA synthesis in the presence of an RNA polymerase (Daubendiek et al., JACS, vol. 117, 7818-7819 (1995)
). An inverse rapid amplification of cDNA ends (RACE) RCA is described by Polidoros et al. A messenger RNA (mRNA) is reverse transcribed into cDNA, followed by RNAse H treatment to separate the cDNA. The cDNA is then circularized by CircLigase into a circular DNA. The amplification of the resulting circular DNA is achieved with RCA (Polidoros et al., BioTechniques, vol. 41, 35-42 (2006),

Any of the foregoing methods may be utilized in the manufacture of one or more regions of the chimeric polynucleotides

Assembling polynucleotides or nucleic acids by a ligase is also widely used. DNA or RNA ligases promote intermolecular ligation of the 5' and 3' ends of polynucleotide chains through the formation of a phosphodiester bond. Ligase chain reaction (LCR) is a promising diagnosing technique based on the principle that two adjacent polynucleotide probes hybridize to one strand of a target gene and couple to each other by a ligase. If a target gene is not present, or if there is a mismatch at the target gene, such as a single-nucleotide polymorphism (SNP), the probes cannot ligase. (Wiedmann et al., PCR Methods and Application, vol.3 (4), s51-s64 (1994) ). LCR may be combined with various amplification techniques to increase sensitivity of detection or to increase the amount of products if it is used in synthesizing polynucleotides and nucleic acids.

Several library preparation kits for nucleic acids are now commercially available. They include enzymes and buffers to convert a small amount of nucleic acid samples into an indexed library for downstream applications. For example, DNA fragments may be placed in a NEBNEXT^{®} ULTRA^{™} DNA Library Prep Kit by NewEngland BioLabs^{®} for end preparation, ligation, size selection, clean-up, PCR amplification and final clean-up.

Continued development is going on to improvement the amplification techniques. For example, US Pat. 8,367,328 to Asada et al. teaches utilizing a reaction enhancer to increase the efficiency of DNA synthesis reactions by DNA polymerases. The reaction enhancer comprises an acidic substance or cationic complexes of an acidic substance. US Pat. 7.384,739 to Kitabayashi et al. teaches a carboxylate ion-supplying substance that promotes enzymatic DNA synthesis, wherein the carboxylate ion-supplying substance is selected from oxalic acid, malonic acid, esters of oxalic acid, esters of malonic acid, salts of malonic acid, and esters of maleic acid. US Pat. 7,378,262 to Sobek et al. discloses an enzyme composition to increase fidelity of DNA amplifications. The composition comprises one enzyme with 3' exonuclease activity but no polymerase activity and another enzyme that is a polymerase. Both of the enzymes are thermostable and are reversibly modified to be inactive at lower temperatures.

US Pat. No. 7,550,264 to Getts et al. teaches multiple round of synthesis of sense RNA molecules are performed by attaching oligodeoxynucleotides tails onto the 3' end of cDNA molecules and initiating RNA transcription using RNA polymerase,

US Pat. Publication No. 2013/0183718 to Rohayem teaches RNA synthesis by RNAdependent RNA polymerases (RdRp) displaying an RNA polymerase activity on single-stranded DNA templates

Oligonucleotides with non-standard nucleotides may be synthesized with enzymatic polymerization by contacting a template comprising non-standard nucleotides with a mixture of nucleotides that are complementary to the nucleotides of the template as disclosed in US Pat. No. 6,617,106 to Benner

### Solid-phase chemical synthesis

Chimeric polynucleotides may be manufactured in whole or in part using solid phase techniques.

Solid-phase chemical synthesis of polynucleotides or nucleic acids is an automated method wherein molecules are immobilized on a solid support and synthesized step by step in a reactant solution. Impurities and excess reagents are washed away and no purification is required after each step. The automation of the process is amenable on a computer-controlled solid-phase synthesizer. Solid-phase synthesis allows rapid production of polynucleotides or nucleic acids in a relatively large scale that leads to the commercial availability of some polynucleotides or nucleic acids. Furthermore, it is useful in site-specific introduction of chemical modifications in the polynucleotide or nucleic acid sequences. It is an indispensable tool in designing modified derivatives of natural nucleic acids.

In automated solid-phase synthesis, the chain is synthesized in 3' to 5' direction. The hydroxyl group in the 3' end of a nucleoside is tethered to a solid support via a chemically cleavable or light-cleavable linker. Activated nucleoside monomers, such as 2'-deoxynucleosides (dA, dC, dG and T), ribonucleosides (A, C, G, and U), or chemically modified nucleosides, are added to the support-bound nucleoside sequentially. Currently most widely utilized monomers are the 3'-phophoramidite derivatives of nucleoside building blocks. The 3' phosphorus atom of the activated monomer couples with the 5' oxygen atom of the support-bound nucleoside to form a phosphate triester. To prevent side reactions, all functional groups not involved in the coupling reaction, such as the 5' hydroxyl group, the hydroxyl group on the 3' phosphorus atom, the 2' hydroxyl group in ribonucleosides monomers, and the amino groups on the purine or pyrimidine bases, are all blocked with protection groups. The next step involves oxidation of the phosphate triester to form a phosphate triester or phosphotriester, where the phosphorus atom is pentavalent. The protection group on the 5' hydroxyl group at the end of the growing chain is then removed, ready to couple with an incoming activated monomer building block. At the end of the synthesis, a cleaving agent such as ammonia or ammonium hydroxide is added to remove all the protecting groups and release the polynucleotide chains from the solid support. Light may also be applied to cleave the polynucleotide chain. The product can then be further purified with high pressure liquid chromatography (HPLC) or electrophoresis.

In solid-phase synthesis, the polynucleotide chain is covalently bound to the solid support via its 3' hydroxyl group. The solid supports are insoluble particles also called resins, typically 50-200 µm in diameter. Many different kinds of resins are now available, as reviewed in "Solid-phase supports for polynucleotide synthesis" by Guzaev [Guzaev, Current Protocols in Nucleic Acid Chemistry, 3.1.1-3.1.60 (2013)]
. The most common materials for the resins include highly cross-linked polystyrene beads and controlled pore glass (CPG) beads. The surface of the beads may be treated to have functional groups, such as amino or aminomethyl groups that can be used as anchoring points for linkers to tether nucleosides. They can be implemented in columns, multi-well plates, microarrays or microchips. The column-based format allows relatively large scale synthesis of the polynucleotides or nucleic acids. The resins are held between filters in columns that enable all reagents and solvents to pass through freely. Multi-well plates, microarrays, or microchips are designed specifically for cost-effective small scale synthesis. Up to a million polynucleotides can be produced on a single microarray chip. However, the error rates of microchipbased synthesis are higher than traditional column-based methods [Borovkov et al., Nucleic Acids Research, vol. 38(19), e180 (2010)] Multi-well plates allow parallel synthesis of polynucleotides or nucleic acids with different sequences simultaneously [Sindelar, et al., Nucleic Acids Research, vol. 23, 982-987 (1995)] The loading on the solid supports is limited. In addition, as the extension progresses, the morphology and bulkiness of the growing chains on the solid supports might hinder the incoming monomers from reacting with the terminal group of the growing chains. Therefore, the number of monomers that can be added to the growing chain is also limited.

Linkers are attached to the solid support for further extension of the chain. They are stable to all the reagents used in the synthesis process, except in the end of the synthesis when the chain is detached from the solid support. Solid supports with a specific nucleoside linker, i.e., A, C, dT, G, or U, can be used to prepare polynucleotides with A, C, T, G, or U as the first nucleotide in the sequence, respectively. Universal solid supports with non-nucleoside linkers can be used for all polynucleotide sequences (US Pat. 6,653,468 to Guzaev et al.
). Various non-nucleoside linkers have been developed for universal supports, a lot of them with two vicinal hydroxyl groups. For example, a succinyl group is a frequently used linker.

As used herein, a linker refers to a group of atoms, e.g., 10-1,000 atoms, and can be comprised of the atoms or groups such as, but not limited to, carbon, amino, alkylamino, oxygen, sulfur, sulfoxide, sulfonyl, carbonyl, and imine. The linker can be attached to a modified nucleoside or nucleotide on the nucleobase or sugar moiety. A linker may be nucleic acid based or non-nucleosidic. The linker may be of sufficient length as to not interfere with incorporation into a nucleic acid sequence. The linker can be used for any useful purpose, such as to form multimers (e.g., through linkage of two or more chimeric polynucleotides molecules) or conjugates, as well as to administer a therapeutic molecule or incorporate a label, as described herein. Examples of chemical groups that can be incorporated into the linker include, but are not limited to, alkyl, alkenyl, alkynyl, amido, amino, ether, thioether, ester, alkylene, heteroalkylene, aryl, or heterocyclyl, each of which can be optionally substituted, as described herein. Examples of linkers include, but are not limited to, unsaturated alkanes, polyethylene glycols (e.g., ethylene or propylene glycol monomeric units, e.g., diethylene glycol, dipropylene glycol, triethylene glycol, tripropylene glycol, tetraethylene glycol, or tetraethylene glycol), and dextran polymers and derivatives thereof., Other examples include, but are not limited to, cleavable moieties within the linker, such as, for example, a disulfide bond (-S-S-) or an azo bond (-N=N-), which can be cleaved using a reducing agent or photolysis. Non-limiting examples of a selectively cleavable bond include an amido bond can be cleaved for example by the use of tris(2-carboxyethyl)phosphine (TCEP), or other reducing agents, and/or photolysis, as well as an ester bond can be cleaved for example by acidic or basic hydrolysis.)

Besides the functional groups on the activated monomer and the growing chain needed for the coupling reaction to extend the chain, all other functional groups need to be protected to avoid side reactions. The conditions for protection and deprotection, and the selection of appropriate protecting groups can be readily determined by one skilled in the art. The chemistry of protecting groups can be found, for example, in Greene, et al., Protective Groups in Organic Synthesis, 2d. Ed., Wiley & Sons, 1991. For example, the 5' hydroxyl group on the activated nucleoside phosphoramidite monomers may be protected with 4,4'-dimethoxytrityl (DMT) and the hydroxyl group on the phosphorus atom may be protected with 2-cyanoethyl. The exocyclic amino groups on the A, C, G bases may be protected with acyl groups.

In a solid-phase synthesis system, the reactivity of the activated monomers is important, because of the heterogeneity of the media. A majority of solid-phase synthesis uses phosphoramidite nucleosides, the mechanism of which is discussed above. Another activated monomer example is nucleoside H-phosphonates. [Abramova, Molecules, vol. 18, 1063-1075 (2013)]. A large excess of reagents, such as monomers, oxidizing agents, and deprotection agents, is required in order to ensure high yields in the solid-phase synthesis system.

Scientific studies and research are going on to further improve the solid-phase synthesis method. For example, instead of the well-established 3 '-to-5' synthesis, US Pat. No. 8,309,707 and US Pat. Publication No. 2013/0072670 to Srivastava et al. disclosed a 5 '-to-3' synthesis of RNA utilizing a novel phosphoramidite and a novel nucleoside derivative, thereby allowing easy modifications of the synthetic RNA at the 3' end. PCT application WO2013123125 to Church et al. describes assembly of a target nucleic acid sequence from a plurality of subsequences, wherein resins with the subsequences are placed in an emulsion droplet. The subsequences are cleaved off the resins and assemble within the emulsion droplet. To reduce the cost of solid supports, a reusable CPG solid support has been developed with a hydroquinone-O, O'-diacetic acid linker (Q-linker) (Pon et al., Nucleic Acid Research, vol. 27, 1531-1538 (1999).

New protecting groups for solid-phase synthesis have also been developed. Nagat et al. has successfully synthesized 110-nt-long RNA with the sequence of a candidate precursor microRNA by using 2-cyanoethoxymethyl (CEM) as the 2'-hydroxy protecting group (Shiba et al., Nucleic Acids Research, vol. 35, 3287-3296 (2007),). Also with CEM as 2'-O-protecting group, a 130-nt mRNA has been synthesized encoding a 33-amino acid peptide that includes the sequence of glucagon-like peptide-1 (GLP-1). The biological activity of the artificial 130-nt mRNA is shown by producing GLP-1 in a cell-free protein synthesis system and in Chinese hamster ovary (CHO) cells (Nagata et al., Nucleic Acids Research, vol. 38(21), 7845-7857 (2010),
). Novel protecting groups for solid-phase synthesis monomers include, but are not limited to, carbonate protecting group disclosed in US Pat. No. 8,309,706 to Dellinger et al., orthoester-type 2' hydroxyl protecting group and an acyl carbonate-type hydroxyl protecting group disclosed in US Pat. No. 8,242,258 to Dellinger et al., 2'-hydroxyl thiocarbon protecting group disclosed in US Pat. No. 8,202,983 to Dellinger et al., 2'-silyl containing thiocarbonate protecting group disclosed in US Pat. No. 7,999,087 to Dellinger et al., 9-fluorenylmethoxycarbonyl (FMOS) derivatives as an amino protecting group disclosed in US Pat. No. 7,667,033 to Alvarado, fluoride-labile 5'sily1 protecting group disclosed in US Pat. No. 5,889,136 to Scaringe et al., and pixyl protecting groups disclosed in US Pat. Publication No. 2008/0119645 to Griffey et al., US Pat. Publication No. 2011/0275793 to Debart et al. teaches RNA synthesis using a protecting group of the hyoxyls in position 2' of the ribose that can be removed by a base Novel solid supports include polymers made from monomers comprising protected hydroxypolyC2-4 alkyleneoxy chain attached to a polymerizable unit taught in US Pat. No. 7.476,709 to Moody et al.

### Liquid Phase Chemical Synthesis

The synthesis of chimeric polynucleotides by the sequential addition of monomer building blocks may be carried out in a liquid phase. A covalent bond is formed between the monomers or between a terminal functional group of the growing chain and an incoming monomer. Functional groups not involved in the reaction must be temporarily protected. After the addition of each monomer building block, the reaction mixture has to be purified before adding the next monomer building block. The functional group at one terminal of the chain has to be deprotected to be able to react with the next monomer building blocks. A liquid phase synthesis is labor- and time-consuming and cannot not be automated. Despite the limitations, liquid phase synthesis is still useful in preparing short polynucleotides in a large scale. Because the system is homogenous, it does not require a large excess of reagents and is cost-effective in this respect.

### Combination of Different Synthetic Methods

The synthetic methods discussed above each has its own advantages and limitations. Attempts have been conducted to combine these methods to overcome the limitations. Such combinations of methods are within the scope of the present disclosure

Short polynucleotide chains with 2-4 nucleotides may be prepared in liquid phase followed by binding to a solid support for extension reactions by solid phase synthesis. A high efficiency liquid phase (HELP) synthesis is developed that uses monomethyl ether of polyethylene glycol (MPEG) beads as a support for the monomer building blocks. MPEG is soluble in methylene chloride and pyridine solvents but precipitates in a diethyl ether solvent. By choosing an appropriate solvent, the coupling reaction between monomers or between a growing chain and an incoming monomer bound on MPEG can be carried out in a homogenous liquid phase system. The mixture can then be washed with a diethyl ether solvent to easily precipitate and purify the product [Bonora et al., Nucleic Acids Research, vol. 18, 3155-3159 (1990)] US Pat. No. 8,304,532 to Adamo et al. teaches a solution phase oligonucleotide synthesis where at least some of the reagents are solid supported.

The use of solid-phase or liquid-phase chemical synthesis in combination with enzymatic ligation provides an efficient way to generate long chain polynucleotides that cannot be obtained by chemical synthesis alone. Moore and Sharp describe preparing RNA fragments 10- to 20-nt long by chemical synthesis, to which site-specific modifications may be introduced, annealing the fragments to a cDNA bridge, and then assemble the fragments with T4 DNA ligase (Moore et al., Science, vol. 256, 992-997 (1992), ).

A solid-phase synthesizer may produce enough polynucleotides or nucleic acids with good purity to preform PCR and other amplification techniques. Agilent Technologies have developed microarrays that are commercially available. Polynucleotides may be synthesized on a microarray substrate, cleaved by a strong base or light, followed by PCR amplification to generate a library of polynucleotides

[Cleary et al., Nature Methods, vol. 1(3), 241-247 (2004)].

### Small Region Synthesis

Regions or subregions of the chimeric polynucleotides

may comprise small RNA molecules such as siRNA, and therefore may be synthesized in the same manner. There are several methods for preparing siRNA, such as chemical synthesis using appropriately protected ribonucleoside phosphoramidites, *in vitro* transcription, siRNA expression vectors, and PCR expression cassettes. Sigma-Aldrich^{®} is one of the siRNA suppliers and synthesizes their siRNA using ribonucleoside phosphoramidite monomers protected at the 2' position with a t-butylmethylsilyl (TBDMS) group. The solid-phase chemical synthesis is carried out with Sigma-Aldrich^{®}'s Ultra Fast Parallel Synthesis (UFPS) and Ultra Fast Parallel Deprotection (UFPD) to achieve high coupling efficiency and fast deprotection. The final siRNA products may be purified with HPLC or PAGE. Such methods may be used to synthesize regions or subregions of chimeric polynucleotides.

*In vitro* transcription and expression from a vector or a PCR-generated siRNA cassette require appropriate templates to produce siRNAs. The commercially available Ambion^{®} Silencer^{®} siRNA construction kit produces siRNA by *in vitro* transcription of DNA templates and contains the enzymes, buffers, primers needed. Such methods may be used to synthesize regions or subregions of chimeric polynucleotides.

### Ligation of chimeric polynucleotide regions or subregions

Ligation is an indispensable tool for assembling polynucleotide or nucleic acid fragments into larger constructs. DNA fragments can be joined by a ligase catalyzed reaction to create recombinant DNA with different functions. Two oligodeoxynucleotides, one with a 5' phosphoryl group and another with a free 3' hydroxyl group, serve as substrates for a DNA ligase. Oligodexoynucleotides with fluorescent or chemiluminescent labels may also serve as DNA ligase substrates. (Martinelli et al., Clinical Chemistry, vol. 42, 14-18 (1996) ). RNA ligases such as T4 RNA ligase catalyze the formation of a phosphodiester bond between two single stranded oligoribonucleotides or RNA fragments. Copies of large DNA constructs have been synthesized with a combination of polynucleotide fragments, thermostable DNA polymerases, and DNA ligases. US Pat. Publication No. 2009/0170090 to Ignatov et al. discloses improving PCT, especially enhancing yield of a long distance PCR and/or a low copy DNA template PCR amplification, by using a DNA ligase in addition to a DNA polymerase.

Ligases may be used with other enzymes to prepare desired chimeric polynucleotide or nucleic acid molecules and to perform genome analysis. For example, ligation-mediated selective PCR amplification is disclosed in EP Pat. Pub. No. 0735144 to Kato. Complementary DNAs (cDNAs) reverse-transcribed from tissue- or cell-derived RNA or DNA are digested into fragments with type IIS restriction enzymes.

Biotinylated adapter sequences are attached to the fragments by *E. coli* DNA ligases. The biotin-labeled DNA fragments are then immobilized onto streptavidin-coated beads for downstream analysis.

A ligation splint or a ligation splint oligo is an oligonucleotide that is used to provide an annealing site or a ligation template for joining two ends of one nucleic acid, i.e., intramolecular joining, or two ends of two nucleic acids, i.e., intermolecular joining, using a ligase or another enzyme with ligase activity. The ligation splint holds the ends adjacent to each other and creates a ligation junction between the 5'-phosphorylated and a 3'-hydroxylated ends that are to be ligated.

In one case, a splint-mediated ligation or splint ligation method may be used to synthesize the chimeric polynucleotides described herein. The chimeric polynucleotide may be assembled using a method that does not rely on the presence of restriction endonuclease cleavage sites such as the method described in International Patent Publication No. WO2012138453. Splint-mediated ligation allows for the rapid synthesis of the construct using controlled concatenation and without the need or with limited need for the introduction of restriction sites at the joining regions. As a non-limiting example, splint ligation may be used to add at least one untranslated region to case a coding region of the chimeric polynucleotide. In one case, splint ligation may be used in combination with other synthesis methods in the synthesis of the chimeric polynucleotides described herein.

If the 5'-phosphorylated and the 3'-hydroxyl ends of nucleic acids are ligated when the ends are annealed to a ligation splint so that the ends are adjacent, enzymes such as, but not limited to, T4 DNA ligase, Ampligase^{®} DNA Ligase (Epicentre^{®} Technologies), *Tth* DNA ligase, *Tƒl* DNA ligase, or *Tsc* DNA Ligase (Prokaria) can be used. U.S. Pat. No. 6,368,801 to Farugui discloses that T4 RNA ligase can efficiently ligate ends of RNA molecules that are adjacent to each other when hybridized to an RNA splint. Thus, T4 RNA ligase is a suitable ligase for joining DNA ends with a ligation splint oligo comprising RNA or modified RNA. Examples of RNA splints include modified RNA containing 2'-fluorine-CTP (2'-F-dCTP) and 2'-fluorine-UTP (2'-F-dUTP) made using the DuraScribe^{®} T7 Transcription Kit (Epicentre^{®} Technologies) disclosed in US Pat. No. 8,137,911 and US Pat. Publication 2012/0156679 to Dahl et al. The modified RNA produced from DuraScribe^{®} T7 Transcription kit is completely resistant to RNase A digestion. DNA splint and DNA ligase may be used to generate RNA-protein fusions disclosed in US Pat. No. 6,258,558 to Szostak et al.

For intramolecular ligation of linear ssDNA, US Pat. No. 7,906,490 to Kool et al. teaches constructing a 83-nucleotide circle by making linear oligodeoxynucleotides fragments on a DNA synthesizer followed by ligation with T4 DNA ligase and two 30 nucleotide splint oligonucleotides. Circulation of linear sense promoter-containing cDNA is disclosed in US Pat. Publication No. 2012/0156679 to Dahl et al.

ThermoPhage^{™} ssDNA ligase (Prokazyme), which is derived from phage TS2126 that infects *Thermus scotoductus,* catalyzes ATP-dependent intra- and inter-molecular ligation of DNA and RNA.

The solid-phase chemical synthesis method that uses phosphoramidite monomers is limited to produce DNA molecules with short strands. The purity of the DNA products and the yield of reactions become poor when the length exceeds 150 bases. For the synthesis of long polynucleotides in high yields, it is more convenient to use enzymatic ligation method in tandem with chemical synthesis. For example, Moore and Sharp describe preparing RNA fragments 10- to 20-nt long by chemical synthesis, to which site-specific modifications may be introduced, annealing the fragments to a cDNAsplint, and then assemble the fragments with T4 DNA ligase. (Moore et al., Science, vol. 256, 992-997 (1992), ). Ligation reactions of oligoribonucleotides with T4 RNA ligase and a DNA splint or a polyribonucleotide to generate large, synthetic RNAs are described in Bain et al., Nucleic Acids Research, vol. 20(16), 4372 (1992), Stark et al., RNA, vol. 12, 2014-2019 (2006), and US Pat. Application No. 2005/0130201 to Deras et al. 5'-cap and 3 '-polyA tail are often added by enzymatic addition to an oligonucleotide synthesized with solid-phase methods. As a non-limiting example, a synthetic capped 42-mer mRNA has been synthesized in three fragments enzymatically ligated as described by Iwase et al. (Nucleic Acids Research, vol. 20, 1643-1648 (1992) ). A 16.3-kilobase mouse mitochondrial genome has been produced from 600 overlapping 60-mer polynucleotides. The method cycles between *in vitro* recombination and amplification until the desired length is reached. (Gibson et al., Nature Methods, vol. 7, 901-903 (2010) ). The assembly of a 1.08 megabase *Mycoplasma mycoides* JCVI-syn1.0 genome has also been reported. 1080 bp cassettes are produced by assembling polynucleotide fragments chemically generated from a polynucleotide synthesizer. The genome is then assembled in three stages by transformation and homologous recombination in yeast. (Gibson, et al., Science, vol. 329, 52-56 (2010).

Studies have been conducted to join short DNA fragments with chemical linkers. 'Click' chemistry or 'click' ligation, the cycloaddition reaction between azide and alkyne, has gained a lot of interest because of its advantages such as mild reaction condition, high yields, and inoffensive byproducts. 'Click' chemistry is reviewed by Nwe et al. in Cancer Biotherapy and Radiopharmaceuticals, vol. 24(3), 289-302 (2009). DNA constructs up to 300 bases in length have been produced with click ligation and longer sequences are feasible. Demonstrated with PCR data, various DNA polymerases are able to amplify the synthesized DNA constructs made by click ligation despite the triazole linkers between the fragments resulting from the cycloaddition reaction. *In vitro* transcription and rolling circle amplification can also be performed on the synthesized DNA constructs. Hairpin ribozymes up to 100 nucleotides in length and cyclic mini-DNA duplexes have also been prepared with click ligation. (El-Sagheer et al., Accounts of Chemical Research, vol. 45(8), 1258-1267 (2012).

For example, polynucleotides of the disclosure having a sequence comprising Formula I:

[Aₙ]-L¹-[Bₒ], Formula I

may be synthesized by reacting a compound having the structure of Formula XVI:

[Aₙ]-(R¹)ₐ-(R²)_{b}-(R³)_{c}-N₃ Formula XVI

with a compound having the structure of Formula XVII:

R²⁷-(R⁵)_{d}-(R⁶)ₑ-(R⁷)_{f}-[Bₒ] Formula XVII

wherein each A and B is independently include any nucleoside (e.g., a nucleotide);

n and o are, independently 10 to 10,000, e.g., 10 to 1000 or 10 to 2000; and

L¹ has the structure of Formula III:

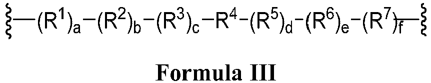

wherein a, b, c, d, e, and f are each, independently, 0 or 1;

R¹, R³, R⁵, and R⁷ each, independently, is selected from optionally substituted C₁-C₆ alkylene, optionally substituted C₁-C₆ heteroalkylene, O, S, and NR⁸;

R² and R⁶ are each, independently, selected from carbonyl, thiocarbonyl, sulfonyl, or phosphoryl;

R⁴ is an optionally substituted triazolene; and

R⁸ is hydrogen, optionally substituted C₁-C₄ alkyl, optionally substituted C₃-C₄ alkenyl, optionally substituted C₂-C₄ alkynyl, optionally substituted C₂-C₆ heterocyclyl, optionally substituted C₆-C₁₂ aryl, or optionally substituted C₁-C₇ heteroalkyl; and

R²⁷ is an optionally substituted C₂-C₃ alkynyl or an optionally substituted C₈-C₁₂ cycloalkynyl,

wherein L¹ is attached to [A_{n]} and [Bₒ] at the sugar of one of the nucleosides.

Chimeric polynucleotides of the disclosure including the structure of Formula XIa, XIb, XIIa, or XIIb: 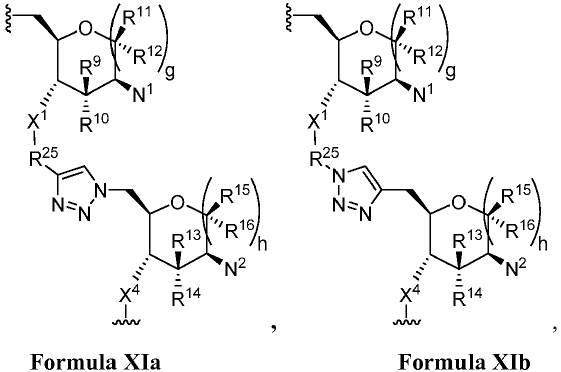 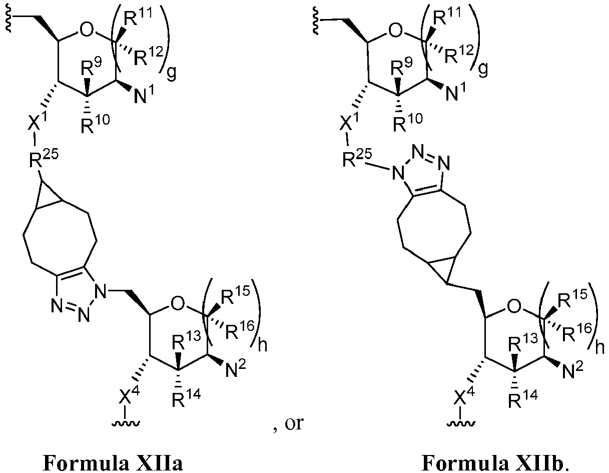 may be synthesized by reacting (e.g., under [3+2] cycloaddition conditions in the presence or absence of a copper source) a compound having the structure of Formula XIIIa, XIIIb, XIVa, or XIVb: 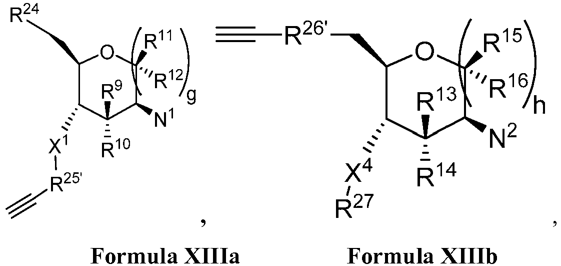 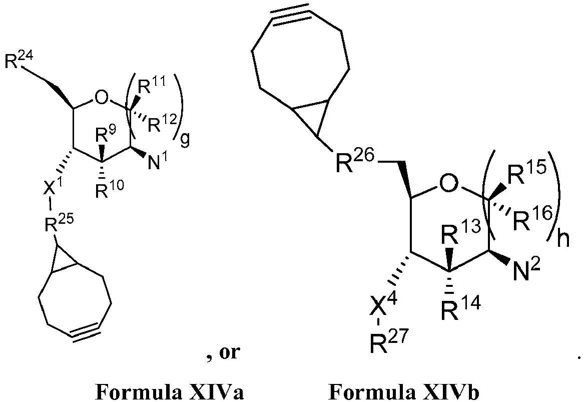 with a compound having the structure of Formula XVa or XVb: 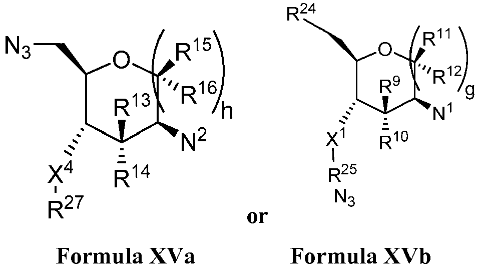 wherein each of N¹ and N² is independently a nucleobase;

each of R⁹, R¹⁰, R¹¹, R¹², R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

each of g and h is, independently, 0 or 1;

each X¹ and X⁴ is, independently, O, NH, or S; and

each of R²⁴ and R²⁷ is, independently, a region of linked nucleosides; and

each of R²⁵, R²⁵, R²⁶ and R^{26'} is, independently, optionally substituted C₁-C₆ alkylene or optionally substituted C₁-C₆ heteroalkylene or R^{25'} or R^{26'} and the alkynyl group together form optionally substituted cycloalkynyl.

For example, the chimeric polynucleotides may be synthesized as shown below 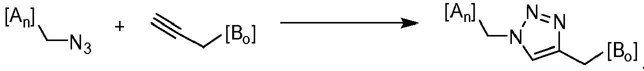 In some cases , the 5' cap structure or poly-A tail may be attached to a chimeric polynucleotide of the disclosure with this method.

A 5' cap structure may be attached to a chimeric polynucleotide of the disclosure as shown below:

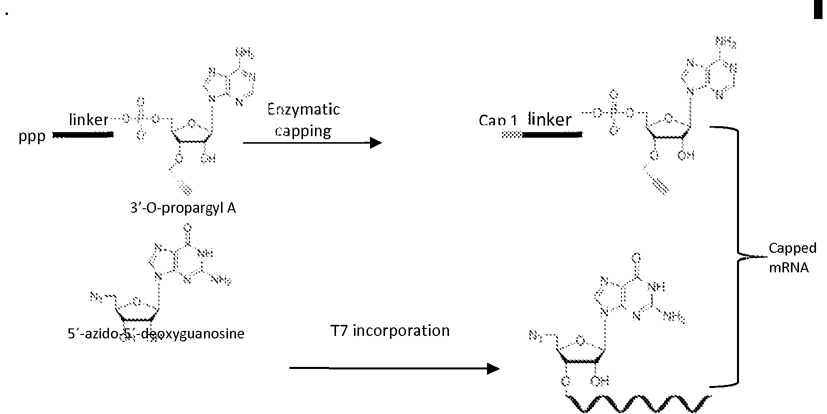

A poly-A tail may be attached to a chimeric polynucleotide of the disclosure as shown below:

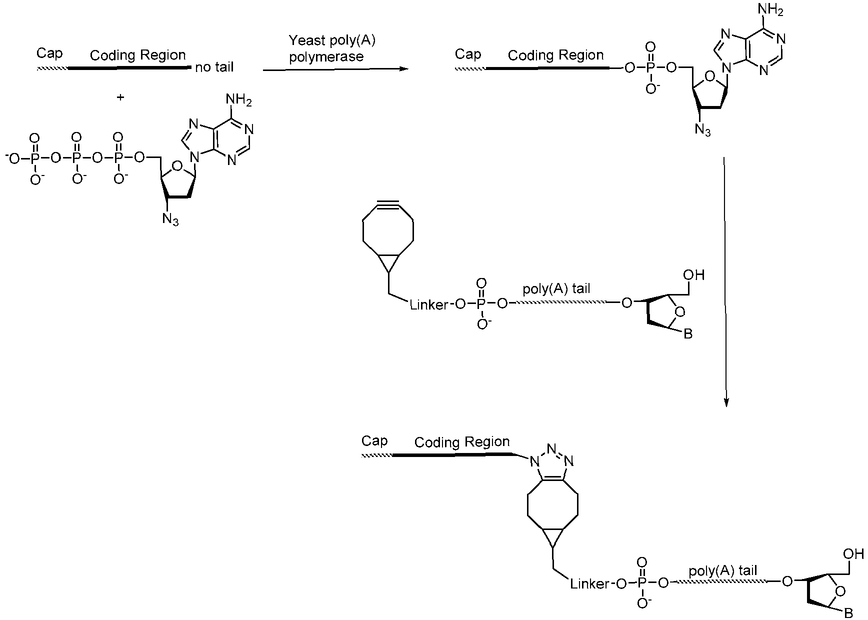

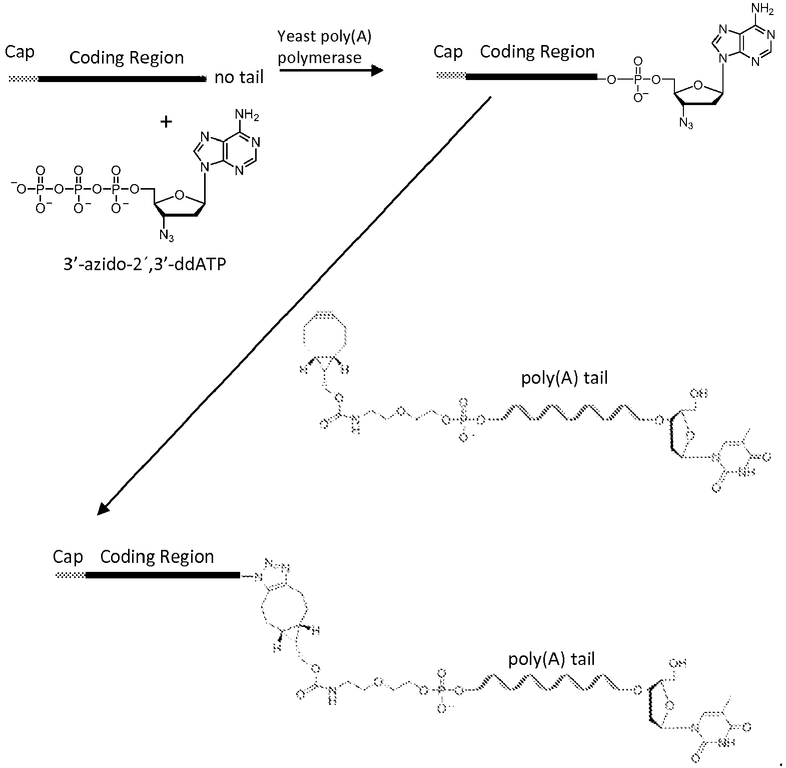

Sequential ligation can be performed on a solid substrate. For example, initial linker DNA molecules modified with biotin at the end are attached to streptavidin-coated beads. The 3 '-ends of the linker DNA molecules are complimentary with the 5 '-ends of the incoming DNA fragments. The beads are washed and collected after each ligation step and the final linear constructs are released by a meganuclease. This method allows rapid and efficient assembly of genes in an optimized order and orientation. (Takita, DNA Research, vol. 20(4), 1-10 (2013). Labeled polynucleotides synthesized on solid-supports are disclosed in US Pat. Pub. No. 2001/0014753 to Soloveichik et al. and US Pat. Pub. No. 2003/0191303 to Vinayak et al.

### Modified and Conjugated Chimeric Polynucleotides

Non-natural modified nucleotides may be introduced to chimeric polynucleotides or nucleic acids during synthesis or post-synthesis of the chains to achieve desired functions or properties. The modifications may be on internucleotide lineage, the purine or pyrimidine bases, or sugar. The modification may be introduced at the terminal of a chain or anywhere else in the chain; with chemical synthesis or with a polymerase enzyme. For example, hexitol nucleic acids (HNAs) are nuclease resistant and provide strong hybridization to RNA. Short messenger RNAs (mRNAs) with hexitol residues in two codons have been constructed. (Lavrik et al., Biochemistry, 40, 11777-11784 (2001).

The antisense effects of a chimeric HNA gapmer oligonucleotide comprising a phosphorothioate central sequence flanked by 5' and 3' HNA sequences have also been studied. (Kang et al., Nucleic Acids Research, vol. 32(4), 4411-4419 (2004).

The preparation and uses of modified nucleotides comprising 6-member rings in RNA interference, antisense therapy or other applications are disclosed in US Pat. Application No. 2008/0261905, US Pat. Application No. 2010/0009865, and PCT Application No. WO97/30064 to Herdewijn et al. Modified nucleic acids and their synthesis are disclosed in copending PCT applications No. PCT/US2012/058519 (Attorney Docket Number M09).

The synthesis and strategy of modified polynucleotides is reviewed by Verma and Eckstein in Annual Review of Biochemistry, vol. 76, 99-134 (1998).

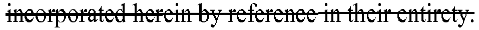

Either enzymatic or chemical ligation methods can be used to conjugate chimeric polynucleotides or their regions with different functional blocks, such as fluorescent labels, liquids, nanoparticles, delivery agents, etc. The conjugates of polynucleotides and modified polynucleotides are reviewed by Goodchild in Bioconjugate Chemistry, vol. 1(3), 165-187 (1990).

US Pat. No. 6,835,827 and US Pat. No. 6,525,183 to Vinayak et al. teach synthesis of labeled oligonucleotides using a labeled solid support.

For example, chimeric polynucleotides may comprise the structure of Formula Va or Vb:

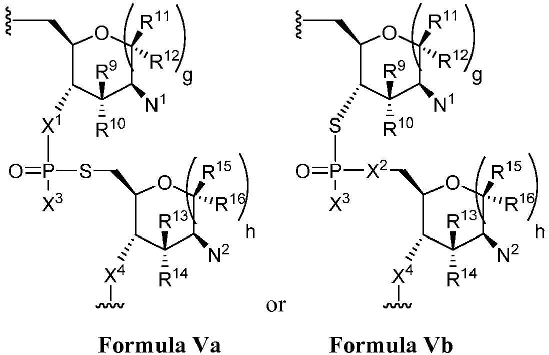

The chimeric polynucleotides may comprise a structure made by a method which includes reacting (e.g., under alkylating conditions) a compound having the structure of Formula VIa or VIb:

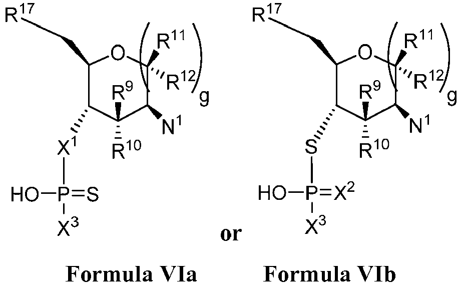

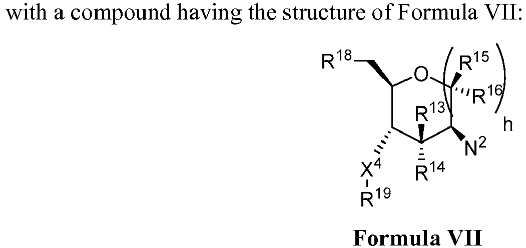

wherein each of N¹ and N² is, independently, a nucleobase;

each of R⁹, R¹⁰, R¹¹, R¹², R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

each of g and h is, independently, 0 or 1;

each X¹ and X⁴ is, independently, O, NH, or S;

each X² is independently O or S; and

each X³ is independently OH or SH, or a salt thereof;

each of R¹⁷ and R¹⁹ is, independently, a region of linked nucleosides; and

R¹⁸ is a halogen.

Chimeric polynucleotides may include the structure of Formula VIIIa or VIIIb:

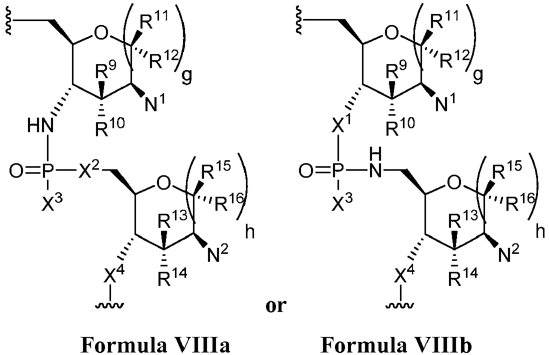

This method includes reacting (e.g., under Staudinger reaction conditions) a compound having the structure of Formula IXa or IXb: 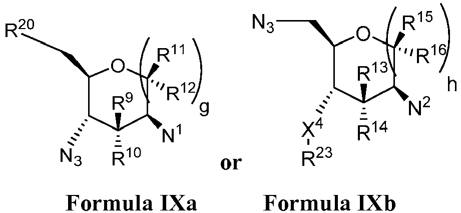 with a compound having the structure of Formula Xa or Xb:

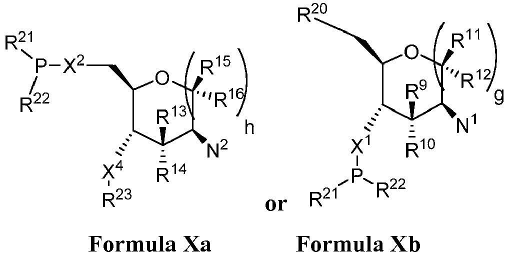

wherein each of N¹ and N² is, independently, a nucleobase;

each of R⁹, R¹⁰, R¹¹, R¹², R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

each of g and h is, independently, 0 or 1;

each X⁴ is, independently, O, NH, or S; and

each X¹ and X² is independently O or S;

each X³ is independently OH, SH, or a salt thereof;

each of R²⁰ and R²³ is, independently, a region of linked nucleosides; and

each of R²¹ and R²² is, independently, optionally substituted C₁-C₆ alkoxy.

Chimeric polynucleotides of the disclosure including the structure of Formula XIa, XIb, XIIa, or XIIa: 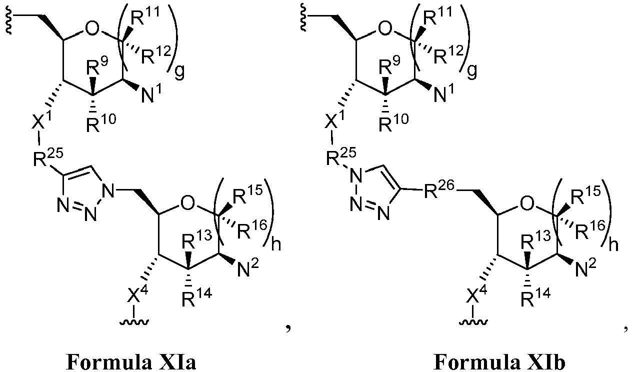 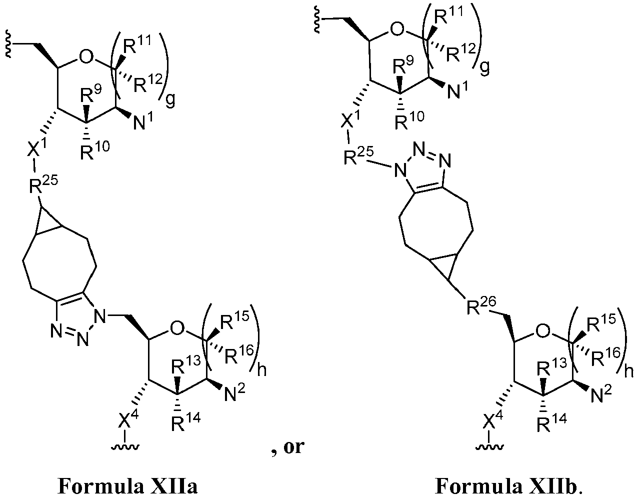 This method includes reacting (e.g., under [3+2] cycloaddition conditions in the presence or absence of a copper source) a compound having the structure of Formula XIIIa, XIIIb, XIVa, or XIVb: 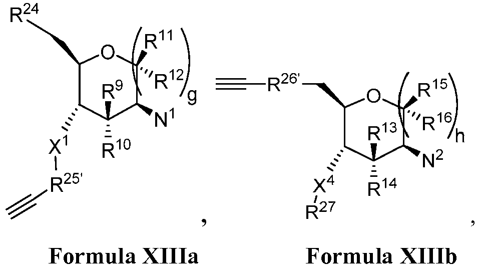 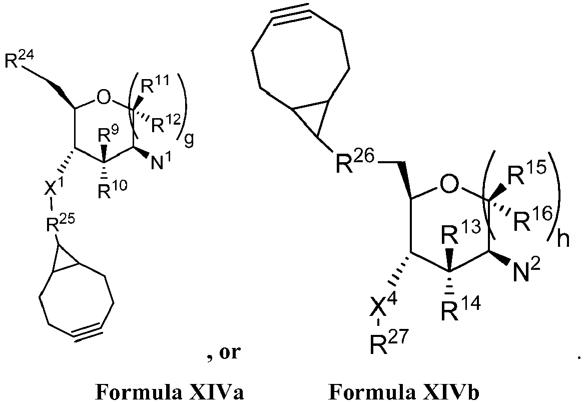 with a compound having the structure of Formula XVa or XVb:

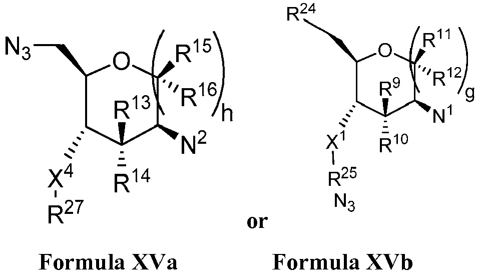

wherein each of N¹ and N² is, independently, a nucleobase;

each of R⁹, R¹⁰, R¹¹, R¹², R¹³, R¹⁴, R¹⁵, and R¹⁶ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;

each of g and h is, independently, 0 or 1;

each X¹ and X⁴ is, independently, absent, O, NH, or S or a salt thereof;

each of R²⁴ and R²⁷ is, independently, a region of linked nucleosides; and

each of R²⁵, R^{25'}, R²⁶ and R^{26'} is independently absent or optionally substituted C₁-C₆ alkylene or optionally substituted C₁-C₆ heteroalkylene or R²⁵ and the alkynyl group together form optionally substituted cycloalkynylene.

Chimeric polynucleotides may be synthesized as shown below:

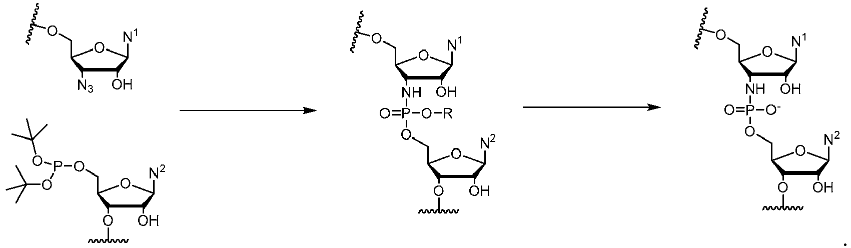

Other methods for the synthesis of the chimeric polynucleotides are shown below: 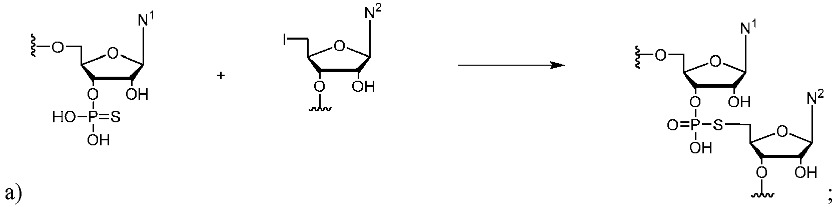 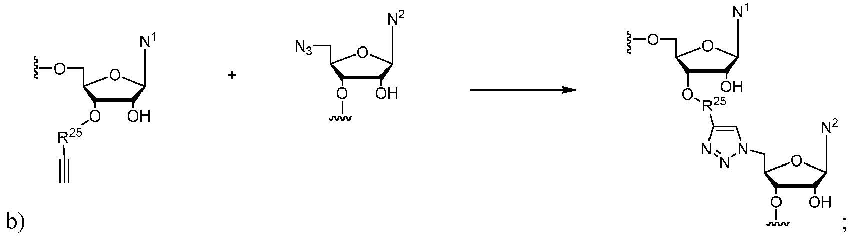 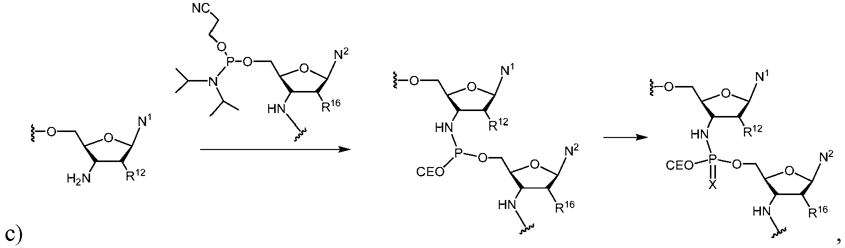 where CEO is 2-cyanoethoxy, and X is O or S.

Other methods for the synthesis of the chimeric polynucleotides of the disclosure are shown below:

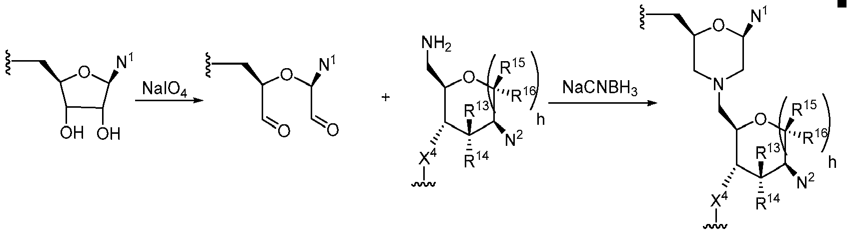

It will be understood that the reactive group shown at the 3' (or 4' position, when g or h is 1) and at the 5' (or 6' position, when g or h is 1) can be reversed. For example, the halogen, azido, or alkynyl group may be attached to the 5' position (or 6' position, when g or h is 1), and the thiophosphate, (thio)phosphoryl, or azido group may be attached to the 3' position (or 4' position, when g or h is 1).

### Quantification

In one case, the chimeric polynucleotides may be quantified in exosomes or when derived from one or more bodily fluid. As used herein "bodily fluids" include peripheral blood, serum, plasma, ascites, urine, cerebrospinal fluid (CSF), sputum, saliva, bone marrow, synovial fluid, aqueous humor, amniotic fluid, cerumen, breast milk, broncheoalveolar lavage fluid, semen, prostatic fluid, cowper's fluid or pre-ejaculatory fluid, sweat, fecal matter, hair, tears, cyst fluid, pleural and peritoneal fluid, pericardial fluid, lymph, chyme, chyle, bile, interstitial fluid, menses, pus, sebum, vomit, vaginal secretions, mucosal secretion, stool water, pancreatic juice, lavage fluids from sinus cavities, bronchopulmonary aspirates, blastocyl cavity fluid, and umbilical cord blood. Alternatively, exosomes may be retrieved from an organ selected from the group consisting of lung, heart, pancreas, stomach, intestine, bladder, kidney, ovary, testis, skin, colon, breast, prostate, brain, esophagus, liver, and placenta.

In the exosome quantification method, a sample of not more than 2mL is obtained from the subject and the exosomes isolated by size exclusion chromatography, density gradient centrifugation, differential centrifugation, nanomembrane ultrafiltration, immunoabsorbent capture, affinity purification, microfluidic separation, or combinations thereof. In the analysis, the level or concentration of a chimeric polynucleotide may be an expression level, presence, absence, truncation or alteration of the administered construct. It is advantageous to correlate the level with one or more clinical phenotypes or with an assay for a human disease biomarker. The assay may be performed using construct specific probes, cytometry, qRT-PCR, real-time PCR, PCR, flow cytometry, electrophoresis, mass spectrometry, or combinations thereof while the exosomes may be isolated using immunohistochemical methods such as enzyme linked immunosorbent assay (ELISA) methods. Exosomes may also be isolated by size exclusion chromatography, density gradient centrifugation, differential centrifugation, nanomembrane ultrafiltration, immunoabsorbent capture, affinity purification, microfluidic separation, or combinations thereof.

These methods afford the investigator the ability to monitor, in real time, the level of chimeric polynucleotides remaining or delivered. This is possible because disclosure the chimeric polynucleotides of the present disclosure differ from the endogenous forms due to the structural or chemical modifications.

In one case, the chimeric polynucleotide may be quantified using methods such as, but not limited to, ultraviolet visible spectroscopy (UV/Vis). A non-limiting example of a UV/Vis spectrometer is a NANODROP^{®} spectrometer (ThermoFisher, Waltham, MA). The quantified chimeric polynucleotide may be analyzed in order to determine if the chimeric polynucleotide may be of proper size, check that no degradation of the chimeric polynucleotide has occurred. Degradation of the chimeric polynucleotide may be checked by methods such as, but not limited to, agarose gel electrophoresis, HPLC based purification methods such as, but not limited to, strong anion exchange HPLC, weak anion exchange HPLC, reverse phase HPLC (RP-HPLC), and hydrophobic interaction HPLC (HIC-HPLC), liquid chromatography-mass spectrometry (LCMS), capillary electrophoresis (CE) and capillary gel electrophoresis (CGE).

### Purification

Chimeric polynucleotide purification may include, but is not limited to, polynucleotide clean-up, quality assurance and quality control. Clean-up may be performed by methods known in the arts such as, but not limited to, AGENCOURT^{®} beads (Beckman Coulter Genomics, Danvers, MA), poly-T beads, LNA^{™} oligo-T capture probes (EXIQON^{®} Inc, Vedbaek, Denmark) or HPLC based purification methods such as, but not limited to, strong anion exchange HPLC, weak anion exchange HPLC, reverse phase HPLC (RP-HPLC), and hydrophobic interaction HPLC (HIC-HPLC). The term "purified" when used in relation to a polynucleotide such as a "purified chimeric polynucleotide" refers to one that is separated from at least one contaminant. As used herein, a "contaminant" is any substance which makes another unfit, impure or inferior. Thus, a purified polynucleotide (e.g., DNA and RNA) is present in a form or setting different from that in which it is found in nature, or a form or setting different from that which existed prior to subjecting it to a treatment or purification method.

A quality assurance and/or quality control check may be conducted using methods such as, but not limited to, gel electrophoresis, UV absorbance, or analytical HPLC.

In another case, the chimeric polynucleotide may be sequenced by methods including, but not limited to reverse-transcriptase-PCR.

### III. Modifications

As used herein in a polynucleotide (such as a chimeric polynucleotide, whether coding or noncoding), the terms "chemical modification" or, as appropriate, "chemically modified" refer to modification with respect to adenosine (A), guanosine (G), uridine (U), thymidine (T) or cytidine (C) ribo- or deoxyribnucleosides in one or more of their position, pattern, percent or population. Generally, herein, these terms are not intended to refer to the ribonucleotide modifications in naturally occurring 5'-terminal mRNA cap moieties.

In a polypeptide, the term "modification" refers to a modification as compared to the canonical set of 20 amino acids.

The modifications may be various distinct modifications. In some cases. the regions may contain one, two, or more (optionally different) nucleoside or nucleotide modifications. In some case, a modified chimeric polynucleotide, introduced to a cell may exhibit reduced degradation in the cell, as compared to an unmodified polynucleotide.

Modifications which are useful in the present disclosure include, but are not limited to those in Table 2. Noted in the table are the symbol of the modification, the nucleobase type and whether the modification is naturally occurring or not.

**Table 2. Modifications**

| **Name** | **Symbol** | **Base** | **Naturally Occurring** |
|---|---|---|---|
| 2-methylthio-N6-(cis-hydroxyisopentenyl)adenosine | ms2i6A | A | YES |
| 2-methylthio-N6-methyladenosine | ms2m6A | A | YES |
| 2-methylthio-N6-threonyl carbamoyladenosine | ms2t6A | A | YES |
| N6-glycinylcarbamoyladenosine | g6A | A | YES |
| N6-isopentenyladenosine | i6A | A | YES |
| N6-methyladenosine | m6A | A | YES |
| N6-threonylcarbamoyladenosine | t6A | A | YES |
| 1,2'-O-dimethyladenosine | mlAm | A | YES |
| 1-methyladenosine | mlA | A | YES |
| 2'-O-methyladenosine | Am | A | YES |
| 2'-O-ribosyladenosine (phosphate) | Ar(p) | A | YES |
| 2-methyladenosine | m2A | A | YES |
| 2-methylthio-N6 isopentenyladenosine | ms2i6A | A | YES |
| 2-methylthio-N6-hydroxynorvalyl carbamoyladenosine | ms2hn6A | A | YES |
| 2'-O-methyladenosine | m6A | A | YES |
| 2'-O-ribosyladenosine (phosphate) | Ar(p) | A | YES |
| isopentenyladenosine | Iga | A | YES |
| N6-(cis-hydroxyisopentenyl)adenosine | io6A | A | YES |
| N6,2'-O-dimethyladenosine | m6Am | A | YES |
| N⁶,2'-O-dimethyladenosine | m⁶Am | A | YES |
| N6,N6,2'-O-trimethyladenosine | m62Am | A | YES |
| N6,N6-dimethyladenosine | m62A | A | YES |
| N6-acetyladenosine | ac6A | A | YES |
| N6-hydroxynorvalylcarbamoyladenosine | hn6A | A | YES |
| N6-methyl-N6-threonylcarbamoyladenosine | m6t6A | A | YES |
| 2-methyladenosine | m²A | A | YES |
| 2-methylthio-N⁶-isopentenyladenosine | ms⁵i⁶A | A | YES |
| 7-deaza-adenosine | -- | A | NO |
| N1-methyl-adenosine | -- | A | NO |
| N6, N6 (dimethyl)adenine | -- | A | NO |
| N6-cis-hydroxy-isopentenyl-adenosine | -- | A | NO |
| α-thio-adenosine | -- | A | NO |
| 2 (amino)adenine | -- | A | NO |
| 2 (aminopropyl)adenine | -- | A | NO |
| 2 (methylthio) N6 (isopentenyl)adenine | -- | A | NO |
| 2-(alkyl)adenine | -- | A | NO |
| 2-(aminoalkyl)adenine | -- | A | NO |
| 2-(aminopropyl)adenine | -- | A | NO |
| 2-(halo)adenine | -- | A | NO |
| 2-(halo)adenine | -- | A | NO |
| 2-(propyl)adenine | -- | A | NO |
| 2'-Amino-2'-deoxy-ATP | -- | A | NO |
| 2'-Azido-2'-deoxy-ATP | -- | A | NO |
| 2'-Deoxy-2'-a-aminoadenosine TP | -- | A | NO |
| 2'-Deoxy-2'-a-azidoadenosine TP | -- | A | NO |
| 6 (alkyl)adenine | -- | A | NO |
| 6 (methyl)adenine | -- | A | NO |
| 6-(alkyl)adenine | -- | A | NO |
| 6-(methyl)adenine | -- | A | NO |
| 7 (deaza)adenine | -- | A | NO |
| 8 (alkenyl)adenine | -- | A | NO |
| 8 (alkynyl)adenine | -- | A | NO |
| 8 (amino)adenine | -- | A | NO |
| 8 (thioalkyl)adenine | -- | A | NO |
| 8-(alkenyl)adenine | -- | A | NO |
| 8-(alkyl)adenine | -- | A | NO |
| 8-(alkynyl)adenine | -- | A | NO |
| 8-(amino)adenine | -- | A | NO |
| 8-(halo)adenine | -- | A | NO |
| 8-(hydroxyl)adenine | -- | A | NO |
| 8-(thioalkyl)adenine | -- | A | NO |
| 8-(thiol)adenine | -- | A | NO |
| 8-azido-adenosine | -- | A | NO |
| aza adenine | -- | A | NO |
| deaza adenine | -- | A | NO |
| N6 (methyl)adenine | -- | A | NO |
| N6-(isopentyl)adenine | -- | A | NO |
| 7-deaza-8-aza-adenosine | -- | A | NO |
| 7-methyladenine | -- | A | NO |
| 1 -Deazaadenosine TP | -- | A | NO |
| 2'Fluoro-N6-Bz-deoxyadenosine TP | -- | A | NO |
| 2'-OMe-2-Amino-ATP | - | A | NO |
| 2'O-methyl-N6-Bz-deoxyadenosine TP | -- | A | NO |
| 2'-a-Ethynyladenosine TP | -- | A | NO |
| 2-aminoadenine | -- | A | NO |
| 2-Aminoadenosine TP | -- | A | NO |
| 2-Amino-ATP | -- | A | NO |
| 2'-a-Trifluoromethyladenosine TP | -- | A | NO |
| 2-Azidoadenosine TP | -- | A | NO |
| 2'-b-Ethynyladenosine TP | -- | A | NO |
| 2-Bromoadenosine TP | -- | A | NO |
| 2'-b-Trifluoromethyladenosine TP | -- | A | NO |
| 2-Chloroadenosine TP | - | A | NO |
| 2'-Deoxy-2',2'-difluoroadenosine TP | -- | A | NO |
| 2'-Deoxy-2'-a-mercaptoadenosine TP | -- | A | NO |
| 2'-Deoxy-2'-a-thiomethoxyadenosine TP | -- | A | NO |
| 2'-Deoxy-2'-b-aminoadenosine TP | -- | A | NO |
| 2'-Deoxy-2'-b-azidoadenosine TP | -- | A | NO |
| 2'-Deoxy-2'-b-bromoadenosine TP | -- | A | NO |
| 2'-Deoxy-2'-b-chloroadenosine TP | -- | A | NO |
| 2'-Deoxy-2'-b-fluoroadenosine TP | -- | A | NO |
| 2'-Deoxy-2'-b-iodoadenosine TP | -- | A | NO |
| 2'-Deoxy-2'-b-mercaptoadenosine TP | -- | A | NO |
| 2'-Deoxy-2'-b-thiomethoxyadenosine TP | -- | A | NO |
| 2-Fluoroadenosine TP | -- | A | NO |
| 2-Iodoadenosine TP | -- | A | NO |
| 2-Mercaptoadenosine TP | -- | A | NO |
| 2-methoxy-adenine | -- | A | NO |
| 2-methylthio-adenine | -- | A | NO |
| 2-Trifluoromethyladenosine TP | -- | A | NO |
| 3-Deaza-3-bromoadenosine TP | -- | A | NO |
| 3-Deaza-3-chloroadenosine TP | -- | A | NO |
| 3-Deaza-3-fluoroadenosine TP | -- | A | NO |
| 3-Deaza-3-iodoadenosine TP | -- | A | NO |
| 3-Deazaadenosine TP | -- | A | NO |
| 4'-Azidoadenosine TP | -- | A | NO |
| 4'-Carbocyclic adenosine TP | -- | A | NO |
| 4'-Ethynyladenosine TP | -- | A | NO |
| 5'-Homo-adenosine TP | -- | A | NO |
| 8-Aza-ATP | -- | A | NO |
| 8-bromo-adenosine TP | -- | A | NO |
| 8-Trifluoromethyladenosine TP | -- | A | NO |
| 9-Deazaadenosine TP | -- | A | NO |
| 2-aminopurine | -- | A/G | NO |
| 7-deaza-2,6-diaminopurine | -- | A/G | NO |
| 7-deaza-8-aza-2,6-diaminopurine | -- | A/G | NO |
| 7-deaza-8-aza-2-aminopurine | -- | A/G | NO |
| 2,6-diaminopurine | -- | A/G | NO |
| 7-deaza-8-aza-adenine, 7-deaza-2-aminopurine | -- | A/G | NO |
| 2-thiocytidine | s2C | C | YES |
| 3-methylcytidine | m3C | C | YES |
| 5-formylcytidine | f5C | C | YES |
| 5-hydroxymethylcytidine | hm5C | C | YES |
| 5-methylcytidine | m5C | C | YES |
| N4-acetylcytidine | ac4C | C | YES |
| 2'-O-methylcytidine | Cm | C | YES |
| 5,2'-O-dimethylcytidine | m5 Cm | C | YES |
| 5-formyl-2'-O-methylcytidine | f5Cm | C | YES |
| lysidine | k2C | C | YES |
| N4,2'-O-dimethylcytidine | m4Cm | C | YES |
| N4-acetyl-2'-O-methylcytidine | ac4Cm | C | YES |
| N4-methylcytidine | m4C | C | YES |
| N4,N4-Dimethyl-2'-OMe-Cytidine TP | -- | C | YES |
| 4-methylcytidine | -- | C | NO |
| 5-aza-cytidine | -- | C | NO |
| Pseudo-iso-cytidine | -- | C | NO |
| pyrrolo-cytidine | -- | C | NO |
| α-thio-cytidine | -- | C | NO |
| 2-(thio)cytosine | -- | C | NO |
| 2'-Amino-2'-deoxy-CTP | -- | C | NO |
| 2'-Azido-2'-deoxy-CTP | -- | C | NO |
| 2'-Deoxy-2'-a-aminocytidine TP | -- | C | NO |
| 2'-Deoxy-2'-a-azidocytidine TP | -- | C | NO |
| 3 (deaza) 5 (aza)cytosine | -- | C | NO |
| 3 (methyl)cytosine | -- | C | NO |
| 3-(alkyl)cytosine | -- | C | NO |
| 3-(deaza) 5 (aza)cytosine | -- | C | NO |
| 3-(methyl)cytidine | -- | C | NO |
| 4,2'-O-dimethylcytidine | -- | C | NO |
| 5 (halo)cytosine | -- | C | NO |
| 5 (methyl)cytosine | -- | C | NO |
| 5 (propynyl)cytosine | -- | C | NO |
| 5 (trifluoromethyl)cytosine | -- | C | NO |
| 5-(alkyl)cytosine | -- | C | NO |
| 5-(alkynyl)cytosine | -- | C | NO |
| 5-(halo)cyto sine | -- | C | NO |
| 5 -(propynyl)cytosine | -- | C | NO |
| 5-(trifluoromethyl)cytosine | -- | C | NO |
| 5-bromo-cytidine | -- | C | NO |
| 5-iodo-cytidine | -- | C | NO |
| 5-propynyl cytosine | -- | C | NO |
| 6-(azo)cytosine | -- | C | NO |
| 6-aza-cytidine | -- | C | NO |
| aza cytosine | -- | C | NO |
| deaza cytosine | -- | C | NO |
| N4 (acetyl)cytosine | -- | C | NO |
| 1-methyl-1-deaza-pseudoisocytidine | -- | C | NO |
| 1-methyl-pseudoisocytidine | -- | C | NO |
| 2-methoxy-5-methyl-cytidine | -- | C | NO |
| 2-methoxy-cytidine | -- | C | NO |
| 2-thio-5-methyl-cytidine | -- | C | NO |
| 4-methoxy-1-methyl-pseudoisocytidine | -- | C | NO |
| 4-methoxy-pseudoisocytidine | -- | C | NO |
| 4-thio-1-methyl-1-deaza-pseudoisocytidine | -- | C | NO |
| 4-thio-1-methyl-pseudoisocytidine | -- | C | NO |
| 4-thio-pseudoisocytidine | -- | C | NO |
| 5-aza-zebularine | -- | C | NO |
| 5-methyl-zebularine | -- | C | NO |
| pyrrolo-pseudoisocytidine | -- | C | NO |
| zebularine | -- | C | NO |
| (E)-5-(2-Bromo-vinyl)cytidine TP | -- | C | NO |
| 2,2'-anhydro-cytidine TP hydrochloride | -- | C | NO |
| 2'Fluor-N4-Bz-cytidine TP | - | C | NO |
| 2'Fluoro-N4-Acetyl-cytidine TP | -- | C | NO |
| 2'-O-Methyl-N4-Acetyl-cytidine TP | -- | C | NO |
| 2'O-methyl-N4-Bz-cytidine TP | -- | C | NO |
| 2'-a-Ethynylcytidine TP | -- | C | NO |
| 2'-a-Trifluoromethylcytidine TP | -- | C | NO |
| 2'-b-Ethynylcytidine TP | -- | C | NO |
| 2'-b-Trifluoromethylcytidine TP | -- | C | NO |
| 2'-Deoxy-2',2'-difluorocytidine TP | -- | C | NO |
| 2'-Deoxy-2'-a-mercaptocytidine TP | -- | C | NO |
| 2'-Deoxy-2'-a-thiomethoxycytidine TP | -- | C | NO |
| 2'-Deoxy-2'-b-aminocytidine TP | -- | C | NO |
| 2'-Deoxy-2'-b-azidocytidine TP | -- | C | NO |
| 2'-Deoxy-2'-b-bromocytidine TP | -- | C | NO |
| 2'-Deoxy-2'-b-chlorocytidine TP | -- | C | NO |
| 2'-Deoxy-2'-b-fluorocytidine TP | -- | C | NO |
| 2'-Deoxy-2'-b-iodocytidine TP | -- | C | NO |
| 2'-Deoxy-2'-b-mercaptocytidine TP | -- | C | NO |
| 2'-Deoxy-2'-b-thiomethoxycytidine TP | -- | C | NO |
| 2'-O-Methyl-5-(1-propynyl)cytidine TP | -- | C | NO |
| 3'-Ethynylcytidine TP | -- | C | NO |
| 4'-Azidocytidine TP | -- | C | NO |
| 4'-Carbocyclic cytidine TP | -- | C | NO |
| 4'-Ethynylcytidine TP | -- | C | NO |
| 5-(1-Propynyl)ara-cytidine TP | -- | C | NO |
| 5-(2-Chloro-phenyl)-2-thiocytidine TP | -- | C | NO |
| 5-(4-Amino-phenyl)-2-thiocytidine TP | -- | C | NO |
| 5-Aminoallyl-CTP | -- | C | NO |
| 5-Cyanocytidine TP | -- | C | NO |
| 5-Ethynylara-cytidine TP | -- | C | NO |
| 5-Ethynylcytidine TP | -- | C | NO |
| 5'-Homo-cvtidine TP | -- | C | NO |
| 5-Methoxycytidine TP | -- | C | NO |
| 5-Trifluoromethyl-Cytidine TP | -- | C | NO |
| N4-Amino-cytidine TP | -- | C | NO |
| N4-Benzoyl-cytidine TP | -- | C | NO |
| pseudoisocytidine | -- | C | NO |
| 7-methylguanosine | m7G | G | YES |
| N2,2'-O-dimethylguanosine | m2Gm | G | YES |
| N2-methylguanosine | m2G | G | YES |
| wyosine | imG | G | YES |
| 1,2'-O-dimethylguanosine | m1Gm | G | YES |
| 1-methylpuanosine | m1G | G | YES |
| 2'-O-methylguanosine | Gm | G | YES |
| 2'-O-ribosylguanosine (phosphate) | Gr(p) | G | YES |
| 2'-O-methylguanosine | Gm | G | YES |
| 2'-O-ribosylguanosine (phosphate) | Gr(p) | G | YES |
| 7-ammomethyl-7-deazaguanosine | preQ1 | G | YES |
| 7-cyano-7-deazaguanosine | preQ0 | G | YES |
| archaeosine | G+ | G | YES |
| methylwyosine | mimG | G | YES |
| N2,7-dimethylguanosine | m2,7G | G | YES |
| N2,N2,2'-O-trimethylguanosine | m22Gm | G | YES |
| N2,N2,7-trimethylguanosine | m2,2,7G | G | YES |
| N2,N2-dimethylguanosine | m22G | G | YES |
| N²,7,2'-O-trimethylguanosine | m^{2,7}Gm | G | YES |
| 6-thio-guanosine | -- | G | NO |
| 7-deaza-guanosine | -- | G | NO |
| 8-oxo-guanosine | -- | G | NO |
| N1-methyl-guanosine | -- | G | NO |
| α-thio-guanosine | -- | G | NO |
| 2 (propyl)guanine | -- | G | NO |
| 2-(alkyl)guanine | -- | G | NO |
| 2'-Amino-2'-deoxy-GTP | -- | G | NO |
| 2'-Azido-2'-deoxy-GTP | -- | G | NO |
| 2'-Deoxy-2'-a-aminoguanosine TP | -- | G | NO |
| 2'-Deoxv-2'-a-azidoguanosine TP | -- | G | NO |
| 6 (methyl)guanine | -- | G | NO |
| 6-(alkyl)guanine | -- | G | NO |
| 6-(methyl)guanine | -- | G | NO |
| 6-methyl-guanosine | -- | G | NO |
| 7 (alkyl)guanine | -- | G | NO |
| 7 (deaza)guanine | -- | G | NO |
| 7 (methyl)guanine | -- | G | NO |
| 7-(alkyl)guanine | -- | G | NO |
| 7-(deaza)guanine | -- | G | NO |
| 7 -(methyl)guanine | -- | G | NO |
| 8 (alkyl)guanine | -- | G | NO |
| 8 (alkynyl)guanine | -- | G | NO |
| 8 (halo)guanine | -- | G | NO |
| 8 (thioalkyl)guanine | -- | G | NO |
| 8-(alkenyl)guanine | -- | G | NO |
| 8-(alkyl)guanine | -- | G | NO |
| 8-(alkynyl)guanine | -- | G | NO |
| 8-(amino)guanine | -- | G | NO |
| 8-(halo)guanine | -- | G | NO |
| 8-(hydroxyl)guanine | -- | G | NO |
| 8-(thioalkyl)guanine | -- | G | NO |
| 8-(thiol)guanine | -- | G | NO |
| aza guanine | -- | G | NO |
| deaza guanine | -- | G | NO |
| N-(methyl)guanine | -- | G | NO |
| 1-methyl-6-thio-guanosine | -- | G | NO |
| 6-methoxy-guanosine | -- | G | NO |
| 6-thio-7-deaza-8-aza-guanosine | -- | G | NO |
| 6-thio-7-deaza-guanosine | -- | G | NO |
| 6-thio-7-methyl-guanosine | -- | G | NO |
| 7-deaza-8-aza-guanosine | -- | G | NO |
| 7-methyl-8-oxo-guanosine | -- | G | NO |
| N2,N2-dimethyl-6-thio-guanosine | -- | G | NO |
| N2-methyl-6-thio-guanosine | -- | G | NO |
| 1-Me-GTP | -- | G | NO |
| 2'Fluoro-N2-isobutyl-guanosine TP | -- | G | NO |
| 2'O-methyl-N2-isobutyl-guanosine TP | -- | G | NO |
| 2 '-a-Ethynylguanosine TP | -- | G | NO |
| 2'-a-Trifluoromethylguanosine TP | -- | G | NO |
| 2'-b-Ethynylguanosine TP | - | G | NO |
| 2'-b-Trifluoromethylguanosine TP | -- | G | NO |
| 2'-Deoxy-2',2'-difluoroguanosine TP | -- | G | NO |
| 2'-Deoxy-2'-a-mercaptoguanosine TP | -- | G | NO |
| 2'-Deoxy-2'-a-thiomethoxyguanosine TP | -- | G | NO |
| 2'-Deoxy-2'-b-aminoguanosine TP | -- | G | NO |
| 2'-Deoxy-2'-b-azidoguanosine TP | -- | G | NO |
| 2'-Deoxy-2'-b-bromoguanosine TP | -- | G | NO |
| 2'-Deoxy-2'-b-chloroguanosine TP | -- | G | NO |
| 2'-Deoxy-2'-b-fluoroguanosine TP | -- | G | NO |
| 2'-Deoxy-2'-b-iodoguanosine TP | -- | G | NO |
| 2'-Deoxy-2'-b-mercaptoguanosine TP | -- | G | NO |
| 2'-Deoxy-2'-b-thiomethoxyguanosine TP | -- | G | NO |
| 4'-Azidoguanosine TP | -- | G | NO |
| 4'-Carbocyclic guanosine TP | -- | G | NO |
| 4'-Etbynylguanosine TP | -- | G | NO |
| 5'-Homo-guanosine TP | -- | G | NO |
| 8-bromo-guanosine TP | -- | G | NO |
| 9-Deazaguanosine TP | -- | G | NO |
| N2-isobutyl-guanosine TP | -- | G | NO |
| 1-methylinosine | m1I | I | YES |
| inosine | I | I | YES |
| 1,2'-O-dimethylinosine | m1Im | I | YES |
| 2'-O-methylinosine | Im | I | YES |
| 7-methylinosine | | I | NO |
| 2'-O-methylinosine | Im | I | YES |
| epoxyqueuosine | oQ | Q | YES |
| galactosyl-queuosine | galQ | Q | YES |
| mannosylqueuosine | manQ | Q | YES |
| queuosine | Q | Q | YES |
| allyamino-thymidine | -- | T | NO |
| aza thymidine | | T | NO |
| deaza thymidine | -- | T | NO |
| deoxy-thymidine | -- | T | NO |
| 2'-O-methyluridine | -- | U | YES |
| 2-thiouridine | s2U | U | YES |
| 3-methyluridine | m3U | U | YES |
| 5-carboxymethyluridine | cm5U | U | YES |
| 5-hydroxyuridine | ho5U | U | YES |
| 5-methyluridine | m5U | U | YES |
| 5-taurinomethyl-2-thiouridine | τm5s2U | U | YES |
| 5-taurinomethyluridine | τm5U | U | YES |
| dihydrouridine | D | U | YES |
| pseudouridine | Ψ | U | YES |
| (3-(3-amino-3-carboxypropyl)uridine | acp3U | U | YES |
| 1-methyl-3-(3-amino-5-carboxypropyl)pseudouridine | m1acp3Ψ | U | YES |
| 1-methylpseduouridine | m1Ψ | U | YES |
| 2'-O-methyluridine | Um | U | YES |
| 2'-O-methylpseudouridine | Ψm | U | YES |
| 2-thio-2'-O-methyluridine | s2Um | U | YES |
| 3-(3-amino-3-carboxypropyl)uridine | acp3U | U | YES |
| 3,2'-O-dimethyluridine | m3Um | U | YES |
| 3-Methyl-pseudo-Uridine TP | -- | U | YES |
| 4-thiouridine | s4U | U | YES |
| 5 -(carboxyhydroxymethyl)uridine | chm5U | U | YES |
| 5-(carboxyhydroxymethyl)uridine methyl ester | mchm5U | U | YES |
| 5,2'-O-dimethyluridine | m5Um | U | YES |
| 5,6-dihydro-uridine | -- | U | YES |
| 5-aminomethyl-2-thiouridine | nm5s2U | U | YES |
| 5-carbamoylmethyl-2'-O-methyluridine | ncm5Um | U | YES |
| 5-carbamoylmethyluridine | ncm5U | U | YES |
| 5-carboxyhydroxymethyluridine | -- | U | YES |
| 5-carboxyhydroxymethyluridine methyl ester | -- | U | YES |
| 5-carboxymethylaminomethyl-2'-O-methyluridine | cmnm5Um | U | YES |
| 5-carboxymethylaminomethyl-2-thiouridine | cmnm5s2U | U | YES |
| 5-carboxymethylaminomethyluridine | cmnm5U | U | YES |
| 5-Carbamoylmethyluridine TP | -- | U | YES |
| 5-methoxycarbonylmethyl-2'-O-methyluridine | mcm5Um | U | YES |
| 5-methoxycarbonylmethyl-2-thiouridine | mcm5s2U | U | YES |
| 5-methoxycarbonylmethyluridine | mcm5U | U | YES |
| 5-methoxyuridine | mo5U | U | YES |
| 5-methyl-2-thiouridine | m5s2U | U | YES |
| 5-methylaminomethyl-2-selenouridine | mnm5se2U | U | YES |
| 5-methylaminomethyl-2-thiouridine | mnm5s2U | U | YES |
| 5-methylaminomethyluridine | mnm5U | U | YES |
| 5-Methyldihydrouridine | -- | U | YES |
| 5-Oxvacetic acid- Uridine TP | -- | U | YES |
| 5-Oxyacetic acid-methyl ester-Uridine TP | -- | U | YES |
| N1-methyl-pseudo-uridine | -- | U | YES |
| uridine 5-oxyacetic acid | cmo5U | U | YES |
| uridine 5-oxyacetic acid methyl ester | mcmo5U | U | YES |
| 3 -(3 -Amino-3 -carboxypropyl)-Uridine TP | -- | U | YES |
| 5-(iso-Pentenylaminomethyl)-2-thiouridine TP | -- | U | YES |
| 5-(iso-Pentenylaminomethyl)-2'-O-methyluridine TP | -- | U | YES |
| 5-(iso-Pentenylaminomethyl)uridine TP | -- | U | YES |
| 5-propynyl uracil | -- | U | NO |
| α-thio-uridine | -- | U | NO |
| 1 (aminoalkylamino-carbonylethylenyl)-2(thio)-pseudouracil | -- | U | NO |
| 1 (aminoalkylaminocarbonylethylenyl)-2,4-(dithio)pseudouracil | -- | U | NO |
| 1 (aminoalkylaminocarbonylethylenyl)-4 (thio)pseudouracil | -- | U | NO |
| 1 (aminoalkylaminocarbonylethylenyl)-pseudouracil | -- | U | NO |
| 1 (aminocarbonylethylenyl)-2(thio)-pseudouracil | -- | U | NO |
| 1 (aminocarbonylethylenyl)-2,4-(dithio)pseudouracil | -- | U | NO |
| 1 (aminocarbonylethylenyl)-4 (thio)pseudouracil | -- | U | NO |
| 1 (aminocarbonylethylenyl)-pseudouracil | -- | U | NO |
| 1 substituted 2(thio)-pseudouracil | -- | U | NO |
| 1 substituted 2,4-(dithio)pseudouracil | -- | U | NO |
| 1 substituted 4 (thio)pseudouracil | -- | U | NO |
| 1 substituted pseudouracil | -- | U | NO |
| 1-(aminoalkylamino-carbonylethylenyl)-2-(thio)-pseudouracil | -- | U | NO |
| 1-Methyl-3 -(3-amino-3 -carboxypropyl) pseudouridine TP | -- | U | NO |
| 1-Methyl-3-(3-amino-3-carboxypropyl)pseudo-UTP | -- | U | NO |
| 1-Methyl-pseudo-UTP | -- | U | NO |
| 2 (thio)pseudouracil | -- | U | NO |
| 2' deoxy uridine | -- | U | NO |
| 2' fluorouridine | -- | U | NO |
| 2-(thio)uracil | -- | U | NO |
| 2,4-(dithio)psuedouracil | -- | U | NO |
| 2' methyl, 2'amino, 2'azido, 2'fluro-guanosine | -- | U | NO |
| 2'-Amino-2'-deoxy-UTP | -- | U | NO |
| 2 '-Azido-2'-deoxy-UTP | -- | U | NO |
| 2'-Azido-deoxyuridine TP | -- | U | NO |
| 2'-O-methylpseudouridine | -- | U | NO |
| 2' deoxy uridine | 2'dU | U | NO |
| 2' fluorouridine | -- | U | NO |
| 2'-Deoxy-2'-a-aminouridine TP | -- | U | NO |
| 2'-Deoxy-2'-a-azidouridine TP | -- | U | NO |
| 2-methylpseudouridine | m3Ψ | U | NO |
| 3 (3 amino-3 carboxypropyl)uracil | -- | U | NO |
| 4 (thio)pseudouracil | -- | U | NO |
| 4-thiouracil | -- | U | NO |
| 5 (1,3-diazole-l-alkyl)uracil | -- | U | NO |
| 5 (2-aminopropyl)uracil | -- | U | NO |
| 5 (aminoalkyl)uracil | -- | U | NO |
| 5 (dimethylaminoalkyl)uracil | -- | U | NO |
| 5 (guanidiniumalkyl)uracil | -- | U | NO |
| 5 (methoxycarbonylmethyl)-2-(thio)uracil | -- | U | NO |
| 5 (methoxycarbonyl-methyl)uracil | -- | U | NO |
| 5 (methyl) 2 (thio)uracil | -- | U | NO |
| 5 (methyl) 2,4 (dithio)uracil | -- | U | NO |
| 5 (methyl) 4 (thio)uracil | -- | U | NO |
| 5 (methylaminomethyl)-2 (thio)uracil | -- | U | NO |
| 5 (methylaminomethyl)-2,4 (dithio)uracil | -- | U | NO |
| 5 (methylaminomethyl)-4 (thio)uracil | -- | U | NO |
| 5 (propynyl)uracil | -- | U | NO |
| 5 (trifluoromethyl)uracil | -- | U | NO |
| 5-(2-aminopropyl)uracil | -- | U | NO |
| 5-(alkyl)-2-(thio)pseudouracil | -- | U | NO |
| 5-(alkyl)-2,4 (dithio)pseudouracil | -- | U | NO |
| 5-(alkyl)-4 (thio)pseudouracil | -- | U | NO |
| 5-(alkyl)pseudouracil | -- | U | NO |
| 5 -(alkyl)uracil | -- | U | NO |
| 5-(alkynyl)uracil | -- | U | NO |
| 5-(allylamino)uracil | -- | U | NO |
| 5-(cyanoalkyl)uracil | -- | U | NO |
| 5 -(dialkylaminoalkyl)uracil | -- | U | NO |
| 5-(dimethylaminoalkyl)uracil | -- | U | NO |
| 5-(guanidiniumalkyl)uracil | -- | U | NO |
| 5-(halo)uracil | -- | U | NO |
| 5-(1,3-diazole-1-alkyl)uracil | -- | U | NO |
| 5 -(methoxv)uracil | -- | U | NO |
| 5-(methoxycarbonylmethyl)-2-(thio)uracil | -- | U | NO |
| 5-(methoxycarbonyl-methyl)uracil | -- | U | NO |
| 5-(metbyl) 2(thio)uracil | -- | U | NO |
| 5-(methyl) 2,4 (dithio)uracil | -- | U | NO |
| 5-(methyl) 4 (thio)uracil | -- | U | NO |
| 5-(methyl)-2-(thio)pseudouracil | -- | U | NO |
| 5-(methyl)-2,4 (dithio)pseudouracil | -- | U | NO |
| 5-(methyl)-4 (thio)pseudouracil | -- | U | NO |
| 5-(methyl)pseudouracil | -- | U | NO |
| 5-(methylaminomethyl)-2 (thio)uracil | -- | U | NO |
| 5-(methylaminomethyl)-2,4(dithio)uracil | -- | U | NO |
| 5-(methylaminomethyl)-4-(thio)uracil | -- | U | NO |
| 5-(propynyl)uracil | -- | U | NO |
| 5-(trifluoromethyl)uracil | -- | U | NO |
| 5-aminoallyl-uridine | -- | U | NO |
| 5-bromo-uridine | -- | U | NO |
| 5-iodo-uridine | -- | U | NO |
| 5-uracil | -- | U | NO |
| 6-(azo)uracil | -- | U | NO |
| 6-aza-uridine | -- | U | NO |
| allyamino-uracil | -- | U | NO |
| aza uracil | -- | U | NO |
| deaza uracil | -- | U | NO |
| N3 (methyl)uracil | -- | U | NO |
| P seudo-UTP-1-2-ethanoic acid | -- | U | NO |
| pseudouracil | -- | U | NO |
| 4-Thio-pseudo-UTP | -- | U | NO |
| 1-carboxymethyl-pseudouridine | -- | U | NO |
| 1-methyl-1-deaza-pseudouridine | -- | U | NO |
| 1-propynyl-uridine | -- | U | NO |
| 1-taurinomethyl-1-methyl-uridine | -- | U | NO |
| 1-taurinomethyl-4-thio-uridine | -- | U | NO |
| 1-taurinomethyl-pseudouridine | -- | U | NO |
| 2-methoxy-4-thio-pseudouridine | -- | U | NO |
| 2-thio-1-methyl-1-deaza-pseudouridine | -- | U | NO |
| 2-thio-1-methyl-pseudouridine | -- | U | NO |
| 2-thio-5-aza-uridine | -- | U | NO |
| 2-thio-dihydropseudouridine | -- | U | NO |
| 2-thio-dihydrouridine | -- | U | NO |
| 2-thio-pseudouridine | -- | U | NO |
| 4-methoxy-2-thio-pseudouridine | -- | U | NO |
| 4-methoxy-pseudouridine | -- | U | NO |
| 4-thio-1-methyl-pseudouridine | -- | U | NO |
| 4-thio-pseudouridine | -- | U | NO |
| 5 -aza-uridine | -- | U | NO |
| dihydropseudouridine | -- | U | NO |
| (±)1-(2-Hydroxypropyl)pseudouridine TP | -- | U | NO |
| (2R)-1-(2-Hydroxypropyl)pseudouridine TP | -- | U | NO |
| (2S)-1-(2-Hydroxypropyl)pseudouridine TP | -- | U | NO |
| (E)-5-(2-Bromo-vinyl)ara-uridine TP | -- | U | NO |
| (E)-5-(2-Bromo-vinyl)uridine TP | -- | U | NO |
| (Z)-5-(2-Bromo-vinyl)ara-uridine TP | -- | U | NO |
| (Z)-5-(2-Bromo-vinyl)uridine TP | -- | U | NO |
| 1-(2,2,2-Trifluoroethyl)-pseudo-UTP | -- | U | NO |
| 1-(2,2,3,3,3-Pentafluoropropyl)pseudouridine TP | -- | U | NO |
| 1-(2,2-Diethoxyethyl)pseudouridine TP | -- | U | NO |
| 1-(2,4,6-Trimethylbenzyl)pseudouridine TP | -- | U | NO |
| 1-(2,4,6-Trimethyl-benzyl)pseudo-UTP | -- | U | NO |
| 1-(2,4,6-Trimethyl-phenyl)pseudo-UTP | -- | U | NO |
| 1-(2-Amino-2-carboxyethyl)pseudo-UTP | -- | U | NO |
| 1-(2-Amino-ethyl)pseudo-UTP | -- | U | NO |
| 1-(2-Hydroxyethyl)pseudouridine TP | -- | U | NO |
| 1-(2-Methoxyethyl)pseudouridine TP | -- | U | NO |
| 1-(3,4-Bis-trifluoromethoxybenzyl)pseudouridine TP | -- | U | NO |
| 1-(3,4-Dimethoxybenzyl)pseudouridine TP | -- | U | NO |
| 1-(3-Amino-3-carboxypropyl)pseudo-UTP | -- | U | NO |
| 1-(3-Amino-propyl)pseudo-UTP | -- | U | NO |
| 1-(3-Cyclopropyl-prop-2-ynyl)pseudouridine TP | -- | U | NO |
| 1-(4-Amino-4-carboxybutyl)pseudo-UTP | -- | U | NO |
| 1-(4-Amino-benzyl)pseudo-UTP | -- | U | NO |
| 1-(4-Amino-butyl)pseudo-UTP | -- | U | NO |
| 1-(4-Amino-phenyl)pseudo-UTP | -- | U | NO |
| 1-(4-Azidobenzyl)pseudouridine TP | -- | U | NO |
| 1-(4-Bromobenzyl)pseudouridine TP | -- | U | NO |
| 1-(4-Chlorobenzyl)pseudouiidine TP | -- | U | NO |
| 1-(4-Fluorobenzyl)pseudouridine TP | -- | U | NO |
| 1-(4-Iodobenzyl)pseudouridine TP | -- | U | NO |
| 1-(4-Methanesulfonylbenzyl)pseudouridine TP | -- | U | NO |
| 1-(4-Methoxybenzyl)pseudouridine TP | -- | U | NO |
| 1-(4-Methoxy-phenyl)pseudo-UTP | -- | U | NO |
| 1-(4-Methylbenzyl)pseudouridine TP | -- | U | NO |
| 1-(4-Nitrobenzyl)pseudouridine TP | -- | U | NO |
| 1(4-Nitro-phenyl)pseudo-UTP | -- | U | NO |
| 1-(4-Thiomethoxybenzyl)pseudouridine TP | -- | U | NO |
| 1-(4- Trifluoromethoxybenzyl)pseudouridine TP | -- | U | NO |
| 1-(4-Trifluoromethylbenzyl)pseudouridine TP | -- | U | NO |
| 1-(5-Amino-pentyl)pseudo-UTP | -- | U | NO |
| 1-(6-Amino-hexyl)pseudo-UTP | -- | U | NO |
| 1,6-Dimethyl-pseudo-UTP | -- | U | NO |
| 1-[3-(2- {2-[2-(2-Aminoethoxy)-ethoxy]-ethoxy}-ethoxy)-propionyl]pseudouridine TP | -- | U | NO |
| 1-{3-[2-(2-Aninoethoxy)-ethoxy]-propionyl} pseudouridine TP | -- | U | NO |
| 1-Acetylpseudouridine TP | -- | U | NO |
| 1-Alkyl-6-(1-propynyl)-pseudo-UTP | -- | U | NO |
| 1-Alkyl-6-(2-propynyl)-pseudo-UTP | -- | U | NO |
| 1-Alkyl-6-allyl-pseudo-UTP | -- | U | NO |
| 1-Alkyl-6-ethynyl-pseudo-UTP | -- | U | NO |
| 1-Alkyl-6-homoallyl-pseudo-UTP | -- | U | NO |
| 1-Alkyl-6-vinyl-pseudo-UTP | -- | U | NO |
| 1-Allylpseudouridine TP | -- | U | NO |
| 1-Aminomethyl-pseudo-UTP | -- | U | NO |
| 1-Benzoylpseudouridine TP | -- | U | NO |
| 1-Benzyloxymethylpseudouridine TP | -- | U | NO |
| 1-Benzyl-pseudo-UTP | -- | U | NO |
| 1-Biotinyl-PEG2-pseudouridine TP | -- | U | NO |
| 1-Biotinylpseudouridine TP | -- | U | NO |
| 1-Butyl-pseudo-UTP | -- | U | NO |
| 1-Cyanomethylpseudouridine TP | -- | U | NO |
| 1 -Cyclobutylmethyl-pseudo-UTP | -- | U | NO |
| 1 -Cyclobutyl-pseudo-UTP | -- | U | NO |
| 1 -Cycloheptylmethyl-pseudo-UTP | -- | U | NO |
| 1 -Cycloheptyl-pseudo-UTP | -- | U | NO |
| 1 -Cyclohexylmethyl-pseudo-UTP | -- | U | NO |
| 1 -Cyclohexyl-pseudo-UTP | -- | U | NO |
| 1 -Cyclooctylmethyl-pseudo-UTP | -- | U | NO |
| 1 -Cyclooctyl-pseudo-UTP | -- | U | NO |
| 1 -Cyclopentylmethyl-pseudo-UTP | -- | U | NO |
| 1 -Cyclopentyl-pseudo-UTP | -- | U | NO |
| 1 -Cyclopropylmethyl-pseudo-UTP | -- | U | NO |
| 1 -Cyclopropyl-pseudo-UTP | -- | U | NO |
| 1-Ethyl-pseudo-UTP | -- | U | NO |
| 1-Hexyl-pseudo-UTP | -- | U | NO |
| 1-Homoallylpseudouridine TP | -- | U | NO |
| 1-Hydroxymethylpseudouridine TP | -- | U | NO |
| 1 -iso-propyl-pseudo-UTP | -- | U | NO |
| 1 -Me-2-thio-pseudo-UTP | -- | U | NO |
| 1 -Me-4-thio-pseudo-UTP | -- | U | NO |
| 1 -Me-alpha-thio-pseudo-UTP | -- | U | NO |
| 1-Methanesulfonylmethylpseudouridine TP | -- | U | NO |
| 1-Methoxymethylpseudouridine TP | -- | U | NO |
| 1-Methyl-6-(2,2,2-Trifluoroethyl)pseudo-UTP | -- | U | NO |
| 1-Methyl-6-(4-morpholino)-pseudo-UTP | -- | U | NO |
| 1-Methyl-6-(4-thiomorpholino)-pseudo-UTP | -- | U | NO |
| 1 -Methyl-6-(substituted phenyl)pseudo-UTP | -- | U | NO |
| 1 -Methyl-6-amino-pseudo-UTP | -- | U | NO |
| 1 -Methyl-6-azido-pseudo-UTP | -- | U | NO |
| 1 -Methyl-6-bromo-pseudo-UTP | -- | U | NO |
| 1 -Methyl-6-butyl-pseudo-UTP | -- | U | NO |
| 1 -Methyl-6-chloro-pseudo-UTP | -- | U | NO |
| 1 -Methyl-6-cyano-pseudo-UTP | -- | U | NO |
| 1-Methyl-6-dimethylamino-pseudo-UTP | -- | U | NO |
| 1 -Methyl-6-ethoxy-pseudo-UTP | -- | U | NO |
| 1-Methyl-6-ethylcarboxylate-pseudo-UTP | -- | U | NO |
| 1 -Methyl-6-ethyl-pseudo-UTP | -- | U | NO |
| 1 -Methyl-6-fluoro-pseudo-UTP | -- | U | NO |
| 1 -Methyl-6-formyl-pseudo-UTP | -- | U | NO |
| 1-Methyl-6-hydroxyamino-pseudo-UTP | -- | U | NO |
| 1 -Methyl-6-hydroxy-pseudo-UTP | -- | U | NO |
| 1 -Methyl-6-iodo-pseudo-UTP | -- | U | NO |
| 1-Methyl-6-iso-propyl-pseudo-UTP | -- | U | NO |
| 1 -Methyl-6-methoxy-pseudo-UTP | -- | U | NO |
| 1-Methyl-6-methylamino-pseudo-UTP | -- | U | NO |
| 1 -Methyl-6-phenyl-pseudo-UTP | -- | U | NO |
| 1 -Methyl-6-propyl-pseudo-UTP | -- | U | NO |
| 1 -Methyl-6-tert-butyl-pseudo-UTP | -- | U | NO |
| 1-Methyl-6-trifluoromethoxy-pseudo-UTP | -- | U | NO |
| 1-Methyl-6-trifluoromethyl-pseudo-UTP | -- | U | NO |
| 1-Morpholinomethylpseudouridine TP | -- | U | NO |
| 1-Pentyl-pseudo-UTP | -- | U | NO |
| 1-Phenyl-pseudo-UTP | -- | U | NO |
| 1 -Pivaloylpseudouridine TP | -- | U | NO |
| 1-Propargylpseudouridine TP | -- | U | NO |
| 1-Propyl-pseudo-UTP | -- | U | NO |
| 1-propynyl-pseudouridine | -- | U | NO |
| 1 -p-tolyl-pseudo-UTP | -- | U | NO |
| 1 -tert-Butyl-pseudo-UTP | -- | U | NO |
| 1 -Thiomethoxymethylpseudouridine TP | -- | U | NO |
| 1 -Thiomorpholinomethylpseudouridine TP | -- | U | NO |
| 1-Trifluoroacetylpseudouridine TP | -- | U | NO |
| 1-Trifluoromethyl-pseudo-UTP | -- | U | NO |
| 1-Vinylpseudouridine TP | -- | U | NO |
| 2,2'-anhydro-uridine TP | -- | U | NO |
| 2'-bromo-deoxyuridine TP | -- | U | NO |
| 2'-F-5-Methyl-2'-deoxy-UTP | -- | U | NO |
| 2'-OMe-5-Me-UTP | -- | U | NO |
| 2 '-OMe-pseudo-UTP | -- | U | NO |
| 2 '-a-Ethynyluridine TP | -- | U | NO |
| 2'-a-Trifluoromethyluridine TP | -- | U | NO |
| 2'-b-Ethynyluridine TP | -- | U | NO |
| 2'-b-Trifluoromethyluridine TP | -- | U | NO |
| 2'-Deoxy-2',2'-difluorouridine TP | -- | U | NO |
| 2'-Deoxy-2'-a-mercaptouridine TP | -- | U | NO |
| 2'-Deoxy-2'-a-thiomethoxyuridine TP | -- | U | NO |
| 2'-Deoxy-2'-b-aminouridine TP | -- | U | NO |
| 2'-Deoxy-2'-b-azidouridine TP | -- | U | NO |
| 2'-Deoxy-2'-b-bromouridine TP | -- | U | NO |
| 2'-Deoxy-2'-b-chlorouridine TP | -- | U | NO |
| 2'-Deoxy-2'-b-fluorouridine TP | -- | U | NO |
| 2'-Deoxy-2'-b-iodouridine TP | -- | U | NO |
| 2'-Deoxy-2'-b-mercaptouridine TP | -- | U | NO |
| 2 '-Deoxy-2 '-b-thiomethoxyuridine TP | -- | U | NO |
| 2-methoxy-4-thio-uridine | -- | U | NO |
| 2-methoxyuridine | -- | U | NO |
| 2'-O-Methyl-5-(1-propynyl)uridine TP | -- | U | NO |
| 3-Alkyl-pseudo-UTP | -- | U | NO |
| 4 '-Azidouridine TP | -- | U | NO |
| 4'-Carbocyclic uridine TP | -- | U | NO |
| 4 '-Ethynyluridine TP | -- | U | NO |
| 5-(1-Propynyl)ara-uridine TP | -- | U | NO |
| 5-(2-Furanyl)uridine TP | -- | U | NO |
| 5-Cyanouridine TP | -- | U | NO |
| 5-Dimethylaminouridine TP | -- | U | NO |
| 5'-Homo-uridine TP | -- | U | NO |
| 5-iodo-2'-fluoro-deoxyuridine TP | -- | U | NO |
| 5-Phenylethynyluridine TP | -- | U | NO |
| 5-Trideuteromethyl-6-deuterouridine TP | -- | U | NO |
| 5-Trifluoromethyl-Uridine TP | -- | U | NO |
| 5-Vinylarauridine TP | -- | U | NO |
| 6-(2,2,2-Trifluoroethyl)-pseudo-UTP | -- | U | NO |
| 6-(4-Morpholino)-pseudo-UTP | -- | U | NO |
| 6-(4-Thiomorpholino)-pseudo-UTP | -- | U | NO |
| 6-(Substituted-Phenyl)-pseudo-UTP | -- | U | NO |
| 6-Amino-pseudo-UTP | -- | U | NO |
| 6-Azido-pseudo-UTP | -- | U | NO |
| 6-Bromo-pseudo-UTP | -- | U | NO |
| 6-Butyl-pseudo-UTP | -- | U | NO |
| 6-Chloro-pseudo-UTP | -- | U | NO |
| 6-Cyano-pseudo-UTP | -- | U | NO |
| 6-Dimethylamino-pseudo-UTP | -- | U | NO |
| 6-Ethoxy-pseudo-UTP | -- | U | NO |
| 6-Ethylcarboxylate-pseudo-UTP | -- | U | NO |
| 6-Ethyl-pseudo-UTP | -- | U | NO |
| 6-Fluoro-pseudo-UTP | -- | U | NO |
| 6-Formyl-pseudo-UTP | -- | U | NO |
| 6-Hydroxyamino-pseudo-UTP | -- | U | NO |
| 6-Hydroxy-pseudo-UTP | -- | U | NO |
| 6-Iodo-pseudo-UTP | -- | U | NO |
| 6-iso-Propyl-pseudo-UTP | -- | U | NO |
| 6-Methoxy-pseudo-UTP | -- | U | NO |
| 6-Methylamino-pseudo-UTP | -- | U | NO |
| 6-Methyl-pseudo-UTP | -- | U | NO |
| 6-Phenyl-pseudo-UTP | -- | U | NO |
| 6-Propyl-pseudo-UTP | -- | U | NO |
| 6-tert-Butyl-pseudo-UTP | -- | U | NO |
| 6-Trifluoromethoxy-pseudo-UTP | -- | U | NO |
| 6-Trifluoromethyl-pseudo-UTP | -- | U | NO |
| Alpha-thio-pseudo-UTP | -- | U | NO |
| Pseudouridine 1 -(4-methylbenzenesulfonic acid) TP | -- | U | NO |
| Pseudouridine 1-(4-methylbenzoic acid) TP | -- | U | NO |
| Pseudouridine TP 1-[3-(2-ethoxy)]propionic acid | -- | U | NO |
| Pseudouridine TP 1-[3-{2-(2-[2-(2-ethoxy )-ethoxy]-ethoxy )-ethoxy}]propionic acid | -- | U | NO |
| Pseudouridine TP 1-[3-{2-(2-[2-{2(2-ethoxy)-ethoxy}-ethoxy]-ethoxy )-ethoxy}]propionic acid | -- | U | NO |
| Pseudouridine TP 1-[3-{2-(2-[2-ethoxy ]-ethoxy)-ethoxy}]propionic acid | -- | U | NO |
| Pseudouridine TP 1-[3-{2-(2-ethoxy)-ethoxy}] propionic acid | -- | U | NO |
| Pseudouridine TP 1-methylphosphonic acid | -- | U | NO |
| Pseudouridine TP 1-methylphosphonic acid diethyl ester | -- | U | NO |
| Pseudo-UTP-N1-3-propionic acid | -- | U | NO |
| Pseudo-UTP-Nl-4-butanoic acid | -- | U | NO |
| Pseudo-UTP-N1-5-pentanoic acid | -- | U | NO |
| Pseudo-UTP-Nl-6-hexanoic acid | -- | U | NO |
| Pseudo-UTP-N1-7-heptanoic acid | -- | U | NO |
| Pseudo-UTP-N1-methyl-p-benzoic acid | -- | U | NO |
| Pseudo-UTP-N1-p-benzoic acid | -- | U | NO |
| wybutosine | yW | W | YES |
| hydroxywybutosine | OHyW | W | YES |
| isowyosine | imG2 | W | YES |
| peroxywybutosine | o2yW | W | YES |
| undermodified hydroxywybutosine | OHyW^{∗} | W | YES |
| 4-demethylwyosine | imG-14 | W | YES |

Other modifications which may be useful in the chimeric polynucleotides disclosure of the present disclosure are listed in Table 3.

**Table 3. Additional Modification types**

| | |
|---|---|
| **Name** | **Type** |
| 2,6-(diamino)purine | Other |
| 1-(aza)-2-(thio)-3-(aza)-phenoxazin-1-yl | Other |
| 1,3-(diaza)-2-(oxo)-phenthiazin-1-yl | Other |
| 1,3-(diaza)-2-(oxo)-phenoxazin-1-yl | Other |
| 1,3,5-(triaza)-2,6-(dioxa)-naphthalene | Other |
| 2 (amino)purine | Other |
| 2,4,5-(trimethyl)phenyl | Other |
| 2' methyl, 2'amino, 2'azido, 2'fluro-cytidine | Other |
| 2' methyl, 2'amino, 2'azido, 2'fluro-adenine | Other |
| 2'methyl, 2'amino, 2'azido, 2'fluro-uridine | Other |
| 2 '-amino-2 '-deoxyribose | Other |
| 2-amino-6-Chloro-purine | Other |
| 2-aza-inosinyl | Other |
| 2 '-azido-2 '-deoxyribose | Other |
| 2 'fluoro-2 '-deoxyribose | Other |
| 2'-fluoro-modified bases | Other |
| 2'-O-methyl-ribose | Other |
| 2-oxo-7-aminopyridopyrimidin-3-yl | Other |
| 2-oxo-pyridopyrimidine-3-yl | Other |
| 2-pyridinone | Other |
| 3 nitropyrrole | Other |
| 3 -(methyl)-7-(propynyl)isocarbostyrilyl | Other |
| 3 -(methyl)isocarbostyrilyl | Other |
| 4-(fluoro)-6-(methyl)benzimidazole | Other |
| 4-(methyl)benzimidazole | Other |
| 4-(methyl)indolyl | Other |
| 4,6-(dimethyl)indolyl | Other |
| 5 nitroindole | Other |
| 5 substituted pyrimidines | Other |
| 5-(methyl)isocarbostyrilyl | Other |
| 5 -nitro indole | Other |
| 6-(aza)pyrimidine | Other |
| 6-(azo)thymine | Other |
| 6-(methyl)-7-(aza)indolyl | Other |
| 6-chloro-purine | Other |
| 6-phenyl-pyrrolo-pyrimidin-2-on-3-yl | Other |
| 7-(-(aza)-2-(thio )-3-(aza)-phenthiazin-l-yl | Other |
| 7-(aminoalkylhydroxy)-1-(aza)-2-(thio)-3-(aza)-phenoxazin-1-yl | Other |
| 7-(aminoalkylhydroxy)-1,3-(diaza)-2-(oxo)-phenoxazin-1-yl | Other |
| 7-(aminoalkylhydroxy)-1,3-(diaza)-2-(oxo)-phenthiazin-1-yl | Other |
| 7-(aminoalkylhydroxy)-1,3-(diaza)-2-(oxo)-phenoxazin-1-yl | Other |
| 7-(aza)indolyl | Other |
| 7-(guanidiniumalkylhydroxy)-1-(aza)-2-(thio )-3-(aza)-phenoxazinl-yl | Other |
| 7-(guanidinimnalkylhydroxy)-1-(aza)-2-(thio )-3-(aza)-phenthiazin-l-yl | Other |
| 7 -(guanidiniumalkylhydroxy)-1-(aza)-2-(thio)-3-(aza)-phenoxazin-1-yl | Other |
| 7 -(guanidiniumalkylhydroxy)-1,3-(diaza)-2-(oxo)-phenoxazin-1-yl | Other |
| 7-(guanidiniumalkyl-hydroxy)-1,3-(diaza)-2-(oxo)-phenthiazin-1-yl | Other |
| 7-(guanidiniumalkylhydroxy)-1,3-(diaza)-2-(oxo)-phenoxazin-1-yl | Other |
| 7-(propynyl)isocarbostyrilyl | Other |
| 7-(propynyl)isocarbostyrilyl, propynyl-7-(aza)indolyl | Other |
| 7-deaza-inosinyl | Other |
| 7-substituted 1-(aza)-2-(thio)-3-(aza)-phenoxazin-1-yl | Other |
| 7-substituted 1,3-(diaza)-2-(oxo)-phenoxazin-1-yl | Other |
| 9-(methyl)-imidizopyridinyl | Other |
| aminoindolyl | Other |
| anthracenyl | Other |
| bis-ortho-(aminoalkylhydroxy)-6-phenyl-pyrrolo-pyrimidin-2-on-3-yl | Other |
| bis-ortho-substituted-6-phenyl-pyrrolo-pyrimidin-2-on-3-yl | Other |
| difluorotolyl | Other |
| hypoxanthine | Other |
| imidizopyridinyl | Other |
| inosinyl | Other |
| isocarbostyrilyl | Other |
| isoguanisine | Other |
| N2-substituted purines | Other |
| N6-methyl-2-amino-purine | Other |
| N6-substituted purines | Other |
| N-alkylated derivative | Other |
| napthalenyl | Other |
| nitro benzimidazolyl | Other |
| nitroimidazolyl | Other |
| nitroindazolyl | Other |
| nitropyrazolyl | Other |
| nubularine | Other |
| O6-substituted purines | Other |
| O-alkylated derivative | Other |
| ortho-(aminoalkylhydroxy)-6-phenyl-pyrrolo-pyrimidin-2-on-3-yl | Other |
| ortho-substituted-6-phenyl-pyrrolo-pyrimidin-2-on-3-yl | Other |
| Oxoformycin TP | Other |
| para-(aminoalkylhydroxy)-6-phenyl-pyrrolo-pyrimidin-2-on-3-yl | Other |
| p ara- substituted- 6-phenyl-pyrrolo-pyrimidin-2 -on-3 -yl | Other |
| pentacenyl | Other |
| phenanthracenyl | Other |
| phenyl | Other |
| propynyl-7-(aza)indolyl | Other |
| pyrenyl | Other |
| pyridopyrimidin-3-yl | Other |
| pyridopyrimidin-3-yl, 2-oxo-7-amino-pyridopyrimidin-3-yl | Other |
| pyrrolo-pyrimidin-2-on-3-yl | Other |
| pyrrolopyrimidinyl | Other |
| pyrrolopyrizinyl | Other |
| stilbenzyl | Other |
| substituted 1,2,4-triazoles | Other |
| tetracenyl | Other |
| tubercidine | Other |
| xanthine | Other |
| Xanthosine-5'-TP | Other |
| 2-thio-zebularine | Other |
| 5-aza-2-thio-zebularine | Other |
| 7-deaza-2-amino-purine | Other |
| pyridin-4-one ribonucleoside | Other |
| 2-Amino-riboside-TP | Other |
| Formycin A TP | Other |
| Formycin B TP | Other |
| Pyrrolosine TP | Other |
| 2'-OH-ara-adenosine TP | Other |
| 2'-OH-ara-cytidine TP | Other |
| 2'-OH-ara-uridine TP | Other |
| 2'-OH-ara-guanosine TP | Other |
| 5-(2-carbomethoxyvinyl)uridine TP | Other |
| N6-(19-Amino-pentaoxanonadecyl)adenosine TP | Other |

The chimeric polynucleotides can include any useful linker between the nucleosides. Such linkers, including backbone modifications are given in Table 4.

**Table 4. Linker modifications**

| **Name** | **TYPE** |
|---|---|
| 3 '-alkylene phosphonates | Linker |
| 3 '-amino phosphoramidate | Linker |
| alkene containing backbones | Linker |
| aminoalkylphosphoramidates | Linker |
| aminoalkylphosphotriesters | Linker |
| boranophosphates | Linker |
| -CH2-0-N(CH3)-CH2- | Linker |
| -CH2-N(CH3)-N(CH3)-CH2- | Linker |
| -CH2-NH-CH2- | Linker |
| chiral phosphonates | Linker |
| chiral phosphorothioates | Linker |
| formacetyl and thioformacetyl backbones | Linker |
| methylene (methylimino) | Linker |
| methylene formacetyl and thioformacetyl backbones | Linker |
| methyleneimino and methylenehydrazino backbones | Linker |
| morpholino linkages | Linker |
| -N(CH3)-CH2-CH2- | Linker |
| oligonucleosides with heteroatom internucleoside linkage | Linker |
| phosphinates | Linker |
| phosphoramidates | Linker |
| phosphorodithioates | Linker |
| phosphorothioate intenucleoside linkages | Linker |
| phosphorothioates | Linker |
| phosphotriesters | Linker |
| PNA | Linker |
| siloxane backbones | Linker |
| sulfamate backbones | Linker |
| sulfide sulfoxide and sulfone backbones | Linker |
| sulfonate and sulfonamide backbones | Linker |
| thionoalkylphosphonates | Linker |
| thionoalkylphosphotriesters | Linker |
| thionophosphoramidates | Linker |

The chimeric polynucleotides can include any useful modification, such as to the sugar, the nucleobase, or the internucleoside linkage (*e.g.* to a linking phosphate / to a phosphodiester linkage / to the phosphodiester backbone). One or more atoms of a pyrimidine nucleobase may be replaced or substituted with optionally substituted amino, optionally substituted thiol, optionally substituted alkyl (e.g., methyl or ethyl), or halo (e.g., chloro or fluoro). In certain cases, modifications (e.g., one or more modifications) are present in each of the sugar and the internucleoside linkage. Modifications may be modifications of ribonucleic acids (RNAs) to deoxyribonucleic acids (DNAs), threose nucleic acids (TNAs), glycol nucleic acids (GNAs), peptide nucleic acids (PNAs), locked nucleic acids (LNAs) or hybrids thereof). Additional modifications are described herein. cases disclosure

In some cases, the chimeric polynucleotides of the disclosure do not substantially induce an innate immune response of a cell into which the mRNA is introduced. Features of an induced innate immune response include 1) increased expression of pro-inflammatory cytokines, 2) activation of intracellular PRRs (RIG-I, MDA5, etc., and/or 3) termination or reduction in protein translation.

In certain cases, it may desirable to intracellularly degrade a chimeric polynucleotide introduced into the cell. For example, degradation of a chimeric polynucleotide may be preferable if precise timing of protein production is desired. Thus, in some cases, described herein is a chimeric polynucleotide containing a degradation domain, which is capable of being acted on in a directed manner within a cell.

Any of the regions of the chimeric polynucleotides may be chemically modified as taught herein or as taught in International Application Number PCT/2012/058519 filed October 3, 2012 (Attorney Docket Number M9) and U.S. Provisional Application Number 61/837297 filed June 20, 2013 (Attorney Docket Number M36)

### Modified Chimeric polynucleotide Molecules

The present disclosure also includes building blocks, e.g., modified ribonucleosides, and modified ribonucleotides, of chimeric polynucleotide molecules. For example, these building blocks can be useful for preparing the chimeric polynucleotides of the disclosure. Such building blocks are taught in International Application WO2013052523 filed October 3, 2012 (Attorney Docket Number M9) and International Application WO2014093924, filed December 13, 2013 (Attorney Docket Number M36)

### Modifications on the Sugar

The modified nucleosides and nucleotides (e.g., building block molecules), which may be incorporated into a chimeric polynucleotide (e.g., RNA or mRNA, as described herein), can be modified on the sugar of the ribonucleic acid. For example, the 2' hydroxyl group (OH) can be modified or replaced with a number of different substituents. Exemplary substitutions at the 2'-position include, but are not limited to, H, halo, optionally substituted C₁₋₆ alkyl; optionally substituted C₁₋₆ alkoxy; optionally substituted C₆₋₁₀ aryloxy; optionally substituted C₃₋₈ cycloalkyl; optionally substituted C₃₋₈ cycloalkoxy; optionally substituted C₆₋₁₀ aryloxy; optionally substituted C₆₋₁₀ aryl-C₁₋₆ alkoxy, optionally substituted C₁₋₁₂ (heterocyclyl)oxy; a sugar (e.g., ribose, pentose, or any described herein); a polyethyleneglycol (PEG), - O(CH₂CH₂O)ₙCH₂CH₂OR, where R is H or optionally substituted alkyl, and n is an integer from 0 to 20 (e.g., from 0 to 4, from 0 to 8, from 0 to 10, from 0 to 16, from 1 to 4, from 1 to 8, from 1 to 10, from 1 to 16, from 1 to 20, from 2 to 4, from 2 to 8, from 2 to 10, from 2 to 16, from 2 to 20, from 4 to 8, from 4 to 10, from 4 to 16, and from 4 to 20); "locked" nucleic acids (LNA) in which the 2'-hydroxyl is connected by a C₁₋₆ alkylene or C₁₋₆ heteroalkylene bridge to the 4'-carbon of the same ribose sugar, where exemplary bridges included methylene, propylene, ether, or amino bridges; aminoalkyl, as defined herein; aminoalkoxy, as defined herein; amino as defined herein; and amino acid, as defined herein

Generally, RNA includes the sugar group ribose, which is a 5-membered ring having an oxygen. Exemplary, non-limiting modified nucleotides include replacement of the oxygen in ribose (e.g., with S, Se, or alkylene, such as methylene or ethylene); addition of a double bond (e.g., to replace ribose with cyclopentenyl or cyclohexenyl); ring contraction of ribose (e.g., to form a 4-membered ring of cyclobutane or oxetane); ring expansion of ribose (e.g., to form a 6- or 7-membered ring having an additional carbon or heteroatom, such as for anhydrohexitol, altritol, mannitol, cyclohexanyl, cyclohexenyl, and morpholino that also has a phosphoramidate backbone); multicyclic forms (e.g., tricyclo; and "unlocked" forms, such as glycol nucleic acid (GNA) (e.g., R-GNA or S-GNA, where ribose is replaced by glycol units attached to phosphodiester bonds), threose nucleic acid (TNA, where ribose is replace with α-L-threofuranosyl-(3'→2')) , and peptide nucleic acid (PNA, where 2-amino-ethyl-glycine linkages replace the ribose and phosphodiester backbone). The sugar group can also contain one or more carbons that possess the opposite stereochemical configuration than that of the corresponding carbon in ribose. Thus, a chimeric polynucleotide molecule can include nucleotides containing, e.g., arabinose, as the sugar. Such sugar modifications are taught International Application Number PCT/2012/058519 filed October 3, 2012 (Attorney Docket Number M9) and International Publication No. WO2014093924 (Attorney Docket Number M36).

### Modifications on the Nucleobase

The present disclosure described modified nucleosides and nucleotides. As described herein "nucleoside" is defined as a compound containing a sugar molecule (e.g., a pentose or ribose) or a derivative thereof in combination with an organic base (e.g., a purine or pyrimidine) or a derivative thereof (also referred to herein as "nucleobase"). As described herein, "nucleotide" is defined as a nucleoside including a phosphate group. The modified nucleotides may by synthesized by any useful method, as described herein (e.g., chemically, enzymatically, or recombinantly to include one or more modified or non-natural nucleosides). The chimeric polynucleotides may comprise a region or regions of linked nucleosides. Such regions may have variable backbone linkages. The linkages may be standard phosphoester linkages, in which case the chimeric polynucleotides would comprise regions of nucleotides.

The modified nucleotide base pairing encompasses not only the standard adenosine-thymine, adenosine-uracil, or guanosine-cytosine base pairs, but also base pairs formed between nucleotides and/or modified nucleotides comprising non-standard or modified bases, wherein the arrangement of hydrogen bond donors and hydrogen bond acceptors permits hydrogen bonding between a non-standard base and a standard base or between two complementary non-standard base structures. One example of such non-standard base pairing is the base pairing between the modified nucleotide inosine and adenine, cytosine or uracil.

The modified nucleosides and nucleotides can include a modified nucleobase. Examples of nucleobases found in RNA include, but are not limited to, adenine, guanine, cytosine, and uracil. Examples of nucleobase found in DNA include, but are not limited to, adenine, guanine, cytosine, and thymine. Such modified nucleobases (including the distinctions between naturally occurring and non-naturally occurring) are taught in International Application Number PCT/2012/058519 filed October 3, 2012 (Attorney Docket Number M9) and International Publication No. WO2014093924 (Attorney Docket Number M36).

### Combinations of Modified Sugars, Nucleobases, and Internucleoside Linkages

The chimeric polynucleotides can include a combination of modifications to the sugar, the nucleobase, and/or the internucleoside linkage. These combinations can include any one or more modifications described herein.

Examples of modified nucleotides and modified nucleotide combinations are described below in Table 5 and Table 6. These combinations of modified nucleotides can be used to form the chimeric polynucleotides of the disclosure. Unless otherwise noted, the modified nucleotides may be completely substituted for the natural nucleotides of the chimeric polynucleotides of the disclosure. As a non-limiting example, the natural nucleotide uridine may be substituted with a modified nucleoside described herein. In another non-limiting example, the natural nucleotide uridine may be partially substituted (e.g., about 0.1%, 1%, 5%, 10%, 15%, 20%, 25%, 30%, 35%, 40%, 45%, 50%, 55%, 60%, 65%, 70%, 75%, 80%, 85%, 90%, 95% or 99.9%) with at least one of the modified nucleoside disclosed herein.

Any combination of base/sugar or linker may be incorporated into the chimeric polynucleotides of the disclosure and such modifications are taught in International Publication No. WO2013052523 (Attorney Docket Number M9); International Application No. PCT/US2013/75177 (Attorney Docket Number M36); International Publication NO. WO2015051173 (Attorney Docket Number M71)

**Table 5. Combinations**

| **Modified Nucleotide** | **Modified Nucleotide Combination** |
|---|---|
| α-thio-cytidine | α-thio-cytidine/5-iodo-uridine |
| | α-thio-cytidine/N1-methyl-pseudouridine |
| | α-thio-cytidine/α-thio-uridine |
| | α-thio-cytidine/5-methyl-uridine |
| | α-thio-cytidine/pseudo-uridine |
| | about 50% of the cytosines are α-thio-cytidine |
| pseudoisocytidine | pseudoisocytidine/5-iodo-uridine |
| | pseudoisocytidine/N1-methyl-pseudouridine |
| | pseudoisocytidine/α-thio-uridine |
| | pseudoisocytidine/5-methyl-uridine |
| | pseudoisocytidine/pseudouridine |
| | about 25% of cytosines are pseudoisocytidine |
| | pseudoisocytidine/about 50% of uridines are N1-methyl-pseudouridine and about 50% of uridines are pseudouridine |
| | pseudoisocytidine/about 25% of uridines are N1-methyl-pseudouridine and about 25% of uridines are pseudouridine |
| pyrrolo-cytidine | pyrrolo-cytidine/5-iodo -uridine |
| | pyrrolo-cytidine/N1-methyl-pseudouridine |
| | pyrrolo-cytidine/α-thio-uridine |
| | pyrrolo-cytidine/5-methyl-uridine |
| | pyrrolo-cytidine/pseudouridine |
| | about 50% of the cytosines are pyrrolo-cytidine |
| 5-methyl-cytidine | 5-methyl-cytidine/5-iodo-uridine |
| | 5-methyl-cytidine/N1-methyl-pseudouridine |
| | 5-methyl-cytidine/α-thio-uridine |
| | 5-methyl-cytidine/5-methyl-uridine |
| | 5 -methyl-cytidine/pseudouridine |
| | about 25% of cytosines are 5-methyl-cytidine |
| | about 50% of cytosines are 5-methyl-cytidine |
| | 5-methyl-cytidine/5-methoxy-uridine |
| | 5-methyl-cytidine/5-bromo-uridine |
| | 5-methyl-cytidine/2-thio-uridine |
| | 5-methyl-cytidine/about 50% of uridines are 2-thio-uridine |
| | about 50% of uridines are 5-methyl-cytidine/ about 50% of uridines are 2-thio-uridine |
| N4-acetyl-cytidine | N4-acetyl-cytidine /5-iodo-uridine |
| | N4-acetyl-cytidine /N1-methyl-pseudouridine |
| | N4-acetyl-cytidine /α-thio-uridine |
| | N4-acetyl-cytidine /5-methyl-uridine |
| | N4-acetyl-cytidine /pseudouridine |
| | about 50% of cytosines are N4-acetyl-cytidine |
| | about 25% of cytosines are N4-acetyl-cytidine |
| | N4-acetyl-cytidine /5-methoxy-uridine |
| | N4-acetyl-cytidine /5-bromo-uridine |
| | N4-acetyl-cytidine /2-thio-uridine |
| | about 50% of cytosines are N4-acetyl-cytidine/ about 50% of uridines are 2-thio-uridine |

**Table 6. Combinations**

| |
|---|
| 1-(2,2,2-Trifluoroethyl)pseudo-UTP |
| 1-Ethyl-pseudo-UTP |
| 1-Methyl-pseudo-U -alpha-thio- TP |
| 1-methyl-pseudouridine TP, ATP, GTP, CTP |
| 1-methyl-pseudo-UTP/5-methyl-CTP/ATP/GTP |
| 1-methyl-pseudo-UTP/CTP/ATP/GTP |
| 1-Propyl-pseudo-UTP |
| 25 % 5-Aminoallyl-CTP + 75 % CTP/ 25 % 5-Methoxy-UTP + 75 % UTP |
| 25 % 5-Aminoallyl-CTP + 75 % CTP/ 75 % 5-Methoxy-UTP + 25 % UTP |
| 25 % 5-Bromo-CTP + 75 % CTP/ 25 % 5-Methoxy-UTP + 75 % UTP |
| 25 % 5-Bromo-CTP + 75 % CTP/ 75 % 5-Methoxy-UTP + 25 % UTP |
| 25 % 5-Bromo-CTP + 75 % CTP/1-Methyl-pseudo-UTP |
| 25 % 5-Carboxy-CTP + 75 % CTP/ 25 % 5-Methoxy-UTP + 75 % UTP |
| 25 % 5-Carboxy-CTP + 75 % CTP/ 75 % 5-Methoxy-UTP + 25 % UTP |
| 25 % 5-Ethyl-CTP + 75 % CTP/ 25 % 5-Methoxy-UTP + 75 % UTP |
| 25 % 5-Ethyl-CTP + 75 % CTP/ 75 % 5-Methoxy-UTP + 25 % UTP |
| 25 % 5-Ethynyl-CTP + 75 % CTP/ 25 % 5-Methoxy-UTP + 75 % UTP |
| 25 % 5-Ethynyl-CTP + 75 % CTP/ 75 % 5-Methoxy-UTP + 25 % UTP |
| 25 % 5-Fluoro-CTP + 75 % CTP/ 25 % 5-Methoxy-UTP + 75 % UTP |
| 25 % 5-Fluoro-CTP + 75 % CTP/ 75 % 5-Methoxy-UTP + 25 % UTP |
| 25 % 5-Formyl-CTP + 75 % CTP/ 25 % 5-Methoxy-UTP + 75 % UTP |
| 25 % 5-Formyl-CTP + 75 % CTP/ 75 % 5-Methoxy-UTP + 25 % UTP |
| 25 % 5-Hydroxymethyl-CTP + 75 % CTP/ 25 % 5-Methoxy-UTP + 75 % UTP |
| 25 % 5-Hydroxymethyl-CTP + 75 % CTP/ 75 % 5-Methoxy-UTP + 25 % UTP |
| 25 % 5-Iodo-CTP + 75 % CTP/ 25 % 5-Methoxy-UTP + 75 % UTP |
| 25 % 5-Iodo-CTP + 75 % CTP/ 75 % 5-Methoxy-UTP + 25 % UTP |
| 25 % 5-Methoxy-CTP + 75 % CTP/ 25 % 5-Methoxy-UTP + 75 % UTP |
| 25 % 5-Methoxy-CTP + 75 % CTP/ 75 % 5-Methoxy-UTP + 25 % UTP |
| 25 % 5-Methyl-CTP + 75 % CTP/25 % 5-Methoxy-UTP + 75 % 1-Methyl-pseudo-UTP |
| 25 % 5-Methyl-CTP + 75 % CTP/25 % 5-Methoxy-UTP + 75 % UTP |
| 25 % 5-Methyl-CTP + 75 % CTP/50 % 5-Methoxy-UTP + 50 % 1-Methyl-pseudo-UTP |
| 25 % 5-Methyl-CTP + 75 % CTP/50 % 5-Methoxy-UTP + 50 % UTP |
| 25 % 5-Methyl-CTP + 75 % CTP/5-Methoxy-UTP |
| 25 % 5-Methyl-CTP + 75 % CTP/75 % 5-Methoxy-UTP + 25 % 1-Methyl-pseudo-UTP |
| 25 % 5-Methyl-CTP + 75 % CTP/75 % 5-Methoxy-UTP + 25 % UTP |
| 25 % 5-Phenyl-CTP + 75 % CTP/ 25 % 5-Methoxy-UTP + 75 % UTP |
| 25 % 5-Phenyl-CTP + 75 % CTP/ 75 % 5-Methoxy-UTP + 25 % UTP |
| 25 % 5-Trifluoromethyl-CTP + 75 % CTP/ 25 % 5-Methoxy-UTP + 75 % UTP |
| 25 % 5-Trifluoromethyl-CTP + 75 % CTP/ 75 % 5-Methoxy-UTP + 25 % UTP |
| 25 % 5-Trifluoromethyl-CTP + 75 % CTP/1-Methyl-pseudo-UTP |
| 25 % N4-Ac-CTP + 75 % CTP/ 25 % 5-Methoxy-UTP + 75 % UTP |
| 25 % N4-Ac-CTP + 75 % CTP/ 75 % 5-Methoxy-UTP + 25 % UTP |
| 25 % N4-Bz-CTP + 75 % CTP/ 25 % 5-Methoxy-UTP + 75 % UTP |
| 25 % N4-Bz-CTP + 75 % CTP/ 75 % 5-Methoxy-UTP + 25 % UTP |
| 25 % N4-Methyl-CTP + 75 % CTP/ 25 % 5-Methoxy-UTP + 75 % UTP |
| 25 % N4-Methyl-CTP + 75 % CTP/ 75 % 5-Methoxy-UTP + 25 % UTP |
| 25 % Pseudo-iso-CTP + 75 % CTP/ 25 % 5-Methoxy-UTP + 75 % UTP |
| 25 % Pseudo-iso-CTP + 75 % CTP/ 75 % 5-Methoxy-UTP + 25 % UTP |
| 25% 5-Bromo-CTP/75% CTP/ Pseudo-UTP |
| 25% 5-methoxy-UTP/25% 5-methyl-CTP/ATP/GTP |
| 25% 5-methoxy-UTP/5-methyl-CTP/ATP/GTP |
| 25% 5-methoxy-UTP/75% 5-methyl-CTP/ATP/GTP |
| 25% 5-methoxy-UTP/CTP/ATP/GTP |
| 25% 5-metoxy-UTP/50% 5-methyl-CTP/ATP/GTP |
| 2-Amino-ATP |
| 2-Thio-CTP |
| 2-thio-pseudouridine TP, ATP, GTP, CTP |
| 2-Thio-pseudo-UTP |
| 2-Thio-UTP |
| 3-Methyl-CTP |
| 3-Methyl-pseudo-UTP |
| 4-Thio-UTP |
| 50 % 5-Bromo-CTP + 50 % CTP/1-Methyl-pseudo-UTP |
| 50 % 5-Hydroxymethyl-CTP + 50 % CTP/1-Methyl-pseudo-UTP |
| 50 % 5-methoxy-UTP/5-methyl-CTP/ATP/GTP |
| 50 % 5-Methyl-CTP + 50 % CTP/25 % 5-Methoxy-UTP + 75 % 1-Methyl-pseudo-UTP |
| 50 % 5-Methyl-CTP + 50 % CTP/25 % 5-Methoxy-UTP + 75 % UTP |
| 50 % 5-Methyl-CTP + 50 % CTP/50 % 5-Methoxy-UTP + 50 % 1-Methyl-pseudo-UTP |
| 50 % 5-Methyl-CTP + 50 % CTP/50 % 5-Methoxy-UTP + 50 % UTP |
| 50 % 5-Methyl-CTP + 50 % CTP/5-Methoxy-UTP |
| 50 % 5-Methyl-CTP + 50 % CTP/75 % 5-Methoxy-UTP + 25 % 1-Methyl-pseudo-UTP |
| 50 % 5-Methyl-CTP + 50 % CTP/75 % 5-Methoxy-UTP + 25 % UTP |
| 50 % 5-Trifluoromethyl-CTP + 50 % CTP/1-Methyl-pseudo-UTP |
| 50% 5-Bromo-CTP/ 50% CTP/Pseudo-UTP |
| 50% 5-methoxy-UTP/25% 5-methyl-CTP/ATP/GTP |
| 50% 5-methoxy-UTP/50% 5-methyl-CTP/ATP/GTP |
| 50% 5-methoxy-UTP/75% 5-methyl-CTP/ATP/GTP |
| 50% 5-methoxy-UTP/CTP/ATP/GTP |
| 5-Aminoallyl-CTP |
| 5-Aminoallyl-CTP/ 5-Methoxy-UTP |
| 5-Aminoallyl-UTP |
| 5-Bromo-CTP |
| 5-Bromo-CTP/ 5-Methoxy-UTP |
| 5-Bromo-CTP/1-Methyl-pseudo-UTP |
| 5-Bromo-CTP/Pseudo-UTP |
| 5-bromocytidine TP, ATP, GTP, UTP |
| 5-Bromo-UTP |
| 5-Carboxy-CTP/ 5-Methoxy-UTP |
| 5-Efliyl-CTP/5-Methoxy-UTP |
| 5-Ethynyl-CTP/5-Methoxy-UTP |
| 5-Fluoro-CTP/ 5-Methoxy-UTP |
| 5-Formyl-CTP/ 5-Methoxy-UTP |
| 5-Hydroxy- methyl-CTP/ 5-Methoxy-UTP |
| 5-Hydroxymethyl-CTP |
| 5-Hydroxymethyl-CTP/1-Methyl-pseudo-UTP |
| 5-Hydroxymethyl-CTP/5-Methoxy-UTP |
| 5-hydroxymethyl-cytidine TP, ATP, GTP, UTP |
| 5-Iodo-CTP/ 5-Methoxy-UTP |
| 5-Me-CTP/5-Methoxy-UTP |
| 5-Methoxy carbonyl methyl-UTP |
| 5-Methoxy-CTP/5-Methoxy-UTP |
| 5-methoxy-uridine TP, ATP, GTP, UTP |
| 5-methoxy-UTP |
| 5-Methoxy-UTP |
| 5-Methoxy-UTP/ N6-Isopentenyl-ATP |
| 5-methoxy-UTP/25% 5-methyl-CTP/ATP/GTP |
| 5-methoxy-UTP/5-methyl-CTP/ATP/GTP |
| 5-methoxy-UTP/75% 5-methyl-CTP/ATP/GTP |
| 5-methoxy-UTP/CTP/ATP/GTP |
| 5-Methyl-2-thio-UTP |
| 5-Methylaminomethyl-UTP |
| 5-Methyl-CTP/ 5-Methoxy-UTP |
| 5-Methyl-CTP/ 5-Methoxy-UTP(cap 0) |
| 5-Methyl-CTP/ 5-Methoxy-UTP(No cap) |
| 5-Methyl-CTP/25 % 5-Methoxy-UTP + 75 % 1-Methyl-pseudo-UTP |
| 5-Methyl-CTP/25 % 5-Methoxy-UTP + 75 % UTP |
| 5-Methyl-CTP/50 % 5-Methoxy-UTP + 50 % 1-Methyl-pseudo-UTP |
| 5-Methyl-CTP/50 % 5-Methoxy-UTP + 50 % UTP |
| 5-Methyl-CTP/5-Methoxy-UTP/N6-Me-ATP |
| 5-Methyl-CTP/75 % 5-Methoxy-UTP + 25 % 1-Methyl-pseudo-UTP |
| 5-Methyl-CTP/75 % 5-Methoxy-UTP + 25 % UTP |
| 5-Phenyl-CTP/ 5-Methoxy-UTP |
| 5-Trifluoro- methyl-CTP/ 5-Methoxy-UTP |
| 5-Trifluoromethyl-CTP |
| 5-Trifluoromethyl-CTP/ 5-Methoxy-UTP |
| 5-Trifluoromethyl-CTP/1-Methyl-pseudo-UTP |
| 5-Trifluoromethyl-CTP/Pseudo-UTP |
| 5-Trifluoromethyl-UTP |
| 5-trifluromethylcytidine TP, ATP, GTP, UTP |
| 75 % 5-Aminoallyl-CTP + 25 % CTP/ 25 % 5-Methoxy-UTP + 75 % UTP |
| 75 % 5-Aminoallyl-CTP + 25 % CTP/ 75 % 5-Methoxy-UTP + 25 % UTP |
| 75 % 5-Bromo-CTP + 25 % CTP/ 25 % 5-Methoxy-UTP + 75 % UTP |
| 75 % 5-Bromo-CTP + 25 % CTP/ 75 % 5-Methoxy-UTP + 25 % UTP |
| 75 % 5-Carboxy-CTP + 25 % CTP/ 25 % 5-Methoxy-UTP + 75 % UTP |
| 75 % 5-Carboxy-CTP + 25 % CTP/ 75 % 5-Methoxy-UTP + 25 % UTP |
| 75 % 5-Ethyl-CTP + 25 % CTP/ 25 % 5-Methoxy-UTP + 75 % UTP |
| 75 % 5-Ethyl-CTP + 25 % CTP/ 75 % 5-Methoxy-UTP + 25 % UTP |
| 75 % 5-Ethynyl-CTP + 25 % CTP/ 25 % 5-Methoxy-UTP + 75 % UTP |
| 75 % 5-Ethynyl-CTP + 25 % CTP/ 75 % 5-Methoxy-UTP + 25 % UTP |
| 75 % 5-Fluoro-CTP + 25 % CTP/ 25 % 5-Methoxy-UTP + 75 % UTP |
| 75 % 5-Fluoro-CTP + 25 % CTP/ 75 % 5-Methoxy-UTP + 25 % UTP |
| 75 % 5-Formyl-CTP + 25 % CTP/ 25 % 5-Methoxy-UTP + 75 % UTP |
| 75 % 5-Formyl-CTP + 25 % CTP/ 75 % 5-Methoxy-UTP + 25 % UTP |
| 75 % 5-Hydroxymethyl-CTP + 25 % CTP/ 25 % 5-Methoxy-UTP + 75 % UTP |
| 75 % 5-Hydroxymethyl-CTP + 25 % CTP/ 75 % 5-Methoxy-UTP + 25 % UTP |
| 75 % 5-Iodo-CTP + 25 % CTP/ 25 % 5-Methoxy-UTP + 75 % UTP |
| 75 % 5-Iodo-CTP + 25 % CTP/ 75 % 5-Methoxy-UTP + 25 % UTP |
| 75 % 5-Methoxy-CTP + 25 % CTP/ 25 % 5-Methoxy-UTP + 75 % UTP |
| 75 % 5-Methoxy-CTP + 25 % CTP/ 75 % 5-Methoxy-UTP + 25 % UTP |
| 75 % 5-methoxy-UTP/5-methyl-CTP/ATP/GTP |
| 75 % 5-Methyl-CTP + 25 % CTP/25 % 5-Methoxy-UTP + 75 % 1-Methyl-pseudo-UTP |
| 75 % 5-Methyl-CTP + 25 % CTP/25 % 5-Methoxy-UTP + 75 % UTP |
| 75 % 5-Methyl-CTP + 25 % CTP/50 % 5-Methoxy-UTP + 50 % 1-Methyl-pseudo-UTP |
| 75 % 5-Methyl-CTP + 25 % CTP/50 % 5-Methoxy-UTP + 50 % UTP |
| 75 % 5-Methyl-CTP + 25 % CTP/5-Methoxy-UTP |
| 75 % 5-Methyl-CTP + 25 % CTP/75 % 5-Methoxy-UTP + 25 % 1-Methyl-pseudo-UTP |
| 75 % 5-Methyl-CTP + 25 % CTP/75 % 5-Methoxy-UTP + 25 % UTP |
| 75 % 5-Phenyl-CTP + 25 % CTP/ 25 % 5-Methoxy-UTP + 75 % UTP |
| 75 % 5-Phenyl-CTP + 25 % CTP/ 75 % 5-Methoxy-UTP + 25 % UTP |
| 75 % 5-Trifluoromethyl-CTP + 25 % CTP/ 25 % 5-Methoxy-UTP + 75 % UTP |
| 75 % 5-Trifluoromethyl-CTP + 25 % CTP/ 75 % 5-Methoxy-UTP + 25 % UTP |
| 75 % 5-Trifluoromethyl-CTP + 25 % CTP/1-Methyl-pseudo-UTP |
| 75 % N4-Ac-CTP + 25 % CTP/ 25 % 5-Methoxy-UTP + 75 % UTP |
| 75 % N4-Ac-CTP + 25 % CTP/ 75 % 5-Methoxy-UTP + 25 % UTP |
| 75 % N4-Bz-CTP + 25 % CTP/ 25 % 5-Methoxy-UTP + 75 % UTP |
| 75 % N4-Bz-CTP + 25 % CTP/ 75 % 5-Methoxy-UTP + 25 % UTP |
| 75 % N4-Methyl-CTP + 25 % CTP/ 25 % 5-Methoxy-UTP + 75 % UTP |
| 75 % N4-Methyl-CTP + 25 % CTP/ 75 % 5-Methoxy-UTP + 25 % UTP |
| 75 % Pseudo-iso-CTP + 25 % CTP/ 25 % 5-Methoxy-UTP + 75 % UTP |
| 75 % Pseudo-iso-CTP + 25 % CTP/ 75 % 5-Methoxy-UTP + 25 % UTP |
| 75% 5-Bromo-CTP/25% CTP/ 1-Methyl-pseudo-UTP |
| 75% 5-Bromo-CTP/25% CTP/ Pseudo-UTP |
| 75% 5-methoxy-UTP/25% 5-methyl-CTP/ATP/GTP |
| 75% 5-methoxy-UTP/50% 5-methyl-CTP/ATP/GTP |
| 75% 5-methoxy-UTP/75% 5-methyl-CTP/ATP/GTP |
| 75% 5-methoxv-UTP/CTP/ATP/GTP |
| 8-Aza-ATP |
| Alpha-thio-CTP |
| CTP/25 % 5-Methoxy-UTP + 75 % 1-Methyl-pseudo-UTP |
| CTP/25 % 5-Methoxy-UTP + 75 % UTP |
| CTP/50 % 5-Methoxy-UTP + 50 % 1-Methyl-pseudo-UTP |
| CTP/50 % 5-Methoxy-UTP + 50 % UTP |
| CTP/5 -Methoxy-UTP |
| CTP/5-Methoxy-UTP (cap 0) |
| CTP/5-Methoxy-UTP(No cap) |
| CTP/75 % 5-Methoxy-UTP + 25 % 1-Methyl-pseudo-UTP |
| CTP/75 % 5-Methoxy-UTP + 25 % UTP |
| CTP/UTP(No cap) |
| Nl-Me-GTP |
| N4-Ac-CTP |
| N4Ac-CTP/1-Methyl-pseudo-UTP |
| N4Ac-CTP/5-Methoxy-UTP |
| N4-acetyl-cytidine TP, ATP, GTP, UTP |
| N4-Bz-CTP/ 5-Methoxy-UTP |
| N4-methyl CTP |
| N4-Methyl-CTP/ 5-Methoxy-UTP |
| Pseudo-iso-CTP/ 5-Methoxy-UTP |
| PseudoU-alpha-thio-TP |
| pseudouridine TP, ATP, GTP, CTP |
| pseudo-UTP/5-methyl-CTP/ATP/GTP |
| UTP-5-oxyacetic acid Me ester |
| Xanthosine |

Polynucleotides may be synthesized to comprise the combinations or single modifications of Table 6.

Where a single modification is listed, the listed nucleoside or nucleotide represents 100 percent of that A, U, G or C nucleotide or nucleoside having been modified. Where percentages are listed, these represent the percentage of that particular A, U, G or C nucleobase triphosphate of the total amount of A, U, G, or C triphosphate present. For example, the combination: 25 % 5-Aminoallyl-CTP + 75 % CTP/ 25 % 5-Methoxy-UTP + 75 % UTP refers to a polynucleotide where 25% of the cytosine triphosphates are 5-Aminoallyl-CTP while 75% of the cytosines are CTP; whereas 25% of the uracils are 5-methoxy UTP while 75% of the uracils are UTP. Where no modified UTP is listed then the naturally occurring ATP, UTP, GTP and/or CTP is used at 100% of the sites of those nucleotides found in the polynucleotide. In this example all of the GTP and ATP nucleotides are left unmodified.

### IV. Pharmaceutical Compositions

### Formulation, Administration, Delivery and Dosing

Described herein are chimeric polynucleotides compositions and complexes in combination with one or more pharmaceutically acceptable excipients. Pharmaceutical compositions may optionally comprise one or more additional active substances, e.g. therapeutically and/or prophylactically active substances. Pharmaceutical compositions may be sterile and/or pyrogen-free. General considerations in the formulation and/or manufacture of pharmaceutical agents may be found, for example, in Remington: The Science and Practice of Pharmacy 21st ed., Lippincott Williams & Wilkins, 2005.

In some cases, compositions are administered to humans, human patients or subjects. For the purposes of the present disclosure, the phrase "active ingredient" generally refers to chimeric polynucleotides to be delivered as described herein.

Although the descriptions of pharmaceutical compositions described herein are principally directed to pharmaceutical compositions which are suitable for administration to humans, it will be understood by the skilled artisan that such compositions are generally suitable for administration to any other animal, e.g., to non-human animals, e.g. non-human mammals. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation. Subjects to which administration of the pharmaceutical compositions is contemplated include, but are not limited to, humans and/or other primates; mammals, including commercially relevant mammals such as cattle, pigs, horses, sheep, cats, dogs, mice, and/or rats; and/or birds, including commercially relevant birds such as poultry, chickens, ducks, geese, and/or turkeys.

Formulations of the pharmaceutical compositions described herein may be prepared by any method known or hereafter developed in the art of pharmacology. In general, such preparatory methods include the step of bringing the active ingredient into association with an excipient and/or one or more other accessory ingredients, and then, if necessary and/or desirable, dividing, shaping and/or packaging the product into a desired single- or multi-dose unit.

Relative amounts of the active ingredient, the pharmaceutically acceptable excipient, and/or any additional ingredients in a pharmaceutical composition in accordance with the disclosure will vary, depending upon the identity, size, and/or condition of the subject treated and further depending upon the route by which the composition is to be administered. By way of example, the composition may comprise between 0.1% and 100%, e.g., between .5 and 50%, between 1-30%, between 5-80%, at least 80% (w/w) active ingredient.

### Formulations

The chimeric polynucleotides of the disclosure can be formulated using one or more excipients to: (1) increase stability; (2) increase cell transfection; (3) permit the sustained or delayed release (e.g., from a depot formulation of the chimeric polynucleotide); (4) alter the biodistribution (e.g., target the chimeric polynucleotide to specific tissues or cell types); (5) increase the translation of encoded protein *in vivo*; and/or (6) alter the release profile of encoded protein in vivo. In addition to traditional excipients such as any and all solvents, dispersion media, diluents, or other liquid vehicles, dispersion or suspension aids, surface active agents, isotonic agents, thickening or emulsifying agents, preservatives, excipients can include, without limitation, lipidoids, liposomes, lipid nanoparticles, polymers, lipoplexes, core-shell nanoparticles, peptides, proteins, cells transfected with chimeric polynucleotides (e.g., for transplantation into a subject), hyaluronidase, nanoparticle mimics and combinations thereof. Accordingly, the formulations can include one or more excipients, each in an amount that together increases the stability of the chimeric polynucleotide, increases cell transfection by the chimeric polynucleotide, increases the expression of chimeric polynucleotides encoded protein, and/or alters the release profile of chimeric polynucleotide encoded proteins. Further, the chimeric polynucleotides may be formulated using self-assembled nucleic acid nanoparticles.

Formulations of the pharmaceutical compositions described herein may be prepared by any method known or hereafter developed in the art of pharmacology. In general, such preparatory methods include the step of associating the active ingredient with an excipient and/or one or more other accessory ingredients.

A pharmaceutical composition in accordance with the present disclosure may be prepared, packaged, and/or sold in bulk, as a single unit dose, and/or as a plurality of single unit doses. As used herein, a "unit dose" refers to a discrete amount of the pharmaceutical composition comprising a predetermined amount of the active ingredient. The amount of the active ingredient is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition in accordance with the present disclosure may vary, depending upon the identity, size, and/or condition of the subject being treated and further depending upon the route by which the composition is to be administered. For example, the composition may comprise between 0.1% and 99% (w/w) of the active ingredient. By way of example, the composition may comprise between 0.1% and 100%, e.g., between .5 and 50%, between 1-30%, between 5-80%, at least 80% (w/w) active ingredient.

In some cases, the formulations described herein may contain at least one chimeric polynucleotide. As a non-limiting example, the formulations may contain 1, 2, 3, 4 or 5 chimeric polynucleotide. In one case the formulation may contain chimeric polynucleotide encoding proteins selected from categories such as, but not limited to, human proteins, veterinary proteins, bacterial proteins, biological proteins, antibodies, immunogenic proteins, therapeutic peptides and proteins, secreted proteins, plasma membrane proteins, cytoplasmic and cytoskeletal proteins, intracellular membrane bound proteins, nuclear proteins, proteins associated with human disease and/or proteins associated with non-human diseases. In one case, the formulation contains at least three chimeric polynucleotides encoding proteins. In one case, the formulation contains at least five chimeric polynucleotide encoding proteins.

Pharmaceutical formulations may additionally comprise a pharmaceutically acceptable excipient, which, as used herein, includes, but is not limited to, any and all solvents, dispersion media, diluents, or other liquid vehicles, dispersion or suspension aids, surface active agents, isotonic agents, thickening or emulsifying agents, preservatives, and the like, as suited to the particular dosage form desired. Various excipients for formulating pharmaceutical compositions and techniques for preparing the composition are known in the art (see Remington: The Science and Practice of Pharmacy, 21st Edition, A. R. Gennaro, Lippincott, Williams & Wilkins, Baltimore, MD, 2006). The use of a conventional excipient medium may be contemplated within the scope of the present disclosure, except insofar as any conventional excipient medium may be incompatible with a substance or its derivatives, such as by producing any undesirable biological effect or otherwise interacting in a deleterious manner with any other component(s) of the pharmaceutical composition.

In some cases, the particle size of the lipid nanoparticle may be increased and/or decreased. The change in particle size may be able to help counter biological reaction such as, but not limited to, inflammation or may increase the biological effect of the modified mRNA delivered to mammals.

Pharmaceutically acceptable excipients used in the manufacture of pharmaceutical compositions include, but are not limited to, inert diluents, surface active agents and/or emulsifiers, preservatives, buffering agents, lubricating agents, and/or oils. Such excipients may optionally be included in the pharmaceutical formulations of the disclosure.

### Lipidoids

The synthesis of lipidoids has been extensively described and formulations containing these compounds are particularly suited for delivery of chimeric polynucleotides (see Mahon et al., Bioconjug Chem. 2010 21:1448-1454; Schroeder et al., J Intern Med. 2010 267:9-21; Akinc et al., Nat Biotechnol. 2008 26:561-569; Love et al., Proc Natl Acad Sci U S A. 2010 107:1864-1869; Siegwart et al., Proc

Natl Acad Sci U S A. 2011 108:12996-3001).

While these lipidoids have been used to effectively deliver double stranded small interfering RNA molecules in rodents and non-human primates (see Akinc et al., Nat Biotechnol. 2008 26:561-569; Frank-Kamenetsky et al., Proc Natl Acad Sci U S A. 2008 105:11915-11920; Akinc et al., Mol Ther. 2009 17:872-879; Love et al., Proc Natl Acad Sci U S A. 2010 107:1864-1869; Leuschner et al., Nat Biotechnol. 2011 29:1005-1010), the present disclosure describes their formulation and use in delivering chimeric polynucleotides.

Complexes, micelles, liposomes or particles can be prepared containing these lipidoids and therefore, can result in an effective delivery of the chimeric polynucleotide, as judged by the production of an encoded protein, following the injection of a lipidoid formulation via localized and/or systemic routes of administration. Lipidoid complexes of chimeric polynucleotides can be administered by various means including, but not limited to, intravenous, intramuscular, or subcutaneous routes.

*In vivo* delivery of nucleic acids may be affected by many parameters, including, but not limited to, the formulation composition, nature of particle PEGylation, degree of loading, polynucleotide to lipid ratio, and biophysical parameters such as, but not limited to, particle size (Akinc et al., Mol Ther. 2009 17:872-879). As an example, small changes in the anchor chain length of poly(ethylene glycol) (PEG) lipids may result in significant effects on in vivo efficacy. Formulations with the different lipidoids, including, but not limited to penta[3-(1-laurylaminopropionyl)]-triethylenetetramine hydrochloride (TETA-5LAP; aka 98N12-5, see Murugaiah et al., Analytical Biochemistry, 401:61 (2010), C12-200 (including derivatives and variants), and MD1, can be tested for *in vivo* activity.

The lipidoid referred to herein as "98N12-5" is disclosed by Akinc et al., Mol Ther. 2009 17:872-879.

The lipidoid referred to herein as "C12-200" is disclosed by Love et al., Proc Natl Acad Sci U S A. 2010 107:1864-1869 and Liu and Huang, Molecular Therapy. 2010 669-670.

The lipidoid formulations can include particles comprising either 3 or 4 or more components in addition to chimeric polynucleotides. As an example, formulations with certain lipidoids, include, but are not limited to, 98N12-5 and may contain 42% lipidoid, 48% cholesterol and 10% PEG (C14 alkyl chain length). As another example, formulations with certain lipidoids, include, but are not limited to, C12-200 and may contain 50% lipidoid, 10% disteroylphosphatidyl choline, 38.5% cholesterol, and 1.5% PEG-DMG.

In one case, a chimeric polynucleotide formulated with a lipidoid for systemic intravenous administration can target the liver. For example, a final optimized intravenous formulation using chimeric polynucleotides, and comprising a lipid molar composition of 42% 98N12-5, 48% cholesterol, and 10% PEG-lipid with a final weight ratio of about 7.5 to 1 total lipid to chimeric polynucleotides, and a C14 alkyl chain length on the PEG lipid, with a mean particle size of roughly 50-60 nm, can result in the distribution of the formulation to be greater than 90% to the liver.(see, Akinc et al., Mol Ther. 2009 17:872-879).

In another example, an intravenous formulation using a C12-200 (see US provisional application 61/175,770 and published international application WO2010129709) lipidoid may have a molar ratio of 50/10/38.5/1.5 of C12-200/disteroylphosphatidyl choline/cholesterol/PEG-DMG, with a weight ratio of 7 to 1 total lipid to chimeric polynucleotides, and a mean particle size of 80 nm may be effective to deliver chimeric polynucleotides to hepatocytes (see, Love et al., Proc Natl Acad Sci U S A. 2010 107:1864-1869). In another case, an MD1 lipidoid-containing formulation may be used to effectively deliver chimeric polynucleotides to hepatocytes *in vivo.*

The characteristics of optimized lipidoid formulations for intramuscular or subcutaneous routes may vary significantly depending on the target cell type and the ability of formulations to diffuse through the extracellular matrix into the blood stream. While a particle size of less than 150 nm may be desired for effective hepatocyte delivery due to the size of the endothelial fenestrae (see, Akinc et al., Mol Ther. 2009 17:872-879), use of a lipidoid-formulated chimeric polynucleotides to deliver the formulation to other cells types including, but not limited to, endothelial cells, myeloid cells, and muscle cells may not be similarly size-limited.

Use of lipidoid formulations to deliver siRNA *in vivo* to other non-hepatocyte cells such as myeloid cells and endothelium has been reported (see Akinc et al., Nat Biotechnol. 2008 26:561-569; Leuschner et al., Nat Biotechnol. 2011 29:1005-1010; Cho et al. Adv. Funct. Mater. 2009 19:3112-3118; 8th International Judah Folkman Conference, Cambridge, MA October 8-9, 2010).

Effective delivery to myeloid cells, such as monocytes, lipidoid formulations may have a similar component molar ratio. Different ratios of lipidoids and other components including, but not limited to, disteroylphosphatidyl choline, cholesterol and PEG-DMG, may be used to optimize the formulation of the chimeric polynucleotide for delivery to different cell types including, but not limited to, hepatocytes, myeloid cells, muscle cells, etc. For example, the component molar ratio may include, but is not limited to, 50% C12-200, 10% disteroylphosphatidyl choline, 38.5% cholesterol, and %1.5 PEG-DMG (see Leuschner et al., Nat Biotechnol 2011 29:1005-1010).

The use of lipidoid formulations for the localized delivery of nucleic acids to cells (such as, but not limited to, adipose cells and muscle cells) via either subcutaneous or intramuscular delivery, may not require all of the formulation components desired for systemic delivery, and as such may comprise only the lipidoid and the chimeric polynucleotide.

Combinations of different lipidoids may be used to improve the efficacy of chimeric polynucleotides directed protein production as the lipidoids may be able to increase cell transfection by the chimeric polynucleotide; and/or increase the translation of encoded protein (see Whitehead et al., Mol. Ther. 2011, 19:1688-1694).

### Liposomes, Lipoplexes, and Lipid Nanoparticles

The chimeric polynucleotides can be formulated using one or more liposomes, lipoplexes, or lipid nanoparticles. In one case, pharmaceutical compositions of chimeric polynucleotides include liposomes. Liposomes are artificially-prepared vesicles which may primarily be composed of a lipid bilayer and may be used as a delivery vehicle for the administration of nutrients and pharmaceutical formulations. Liposomes can be of different sizes such as, but not limited to, a multilamellar vesicle (MLV) which may be hundreds of nanometers in diameter and may contain a series of concentric bilayers separated by narrow aqueous compartments, a small unicellular vesicle (SUV) which may be smaller than 50 nm in diameter, and a large unilamellar vesicle (LUV) which may be between 50 and 500 nm in diameter. Liposome design may include, but is not limited to, opsonins or ligands in order to improve the attachment of liposomes to unhealthy tissue or to activate events such as, but not limited to, endocytosis. Liposomes may contain a low or a high pH in order to improve the delivery of the pharmaceutical formulations.

The formation of liposomes may depend on the physicochemical characteristics such as, but not limited to, the pharmaceutical formulation entrapped and the liposomal ingredients , the nature of the medium in which the lipid vesicles are dispersed, the effective concentration of the entrapped substance and its potential toxicity, any additional processes involved during the application and/or delivery of the vesicles, the optimization size, polydispersity and the shelf-life of the vesicles for the intended application, and the batch-to-batch reproducibility and possibility of large-scale production of safe and efficient liposomal products.

As a non-limiting example, liposomes such as synthetic membrane vesicles may be prepared by the methods, apparatus and devices described in US

Patent Publication No. US20130177638, US20130177637, US20130177636, US20130177635, US20130177634, US20130177633, US20130183375, US20130183373 and US20130183372.

In one case, pharmaceutical compositions described herein may include, without limitation, liposomes such as those formed from 1,2-dioleyloxy-*N*,*N-*dimethylaminopropane (DODMA) liposomes, DiLa2 liposomes from Marina Biotech (Bothell, WA), 1,2-dilinoleyloxy-3-dimethylaminopropane (DLin-DMA), 2,2-dilinoleyl-4-(2-dimethylaminoethyl)-[1,3]-dioxolane (DLin-KC2-DMA), and MC3 (US20100324120) and liposomes which may deliver small molecule drugs such as, but not limited to, DOXIL^{®} from Janssen Biotech, Inc. (Horsham, PA).

In one case, pharmaceutical compositions described herein may include, without limitation, liposomes such as those formed from the synthesis of stabilized plasmid-lipid particles (SPLP) or stabilized nucleic acid lipid particle (SNALP) that have been previously described and shown to be suitable for oligonucleotide delivery *in vitro* and *in vivo* (see Wheeler et al. Gene Therapy. 1999 6:271-281; Zhang et al. Gene Therapy. 1999 6:1438-1447; Jeffs et al. Pharm Res. 2005 22:362-372; Morrissey et al., Nat Biotechnol. 2005 2:1002-1007; Zimmermann et al., Nature. 2006 441:111-114; Heyes et al. J Contr Rel. 2005 107:276-287; Semple et al. Nature Biotech. 2010 28:172-176; Judge et al. J Clin Invest. 2009 119:661-673; deFougerolles Hum Gene Ther. 2008 19:125-132; U.S. Patent Publication No US20130122104).

The original manufacture method by Wheeler et al. was a detergent dialysis method, which was later improved by Jeffs et al. and is referred to as the spontaneous vesicle formation method. The liposome formulations are composed of 3 to 4 lipid components in addition to the chimeric polynucleotide. As an example a liposome can contain, but is not limited to, 55% cholesterol, 20% disteroylphosphatidyl choline (DSPC), 10% PEG-S-DSG, and 15% 1,2-dioleyloxy-*N*,*N*-dimethylaminopropane (DODMA), as described by Jeffs et al. As another example, certain liposome formulations may contain, but are not limited to, 48% cholesterol, 20% DSPC, 2% PEG-c-DMA, and 30% cationic lipid, where the cationic lipid can be 1,2-distearloxy-*N*,*N*-dimethylaminopropane (DSDMA), DODMA, DLin-DMA, or 1,2-dilinolenyloxy-3-dimethylaminopropane (DLenDMA), as described by Heyes et al.

In some cases, liposome formulations may comprise from about 25.0% cholesterol to about 40.0% cholesterol, from about 30.0% cholesterol to about 45.0% cholesterol, from about 35.0% cholesterol to about 50.0% cholesterol and/or from about 48.5% cholesterol to about 60% cholesterol. In a preferred case, formulations may comprise a percentage of cholesterol selected from the group consisting of 28.5%, 31.5%, 33.5%, 36.5%, 37.0%, 38.5%, 39.0% and 43.5%. In some cases, formulations may comprise from about 5.0% to about 10.0% DSPC and/or from about 7.0% to about 15.0% DSPC.

In one case, pharmaceutical compositions may include liposomes which may be formed to deliver chimeric polynucleotides which may encode at least one immunogen or any other polypeptide of interest. The chimeric polynucleotide may be encapsulated by the liposome and/or it may be contained in an aqueous core which may then be encapsulated by the liposome (see International Pub. Nos. WO2012031046, WO2012031043, WO2012030901 and WO2012006378 and US

Patent Publication No. US20130189351, US20130195969 and US20130202684).

In another case, liposomes may be formulated for targeted delivery. As a non-limiting example, the liposome may be formulated for targeted delivery to the liver. The liposome used for targeted delivery may include, but is not limited to, the liposomes described in and methods of making liposomes described in US Patent Publication No. US20130195967.

In another case, the chimeric polynucleotide which may encode an immunogen may be formulated in a cationic oil-in-water emulsion where the emulsion particle comprises an oil core and a cationic lipid which can interact with the chimeric polynucleotide anchoring the molecule to the emulsion particle (see International Pub. No. WO2012006380).

In one case, the chimeric polynucleotides may be formulated in a water-in-oil emulsion comprising a continuous hydrophobic phase in which the hydrophilic phase is dispersed. As a non-limiting example, the emulsion may be made by the methods described in International Publication No. WO201087791.

In another case, the lipid formulation may include at least cationic lipid, a lipid which may enhance transfection and a least one lipid which contains a hydrophilic head group linked to a lipid moiety (International Pub. No. WO2011076807 and U.S. Pub. No. 20110200582). In another case, the chimeric polynucleotides encoding an immunogen may be formulated in a lipid vesicle which may have crosslinks between functionalized lipid bilayers (see U.S. Pub. No. 20120177724).

In one case, the chimeric polynucleotides may be formulated in a liposome as described in International Patent Publication No. WO2013086526. The chimeric polynucleotides may be encapsulated in a liposome using reverse pH gradients and/or optimized internal buffer compositions as described in International Patent Publication No. WO2013086526.

In one case, the pharmaceutical compositions may be formulated in liposomes such as, but not limited to, DiLa2 liposomes (Marina Biotech, Bothell, WA), SMARTICLES^{®} (Marina Biotech, Bothell, WA), neutral DOPC (1,2-dioleoyl-sn-glycero-3-phosphocholine) based liposomes (e.g., siRNA delivery for ovarian cancer (Landen et al. Cancer Biology & Therapy 2006 5(12)1708-1713)
) and hyaluronan-coated liposomes (Quiet Therapeutics, Israel).

In one case, the cationic lipid may be a low molecular weight cationic lipid such as those described in US Patent Application No. 20130090372.

In one case, the chimeric polynucleotides may be formulated in a lipid vesicle which may have crosslinks between functionalized lipid bilayers.

In one case, the chimeric polynucleotides may be formulated in a liposome comprising a cationic lipid. The liposome may have a molar ratio of nitrogen atoms in the cationic lipid to the phosphates in the RNA (N:P ratio) of between 1:1 and 20:1 as described in International Publication No. WO2013006825.

In another case, the liposome may have a N:P ratio of greater than 20:1 or less than 1:1.

In one case, the chimeric polynucleotides may be formulated in a lipid-polycation complex. The formation of the lipid-polycation complex may be accomplished by methods known in the art and/or as described in U.S. Pub. No. 20120178702. As a non-limiting example, the polycation may include a cationic peptide or a polypeptide such as, but not limited to, polylysine, polyornithine and/or polyarginine and the cationic peptides described in International Pub. No. WO2012013326 or US Patent Pub. No. US20130142818. In another case, the chimeric polynucleotides may be formulated in a lipid-polycation complex which may further include a neutral lipid such as, but not limited to, cholesterol or dioleoyl phosphatidylethanolamine (DOPE).

In one case, the chimeric polynucleotide may be formulated in an aminoalcohol lipidoid. Aminoalcohol lipidoids which may be used may be prepared by the methods described in U.S. Patent No. 8,450,298.

The liposome formulation may be influenced by, but not limited to, the selection of the cationic lipid component, the degree of cationic lipid saturation, the nature of the PEGylation, ratio of all components and biophysical parameters such as size. In one example by Semple et al. (Semple et al. Nature Biotech. 2010 28:172-176), the liposome formulation was composed of 57.1 % cationic lipid, 7.1% dipalmitoylphosphatidylcholine, 34.3 % cholesterol, and 1.4% PEG-c-DMA. As another example, changing the composition of the cationic lipid could more effectively deliver siRNA to various antigen presenting cells (Basha et al. Mol Ther. 2011 19:2186-2200).

In some cases, liposome formulations may comprise from about 35 to about 45% cationic lipid, from about 40% to about 50% cationic lipid, from about 50% to about 60% cationic lipid and/or from about 55% to about 65% cationic lipid. In some cases, the ratio of lipid to mRNA in liposomes may be from about 5:1 to about 20:1, from about 10:1 to about 25:1, from about 15:1 to about 30:1 and/or at least 30:1.

In some cases, the ratio of PEG in the lipid nanoparticle (LNP) formulations may be increased or decreased and/or the carbon chain length of the PEG lipid may be modified from C14 to C18 to alter the pharmacokinetics and/or biodistribution of the LNP formulations. As a non-limiting example, LNP formulations may contain from about 0.5% to about 3.0%, from about 1.0% to about 3.5%, from about 1.5% to about 4.0%, from about 2.0% to about 4.5%, from about 2.5% to about 5.0% and/or from about 3.0% to about 6.0% of the lipid molar ratio of PEG-c-DOMG as compared to the cationic lipid, DSPC and cholesterol. In another case the PEG-c-DOMG may be replaced with a PEG lipid such as, but not limited to, PEG- DSG (1,2-Distearoyl-sn-glycerol, methoxypolyethylene glycol), PEG-DMG (1,2-Dimyristoyl-sn-glycerol) and/or PEG-DPG (1,2-Dipalmitoyl-sn-glycerol, methoxypolyethylene glycol). The cationic lipid may be selected from any lipid known in the art such as, but not limited to, DLin-MC3-DMA, DLin-DMA, C12-200 and DLin-KC2-DMA.

In one case, the chimeric polynucleotides may be formulated in a lipid nanoparticle such as those described in International Publication No. WO2012170930.

In one case, the formulation comprising the chimeric polynucleotide is a nanoparticle which may comprise at least one lipid. The lipid may be selected from, but is not limited to, DLin-DMA, DLin-K-DMA, 98N12-5, C12-200, DLin-MC3-DMA, DLin-KC2-DMA, DODMA, PLGA, PEG, PEG-DMG, PEGylated lipids and amino alcohol lipids. In another case, the lipid may be a cationic lipid such as, but not limited to, DLin-DMA, DLin-D-DMA, DLin-MC3-DMA, DLin-KC2-DMA, DODMA and amino alcohol lipids. The amino alcohol cationic lipid may be the lipids described in and/or made by the methods described in US Patent Publication No. US20130150625. As a non-limiting example, the cationic lipid may be 2-amino-3-[(9Z,12Z)-octadeca-9,12-dien-1-yloxy-2-{[(9Z,2Z)-octadeca-9,12-dien-1-yloxy]methyl}propan-1-ol (Compound 1 in US20130150625); 2-amino-3-[(9Z)-octadec-9-en-1-yloxy]-2-{[(9Z)-octadec-9-en-1-yloxy]methyl}propan-1-ol (Compound 2 in US20130150625); 2-amino-3-[(9Z,12Z)-octadeca-9,12-dien-1-yloxy]-2-[(octyloxy)methyl]propan-1-ol (Compound 3 in US20130150625); and 2-(dimethylamino)-3-[(9Z,12Z)-octadeca-9,12-dien-1-yloxy]-2-1[(9Z,12Z)-octadeca-9,12-dien-1-yloxy]methyl}propan-1-ol (Compound 4 in US20130150625); or any pharmaceutically acceptable salt or stereoisomer thereof.

In one case, the cationic lipid may be selected from, but not limited to, a cationic lipid described in paragraph [000398] in co-pending International Publication No. WO2015034928.

In one case, the lipid may be a cleavable lipid such as those described in International Publication No. WO2012170889.

In another case, the lipid may be a cationic lipid such as, but not limited to, Formula (I) of U.S. Patent Application No. US20130064894.

In one case, the cationic lipid may be synthesized by methods known in the art and/or as described in International Publication Nos. WO2012040184, WO2011153120, WO2011149733, WO2011090965, WO2011043913, WO2011022460, WO2012061259, WO2012054365, WO2012044638, WO2010080724, WO201021865, WO2013086373 and WO2013086354.

In another case, the cationic lipid may be a trialkyl cationic lipid. Non-limiting examples of trialkyl cationic lipids and methods of making and using the trialkyl cationic lipids are described in International Patent Publication No. WO2013126803.

In one case, the LNP formulations of the chimeric polynucleotides may contain PEG-c-DOMG at 3% lipid molar ratio. In another embodiment, the LNP formulations chimeric polynucleotides may contain PEG-c-DOMG at 1.5% lipid molar ratio.

In one case, the pharmaceutical compositions of the chimeric polynucleotides may include at least one of the PEGylated lipids described in International Publication No. WO2012099755

In one case, the LNP formulation may contain PEG-DMG 2000 (1,2-dimyristoyl-sn-glycero-3-phophoethanolamine-N-[methoxy(polyethylene glycol)-2000). In one case, the LNP formulation may contain PEG-DMG 2000, a cationic lipid known in the art and at least one other component. In another case, the LNP formulation may contain PEG-DMG 2000, a cationic lipid known in the art, DSPC and cholesterol. As a non-limiting example, the LNP formulation may contain PEG-DMG 2000, DLin-DMA, DSPC and cholesterol. As another non-limiting example the LNP formulation may contain PEG-DMG 2000, DLin-DMA, DSPC and cholesterol in a molar ratio of 2:40:10:48 (see e.g., Geall et al., Nonviral delivery of self-amplifying RNA vaccines, PNAS 2012; PMID: 22908294).

In one case, the LNP formulation may be formulated by the methods described in International Publication Nos. WO2011127255 or WO2008103276.

As a non-limiting example, the chimeric polynucleotides described herein may be encapsulated in LNP formulations as described in WO2011127255 and/or WO2008103276.

In one case, the chimeric polynucleotides described herein may be formulated in a nanoparticle to be delivered by a parenteral route as described in U.S. Pub. No. US20120207845.

In one case, the chimeric polynucleotides may be formulated in a lipid nanoparticle made by the methods described in US Patent Publication No US20130156845 or International Publication No WO2013093648 or WO2012024526.

The lipid nanoparticles described herein may be made in a sterile environment by the system and/or methods described in US Patent Publication No. US20130164400.

In one case, the LNP formulation may be formulated in a nanoparticle such as a nucleic acid-lipid particle described in US Patent No. 8,492,359. As a non-limiting example, the lipid particle may comprise one or more active agents or therapeutic agents; one or more cationic lipids comprising from about 50 mol % to about 85 mol % of the total lipid present in the particle; one or more non-cationic lipids comprising from about 13 mol % to about 49.5 mol % of the total lipid present in the particle; and one or more conjugated lipids that inhibit aggregation of particles comprising from about 0.5 mol % to about 2 mol % of the total lipid present in the particle. The nucleic acid in the nanoparticle may be the chimeric polynucleotides described herein and/or are known in the art.

In one case, the LNP formulation may be formulated by the methods described in International Publication Nos. WO2011127255 or WO2008103276.

As a non-limiting example, modified RNA described herein may be encapsulated in LNP formulations as described in WO2011127255 and/or WO2008103276.

In one case, LNP formulations described herein may comprise a polycationic composition. As a non-limiting example, the polycationic composition may be selected from formula 1-60 of US Patent Publication No. US20050222064.

In another case, the LNP formulations comprising a polycationic composition may be used for the delivery of the modified RNA described herein *in vivo* and/or *in vitro.*

In one case, the LNP formulations described herein may additionally comprise a permeability enhancer molecule. Non-limiting permeability enhancer molecules are described in US Patent Publication No. US20050222064.

In one case, the pharmaceutical compositions may be formulated in liposomes such as, but not limited to, DiLa2 liposomes (Marina Biotech, Bothell, WA), SMARTICLES^{®} (Marina Biotech, Bothell, WA), neutral DOPC (1,2-dioleoylsn-glycero-3-phosphocholine) based liposomes (e.g., siRNA delivery for ovarian cancer (Landen et al. Cancer Biology & Therapy 2006 5(12)1708-1713) ) and hyaluronan-coated liposomes (Quiet Therapeutics, Israel).

In one case, the chimeric polynucleotides may be formulated in a lyophilized gel-phase liposomal composition as described in US Publication No. US2012060293.

The nanoparticle formulations may comprise a phosphate conjugate. The phosphate conjugate may increase *in vivo* circulation times and/or increase the targeted delivery of the nanoparticle. Phosphate conjugates for use with the present disclosure may be made by the methods described in International Application No. WO2013033438 or US Patent Publication No. US20130196948.

As a non-limiting example, the phosphate conjugates may include a compound of any one of the formulas described in International Application No. WO2013033438.

The nanoparticle formulation may comprise a polymer conjugate. The polymer conjugate may be a water soluble conjugate. The polymer conjugate may have a structure as described in U.S. Patent Application No. 20130059360. In one case, polymer conjugates with the chimeric polynucleotides may be made using the methods and/or segmented polymeric reagents described in U.S. Patent Application No. 20130072709. In another case, the polymer conjugate may have pendant side groups comprising ring moieties such as, but not limited to, the polymer conjugates described in US Patent Publication No. US20130196948.

The nanoparticle formulations may comprise a conjugate to enhance the delivery of nanoparticles of the present disclosure in a subject. Further, the conjugate may inhibit phagocytic clearance of the nanoparticles in a subject. In one case, the conjugate may be a "self' peptide designed from the human membrane protein CD47 (e.g., the "self' particles described by Rodriguez et al (Science 2013 339, 971-975).

As shown by Rodriguez et al. the self-peptides delayed macrophage-mediated clearance of nanoparticles which enhanced delivery of the nanoparticles. In another case, the conjugate may be the membrane protein CD47 (e.g., see Rodriguez et al. Science 2013 339, 971-975

Rodriguez et al. showed that, similarly to "self' peptides, CD47 can increase the circulating particle ratio in a subject as compared to scrambled peptides and PEG coated nanoparticles.

In one case, the chimeric polynucleotides of the present disclosure are formulated in nanoparticles which comprise a conjugate to enhance the delivery of the nanoparticles of the present disclosure in a subject. The conjugate may be the CD47 membrane or the conjugate may be derived from the CD47 membrane protein, such as the "self' peptide described previously. In another case the nanoparticle may comprise PEG and a conjugate of CD47 or a derivative thereof. In yet another case, the nanoparticle may comprise both the "self' peptide described above and the membrane protein CD47.

In another case, a "self' peptide and/or CD47 protein may be conjugated to a virus-like particle or pseudovirion, as described herein for delivery of the chimeric polynucleotides of the present disclosure.

In another case, pharmaceutical compositions comprising the chimeric polynucleotides of the present disclosure and a conjugate which may have a degradable linkage. Non-limiting examples of conjugates include an aromatic moiety comprising an ionizable hydrogen atom, a spacer moiety, and a water-soluble polymer. As a non-limiting example, pharmaceutical compositions comprising a conjugate with a degradable linkage and methods for delivering such pharmaceutical compositions are described in US Patent Publication No. US20130184443.

The nanoparticle formulations may be a carbohydrate nanoparticle comprising a carbohydrate carrier and a chimeric polynucleotide. As a non-limiting example, the carbohydrate carrier may include, but is not limited to, an anhydride-modified phytoglycogen or glycogen-type material, phtoglycogen octenyl succinate, phytoglycogen beta-dextrin, anhydride-modified phytoglycogen beta-dextrin. (See e.g., International Publication No. WO2012109121).

Nanoparticle formulations may be coated with a surfactant or polymer in order to improve the delivery of the particle. In one case, the nanoparticle may be coated with a hydrophilic coating such as, but not limited to, PEG coatings and/or coatings that have a neutral surface charge. The hydrophilic coatings may help to deliver nanoparticles with larger payloads such as, but not limited to, chimeric polynucleotides within the central nervous system. As a non-limiting example nanoparticles comprising a hydrophilic coating and methods of making such nanoparticles are described in US Patent Publication No. US20130183244.

In one case, the lipid nanoparticles may be hydrophilic polymer particles. Non-limiting examples of hydrophilic polymer particles and methods of making hydrophilic polymer particles are described in US Patent Publication No. US20130210991

In another case, the lipid nanoparticles may be hydrophobic polymer particles.

Lipid nanoparticle formulations may be improved by replacing the cationic lipid with a biodegradable cationic lipid which is known as a rapidly eliminated lipid nanoparticle (reLNP). Ionizable cationic lipids, such as, but not limited to, DLinDMA, DLin-KC2-DMA, and DLin-MC3-DMA, have been shown to accumulate in plasma and tissues over time and may be a potential source of toxicity. The rapid metabolism of the rapidly eliminated lipids can improve the tolerability and therapeutic index of the lipid nanoparticles by an order of magnitude from a 1 mg/kg dose to a 10 mg/kg dose in rat. Inclusion of an enzymatically degraded ester linkage can improve the degradation and metabolism profile of the cationic component, while still maintaining the activity of the reLNP formulation. The ester linkage can be internally located within the lipid chain or it may be terminally located at the terminal end of the lipid chain. The internal ester linkage may replace any carbon in the lipid chain.

In one case, the internal ester linkage may be located on either side of the saturated carbon, such as the reLNPs described in paragraph [000426] of copending International Publication No. WO2015034928.

In one case, an immune response may be elicited by delivering a lipid nanoparticle which may include a nanospecies, a polymer and an immunogen. (U.S. Publication No. 20120189700 and International Publication No. WO2012099805; The polymer may encapsulate the nanospecies or partially encapsulate the nanospecies. The immunogen may be a recombinant protein, a modified RNA and/or a chimeric polynucleotide described herein. In one case, the lipid nanoparticle may be formulated for use in a vaccine such as, but not limited to, against a pathogen.

Lipid nanoparticles may be engineered to alter the surface properties of particles so the lipid nanoparticles may penetrate the mucosal barrier. Mucus is located on mucosal tissue such as, but not limited to, oral (e.g., the buccal and esophageal membranes and tonsil tissue), ophthalmic, gastrointestinal (e.g., stomach, small intestine, large intestine, colon, rectum), nasal, respiratory (e.g., nasal, pharyngeal, tracheal and bronchial membranes), genital (e.g., vaginal, cervical and urethral membranes). Nanoparticles larger than 10-200 nm which are preferred for higher drug encapsulation efficiency and the ability to provide the sustained delivery of a wide array of drugs have been thought to be too large to rapidly diffuse through mucosal barriers. Mucus is continuously secreted, shed, discarded or digested and recycled so most of the trapped particles may be removed from the mucosla tissue within seconds or within a few hours. Large polymeric nanoparticles (200nm -500nm in diameter) which have been coated densely with a low molecular weight polyethylene glycol (PEG) diffused through mucus only 4 to 6-fold lower than the same particles diffusing in water (Lai et al. PNAS 2007 104(5):1482-487; Lai et al. Adv Drug Deliv Rev. 2009 61(2): 158-171).

The transport of nanoparticles may be determined using rates of permeation and/or fluorescent microscopy techniques including, but not limited to, fluorescence recovery after photobleaching (FRAP) and high resolution multiple particle tracking (MPT). As a non-limiting example, compositions which can penetrate a mucosal barrier may be made as described in U.S. Pat. No. 8,241,670 or International Patent Publication No. WO2013110028.

The lipid nanoparticle engineered to penetrate mucus may comprise a polymeric material (i.e. a polymeric core) and/or a polymer-vitamin conjugate and/or a tri-block co-polymer. The polymeric material may include, but is not limited to, polyamines, polyethers, polyamides, polyesters, polycarbamates, polyureas, polycarbonates, poly(styrenes), polyimides, polysulfones, polyurethanes, polyacetylenes, polyethylenes, polyethyeneimines, polyisocyanates, polyacrylates, polymethacrylates, polyacrylonitriles, and polyarylates. The polymeric material may be biodegradable and/or biocompatible. Non-limiting examples of biocompatible polymers are described in International Patent Publication No. WO2013116804. The polymeric material may additionally be irradiated. As a non-limiting example, the polymeric material may be gamma irradiated (See e.g., International App. No. WO201282165). Non-limiting examples of specific polymers include poly(caprolactone) (PCL), ethylene vinyl acetate polymer (EVA), poly(lactic acid) (PLA), poly(L-lactic acid) (PLLA), poly(glycolic acid) (PGA), poly(lactic acid-co-glycolic acid) (PLGA), poly(L-lactic acid-co-glycolic acid) (PLLGA), poly(D,L-lactide) (PDLA), poly(L-lactide) (PLLA), poly(D,L-lactide-co-caprolactone), poly(D,L-lactide-co-caprolactone-co-glycolide), poly(D,L-lactide-co-PEO-co-D,L-lactide), poly(D,L-lactide-co-PPO-co-D,L-lactide), polyalkyl cyanoacralate, polyurethane, poly-L-lysine (PLL), hydroxypropyl methacrylate (HPMA), polyethyleneglycol, poly-L-glutamic acid, poly(hydroxy acids), polyanhydrides, polyorthoesters, poly(ester amides), polyamides, poly(ester ethers), polycarbonates, polyalkylenes such as polyethylene and polypropylene, polyalkylene glycols such as poly(ethylene glycol) (PEG), polyalkylene oxides (PEO), polyalkylene terephthalates such as poly(ethylene terephthalate), polyvinyl alcohols (PVA), polyvinyl ethers, polyvinyl esters such as poly(vinyl acetate), polyvinyl halides such as poly(vinyl chloride) (PVC), polyvinylpyrrolidone, polysiloxanes, polystyrene (PS), polyurethanes, derivatized celluloses such as alkyl celluloses, hydroxyalkyl celluloses, cellulose ethers, cellulose esters, nitro celluloses, hydroxypropylcellulose, carboxymethylcellulose, polymers of acrylic acids, such as poly(methyl(meth)acrylate) (PMMA), poly(ethyl(meth)acrylate), poly(butyl(meth)acrylate), poly(isobutyl(meth)acrylate), poly(hexyl(meth)acrylate), poly(isodecyl(meth)acrylate), poly(lauryl(meth)acrylate), poly(phenyl(meth)acrylate), poly(methyl acrylate), poly(isopropyl acrylate), poly(isobutyl acrylate), poly(octadecyl acrylate) and copolymers and mixtures thereof, polydioxanone and its copolymers, polyhydroxyalkanoates, polypropylene fumarate, polyoxymethylene, poloxamers, poly(ortho)esters, poly(butyric acid), poly(valeric acid), poly(lactide-co-caprolactone), PEG-PLGA-PEG and trimethylene carbonate, polyvinylpyrrolidone. The lipid nanoparticle may be coated or associated with a co-polymer such as, but not limited to, a block co-polymer (such as a branched polyether-polyamide block copolymer described in International Publication No. WO2013012476 ), and (poly(ethylene glycol))-(poly(propylene oxide))-(poly(ethylene glycol)) triblock copolymer (see e.g., US Publication 20120121718 and US Publication 20100003337 and U.S. Pat. No. 8,263,665). The co-polymer may be a polymer that is generally regarded as safe (GRAS) and the formation of the lipid nanoparticle may be in such a way that no new chemical entities are created. For example, the lipid nanoparticle may comprise poloxamers coating PLGA nanoparticles without forming new chemical entities which are still able to rapidly penetrate human mucus (Yang et al. Angew. Chem. Int. Ed. 2011 50:2597-2600). A non-limiting scalable method to produce nanoparticles which can penetrate human mucus is described by Xu et al. (See e.g., J Control Release 2013, 170(2):279-86).

The vitamin of the polymer-vitamin conjugate may be vitamin E. The vitamin portion of the conjugate may be substituted with other suitable components such as, but not limited to, vitamin A, vitamin E, other vitamins, cholesterol, a hydrophobic moiety, or a hydrophobic component of other surfactants (e.g., sterol chains, fatty acids, hydrocarbon chains and alkylene oxide chains).

The lipid nanoparticle engineered to penetrate mucus may include surface altering agents such as, but not limited to, chimeric polynucleotides, anionic proteins (e.g., bovine serum albumin), surfactants (e.g., cationic surfactants such as for example dimethyldioctadecyl-ammonium bromide), sugars or sugar derivatives (e.g., cyclodextrin), nucleic acids, polymers (e.g., heparin, polyethylene glycol and poloxamer), mucolytic agents (e.g., N-acetylcysteine, mugwort, bromelain, papain, clerodendrum, acetylcysteine, bromhexine, carbocisteine, eprazinone, mesna, ambroxol, sobrerol, domiodol, letosteine, stepronin, tiopronin, gelsolin, thymosin β4 dornase alfa, neltenexine, erdosteine) and various DNases including rhDNase. The surface altering agent may be embedded or enmeshed in the particle's surface or disposed (e.g., by coating, adsorption, covalent linkage, or other process) on the surface of the lipid nanoparticle. (see e.g., US Publication 20100215580 and US Publication 20080166414 and US20130164343).

In one case, the mucus penetrating lipid nanoparticles may comprise at least one chimeric polynucleotide described herein. The chimeric polynucleotide may be encapsulated in the lipid nanoparticle and/or disposed on the surface of the particle. The chimeric polynucleotide may be covalently coupled to the lipid nanoparticle. Formulations of mucus penetrating lipid nanoparticles may comprise a plurality of nanoparticles. Further, the formulations may contain particles which may interact with the mucus and alter the structural and/or adhesive properties of the surrounding mucus to decrease mucoadhesion which may increase the delivery of the mucus penetrating lipid nanoparticles to the mucosal tissue.

In another case, the mucus penetrating lipid nanoparticles may be a hypotonic formulation comprising a mucosal penetration enhancing coating. The formulation may be hypotonic for the epithelium to which it is being delivered. Non-limiting examples of hypotonic formulations may be found in International Patent Publication No. WO2013110028.

In one case, in order to enhance the delivery through the mucosal barrier the formulation may comprise or be a hypotonic solution. Hypotonic solutions were found to increase the rate at which mucoinert particles such as, but not limited to, mucus-penetrating particles, were able to reach the vaginal epithelial surface (See e.g., Ensign et al. Biomaterials 2013 34(28):6922-9).

In one case, the chimeric polynucleotide is formulated as a lipoplex, such as, without limitation, the ATUPLEX^{™} system, the DACC system, the DBTC system and other siRNA-lipoplex technology from Silence Therapeutics (London, United Kingdom), STEMFECT^{™} from STEMGENT^{®} (Cambridge, MA), and polyethylenimine (PEI) or protamine-based targeted and non-targeted delivery of nucleic acids (Aleku et al. Cancer Res. 2008 68:9788-9798; Strumberg et al. Int J Clin Pharmacol Ther 2012 50:76-78; Santel et al., Gene Ther 2006 13:1222-1234; Santel et al., Gene Ther 2006 13:1360-1370; Gutbier et al., Pulm Pharmacol. Ther. 2010 23:334-344; Kaufmann et al. Microvasc Res 2010 80:286-293Weide et al. J Immunother. 2009 32:498-507; Weide et al. J Immunother. 2008 31:180-188; Pascolo Expert Opin. Biol. Ther. 4:1285-1294; Fotin-Mleczek et al., 2011 J. Immunother. 34:1-15; Song et al., Nature Biotechnol. 2005, 23:709-717; Peer et al., Proc Natl Acad Sci U S A. 2007 6;104:4095-4100; deFougerolles Hum Gene Ther. 2008 19:125-132).

In one case such formulations may also be constructed or compositions altered such that they passively or actively are directed to different cell types *in vivo,* including but not limited to hepatocytes, immune cells, tumor cells, endothelial cells, antigen presenting cells, and leukocytes (Akinc et al. Mol Ther. 2010 18:1357-1364; Song et al., Nat Biotechnol. 2005 23:709-717; Judge et al., J Clin Invest. 2009 119:661-673; Kaufmann et al., Microvasc Res 2010 80:286-293; Santel et al., Gene Ther 2006 13:1222-1234; Santel et al., Gene Ther 2006 13:1360-1370; Gutbier et al., Pulm Pharmacol. Ther. 2010 23:334-344; Basha et al., Mol. Ther. 2011 19:2186-2200; Fenske and Cullis, Expert Opin Drug Deliv. 2008 5:25-44; Peer et al., Science. 2008 319:627-630; Peer and Lieberman, Gene Ther. 2011 18:1127-1133). One example of passive targeting of formulations to liver cells includes the DLin-DMA, DLin-KC2-DMA and DLin-MC3-DMA-based lipid nanoparticle formulations which have been shown to bind to apolipoprotein E and promote binding and uptake of these formulations into hepatocytes in vivo (Akinc et al. Mol Ther. 2010 18:1357-1364). Formulations can also be selectively targeted through expression of different ligands on their surface as exemplified by, but not limited by, folate, transferrin, N-acetylgalactosamine (GalNAc), and antibody targeted approaches (Kolhatkar et al., Curr Drug Discov Technol. 2011 8:197-206; Musacchio and Torchilin, Front Biosci. 2011 16:1388-1412; Yu et al., Mol Membr Biol. 2010 27:286-298; Patil et al., Crit Rev Ther Drug Carrier Syst. 2008 25:1-61; Benoit et al., Biomacromolecules. 2011 12:2708-2714; Zhao et al., Expert Opin Drug Deliv. 2008 5:309-319; Akinc et al., Mol Ther. 2010 18:1357-1364; Srinivasan et al., Methods Mol Biol. 2012 820:105-116; Ben-Arie et al., Methods Mol Biol. 2012 757:497-507; Peer 2010 J Control Release. 20:63-68; Peer et al., Proc Natl Acad Sci U S A. 2007 104:4095-4100; Kim et al., Methods Mol Biol. 2011 721:339-353; Subramanya et al., Mol Ther. 2010 18:2028-2037; Song et al., Nat Biotechnol. 2005 23:709-717; Peer et al., Science. 2008 319:627-630; Peer and Lieberman, Gene Ther. 2011 18:1127-1133).

In one case, the chimeric polynucleotide is formulated as a solid lipid nanoparticle. A solid lipid nanoparticle (SLN) may be spherical with an average diameter between 10 to 1000 nm. SLN possess a solid lipid core matrix that can solubilize lipophilic molecules and may be stabilized with surfactants and/or emulsifiers. In a further case, the lipid nanoparticle may be a self-assembly lipid-polymer nanoparticle (see Zhang et al., ACS Nano, 2008, 2 (8), pp 1696-1702).

As a non-limiting example, the SLN may be the SLN described in International Patent Publication No. WO2013105101.

As another non-limiting example, the SLN may be made by the methods or processes described in International Patent Publication No. WO2013105101.

Liposomes, lipoplexes, or lipid nanoparticles may be used to improve the efficacy of chimeric polynucleotides directed protein production as these formulations may be able to increase cell transfection by the chimeric polynucleotide; and/or increase the translation of encoded protein. One such example involves the use of lipid encapsulation to enable the effective systemic delivery of polyplex plasmid DNA (Heyes et al., Mol Ther. 2007 15:713-720).

The liposomes, lipoplexes, or lipid nanoparticles may also be used to increase the stability of the chimeric polynucleotide.

In one case, the chimeric polynucleotides of the present disclosure can be formulated for controlled release and/or targeted delivery. As used herein, "controlled release" refers to a pharmaceutical composition or compound release profile that conforms to a particular pattern of release to effect a therapeutic outcome. In one case, the chimeric polynucleotides may be encapsulated into a delivery agent described herein and/or known in the art for controlled release and/or targeted delivery. As used herein, the term "encapsulate" means to enclose, surround or encase. As it relates to the formulation of the compounds of the disclosure, encapsulation may be substantial, complete or partial. The term "substantially encapsulated" means that at least greater than 50, 60, 70, 80, 85, 90, 95, 96, 97, 98, 99, 99.9, 99.9 or greater than 99.999% of the pharmaceutical composition or compound may be enclosed, surrounded or encased within the delivery agent. "Partially encapsulation" means that less than 10, 10, 20, 30, 40 50 or less of the pharmaceutical composition or compound may be enclosed, surrounded or encased within the delivery agent. Advantageously, encapsulation may be determined by measuring the escape or the activity of the pharmaceutical composition or compound of the disclosure using fluorescence and/or electron micrograph. For example, at least 1, 5, 10, 20, 30, 40, 50, 60, 70, 80, 85, 90, 95, 96, 97, 98, 99, 99.9, 99.99 or greater than 99.99% of the pharmaceutical composition or compound of the disclosure are encapsulated in the delivery agent.

In one case, the controlled release formulation may include, but is not limited to, tri-block co-polymers. As a non-limiting example, the formulation may include two different types of tri-block co-polymers (International Pub. No. WO2012131104 and WO2012131106).

In another case, the chimeric polynucleotides may be encapsulated into a lipid nanoparticle or a rapidly eliminated lipid nanoparticle and the lipid nanoparticles or a rapidly eliminated lipid nanoparticle may then be encapsulated into a polymer, hydrogel and/or surgical sealant described herein and/or known in the art. As a non-limiting example, the polymer, hydrogel or surgical sealant may be PLGA, ethylene vinyl acetate (EVAc), poloxamer, GELSITE^{®} (Nanotherapeutics, Inc. Alachua, FL), HYLENEX^{®} (Halozyme Therapeutics, San Diego CA), surgical sealants such as fibrinogen polymers (Ethicon Inc. Cornelia, GA), TISSELL^{®} (Baxter International, Inc Deerfield, IL), PEG-based sealants, and COSEAL^{®} (Baxter International, Inc Deerfield, IL).

In another case, the lipid nanoparticle may be encapsulated into any polymer known in the art which may form a gel when injected into a subject. As another non-limiting example, the lipid nanoparticle may be encapsulated into a polymer matrix which may be biodegradable.

In one case, the chimeric polynucleotide formulation for controlled release and/or targeted delivery may also include at least one controlled release coating. Controlled release coatings include, but are not limited to, OPADRY^{®}, polyvinylpyrrolidone/vinyl acetate copolymer, polyvinylpyrrolidone, hydroxypropyl methylcellulose, hydroxypropyl cellulose, hydroxyethyl cellulose, EUDRAGIT RL^{®}, EUDRAGIT RS^{®} and cellulose derivatives such as ethylcellulose aqueous dispersions (AQUACOAT^{®} and SURELEASE^{®}).

In one case, the controlled release and/or targeted delivery formulation may comprise at least one degradable polyester which may contain polycationic side chains. Degradable polyesters include, but are not limited to, poly(serine ester), poly(L-lactide-co-L-lysine), poly(4-hydroxy-L-proline ester), and combinations thereof. In another case, the degradable polyesters may include a PEG conjugation to form a PEGylated polymer.

In one case, the controlled release and/or targeted delivery formulation comprising at least one chimeric polynucleotide may comprise at least one PEG and/or PEG related polymer derivatives as described in US Patent No. 8,404,222.

In another case, the controlled release delivery formulation comprising at least one chimeric polynucleotide may be the controlled release polymer system described in US20130130348.

In one case, the chimeric polynucleotides may be encapsulated in a therapeutic nanoparticle. Therapeutic nanoparticles may be formulated by methods described herein and known in the art such as, but not limited to, International Pub Nos. WO2010005740, WO2010030763, WO2010005721, WO2010005723, WO2012054923, US Pub. Nos. US20110262491, US20100104645, US20100087337, US20100068285, US20110274759, US20100068286, US20120288541, US20130123351 and US20130230567 and US Pat No. 8,206,747, 8,293,276, 8,318,208 and 8,318,211. In another case, therapeutic polymer nanoparticles may be identified by the methods described in US Pub No. US20120140790.

In one case, the therapeutic nanoparticle may be formulated for sustained release. As used herein, "sustained release" refers to a pharmaceutical composition or compound that conforms to a release rate over a specific period of time. The period of time may include, but is not limited to, hours, days, weeks, months and years. As a non-limiting example, the sustained release nanoparticle may comprise a polymer and a therapeutic agent such as, but not limited to, the chimeric polynucleotides of the present disclosure (see International Pub No. 2010075072 and US Pub No. US20100216804, US20110217377 and US20120201859 ). In another non-limiting example, the sustained release formulation may comprise agents which permit persistent bioavailability such as, but not limited to, crystals, macromolecular gels and/or particulate suspensions (see US Patent Publication No US20130150295 ).

In one case, the therapeutic nanoparticles may be formulated to be target specific. As a non-limiting example, the therapeutic nanoparticles may include a corticosteroid (see International Pub. No. WO2011084518). ). In one case, the therapeutic nanoparticles may be formulated to be cancer specific. As a non-limiting example, the therapeutic nanoparticles may be formulated in nanoparticles described in International Pub No. WO2008121949, WO2010005726, WO2010005725, WO2011084521 and US Pub No. US20100069426, US20120004293 and US20100104655.

In one case the nanoparticles may comprise a polymeric matrix. As a non-limiting example, the nanoparticle may comprise two or more polymers such as, but not limited to, polyethylenes, polycarbonates, polyanhydrides, polyhydroxyacids, polypropylfumerates, polycaprolactones, polyamides, polyacetals, polyethers, polyesters, poly(orthoesters), polycyanoacrylates, polyvinyl alcohols, polyurethanes, polyphosphazenes, polyacrylates, polymethacrylates, polycyanoacrylates, polyureas, polystyrenes, polyamines, polylysine, poly(ethylene imine), poly(serine ester), poly(L-lactide-co-L-lysine), poly(4-hydroxy-L-proline ester) or combinations thereof.

In one case, the therapeutic nanoparticle comprises a diblock copolymer. In one case, the diblock copolymer may include PEG in combination with a polymer such as, but not limited to, polyethylenes, polycarbonates, polyanhydrides, polyhydroxyacids, polypropylfumerates, polycaprolactones, polyamides, polyacetals, polyethers, polyesters, poly(orthoesters), polycyanoacrylates, polyvinyl alcohols, polyurethanes, polyphosphazenes, polyacrylates, polymethacrylates, polycyanoacrylates, polyureas, polystyrenes, polyamines, polylysine, poly(ethylene imine), poly(serine ester), poly(L-lactide-co-L-lysine), poly(4-hydroxy-L-proline ester) or combinations thereof. In another case, the diblock copolymer may comprise the diblock copolymers described in European Patent Publication No. . In yet another case, the diblock copolymer may be a high-X diblock copolymer such as those described in International Patent Publication No. WO2013120052.

As a non-limiting example the therapeutic nanoparticle comprises a PLGA-PEG block copolymer (see US Pub. No. US20120004293 and US Pat No. 8,236,330). In another non-limiting example, the therapeutic nanoparticle is a stealth nanoparticle comprising a diblock copolymer of PEG and PLA or PEG and PLGA (see US Pat No 8,246,968 and International Publication No. WO2012166923 ). In yet another non-limiting example, the therapeutic nanoparticle is a stealth nanoparticle or a target-specific stealth nanoparticle as described in US Patent Publication No. US20130172406.

In one case, the therapeutic nanoparticle may comprise a multiblock copolymer (See e.g., U.S. Pat. No. 8,263,665 and 8,287,910 and US Patent Pub. No. US20130195987).

In yet another non-limiting example, the lipid nanoparticle comprises the block copolymer PEG-PLGA-PEG (see e.g., the thermosensitive hydrogel (PEG-PLGA-PEG) was used as a TGF-beta1 gene delivery vehicle in Lee et al. Thermosensitive Hydrogel as a Tgf-β1 Gene Delivery Vehicle Enhances Diabetic Wound Healing. Pharmaceutical Research, 2003 20(12): 1995-2000; as a controlled gene delivery system in Li et al. Controlled Gene Delivery System Based on Thermosensitive Biodegradable Hydrogel. Pharmaceutical Research 2003 20(6):884-888; and Chang et al., Non-ionic amphiphilic biodegradable PEG-PLGA-PEG copolymer enhances gene delivery efficiency in rat skeletal muscle. J Controlled Release. 2007 118:245-253).

The chimeric polynucleotides may be formulated in lipid nanoparticles comprising the PEG-PLGA-PEG block copolymer.

In one case, the therapeutic nanoparticle may comprise a multiblock copolymer (See e.g., U.S. Pat. No. 8,263,665 and 8,287,910 and US Patent Pub. No. US20130195987).

In one case, the block copolymers described herein may be included in a polyion complex comprising a non-polymeric micelle and the block copolymer. (See e.g., U.S. Pub. No. 20120076836).

In one case, the therapeutic nanoparticle may comprise at least one acrylic polymer. Acrylic polymers include but are not limited to, acrylic acid, methacrylic acid, acrylic acid and methacrylic acid copolymers, methyl methacrylate copolymers, ethoxyethyl methacrylates, cyanoethyl methacrylate, amino alkyl methacrylate copolymer, poly(acrylic acid), poly(methacrylic acid), polycyanoacrylates and combinations thereof.

In one case, the therapeutic nanoparticles may comprise at least one poly(vinyl ester) polymer. The poly(vinyl ester) polymer may be a copolymer such as a random copolymer. As a non-limiting example, the random copolymer may have a structure such as those described in International Application No. WO2013032829 or US Patent Publication No US20130121954. . In one case, the poly(vinyl ester) polymers may be conjugated to the chimeric polynucleotides described herein. In another case, the poly(vinyl ester) polymer which may be used may be those described in

In one case, the therapeutic nanoparticle may comprise at least one diblock copolymer. The diblock copolymer may be, but it not limited to, a poly(lactic) acid-poly(ethylene)glycol copolymer (see e.g., International Patent Publication No. WO2013044219). As a non-limiting example, the therapeutic nanoparticle may be used to treat cancer (see International publication No. WO2013044219 ).

In one case, the therapeutic nanoparticles may comprise at least one cationic polymer described herein and/or known in the art.

In one case, the therapeutic nanoparticles may comprise at least one amine-containing polymer such as, but not limited to polylysine, polyethylene imine, poly(amidoamine) dendrimers, poly(beta-amino esters) (See e.g., U.S. Pat. No. 8,287,849) and combinations thereof.

In another case, the nanoparticles described herein may comprise an amine cationic lipid such as those described in International Patent Application No. WO2013059496.

In one case the cationic lipids may have a amino-amine or an amino-amide moiety.

In one case, the therapeutic nanoparticles may comprise at least one degradable polyester which may contain polycationic side chains. Degradable polyesters include, but are not limited to, poly(serine ester), poly(L-lactide-co-L-lysine), poly(4-hydroxy-L-proline ester), and combinations thereof. In another case, the degradable polyesters may include a PEG conjugation to form a PEGylated polymer.

In another case, the therapeutic nanoparticle may include a conjugation of at least one targeting ligand. The targeting ligand may be any ligand known in the art such as, but not limited to, a monoclonal antibody. (Kirpotin et al, Cancer Res. 2006 66:6732-6740).

In one case, the therapeutic nanoparticle may be formulated in an aqueous solution which may be used to target cancer (see International Pub No. WO2011084513 and US Pub No. US20110294717 ).

In one case, the therapeutic nanoparticle comprising at least one chimeric polynucleotide may be formulated using the methods described by Podobinski et al in US Patent No. 8,404,799 .

In one case, the chimeric polynucleotides may be encapsulated in, linked to and/or associated with synthetic nanocarriers. Synthetic nanocarriers include, but are not limited to, those described in paragraphs [000468] - [000477] of copending International Publication No. WO2015034928 .

In one case, the chimeric polynucleotides may be encapsulated in, linked to and/or associated with zwitterionic lipids. Non-limiting examples of zwitterionic lipids and methods of using zwitterionic lipids are described in US Patent Publication No. US20130216607.

In one case, the zwitterionic lipids may be used in the liposomes and lipid nanoparticles described herein.

In one case, the chimeric polynucleotides may be formulated in colloid nanocarriers as described in US Patent Publication No. US20130197100.

In one case, the nanoparticle may be optimized for oral administration. The nanoparticle may comprise at least one cationic biopolymer such as, but not limited to, chitosan or a derivative thereof. As a non-limiting example, the nanoparticle may be formulated by the methods described in U.S. Pub. No. 20120282343.

In some cases, LNPs comprise the lipid KL52 (an amino-lipid disclosed in U.S. Application Publication No. 2012/0295832). Activity and/or safety (as measured by examining one or more of ALT/AST, white blood cell count and cytokine induction) of LNP administration may be improved by incorporation of such lipids. LNPs comprising KL52 may be administered intravenously and/or in one or more doses. In some cases cases, administration of LNPs comprising KL52 results in equal or improved mRNA and/or protein expression as compared to LNPs comprising MC3.

In some cases, chimeric polynucleotides may be delivered using smaller LNPs. Such particles may comprise a diameter from below 0.1 um up to 100 nm such as, but not limited to, less than 0.1 um, less than 1.0 um, less than 5 um, less than 10 um, less than 15 um, less than 20 um, less than 25 um, less than 30 um, less than 35 um, less than 40 um, less than 50 um, less than 55 um, less than 60 um, less than 65 um, less than 70 um, less than 75 um, less than 80 um, less than 85 um, less than 90 um, less than 95 um, less than 100 um, less than 125 um, less than 150 um, less than 175 um, less than 200 um, less than 225 um, less than 250 um, less than 275 um, less than 300 um, less than 325 um, less than 350 um, less than 375 um, less than 400 um, less than 425 um, less than 450 um, less than 475 um, less than 500 um, less than 525 um, less than 550 um, less than 575 um, less than 600 um, less than 625 um, less than 650 um, less than 675 um, less than 700 um, less than 725 um, less than 750 um, less than 775 um, less than 800 um, less than 825 um, less than 850 um, less than 875 um, less than 900 um, less than 925 um, less than 950 um, less than 975 um.

In another cases, chimeric polynucleotides may be delivered using smaller LNPs which may comprise a diameter from about 1 nm to about 100 nm, from about 1 nm to about 10 nm, about 1 nm to about 20 nm, from about 1 nm to about 30 nm, from about 1 nm to about 40 nm, from about 1 nm to about 50 nm, from about 1 nm to about 60 nm, from about 1 nm to about 70 nm, from about 1 nm to about 80 nm, from about 1 nm to about 90 nm, from about 5 nm to about from 100 nm, from about 5 nm to about 10 nm, about 5 nm to about 20 nm, from about 5 nm to about 30 nm, from about 5 nm to about 40 nm, from about 5 nm to about 50 nm, from about 5 nm to about 60 nm, from about 5 nm to about 70 nm, from about 5 nm to about 80 nm, from about 5 nm to about 90 nm, about 10 to about 50 nM, from about 20 to about 50 nm, from about 30 to about 50 nm, from about 40 to about 50 nm, from about 20 to about 60 nm, from about 30 to about 60 nm, from about 40 to about 60 nm, from about 20 to about 70 nm, from about 30 to about 70 nm, from about 40 to about 70 nm, from about 50 to about 70 nm, from about 60 to about 70 nm, from about 20 to about 80 nm, from about 30 to about 80 nm, from about 40 to about 80 nm, from about 50 to about 80 nm, from about 60 to about 80 nm, from about 20 to about 90 nm, from about 30 to about 90 nm, from about 40 to about 90 nm, from about 50 to about 90 nm, from about 60 to about 90 nm and/or from about 70 to about 90 nm.

In some cases, such LNPs are synthesized using methods comprising microfluidic mixers. Exemplary microfluidic mixers may include, but are not limited to a slit interdigital micromixer including, but not limited to those manufactured by Microinnova (Allerheiligen bei Wildon, Austria) and/or a staggered herringbone micromixer (SHM) (Zhigaltsev, I.V. et al., Bottom-up design and synthesis of limit size lipid nanoparticle systems with aqueous and triglyceride cores using millisecond microfluidic mixing have been published (Langmuir. 2012. 28:3633-40; Belliveau, N.M. et al., Microfluidic synthesis of highly potent limit-size lipid nanoparticles for in vivo delivery of siRNA. Molecular Therapy-Nucleic Acids. 2012. 1:e37; Chen, D. et al., Rapid discovery of potent siRNA-containing lipid nanoparticles enabled by controlled microfluidic formulation. J Am Chem Soc. 2012. 134(16):6948-51). In some cases, methods of LNP generation comprising SHM, further comprise the mixing of at least two input streams wherein mixing occurs by microstructure-induced chaotic advection (MICA). According to this method, fluid streams flow through channels present in a herringbone pattern causing rotational flow and folding the fluids around each other. This method may also comprise a surface for fluid mixing wherein the surface changes orientations during fluid cycling. Methods of generating LNPs using SHM include those disclosed in U.S. Application Publication Nos. 2004/0262223 and 2012/0276209.

In one cases, the chimeric polynucleotides may be formulated in lipid nanoparticles created using a micromixer such as, but not limited to, a Slit Interdigital Microstructured Mixer (SIMM-V2) or a Standard Slit Interdigital Micro Mixer (SSIMM) or Caterpillar (CPMM) or Impinging-jet (IJMM)from the Institut fur Mikrotechnik Mainz GmbH, Mainz Germany).

In one case the chimeric polynucleotides may be formulated in lipid nanoparticles created using microfluidic technology (see Whitesides, George M. The Origins and the Future of Microfluidics. Nature, 2006 442: 368-373; and Abraham et al. Chaotic Mixer for Microchannels. Science, 2002 295: 647-651). As a non-limiting example, controlled microfluidic formulation includes a passive method for mixing streams of steady pressure-driven flows in micro channels at a low Reynolds number (See e.g., Abraham et al. Chaotic Mixer for Microchannels. Science, 2002 295: 647-651).

In one case, the chimeric polynucleotides may be formulated in lipid nanoparticles created using a micromixer chip such as, but not limited to, those from Harvard Apparatus (Holliston, MA) or Dolomite Microfluidics (Royston, UK). A micromixer chip can be used for rapid mixing of two or more fluid streams with a split and recombine mechanism.

In one case, the chimeric polynucleotides may be formulated for delivery using the drug encapsulating microspheres described in International Patent Publication No. WO2013063468 or U.S. Patent No. 8,440,614.

The microspheres may comprise a compound of the formula (I), (II), (III), (IV), (V) or (VI) as described in International patent application No. WO2013063468.

In another case, the amino acid, peptide, polypeptide, lipids (APPL) are useful in delivering the chimeric polynucleotides of the disclosure to cells (see International Patent Publication No. WO201306346).

In one case, the chimeric polynucleotides may be formulated in lipid nanoparticles having a diameter from about 10 to about 100 nm such as, but not limited to, about 10 to about 20 nm, about 10 to about 30 nm, about 10 to about 40 nm, about 10 to about 50 nm, about 10 to about 60 nm, about 10 to about 70 nm, about 10 to about 80 nm, about 10 to about 90 nm, about 20 to about 30 nm, about 20 to about 40 nm, about 20 to about 50 nm, about 20 to about 60 nm, about 20 to about 70 nm, about 20 to about 80 nm, about 20 to about 90 nm, about 20 to about 100 nm, about 30 to about 40 nm, about 30 to about 50 nm, about 30 to about 60 nm, about 30 to about 70 nm, about 30 to about 80 nm, about 30 to about 90 nm, about 30 to about 100 nm, about 40 to about 50 nm, about 40 to about 60 nm, about 40 to about 70 nm, about 40 to about 80 nm, about 40 to about 90 nm, about 40 to about 100 nm, about 50 to about 60 nm, about 50 to about 70 nm about 50 to about 80 nm, about 50 to about 90 nm, about 50 to about 100 nm, about 60 to about 70 nm, about 60 to about 80 nm, about 60 to about 90 nm, about 60 to about 100 nm, about 70 to about 80 nm, about 70 to about 90 nm, about 70 to about 100 nm, about 80 to about 90 nm, about 80 to about 100 nm and/or about 90 to about 100 nm.

In one case, the lipid nanoparticles may have a diameter from about 10 to 500 nm.

In one case, the lipid nanoparticle may have a diameter greater than 100 nm, greater than 150 nm, greater than 200 nm, greater than 250 nm, greater than 300 nm, greater than 350 nm, greater than 400 nm, greater than 450 nm, greater than 500 nm, greater than 550 nm, greater than 600 nm, greater than 650 nm, greater than 700 nm, greater than 750 nm, greater than 800 nm, greater than 850 nm, greater than 900 nm, greater than 950 nm or greater than 1000 nm.

In one case, the lipid nanoparticle may be a limit size lipid nanoparticle described in International Patent Publication No. WO2013059922. The limit size lipid nanoparticle may comprise a lipid bilayer surrounding an aqueous core or a hydrophobic core; where the lipid bilayer may comprise a phospholipid such as, but not limited to, diacylphosphatidylcholine, a diacylphosphatidylethanolamine, a ceramide, a sphingomyelin, a dihydrosphingomyelin, a cephalin, a cerebroside, a C8-C20 fatty acid diacylphophatidylcholine, and 1-palmitoyl-2-oleoyl phosphatidylcholine (POPC). In another case the limit size lipid nanoparticle may comprise a polyethylene glycol-lipid such as, but not limited to, DLPE-PEG, DMPE-PEG, DPPC-PEG and DSPE-PEG.

In one case, the chimeric polynucleotides may be delivered, localized and/or concentrated in a specific location using the delivery methods described in International Patent Publication No. WO2013063530. As a non-limiting example, a subject may be administered an empty polymeric particle prior to, simultaneously with or after delivering the chimeric polynucleotides to the subject. The empty polymeric particle undergoes a change in volume once in contact with the subject and becomes lodged, embedded, immobilized or entrapped at a specific location in the subject. case

In one case, the chimeric polynucleotides may be formulated in an active substance release system (See e.g., US Patent Publication No. US20130102545). The active substance release system may comprise 1) at least one nanoparticle bonded to an oligonucleotide inhibitor strand which is hybridized with a catalytically active nucleic acid and 2) a compound bonded to at least one substrate molecule bonded to a therapeutically active substance (e.g., chimeric polynucleotides described herein), where the therapeutically active substance is released by the cleavage of the substrate molecule by the catalytically active nucleic acid.

In one case, the chimeric polynucleotides may be formulated in a nanoparticle comprising an inner core comprising a non-cellular material and an outer surface comprising a cellular membrane. The cellular membrane may be derived from a cell or a membrane derived from a virus. As a non-limiting example, the nanoparticle may be made by the methods described in International Patent Publication No. WO2013052167. As another non-limiting example, the nanoparticle described in International Patent Publication No. WO2013052167, may be used to deliver the chimeric polynucleotides described herein.

In one case, the chimeric polynucleotides may be formulated in porous nanoparticle-supported lipid bilayers (protocells). Protocells are described in International Patent Publication No. WO2013056132.

In one case, the chimeric polynucleotides described herein may be formulated in polymeric nanoparticles as described in or made by the methods described in US Patent No. 8,420,123 and 8,518,963 and European Patent No. EP2073 848B1.

As a non-limiting example, the polymeric nanoparticle may have a high glass transition temperature such as the nanoparticles described in or nanoparticles made by the methods described in US Patent No. 8,518,963.

As another non-limiting example, the polymer nanoparticle for oral, parenteral and topical formulations may be made by the methods described in European Patent No. EP2073848B1.

In another case, the chimeric polynucleotides described herein may be formulated in nanoparticles used in imaging. The nanoparticles may be liposome nanoparticles such as those described in US Patent Publication No US20130129636. As a non-limiting example, the liposome may comprise gadolinium(III)2-{4,7-bis-carboxymethyl-10-[(N,N-distearylamidomethyl-N'-amido-methyl]-1,4,7,10-tetra-azacyclododec-1-yl}-acetic acid and a neutral, fully saturated phospholipid component (see e.g., US Patent Publication No US20130129636).

In one case, the nanoparticles which may be used in the present disclosure are formed by the methods described in U.S. Patent Application No. US20130130348

The nanoparticles may further include nutrients such as, but not limited to, those which deficiencies can lead to health hazards from anemia to neural tube defects (see e.g, the nanoparticles described in International Patent Publication No WO2013072929). As a non-limiting example, the nutrient may be iron in the form of ferrous, ferric salts or elemental iron, iodine, folic acid, vitamins or micronutrients.

In one case, the chimeric polynucleotides may be formulated in a swellable nanoparticle. The swellable nanoparticle may be, but is not limited to, those described in U.S. Patent No. 8,440,231. As a non-limiting case, the swellable nanoparticle may be used for delivery of the chimeric polynucleotides of the present disclosure to the pulmonary system (see e.g., U.S. Patent No. 8,440,231).

The chimeric polynucleotides may be formulated in polyanhydride nanoparticles such as, but not limited to, those described in U.S. Patent No. 8,449,916.

The nanoparticles and microparticles may be geometrically engineered to modulate macrophage and/or the immune response. In one case, the geometrically engineered particles may have varied shapes, sizes and/or surface charges in order to incorporated the chimeric polynucleotides of the present disclosure for targeted delivery such as, but not limited to, pulmonary delivery (see e.g., International Publication No WO2013082111).

Other physical features the geometrically engineering particles may have include, but are not limited to, fenestrations, angled arms, asymmetry and surface roughness, charge which can alter the interactions with cells and tissues. As a non-limiting example, nanoparticles may be made by the methods described in International Publication No WO2013082111.

In one case, the nanoparticles may be water soluble nanoparticles such as, but not limited to, those described in International Publication No. WO2013090601.

The nanoparticles may be inorganic nanoparticles which have a compact and zwitterionic ligand in order to exhibit good water solubility. The nanoparticles may also have small hydrodynamic diameters (HD), stability with respect to time, pH, and salinity and a low level of non-specific protein binding.

In one case the nanoparticles may be developed by the methods described in US Patent Publication No. US20130172406.

In one case, the nanoparticles of the present disclosure are stealth nanoparticles or target-specific stealth nanoparticles such as, but not limited to, those described in US Patent Publication No. US20130172406. The nanoparticles may be made by the methods described in US Patent Publication No. S20130172406.

In another case, the stealth or target-specific stealth nanoparticles may comprise a polymeric matrix. The polymeric matrix may comprise two or more polymers such as, but not limited to, polyethylenes, polycarbonates, polyanhydrides, polyhydroxyacids, polypropylfumerates, polycaprolactones, polyamides, polyacetals, polyethers, polyesters, poly(orthoesters), polycyanoacrylates, polyvinyl alcohols, polyurethanes, polyphosphazenes, polyacrylates, polymethacrylates, polycyanoacrylates, polyureas, polystyrenes, polyamines, polyesters, polyanhydrides, polyethers, polyurethanes, polymethacrylates, polyacrylates, polycyanoacrylates or combinations thereof.

In one case, the nanoparticle may be a nanoparticle-nucleic acid hybrid structure having a high density nucleic acid layer. As a non-limiting example, the nanoparticle-nucleic acid hybrid structure may made by the methods described in US Patent Publication No. US20130171646.

The nanoparticle may comprise a nucleic acid such as, but not limited to, chimeric polynucleotides described herein and/or known in the art.

At least one of the nanoparticles may be embedded in in the core a nanostructure or coated with a low density porous 3-D structure or coating which is capable of carrying or associating with at least one payload within or on the surface of the nanostructure. Non-limiting examples of the nanostructures comprising at least one nanoparticle are described in International Patent Publication No. WO2013123523.

### Polymers, Biodegradable Nanoparticles, and Core-Shell Nanoparticles

The chimeric polynucleotides can be formulated using natural and/or synthetic polymers. Non-limiting examples of polymers which may be used for delivery include, but are not limited to, DYNAMIC POLYCONJUGATE^{®} (Arrowhead Research Corp., Pasadena, CA) formulations from MIRUS^{®} Bio (Madison, WI) and Roche Madison (Madison, WI), PHASERX^{™} polymer formulations such as, without limitation, SMARTT POLYMER TECHNOLOGY^{™} (PHASERX^{®}, Seattle, WA), DMRI/DOPE, poloxamer, VAXFECTIN^{®} adjuvant from Vical (San Diego, CA), chitosan, cyclodextrin from Calando Pharmaceuticals (Pasadena, CA), dendrimers and poly(lactic-co-glycolic acid) (PLGA) polymers. RONDEL^{™} (RNAi/Oligonucleotide Nanoparticle Delivery) polymers (Arrowhead Research Corporation, Pasadena, CA) and pH responsive co-block polymers such as, but not limited to, PHASERX^{®} (Seattle, WA).

A non-limiting example of chitosan formulation includes a core of positively charged chitosan and an outer portion of negatively charged substrate (U.S. Pub. No. 20120258176). Chitosan includes, but is not limited to N-trimethyl chitosan, mono-N-carboxymethyl chitosan (MCC), N-palmitoyl chitosan (NPCS), EDTA-chitosan, low molecular weight chitosan, chitosan derivatives, or combinations thereof.

In one case, the polymers used in the present disclosure have undergone processing to reduce and/or inhibit the attachment of unwanted substances such as, but not limited to, bacteria, to the surface of the polymer. The polymer may be processed by methods known and/or described in the art and/or described in International Pub. No. WO2012150467

A non-limiting example of PLGA formulations include, but are not limited to, PLGA injectable depots (e.g., ELIGARD^{®} which is formed by dissolving PLGA in 66% N-methyl-2-pyrrolidone (NMP) and the remainder being aqueous solvent and leuprolide. Once injected, the PLGA and leuprolide peptide precipitates into the subcutaneous space).

Many of these polymer approaches have demonstrated efficacy in delivering oligonucleotides *in vivo* into the cell cytoplasm (reviewed in deFougerolles Hum Gene Ther. 2008 19:125-132). Two polymer approaches that have yielded robust *in vivo* delivery of nucleic acids, in this case with small interfering RNA (siRNA), are dynamic polyconjugates and cyclodextrin-based nanoparticles (see e.g., US Patent Publication No. US20130156721). The first of these delivery approaches uses dynamic polyconjugates and has been shown *in vivo* in mice to effectively deliver siRNA and silence endogenous target mRNA in hepatocytes (Rozema et al., Proc Natl Acad Sci U S A. 2007 104:12982-12887).

This particular approach is a multicomponent polymer system whose key features include a membrane-active polymer to which nucleic acid, in this case siRNA, is covalently coupled via a disulfide bond and where both PEG (for charge masking) and *N*-acetylgalactosamine (for hepatocyte targeting) groups are linked via pH-sensitive bonds (Rozema et al., Proc Natl Acad Sci U S A. 2007 104:12982-12887).

On binding to the hepatocyte and entry into the endosome, the polymer complex disassembles in the low-pH environment, with the polymer exposing its positive charge, leading to endosomal escape and cytoplasmic release of the siRNA from the polymer. Through replacement of the *N*-acetylgalactosamine group with a mannose group, it was shown one could alter targeting from asialoglycoprotein receptor-expressing hepatocytes to sinusoidal endothelium and Kupffer cells. Another polymer approach involves using transferrin-targeted cyclodextrin-containing polycation nanoparticles. These nanoparticles have demonstrated targeted silencing of the *EWS-FLI1* gene product in transferrin receptor-expressing Ewing's sarcoma tumor cells (Hu-Lieskovan et al., Cancer Res.2005 65: 8984-8982)
and siRNA formulated in these nanoparticles was well tolerated in non-human primates (Heidel et al., Proc Natl Acad Sci USA 2007 104:5715-21). Both of these delivery strategies incorporate rational approaches using both targeted delivery and endosomal escape mechanisms.

The polymer formulation can permit the sustained or delayed release of chimeric polynucleotides (e.g., following intramuscular or subcutaneous injection). The altered release profile for the chimeric polynucleotide can result in, for example, translation of an encoded protein over an extended period of time. The polymer formulation may also be used to increase the stability of the chimeric polynucleotide. Biodegradable polymers have been previously used to protect nucleic acids other than chimeric polynucleotide from degradation and been shown to result in sustained release of payloads in vivo (Rozema et al., Proc Natl Acad Sci U S A. 2007 104:12982-12887; Sullivan et al., Expert Opin Drug Deliv. 2010 7:1433-1446; Convertine et al., Biomacromolecules. 2010 Oct 1; Chu et al., Acc Chem Res. 2012 Jan 13; Manganiello et al., Biomaterials. 2012 33:2301-2309; Benoit et al., Biomacromolecules. 2011 12:2708-2714; Singha et al., Nucleic Acid Ther. 2011 2:133-147; deFougerolles Hum Gene Ther. 2008 19:125-132; Schaffert and Wagner, Gene Ther. 2008 16:1131-1138; Chaturvedi et al., Expert Opin Drug Deliv. 2011 8:1455-1468; Davis, Mol Pharm. 2009 6:659-668; Davis, Nature 2010 464:1067-1070).

In one case, the pharmaceutical compositions may be sustained release formulations. In a further case, the sustained release formulations may be for subcutaneous delivery. Sustained release formulations may include, but are not limited to, PLGA microspheres, ethylene vinyl acetate (EVAc), poloxamer, GELSITE^{®} (Nanotherapeutics, Inc. Alachua, FL), HYLENEX^{®} (Halozyme Therapeutics, San Diego CA), surgical sealants such as fibrinogen polymers (Ethicon Inc. Cornelia, GA), TISSELL^{®} (Baxter International, Inc Deerfield, IL), PEG-based sealants, and COSEAL^{®} (Baxter International, Inc Deerfield, IL).

As a non-limiting example modified mRNA may be formulated in PLGA microspheres by preparing the PLGA microspheres with tunable release rates (e.g., days and weeks) and encapsulating the modified mRNA in the PLGA microspheres while maintaining the integrity of the modified mRNA during the encapsulation process. EVAc are non-biodegradable, biocompatible polymers which are used extensively in pre-clinical sustained release implant applications (e.g., extended release products Ocusert a pilocarpine ophthalmic insert for glaucoma or progestasert a sustained release progesterone intrauterine device; transdermal delivery systems Testoderm, Duragesic and Selegiline; catheters). Poloxamer F-407 NF is a hydrophilic, non-ionic surfactant triblock copolymer of polyoxyethylene-polyoxypropylene-polyoxyethylene having a low viscosity at temperatures less than 5°C and forms a solid gel at temperatures greater than 15°C. PEG-based surgical sealants comprise two synthetic PEG components mixed in a delivery device which can be prepared in one minute, seals in 3 minutes and is reabsorbed within 30 days. GELSITE^{®} and natural polymers are capable of in-situ gelation at the site of administration. They have been shown to interact with protein and peptide therapeutic candidates through ionic interaction to provide a stabilizing effect.

Polymer formulations can also be selectively targeted through expression of different ligands as exemplified by, but not limited by, folate, transferrin, and N-acetylgalactosamine (GalNAc) (Benoit et al., Biomacromolecules. 2011 12:2708-2714; Rozema et al., Proc Natl Acad Sci U S A. 2007 104:12982-12887; Davis, Mol Pharm. 2009 6:659-668; Davis, Nature 2010 464:1067-1070).

The chimeric polynucleotides may be formulated with or in a polymeric compound. The polymer may include at least one polymer such as, but not limited to, polyethenes, polyethylene glycol (PEG), poly(1-lysine)(PLL), PEG grafted to PLL, cationic lipopolymer, biodegradable cationic lipopolymer, polyethyleneimine (PEI), cross-linked branched poly(alkylene imines), a polyamine derivative, a modified poloxamer, a biodegradable polymer, elastic biodegradable polymer, biodegradable block copolymer, biodegradable random copolymer, biodegradable polyester copolymer, biodegradable polyester block copolymer, biodegradable polyester block random copolymer, multiblock copolymers, linear biodegradable copolymer, poly[α-(4-aminobutyl)-L-glycolic acid) (PAGA), biodegradable cross-linked cationic multi-block copolymers, polycarbonates, polyanhydrides, polyhydroxyacids, polypropylfumerates, polycaprolactones, polyamides, polyacetals, polyethers, polyesters, poly(orthoesters), polycyanoacrylates, polyvinyl alcohols, polyurethanes, polyphosphazenes, polyacrylates, polymethacrylates, polycyanoacrylates, polyureas, polystyrenes, polyamines, polylysine, poly(ethylene imine), poly(serine ester), poly(L-lactide-co-L-lysine), poly(4-hydroxy-L-proline ester), acrylic polymers, amine-containing polymers, dextran polymers, dextran polymer derivatives or combinations thereof.

As a non-limiting example, the chimeric polynucleotides may be formulated with the polymeric compound of PEG grafted with PLL as described in U.S. Pat. No. 6,177,274. The formulation may be used for transfecting cells *in vitro* or for *in vivo* delivery of chimeric polynucleotide. In another example, the chimeric polynucleotide may be suspended in a solution or medium with a cationic polymer, in a dry pharmaceutical composition or in a solution that is capable of being dried as described in U.S. Pub. Nos. 20090042829 and 20090042825.

As another non-limiting example the chimeric polynucleotides may be formulated with a PLGA-PEG block copolymer (see US Pub. No. US20120004293 and US Pat No. 8,236,330) or PLGA-PEG-PLGA block copolymers (See U.S. Pat. No. 6,004,573). As a non-limiting example, the chimeric polynucleotides may be formulated with a diblock copolymer of PEG and PLA or PEG and PLGA (see US Pat No 8,246,968).

A polyamine derivative may be used to deliver nucleic acids or to treat and/or prevent a disease or to be included in an implantable or injectable device (U.S. Pub. No. 20100260817 (now U.S. Patent No. 8,460,696). As a non-limiting example, a pharmaceutical composition may include the chimeric polynucleotide and the polyamine derivative described in U.S. Pub. No. 20100260817 (now U.S. Patent No. 8,460,696. As a non-limiting example the chimeric polynucleotides may be delivered using a polyaminde polymer such as, but not limited to, a polymer comprising a 1,3-dipolar addition polymer prepared by combining a carbohydrate diazide monomer with a dilkyne unite comprising oligoamines (U.S. Pat. No. 8,236,280).

The chimeric polynucleotides may be formulated with at least one acrylic polymer. Acrylic polymers include but are not limited to, acrylic acid, methacrylic acid, acrylic acid and methacrylic acid copolymers, methyl methacrylate copolymers, ethoxyethyl methacrylates, cyanoethyl methacrylate, amino alkyl methacrylate copolymer, poly(acrylic acid), poly(methacrylic acid), polycyanoacrylates and combinations thereof.

In one case, the chimeric polynucleotides may be formulated with at least one polymer and/or derivatives thereof described in International Publication Nos. WO2011115862, WO2012082574 and WO2012068187 and U.S. Pub. No. 20120283427. In another case, the chimeric polynucleotides may be formulated with a polymer of formula Z as described in WO2011115862. In yet another case, the chimeric polynucleotides may be formulated with a polymer of formula Z, Z' or Z" as described in International Pub. Nos. WO2012082574 or WO2012068187 and U.S. Pub. No. 2012028342. The polymers formulated with the modified RNA may be synthesized by the methods described in International Pub. Nos. WO2012082574 or WO2012068187.

The chimeric polynucleotides may be formulated with at least one acrylic polymer. Acrylic polymers include but are not limited to, acrylic acid, methacrylic acid, acrylic acid and methacrylic acid copolymers, methyl methacrylate copolymers, ethoxyethyl methacrylates, cyanoethyl methacrylate, amino alkyl methacrylate copolymer, poly(acrylic acid), poly(methacrylic acid), polycyanoacrylates and combinations thereof.

Formulations of chimeric polynucleotides may include at least one amine-containing polymer such as, but not limited to polylysine, polyethylene imine, poly(amidoamine) dendrimers, poly(amine-co-esters) or combinations thereof. As a non-limiting example, the poly(amine-co-esters) may be the polymers described in and/or made by the methods described in International Publication No WO2013082529.

For example, the chimeric polynucleotides may be formulated in a pharmaceutical compound including a poly(alkylene imine), a biodegradable cationic lipopolymer, a biodegradable block copolymer, a biodegradable polymer, or a biodegradable random copolymer, a biodegradable polyester block copolymer, a biodegradable polyester polymer, a biodegradable polyester random copolymer, a linear biodegradable copolymer, PAGA, a biodegradable cross-linked cationic multi-block copolymer or combinations thereof. The biodegradable cationic lipopolymer may be made by methods known in the art and/or described in U.S. Pat. No. 6,696,038, U.S. App. Nos. 20030073619 and 20040142474. The poly(alkylene imine) may be made using methods known in the art and/or as described in U.S. Pub. No. 20100004315.

The biodegradable polymer, biodegradable block copolymer, the biodegradable random copolymer, biodegradable polyester block copolymer, biodegradable polyester polymer, or biodegradable polyester random copolymer may be made using methods known in the art and/or as described in U.S. Pat. Nos. 6,517,869 and 6,267,987.

The linear biodegradable copolymer may be made using methods known in the art and/or as described in U.S. Pat. No. 6,652,886. The PAGA polymer may be made using methods known in the art and/or as described in U.S. Pat. No. 6,217,912. The PAGA polymer may be copolymerized to form a copolymer or block copolymer with polymers such as but not limited to, poly-L-lysine, polyargine, polyornithine, histones, avidin, protamines, polylactides and poly(lactide-co-glycolides). The biodegradable cross-linked cationic multi-block copolymers may be made my methods known in the art and/or as described in U.S. Pat. No. 8,057,821, 8,444,992 or U.S. Pub. No. 2012009145. For example, the multi-block copolymers may be synthesized using linear polyethyleneimine (LPEI) blocks which have distinct patterns as compared to branched polyethyleneimines. Further, the composition or pharmaceutical composition may be made by the methods known in the art, described herein, or as described in U.S. Pub. No. 20100004315 or U.S. Pat. Nos. 6,267,987 and 6,217,912.

The chimeric polynucleotides may be formulated with at least one degradable polyester which may contain polycationic side chains. Degradable polyesters include, but are not limited to, poly(serine ester), poly(L-lactide-co-L-lysine), poly(4-hydroxy-L-proline ester), and combinations thereof. In another case, the degradable polyesters may include a PEG conjugation to form a PEGylated polymer.

The chimeric polynucleotides may be formulated with at least one crosslinkable polyester. Crosslinkable polyesters include those known in the art and described in US Pub. No. 2012026976-1.

The chimeric polynucleotides may be formulated in or with at least one cyclodextrin polymer. Cyclodextrin polymers and methods of making cyclodextrin polymers include those known in the art and described in US Pub. No. 20130184453.

In one case, the chimeric polynucleotides may be formulated in or with at least one crosslinked cation-binding polymers. Crosslinked cation-binding polymers and methods of making crosslinked cation-binding polymers include those known in the art and described in International Patent Publication No. WO2013106072, WO2013106073 and WO2013106086.

In one case, the chimeric polynucleotides may be formulated in or with at least one branched polymer. Branched polymers and methods of making branched polymers include those known in the art and described in International Patent Publication No. WO2013113071.

In one case, the chimeric polynucleotides may be formulated in or with at least PEGylated albumin polymer. PEGylated albumin polymer and methods of making PEGylated albumin polymer include those known in the art and described in US Patent Publication No. US20130231287.

In one case, the polymers described herein may be conjugated to a lipid-terminating PEG. As a non-limiting example, PLGA may be conjugated to a lipid-terminating PEG forming PLGA-DSPE-PEG. As another non-limiting example, PEG conjugates for use with the present disclosure are described in International Publication No. WO2008103276. The polymers may be conjugated using a ligand conjugate such as, but not limited to, the conjugates described in U.S. Pat. No. 8,273,363.

In one case, the chimeric polynucleotides disclosed herein may be mixed with the PEGs or the sodium phosphate/sodium carbonate solution prior to administration. In another case, a chimeric polynucleotides encoding a protein of interest may be mixed with the PEGs and also mixed with the sodium phosphate/sodium carbonate solution. In yet another case, chimeric polynucleotides encoding a protein of interest may be mixed with the PEGs and a chimeric polynucleotides encoding a second protein of interest may be mixed with the sodium phosphate/sodium carbonate solution.

In one case, the chimeric polynucleotides described herein may be conjugated with another compound. Non-limiting examples of conjugates are described in US Patent Nos. 7,964,578 and 7,833,992. In another case, modified RNA may be conjugated with conjugates of formula 1-122 as described in US Patent Nos. 7,964,578 and 7,833,992. The chimeric polynucleotides described herein may be conjugated with a metal such as, but not limited to, gold. (See e.g., Giljohann et al. Journ. Amer. Chem. Soc. 2009 131(6): 2072-2073). In another case, the chimeric polynucleotides described herein may be conjugated and/or encapsulated in gold-nanoparticles. (International Pub. No. WO201216269 and U.S. Pub. No. 20120302940 and US20130177523).

As described in U.S. Pub. No. 20100004313 a gene delivery composition may include a nucleotide sequence and a poloxamer. For example, the chimeric polynucleotides may be used in a gene delivery composition with the poloxamer described in U.S. Pub. No. 20100004313.

In one case, the polymer formulation may be stabilized by contacting the polymer formulation, which may include a cationic carrier, with a cationic lipopolymer which may be covalently linked to cholesterol and polyethylene glycol groups. The polymer formulation may be contacted with a cationic lipopolymer using the methods described in U.S. Pub. No. 20090042829.

The cationic carrier may include, but is not limited to, polyethylenimine, poly(trimethylenimine), poly(tetramethylenimine), polypropylenimine, aminoglycoside-polyamine, dideoxy-diamino-b-cyclodextrin, spermine, spermidine, poly(2-dimethylamino)ethyl methacrylate, poly(lysine), poly(histidine), poly(arginine), cationized gelatin, dendrimers, chitosan, 1,2-Dioleoyl-3-Trimethylammonium-Propane(DOTAP), N-[1-(2,3-dioleoyloxy)propyl]-N,N,N-trimethylammonium chloride (DOTMA), 1-[2-(oleoyloxy)ethyl]-2-oleyl-3-(2-hydroxyethyl)imidazolinium chloride (DOTIM), 2,3-dioleyloxy-N-[2(sperminecarboxamido)ethyl]-N,N-dimethyl-1-propanaminium trifluoroacetate (DOSPA), 3B-[N-(N',N'-Dimethylaminoethane)-carbamoyl]Cholesterol Hydrochloride (DC-Cholesterol HCl) diheptadecylamidoglycyl spermidine (DOGS), N,N-distearyl-N,N-dimethylammonium bromide (DDAB), N-(1,2-dimyristyloxyprop-3-yl)-N,N-dimethyl-N-hydroxyethyl ammonium bromide (DMRIE), N,N-dioleyl-N,N-dimethylammonium chloride DODAC) and combinations thereof. As a non-limiting example, the chimeric polynucleotides may be formulated with a cationic lipopolymer such as those described in U.S. Patent Application No. 20130065942.

The chimeric polynucleotides may be formulated in a polyplex of one or more polymers (See e.g., U.S. Pat. No. 8,501,478, U.S. Pub. No. 20120237565 and 20120270927 and 20130149783 and International Patent Pub. No. WO2013090861).

As a non-limiting example, the polyplex may be formed using the novel alpha-aminoamidine polymers described in International Publication No. WO2013090861.

As another non-limiting example, the polyplex may be formed using the click polymers described in US Patent No. 8,501,478.

In one case, the polyplex comprises two or more cationic polymers. The cationic polymer may comprise a poly(ethylene imine) (PEI) such as linear PEI. In another case, the polyplex comprises p(TETA/CBA) its PEGylated analog p(TETA/CBA)-g-PEG2k and mixtures thereof (see e.g., US Patent Publication No. US20130149783.

The chimeric polynucleotides can also be formulated as a nanoparticle using a combination of polymers, lipids, and/or other biodegradable agents, such as, but not limited to, calcium phosphate. Components may be combined in a core-shell, hybrid, and/or layer-by-layer architecture, to allow for fine-tuning of the nanoparticle so to delivery of the polynucleotide, chimeric polynucleotides may be enhanced (Wang et al., Nat Mater. 2006 5:791-796; Fuller et al., Biomaterials. 2008 29:1526-1532; DeKoker et al., Adv Drug Deliv Rev. 2011 63:748-761; Endres et al., Biomaterials. 2011 32:7721-7731; Su et al., Mol Pharm. 2011 Jun 6;8(3):774-87). As a non-limiting example, the nanoparticle may comprise a plurality of polymers such as, but not limited to hydrophilic-hydrophobic polymers (e.g., PEG-PLGA), hydrophobic polymers (e.g., PEG) and/or hydrophilic polymers (International Pub. No. WO20120225129).

As another non-limiting example the nanoparticle comprising hydrophilic polymers for the chimeric polynucleotides may be those described in or made by the methods described in International Patent Publication No. WO2013119936.

In one case, the biodegradable polymers which may be used in the present disclosure are poly(ether-anhydride) block copolymers. As a non-limiting example, the biodegradable polymers used herein may be a block copolymer as described in International Patent Publication No WO2006063249
or made by the methods described in International Patent Publication No WO2006063249.

In another case, the biodegradable polymers which may be used in the present disclosure are alkyl and cycloalkyl terminated biodegradable lipids. As a non-limiting example, the alkyl and cycloalkyl terminated biodegradable lipids may be those described in International Publication No. WO2013086322 and/or made by the methods described in International Publication No. WO2013086322.

In yet another case, the biodegradable polymers which may be used in the present disclosure are cationic lipids having one or more biodegradable group located in a lipid moiety. As a non-limiting example, the biodegradable lipids may be those described in US Patent Publication No. US20130195920.

Biodegradable calcium phosphate nanoparticles in combination with lipids and/or polymers have been shown to deliver chimeric polynucleotides *in vivo.* In one case, a lipid coated calcium phosphate nanoparticle, which may also contain a targeting ligand such as anisamide, may be used to deliver the polynucleotide, chimeric polynucleotides of the present disclosure. For example, to effectively deliver siRNA in a mouse metastatic lung model a lipid coated calcium phosphate nanoparticle was used (Li et al., J Contr Rel. 2010 142: 416-421; Li et al., J Contr Rel. 2012 158:108-114; Yang et al., Mol Ther. 2012 20:609-615).

This delivery system combines both a targeted nanoparticle and a component to enhance the endosomal escape, calcium phosphate, in order to improve delivery of the siRNA.

In one case, calcium phosphate with a PEG-polyanion block copolymer may be used to delivery chimeric polynucleotides (Kazikawa et al., J Contr Rel. 2004 97:345-356; Kazikawa et al., J Contr Rel. 2006 111:368-370).

In one case, a PEG-charge-conversional polymer (Pitella et al., Biomaterials. 2011 32:3106-3114) may be used to form a nanoparticle to deliver the chimeric polynucleotides of the present disclosure. The PEG-charge-conversional polymer may improve upon the PEG-polyanion block copolymers by being cleaved into a polycation at acidic pH, thus enhancing endosomal escape.

In one case, a polymer used may be a pentablock polymer such as, but not limited to, the pentablock polymers described in International Patent Publication No. WO2013055331. As a non-limiting example, the pentablock polymer comprises PGA-PCL-PEG-PCL-PGA, wherein PEG is polyethylene glycol, PCL is poly(E-caprolactone), PGA is poly(glycolic acid), and PLA is poly(lactic acid). As another non-limiting example, the pentablock polymer comprises PEG-PCL- PLA-PCL-PEG, wherein PEG is polyethylene glycol, PCL is poly(E-caprolactone), PGA is poly(glycolic acid), and PLA is poly(lactic acid).

In one case, a polymer which may be used in the present disclosure comprises at least one diepoxide and at least one aminoglycoside (See e.g., International Patent Publication No. WO2013055971). The diepoxide may be selected from, but is not limited to, 1,4 butanediol diglycidyl ether (1,4 B), 1,4-cyclohexanedimethanol diglycidyl ether (1,4 C), 4-vinylcyclohexene diepoxide (4VCD), ethyleneglycol diglycidyl ether (EDGE), glycerol diglycidyl ether (GDE), neopentylglycol diglycidyl ether (NPDGE), poly(ethyleneglycol) diglycidyl ether (PEGDE), poly(propyleneglycol) diglycidyl ether (PPGDE) and resorcinol diglycidyl ether (RDE). The aminoglycoside may be selected from, but is not limited to, streptomycin, neomycin, framycetin, paromomycin, ribostamycin, kanamycin, amikacin, arbekacin, bekanamycin, dibekacin, tobramycin, spectinomycin, hygromycin, gentamicin, netilmicin, sisomicin, isepamicin, verdamicin, astromicin, and apramycin. As a non-limiting example, the polymers may be made by the methods described in International Patent Publication No. WO2013055971. As another non-limiting example, compositions comprising any of the polymers comprising at least one least one diepoxide and at least one aminoglycoside may be made by the methods described in International Patent Publication No. WO2013055971.

In one case, a polymer which may be used in the present disclosure may be a cross-linked polymer. As a non-limiting example, the cross-linked polymers may be used to form a particle as described in US Patent No. 8,414,927. As another non-limiting example, the cross-linked polymer may be obtained by the methods described in US Patent Publication No. US20130172600.

In another case, a polymer which may be used in the present disclosure may be a cross-linked polymer such as those described in US Patent No. 8,461,132. As a non-limiting example, the cross-linked polymer may be used in a therapeutic composition for the treatment of a body tissue. The therapeutic composition may be administered to damaged tissue using various methods known in the art and/or described herein such as injection or catheterization.

In one case, a polymer which may be used in the present disclosure may be a di-alphatic substituted pegylated lipid such as, but not limited to, those described in International Patent Publication No. WO2013049328.

In one case, a block copolymer is PEG-PLGA-PEG (see e.g., the thermosensitive hydrogel (PEG-PLGA-PEG) was used as a TGF-betal gene delivery vehicle in Lee et al. Thermosensitive Hydrogel as a Tgf-β1 Gene Delivery Vehicle Enhances Diabetic Wound Healing. Pharmaceutical Research, 2003 20(12): 1995-2000; as a controlled gene delivery system in Li et al. Controlled Gene Delivery System Based on Thermosensitive Biodegradable Hydrogel. Pharmaceutical Research 2003 20(6):884-888; and Chang et al., Non-ionic amphiphilic biodegradable PEG-PLGA-PEG copolymer enhances gene delivery efficiency in rat skeletal muscle. J Controlled Release. 2007 118:245-253) may be used in the present disclosure. The present disclosure may be formulated with PEG-PLGA-PEG for administration such as, but not limited to, intramuscular and subcutaneous administration.

In another case, the PEG-PLGA-PEG block copolymer is used in the present disclosure to develop a biodegradable sustained release system. In one case, the chimeric polynucleotides of the present disclosure are mixed with the block copolymer prior to administration. In another case, the chimeric polynucleotides acids of the present disclosure are co-administered with the block copolymer.

In one case, the polymer used may be a multi-functional polymer derivative such as, but not limited to, a multi-functional N-maleimidyl polymer derivatives as described in US Patent No US8454946.

The use of core-shell nanoparticles has additionally focused on a high-throughput approach to synthesize cationic cross-linked nanogel cores and various shells (Siegwart et al., Proc Natl Acad Sci U S A. 2011 108:12996-13001).

The complexation, delivery, and internalization of the polymeric nanoparticles can be precisely controlled by altering the chemical composition in both the core and shell components of the nanoparticle. For example, the core-shell nanoparticles may efficiently deliver siRNA to mouse hepatocytes after they covalently attach cholesterol to the nanoparticle.

In one case, a hollow lipid core comprising a middle PLGA layer and an outer neutral lipid layer containing PEG may be used to delivery of the polynucleotide, chimeric polynucleotides of the present disclosure. As a non-limiting example, in mice bearing a luciferase-expressing tumor, it was determined that the lipid-polymer-lipid hybrid nanoparticle significantly suppressed luciferase expression, as compared to a conventional lipoplex (Shi et al, Angew Chem Int Ed. 2011 50:7027-7031).

In one case, the lipid nanoparticles may comprise a core of the chimeric polynucleotides disclosed herein and a polymer shell. The polymer shell may be any of the polymers described herein and are known in the art. In an additional case, the polymer shell may be used to protect the chimeric polynucleotides in the core.

Core-shell nanoparticles for use with the chimeric polynucleotides of the present disclosure are described and may be formed by the methods described in U.S. Pat. No. 8,313,777 or International Patent Publication No. WO2013124867.

In one case, the core-shell nanoparticles may comprise a core of the chimeric polynucleotides disclosed herein and a polymer shell. The polymer shell may be any of the polymers described herein and are known in the art. In an additional case, the polymer shell may be used to protect the chimeric polynucleotides in the core.

In one case, the polymer used with the formulations described herein may be a modified polymer (such as, but not limited to, a modified polyacetal) as described in International Publication No. WO2011120053.

In one case, the formulation may be a polymeric carrier cargo complex comprising a polymeric carrier and at least one nucleic acid molecule. Non-limiting examples of polymeric carrier cargo complexes are described in International Patent Publications Nos. WO2013113326, WO2013113501, WO2013113325, WO2013113502 and WO2013113736 and European Patent Publication No. EP2623121, In one case the polymeric carrier cargo complexes may comprise a negatively charged nucleic acid molecule such as, but not limited to, those described in International Patent Publication Nos. WO2013113325 and WO2013113502,

In one case, a pharmaceutical composition may comprise chimeric polynucleotides of the disclosure and a polymeric carrier cargo complex. The chimeric polynucleotides may encode a protein of interest such as, but not limited to, an antigen from a pathogen associated with infectious disease, an antigen associated with allergy or allergic disease, an antigen associated with autoimmune disease or an antigen associated with cancer or tumor disease (See e.g., the antigens described in International Patent Publications Nos. WO2013113326, WO2013113501, WO2013113325, WO2013113502 and WO2013113736 and European Patent Publication No. EP2623121).

As a non-limiting example, the core-shell nanoparticle may be used to treat an eye disease or disorder (See e.g. US Publication No. 20120321719).

In one case, the polymer used with the formulations described herein may be a modified polymer (such as, but not limited to, a modified polyacetal) as described in International Publication No. WO2011120053.

### Peptides and Proteins

The chimeric polynucleotides can be formulated with peptides and/or proteins in order to increase transfection of cells by the chimeric polynucleotide. Peptides and/or proteins which may be used in the present disclosure are described in paragraphs [000567] - [000570] of co-pending International Publication No. WO2015034928.

### Cells

The chimeric polynucleotides can be transfected *ex vivo* into cells, which are subsequently transplanted into a subject. As non-limiting examples, the pharmaceutical compositions may include red blood cells to deliver modified RNA to liver and myeloid cells, virosomes to deliver modified RNA in virus-like particles (VLPs), and electroporated cells such as, but not limited to, those described in paragraphs [000571] - [000573] of co-pending International Publication No. WO2015034928.

### Introduction into Cells

A variety of methods are known in the art and suitable for introduction of nucleic acid into a cell, including viral and non-viral mediated techniques. Examples of introduction methods which may be used in the present disclosure are described in paragraphs [000574] - [000576] of co-pending International Publication No. WO2015034928.

### Micro-Organ

The chimeric polynucleotides may be contained in a micro-organ which can then express an encoded polypeptide of interest in a long-lasting therapeutic formulation. Micro-organs which may be used in the present invention are described in paragraphs [000577] - [000580] of co-pending International Publication No. WO2015034928.

### Hyaluronidase

The intramuscular or subcutaneous localized injection of chimeric polynucleotides can include hyaluronidase, which catalyzes the hydrolysis of hyaluronan. By catalyzing the hydrolysis of hyaluronan, a constituent of the interstitial barrier, hyaluronidase lowers the viscosity of hyaluronan, thereby increasing tissue permeability (Frost, Expert Opin. Drug Deliv. (2007) 4:427-440
). It is useful to speed their dispersion and systemic distribution of encoded proteins produced by transfected cells. Alternatively, the hyaluronidase can be used to increase the number of cells exposed to a chimeric polynucleotide of the disclosure administered intramuscularly or subcutaneously.

### Nanoparticle Mimics

The chimeric polynucleotides may be encapsulated within and/or absorbed to a nanoparticle mimic. A nanoparticle mimic can mimic the delivery function organisms or particles such as, but not limited to, pathogens, viruses, bacteria, fungus, parasites, prions and cells. As a non-limiting example the chimeric polynucleotides may be encapsulated in a non-viron particle which can mimic the delivery function of a virus (see International Pub. No. WO2012006376 and US Patent Publication No. US20130171241 and US20130195968).

### Nanotubes

The chimeric polynucleotides can be attached or otherwise bound to at least one nanotube such as, but not limited to, rosette nanotubes, rosette nanotubes having twin bases with a linker, carbon nanotubes and/or single-walled carbon nanotubes. Nanotubes which may be used in the present disclosure are described in paragraphs [000583] - [000587] of co-pending International Publication No. WO201503492.

### Conjugates

The chimeric polynucleotides of the disclosure include conjugates, such as a chimeric polynucleotide covalently linked to a carrier or targeting group, or including two encoding regions that together produce a fusion protein (e.g., bearing a targeting group and therapeutic protein or peptide).

The conjugates of the disclosure include a naturally occurring substance, such as a protein (e.g., human serum albumin (HSA), low-density lipoprotein (LDL), high-density lipoprotein (HDL), or globulin); an carbohydrate (e.g., a dextran, pullulan, chitin, chitosan, inulin, cyclodextrin or hyaluronic acid); or a lipid. The ligand may also be a recombinant or synthetic molecule, such as a synthetic polymer, e.g., a synthetic polyamino acid, an oligonucleotide (e.g. an aptamer). Examples of polyamino acids include polyamino acid is a polylysine (PLL), poly L-aspartic acid, poly L-glutamic acid, styrene-maleic acid anhydride copolymer, poly(L-lactide-co-glycolied) copolymer, divinyl ether-maleic anhydride copolymer, N-(2-hydroxypropyl)methacrylamide copolymer (HMPA), polyethylene glycol (PEG), polyvinyl alcohol (PVA), polyurethane, poly(2-ethylacryllic acid), N-isopropylacrylamide polymers, or polyphosphazine. Example of polyamines include: polyethylenimine, polylysine (PLL), spermine, spermidine, polyamine, pseudopeptide-polyamine, peptidomimetic polyamine, dendrimer polyamine, arginine, amidine, protamine, cationic lipid, cationic porphyrin, quaternary salt of a polyamine, or an alpha helical peptide.

Representative U.S. patents that teach the preparation of polynucleotide conjugates, particularly to RNA, include, but are not limited to, U.S. Pat. Nos. 4,828,979; 4,948,882; 5,218,105; 5,525,465; 5,541,313; 5,545,730; 5,552,538; 5,578,717, 5,580,731; 5,591,584; 5,109,124; 5,118,802; 5,138,045; 5,414,077; 5,486,603; 5,512,439; 5,578,718; 5,608,046; 4,587,044; 4,605,735; 4,667,025; 4,762,779; 4,789,737; 4,824,941; 4,835,263; 4,876,335; 4,904,582; 4,958,013; 5,082,830; 5,112,963; 5,214,136; 5,082,830; 5,112,963; 5,214,136; 5,245,022; 5,254,469; 5,258,506; 5,262,536; 5,272,250; 5,292,873; 5,317,098; 5,371,241, 5,391,723; 5,416,203, 5,451,463; 5,510,475; 5,512,667; 5,514,785; 5,565,552; 5,567,810; 5,574,142; 5,585,481; 5,587,371; 5,595,726; 5,597,696; 5,599,923; 5,599,928 and 5,688,941; 6,294,664; 6,320,017; 6,576,752; 6,783,931; 6,900,297; 7,037,646

In one case, the conjugate may function as a carrier for the chimeric polynucleotides of the present disclosure. The conjugate may comprise a cationic polymer such as, but not limited to, polyamine, polylysine, polyalkylenimine, and polyethylenimine which may be grafted to with poly(ethylene glycol). As a non-limiting example, the conjugate may be similar to the polymeric conjugate and the method of synthesizing the polymeric conjugate described in U.S. Pat. No. 6,586,524.

A non-limiting example of a method for conjugation to a substrate is described in US Patent Publication No. US20130211249. The method may be used to make a conjugated polymeric particle comprising a chimeric polynucleotide.

The conjugates can also include targeting groups, e.g., a cell or tissue targeting agent, e.g., a lectin, glycoprotein, lipid or protein, e.g., an antibody, that binds to a specified cell type such as a kidney cell. A targeting group can be a thyrotropin, melanotropin, lectin, glycoprotein, surfactant protein A, Mucin carbohydrate, multivalent lactose, multivalent galactose, N-acetyl-galactosamine, N-acetyl-gulucosamine multivalent mannose, multivalent fucose, glycosylated polyaminoacids, multivalent galactose, transferrin, bisphosphonate, polyglutamate, polyaspartate, a lipid, cholesterol, a steroid, bile acid, folate, vitamin B12, biotin, an RGD peptide, an RGD peptide mimetic or an aptamer.

Targeting groups can be proteins, e.g., glycoproteins, or peptides, e.g., molecules having a specific affinity for a co-ligand, or antibodies e.g., an antibody, that binds to a specified cell type such as a cancer cell, endothelial cell, or bone cell. Targeting groups may also include hormones and hormone receptors. They can also include non-peptidic species, such as lipids, lectins, carbohydrates, vitamins, cofactors, multivalent lactose, multivalent galactose, N-acetyl-galactosamine, N-acetyl-gulucosamine multivalent mannose, multivalent fucose, or aptamers. The ligand can be, for example, a lipopolysaccharide, or an activator of p38 MAP kinase.

The targeting group can be any ligand that is capable of targeting a specific receptor. Examples include, without limitation, folate, GalNAc, galactose, mannose, mannose-6P, aptamers, integrin receptor ligands, chemokine receptor ligands, transferrin, biotin, serotonin receptor ligands, PSMA, endothelin, GCPII, somatostatin, LDL, and HDL ligands. In particular cases, the targeting group is an aptamer. The aptamer can be unmodified or have any combination of modifications disclosed herein.

As a non-limiting example, the targeting group may be a glutathione receptor (GR)-binding conjugate for targeted delivery across the blood-central nervous system barrier (See e.g., US Patent Publication No. US2013021661012.

In one case, the conjugate may be a synergistic biomolecule-polymer conjugate. The synergistic biomolecule-polymer conjugate may be long-acting continuous-release system to provide a greater therapeutic efficacy. The synergistic biomolecule-polymer conjugate may be those described in US Patent Publication No. US20130195799.

In another case, the conjugate which may be used invention may be an aptamer conjugate. Non-limiting examples of aptamer conjugates are described in International Patent Publication No. WO2012040524. The aptamer conjugates may be used to provide targeted delivery of formulations comprising chimeric polynucleotides.

In one case, the conjugate which may be used invention may be an amine containing polymer conjugate. Non-limiting examples of amine containing polymer conjugate are described in US Patent No. US 8,507,653. The factor IX moiety polymer conjugate may comprise releasable linkages to release the chimeric polynucleotides upon and/or after delivery to a subject.

In one case, pharmaceutical compositions of the present disclosure may include chemical modifications such as, but not limited to, modifications similar to locked nucleic acids.

Representative U.S. Patents that teach the preparation of locked nucleic acid (LNA) such as those from Santaris, include, but are not limited to, the following: U.S. Pat. Nos. 6,268,490; 6,670,461; 6,794,499; 6,998,484; 7,053,207; 7,084,125; and 7,399,845.

Representative U.S. patents that teach the preparation of PNA compounds include, but are not limited to, U.S. Pat. Nos. 5,539,082; 5,714,331; and 5,719,262. Further teaching of PNA compounds can be found, for example, in Nielsen et al., Science, 1991, 254, 1497-1500.

Also described herein are chimeric polynucleotides with phosphorothioate backbones and oligonucleosides with other modified backbones, and in particular --CH₂--NH--CH₂--, --CH₂--N(CH₃)--O--CH₂-[known as a methylene (methylimino) or MMI backbone], --CH₂--O--N(CH₃)--CH₂--, --CH₂--N(CH₃)--N(CH₃)--CH₂-- and --N(CH₃)--CH₂--CH₂--[wherein the native phosphodiester backbone is represented as --O-P(O)₂--O--CH₂--] of U.S. Pat. No. 5,489,677, and the amide backbones of U.S. Pat. No. 5,602,240. In some cases, the polynucleotides featured herein have morpholino backbone structures of U.S. Pat. No. 5,034,506.

Modifications at the 2' position may also aid in delivery. Preferably, modifications at the 2' position are not located in a polypeptide-coding sequence, i.e., not in a translatable region. Modifications at the 2' position may be located in a 5'UTR, a 3'UTR and/or a tailing region. Modifications at the 2' position can include one of the following at the 2' position: H (i.e., 2'-deoxy); F; O-, S-, or N-alkyl; O-, S-, or N-alkenyl; O-, S- or N-alkynyl; or O-alkyl-O-alkyl, wherein the alkyl, alkenyl and alkynyl may be substituted or unsubstituted C₁ to C₁₀ alkyl or C₂ to C₁₀ alkenyl and alkynyl. Exemplary suitable modifications include O[(CH₂)ₙO] ₘCH₃, O(CH₂).ₙOCH₃, O(CH₂)ₙNH₂, O(CH₂)ₙCH₃, O(CH₂)ₙONH₂, and O(CH₂)ₙON[(CH₂)ₙCH₃)]₂, where n and m are from 1 to about 10. In other cases, the chimeric polynucleotides include one of the following at the 2' position: C₁ to C₁₀ lower alkyl, substituted lower alkyl, alkaryl, aralkyl, O-alkaryl or O-aralkyl, SH, SCH₃, OCN, Cl, Br, CN, CF₃, OCF₃, SOCH₃, SO₂CH₃, ONO₂, NO₂, N₃, NH₂, heterocycloalkyl, heterocycloalkaryl, aminoalkylamino, polyalkylamino, substituted silyl, an RNA cleaving group, a reporter group, an intercalator, a group for improving the pharmacokinetic properties, or a group for improving the pharmacodynamic properties, and other substituents having similar properties. In some cases, the modification includes a 2'-methoxyethoxy (2'-O-CH₂CH₂OCH₃, also known as 2'-O-(2-methoxyethyl) or 2'-MOE) (Martin et al., Helv. Chim. Acta, 1995, 78:486-504) *i.e.,* an alkoxy-alkoxy group. Another exemplary modification is 2'-dimethylaminooxyethoxy, *i.e.,* a O(CH₂)₂ON(CH₃)₂ group, also known as 2'-DMAOE, as described in examples herein below, and 2'-dimethylaminoethoxyethoxy (also known in the art as 2'-O-dimethylaminoethoxyethyl or 2'-DMAEOE), *i.e.,* 2'-O--CH₂--O--CH₂--N(CH₂)₂, also described in examples herein below. Other modifications include 2'-methoxy (2'-OCH₃), 2'-aminopropoxy (2'-OCH₂CH₂CH₂NH₂) and 2'-fluoro (2'-F). Similar modifications may also be made at other positions, particularly the 3' position of the sugar on the 3' terminal nucleotide or in 2'-5' linked dsRNAs and the 5' position of 5' terminal nucleotide. Polynucleotides may also have sugar mimetics such as cyclobutyl moieties in place of the pentofuranosyl sugar. Representative U.S. patents that teach the preparation of such modified sugar structures include, but are not limited to, U.S. Pat. Nos. 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; and 5,700,920.

In still other cases, the chimeric polynucleotide is covalently conjugated to a cell penetrating polypeptide. The cell-penetrating peptide may also include a signal sequence. The conjugates can be designed to have increased stability; increased cell transfection; and/or altered the biodistribution (e.g., targeted to specific tissues or cell types).

In one case, the chimeric polynucleotides may be conjugated to an agent to enhance delivery. As a non-limiting example, the agent may be a monomer or polymer such as a targeting monomer or a polymer having targeting blocks as described in International Publication No. WO2011062965.

In another non-limiting example, the agent may be a transport agent covalently coupled to the chimeric polynucleotides of the present disclosure (See e.g., U.S. Pat. Nos. 6,835.393 and 7,374,778). In yet another non-limiting example, the agent may be a membrane barrier transport enhancing agent such as those described in U.S. Pat. Nos. 7,737,108 and 8,003,129.

In another case, chimeric polynucleotides may be conjugated to SMARTT POLYMER TECHNOLOGY^{®} (PHASERX^{®}, Inc. Seattle, WA).

In another case, the conjugate may be a peptide that selectively directs the nanoparticle to neurons in a tissue or organism. As a non-limiting example, the peptide used may be, but is not limited to, the peptides described in US Patent Publication No US20130129627.

In yet another case, the conjugate may be a peptide that can assist in crossing the blood-brain barrier.

### Self-Assembled Nanoparticles

Self-assembled nanoparticles including nucleic acid self-assembled nanoparticles, and polymer-based self-assembled nanoparticles, which may be used in the present disclosure are described in paragraphs [000610] - [000619] co-pending International Publication No. WO2015034928.

### Self-Assembled Macromolecules

The chimeric polynucleotides may be formulated in amphiphilic macromolecules (AMs) for delivery. AMs comprise biocompatible amphiphilic polymers which have an alkylated sugar backbone covalently linked to poly(ethylene glycol). In aqueous solution, the AMs self-assemble to form micelles. Non-limiting examples of methods of forming AMs and AMs are described in US Patent Publication No. US20130217753.

### Inorganic Nanoparticles

The chimeric polynucleotides may be formulated in inorganic nanoparticles (U.S. Pat. No. 8,257,745). The inorganic nanoparticles may include, but are not limited to, clay substances that are water swellable. As a non-limiting example, the inorganic nanoparticle may include synthetic smectite clays which are made from simple silicates (See e.g., U.S. Pat. No. 5,585,108 and 8,257,745).

In one case, the inorganic nanoparticles may comprise a core of the chimeric polynucleotides disclosed herein and a polymer shell. The polymer shell may be any of the polymers described herein and are known in the art. In an additional case, the polymer shell may be used to protect the chimeric polynucleotides in the core.

### Semi-conductive and Metallic Nanoparticles

The chimeric polynucleotides may be formulated in water-dispersible nanoparticle comprising a semiconductive or metallic material (U.S. Pub. No. 20120228565) or formed in a magnetic nanoparticle (U.S. Pub. No. 20120265001 and 20120283503). The water-dispersible nanoparticles may be hydrophobic nanoparticles or hydrophilic nanoparticles.

In one case, the semi-conductive and/or metallic nanoparticles may comprise a core of the chimeric polynucleotides disclosed herein and a polymer shell. The polymer shell may be any of the polymers described herein and are known in the art. In an additional case, the polymer shell may be used to protect the chimeric polynucleotides in the core.

### Surgical Sealants: Gels and Hydrogels

In one case, the chimeric polynucleotides disclosed herein may be encapsulated into any hydrogel known in the art which may form a gel when injected into a subject. Hydrogels are a network of polymer chains that are hydrophilic, and are sometimes found as a colloidal gel in which water is the dispersion medium. Hydrogels are highly absorbent (they can contain over 99% water) natural or synthetic polymers. Hydrogels also possess a degree of flexibility very similar to natural tissue, due to their significant water content. The hydrogel described herein may be used to encapsulate lipid nanoparticles which are biocompatible, biodegradable and/or porous. A hydrogel can be made in situ from solution injection or implanted. Gels and hydrogels which may be used in the present disclosure are described in paragraphs [000624] - [000663] of co-pending International Publication No. WO2015034928.

### Suspension formulations

In some cases, suspension formulations are described comprising chimeric polynucleotides, water immiscible oil depots, surfactants and/or co-surfactants and/or co-solvents. Combinations of oils and surfactants may enable suspension formulation with chimeric polynucleotides. Delivery of chimeric polynucleotides in a water immiscible depot may be used to improve bioavailability through sustained release of mRNA from the depot to the surrounding physiologic environment and prevent chimeric polynucleotides degradation by nucleases. Suspension formulations which may be used in the present disclosure are described in paragraphs [000664] - [000670] of co-pending International Publication No. WO2015034928.

### Cations and Anions

Formulations of chimeric polynucleotides disclosed herein may include cations or anions. In one case, the formulations include metal cations such as, but not limited to, Zn2+, Ca2+, Cu2+, Mg+ and combinations thereof. As a non-limiting example, formulations may include polymers and a chimeric polynucleotides complexed with a metal cation (See e.g., U.S. Pat. Nos. 6,265,389 and 6,555,525).

In some cases, cationic nanoparticles comprising combinations of divalent and monovalent cations may be formulated with chimeric polynucleotides. Such nanoparticles may form spontaneously in solution over a given period (e.g. hours, days, etc.). Such nanoparticles do not form in the presence of divalent cations alone or in the presence of monovalent cations alone. The delivery of chimeric polynucleotides in cationic nanoparticles or in one or more depot comprising cationic nanoparticles may improve chimeric polynucleotide bioavailability by acting as a long-acting depot and/or reducing the rate of degradation by nucleases.

### Molded Nanoparticles and Microparticles

The chimeric polynucleotides disclosed herein may be formulated in nanoparticles and/or microparticles. These nanoparticles and/or microparticles may be molded into any size shape and chemistry. As an example, the nanoparticles and/or microparticles may be made using the PRINT^{®} technology by LIQUIDA TECHNOLOGIES^{®} (Morrisville, NC) (See e.g., International Pub. No. WO2007024323).

In one case, the molded nanoparticles may comprise a core of the chimeric polynucleotides disclosed herein and a polymer shell. The polymer shell may be any of the polymers described herein and are known in the art. In an additional case, the polymer shell may be used to protect the chimeric polynucleotides in the core.

In one case, the chimeric polynucleotides may be formulated in microparticles. The microparticles may contain a core of the chimeric polynucleotides and a cortext of a biocompatible and/or biodegradable polymer. As a non-limiting example, the microparticles which may be used with the present disclosure may be those described in U.S. Patent No. 8,460,709, U.S. Patent Publication No. US20130129830 and International Patent Publication No WO2013075068. As another non-limiting example, the microparticles may be designed to extend the release of the chimeric polynucleotides of the present disclosure over a desired period of time (see e.g., extended release of a therapeutic protein in U.S. Patent Publication No. US20130129830).

The microparticle for use with the present disclosure may have a diameter of at least 1 micron to at least 100 microns (e.g., at least 1 micron, at least 5 micron, at least 10 micron, at least 15 micron, at least 20 micron, at least 25 micron, at least 30 micron, at least 35 micron, at least 40 micron, at least 45 micron, at least 50 micron, at least 55 micron, at least 60 micron, at least 65 micron, at least 70 micron, at least 75 micron, at least 80 micron, at least 85 micron, at least 90 micron, at least 95 micron, at least 97 micron, at least 99 micron, and at least 100 micron).

### NanoJackets and NanoLiposomes

The chimeric polynucleotides disclosed herein may be formulated in NanoJackets and NanoLiposomes by Keystone Nano (State College, PA). NanoJackets are made of compounds that are naturally found in the body including calcium, phosphate and may also include a small amount of silicates. Nanojackets may range in size from 5 to 50 nm and may be used to deliver hydrophilic and hydrophobic compounds such as, but not limited to, chimeric polynucleotides.

NanoLiposomes are made of lipids such as, but not limited to, lipids which naturally occur in the body. NanoLiposomes may range in size from 60-80 nm and may be used to deliver hydrophilic and hydrophobic compounds such as, but not limited to, chimeric polynucleotides. In one case, the chimeric polynucleotides disclosed herein are formulated in a NanoLiposome such as, but not limited to, Ceramide NanoLiposomes.

### Pseudovirions

In one case, the chimeric polynucleotides disclosed herein may be formulated in Pseudovirions (e.g., pseudo-virions). Pseudovirions which may be used in the present disclosure are described in paragraphs [000679] - [000684] of co-pending International Publication No. WO2015034928.

### Minicells

In one case, the chimeric polynucleotides may be formulated in bacterial minicells. As a non-limiting example, bacterial minicells may be those described in International Publication No. WO2013088250 or US Patent Publication No. US20130177499.

The bacterial minicells comprising therapeutic agents such as chimeric polynucleotides described herein may be used to deliver the therapeutic agents to brain tumors.

### Semi-solid Compositions

In one case, the chimeric polynucleotides may be formulated with a hydrophobic matrix to form a semi-solid composition. As a non-limiting example, the semi-solid composition or paste-like composition may be made by the methods described in International Patent Publication No WO201307604.

The semi-solid composition may be a sustained release formulation as described in International Patent Publication No WO201307604, .

In another case, the semi-solid composition may further have a micro-porous membrane or a biodegradable polymer formed around the composition (see e.g., International Patent Publication No WO201307604).

The semi-solid composition using the chimeric polynucleotides of the present disclosure may have the characteristics of the semi-solid mixture as described in International Patent Publication No WO201307604 (e.g., a modulus of elasticity of at least 10⁻⁴ N·mm⁻², and/or a viscosity of at least 100mPa·s).

### Exosomes

In one case, the chimeric polynucleotides may be formulated in exosomes. The exosomes may be loaded with at least one chimeric polynucleotide and delivered to cells, tissues and/or organisms. As a non-limiting example, the chimeric polynucleotides may be loaded in the exosomes described in International Publication No. WO2013084000.

### Silk-Based Delivery

In one case, the chimeric polynucleotides may be formulated in a sustained release silk-based delivery system. The silk-based delivery system may be formed by contacting a silk fibroin solution with a therapeutic agent such as, but not limited to, the chimeric polynucleotides described herein and/or known in the art. As a non-limiting example, the sustained release silk-based delivery system which may be used in the present disclosure and methods of making such system are described in US Patent Publication No. US20130177611.

### Microparticles

In one case, formulations comprising chimeric polynucleotides may comprise microparticles. The microparticles may comprise a polymer described herein and/or known in the art such as, but not limited to, poly(α-hydroxy acid), a polyhydroxy butyric acid, a polycaprolactone, a polyorthoester and a polyanhydride. The microparticle may have adsorbent surfaces to adsorb biologically active molecules such as chimeric polynucleotides. As a non-limiting example microparticles for use with the present disclosure and methods of making microparticles are described in US Patent Publication No. US2013195923 and US20130195898 and US Patent No. 8,309,139 and 8,206,749.

In another case, the formulation may be a microemulsion comprising microparticles and chimeric polynucleotides. As a non-limiting example, microemulsions comprising microparticles are described in US Patent Publication No. US2013195923 and US20130195898 and US Patent No. 8,309,139 and 8,206,749.

### Amino Acid Lipids

In one case, the chimeric polynucleotides may be formulated in amino acid lipids. Amino acid lipids are lipophilic compounds comprising an amino acid residue and one or more lipophilic tails. Non-limiting examples of amino acid lipids and methods of making amino acid lipids are described in US Patent No. 8,501,824.

In one case, the amino acid lipids have a hydrophilic portion and a lipophilic portion. The hydrophilic portion may be an amino acid residue and a lipophilic portion may comprise at least one lipophilic tail.

In one case, the amino acid lipid formulations may be used to deliver the chimeric polynucleotides to a subject.

In another case, the amino acid lipid formulations may deliver a chimeric polynucleotide in releasable form which comprises an amino acid lipid that binds and releases the chimeric polynucleotides. As a non-limiting example, the release of the chimeric polynucleotides may be provided by an acid-labile linker such as, but not limited to, those described in U.S. Patent Nos. 7,098,032, 6,897,196, 6,426,086, 7,138,382, 5,563,250, and 5,505,93.

### Microvesicles

In one case, chimeric polynucleotides may be formulated in microvesicles. Non-limiting examples of microvesicles include those described in US Patent Publication No. US20130209544.

In one case, the microvesicle is an ARRDC1-mediated microvesicles (ARMMs). Non-limiting examples of ARMMs and methods of making ARMMs are described in International Patent Publication No. WO2013119602.

### Interpolyelectrolyte Complexes

In one case, the chimeric polynucleotides may be formulated in an interpolyelectrolyte complex. Interpolyelectrolyte complexes are formed when charge-dynamic polymers are complexed with one or more anionic molecules. Non-limiting examples of charge-dynamic polymers and interpolyelectrolyte complexes and methods of making interpolyelectrolyte complexes are described in US Patent No. 8,524,368.

### Crystalline Polymeric Systems

In one case, the chimeric polynucleotides may be formulated in crystalline polymeric systems. Crystalline polymeric systems are polymers with crystalline moieties and/or terminal units comprising crystalline moieties. Non-limiting examples of polymers with crystalline moieties and/or terminal units comprising crystalline moieties termed "CYC polymers," crystalline polymer systems and methods of making such polymers and systems are described in US Patent No. US 8,524,259.

### Excipients

Pharmaceutical formulations may additionally comprise a pharmaceutically acceptable excipient, which, as used herein, includes, but are not limited to, any and all solvents, dispersion media, diluents, or other liquid vehicles, dispersion or suspension aids, surface active agents, isotonic agents, thickening or emulsifying agents, preservatives, solid binders, lubricants, flavoring agents, stabilizers, antioxidants, osmolality adjusting agents, pH adjusting agents and the like, as suited to the particular dosage form desired. Various excipients for formulating pharmaceutical compositions and techniques for preparing the composition are known in the art (see Remington: The Science and Practice of Pharmacy, 21st Edition, A. R. Gennaro (Lippincott, Williams & Wilkins, Baltimore, MD, 2006).

The use of a conventional excipient medium may be contemplated within the scope of the present disclosure, except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this disclosure.

In some cases, a pharmaceutically acceptable excipient may be at least 95%, at least 96%, at least 97%, at least 98%, at least 99%, or 100% pure. In some cases, an excipient is approved for use for humans and for veterinary use. In some cases, an excipient may be approved by United States Food and Drug Administration. In some cases, an excipient may be of pharmaceutical grade. In some cases, an excipient may meet the standards of the United States Pharmacopoeia (USP), the European Pharmacopoeia (EP), the British Pharmacopoeia, and/or the International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in pharmaceutical compositions. The composition may also include excipients such as cocoa butter and suppository waxes, coloring agents, coating agents, sweetening, flavoring, and/or perfuming agents.

Exemplary diluents, granulating and/or dispersing agents, surface active agents and/or emulsifiers, binding agents, preservatives, buffers, lubricating agents, oils, additives, cocoa butter and suppository waxes, coloring agents, coating agents, sweetening, flavoring, and/or perfuming agents are described in co-pending International Patent Publication No. WO2015038892, such as, but not limited to, in paragraphs [000828] - [000838]. Cryoprotectants for mRNA

In some cases, chimeric polynucleotide formulations may comprise cyroprotectants. As used herein, there term "cryoprotectant" refers to one or more agent that when combined with a given substance, helps to reduce or eliminate damage to that substance that occurs upon freezing. In some cases, cryoprotectants are combined with chimeric polynucleotides in order to stabilize them during freezing. Frozen storage of mRNA between -20°C and -80°C may be advantageous for long term (e.g. 36 months) stability of chimeric polynucleotide. In some cases, cryoprotectants are included in chimeric polynucleotide formulations to stabilize chimeric polynucleotide through freeze/thaw cycles and under frozen storage conditions. Cryoprotectants may include, but are not limited to sucrose, trehalose, lactose, glycerol, dextrose, raffinose and/or mannitol. Trehalose is listed by the Food and Drug Administration as being generally regarded as safe (GRAS) and is commonly used in commercial pharmaceutical formulations.

### Bulking agents

In some cases, chimeric polynucleotide formulations may comprise bulking agents. As used herein, ther term "bulking agent" refers to one or more agents included in formulations to impart a desired consistency to the formulation and/or stabilization of formulation components. In some cases, bulking agents are included in lyophilized chimeric polynucleotide formulations to yield a "pharmaceutically elegant" cake, stabilizing the lyophilized chimeric polynucleotides during long term (e.g. 36 month) storage. Bulking agents may include, but are not limited to sucrose, trehalose, mannitol, glycine, lactose and/or raffinose. In some cases, combinations of cryoprotectants and bulking agents (for example, sucrose/glycine or trehalose/mannitol) may be included to both stabilize chimeric polynucleotides during freezing and provide a bulking agent for lyophilization.

Non-limiting examples of formulations and methods for formulating the chimeric polynucleotides of the present disclosure are also described in International Publication No WO2013090648 filed December 14, 2012.

### Inactive Ingredients

In some cases, chimeric polynucleotide formulations may comprise at least one excipient which is an inactive ingredient. As used herein, ther term "inactive ingredient" refers to one or more inactive agents included in formulations. In some cases, all, none or some of the inactive ingredients which may be used in the formulations of the present disclosure may be approved by the US Food and Drug Administration (FDA).

A non-exhaustive list of inactive ingredients and the routes of administration the inactive ingredients may be formulated in are described in Table 4 of co-pending International Publication No. WO2014152211 (Attorney Docket No. M030).

### Delivery

The present disclosure encompasses the delivery of chimeric polynucleotides for any of therapeutic, pharmaceutical, diagnostic or imaging by any appropriate route taking into consideration likely advances in the sciences of drug delivery. Delivery may be naked or formulated.

### Naked Delivery

The chimeric polynucleotides may be delivered to a cell naked. As used herein in, "naked" refers to delivering chimeric polynucleotides free from agents which promote transfection. For example, the chimeric polynucleotides delivered to the cell may contain no modifications. The naked chimeric polynucleotides may be delivered to the cell using routes of administration known in the art and described herein.

### Formulated Delivery

The chimeric polynucleotides may be formulated, using the methods described herein. The formulations may contain chimeric polynucleotides which may be modified and/or unmodified. The formulations may further include, but are not limited to, cell penetration agents, a pharmaceutically acceptable carrier, a delivery agent, a bioerodible or biocompatible polymer, a solvent, and a sustained-release delivery depot. The formulated chimeric polynucleotides may be delivered to the cell using routes of administration known in the art and described herein.

The compositions may also be formulated for direct delivery to an organ or tissue in any of several ways in the art including, but not limited to, direct soaking or bathing, via a catheter, by gels, powder, ointments, creams, gels, lotions, and/or drops, by using substrates such as fabric or biodegradable materials coated or impregnated with the compositions, and the like.

### Administration

The chimeric polynucleotides may be administered by any route which results in a therapeutically effective outcome. These include, but are not limited to enteral (into the intestine), gastroenteral, epidural (into the dura matter), oral (by way of the mouth), transdermal, peridural, intracerebral (into the cerebrum), intracerebroventricular (into the cerebral ventricles), epicutaneous (application onto the skin), intradermal, (into the skin itself), subcutaneous (under the skin), nasal administration (through the nose), intravenous (into a vein), intravenous bolus, intravenous drip, intraarterial (into an artery), intramuscular (into a muscle), intracardiac (into the heart), intraosseous infusion (into the bone marrow), intrathecal (into the spinal canal), intraperitoneal, (infusion or injection into the peritoneum), intravesical infusion, intravitreal, (through the eye), intracavernous injection (into a pathologic cavity) intracavitary (into the base of the penis), intravaginal administration, intrauterine, extra-amniotic administration, transdermal (diffusion through the intact skin for systemic distribution), transmucosal (diffusion through a mucous membrane), transvaginal, insufflation (snorting), sublingual, sublabial, enema, eye drops (onto the conjunctiva), in ear drops, auricular (in or by way of the ear), buccal (directed toward the cheek), conjunctival, cutaneous, dental (to a tooth or teeth), electro-osmosis, endocervical, endosinusial, endotracheal, extracorporeal, hemodialysis, infiltration, interstitial, intra-abdominal, intra-amniotic, intra-articular, intrabiliary, intrabronchial, intrabursal, intracartilaginous (within a cartilage), intracaudal (within the cauda equine), intracisternal (within the cisterna magna cerebellomedularis), intracorneal (within the cornea), dental intracornal, intracoronary (within the coronary arteries), intracorporus cavernosum (within the dilatable spaces of the corporus cavernosa of the penis), intradiscal (within a disc), intraductal (within a duct of a gland), intraduodenal (within the duodenum), intradural (within or beneath the dura), intraepidermal (to the epidermis), intraesophageal (to the esophagus), intragastric (within the stomach), intragingival (within the gingivae), intraileal (within the distal portion of the small intestine), intralesional (within or introduced directly to a localized lesion), intraluminal (within a lumen of a tube), intralymphatic (within the lymph), intramedullary (within the marrow cavity of a bone), intrameningeal (within the meninges), intraocular (within the eye), intraovarian (within the ovary), intrapericardial (within the pericardium), intrapleural (within the pleura), intraprostatic (within the prostate gland), intrapulmonary (within the lungs or its bronchi), intrasinal (within the nasal or periorbital sinuses), intraspinal (within the vertebral column), intrasynovial (within the synovial cavity of a joint), intratendinous (within a tendon), intratesticular (within the testicle), intrathecal (within the cerebrospinal fluid at any level of the cerebrospinal axis), intrathoracic (within the thorax), intratubular (within the tubules of an organ), intratumor (within a tumor), intratympanic (within the aurus media), intravascular (within a vessel or vessels), intraventricular (within a ventricle), iontophoresis (by means of electric current where ions of soluble salts migrate into the tissues of the body), irrigation (to bathe or flush open wounds or body cavities), laryngeal (directly upon the larynx), nasogastric (through the nose and into the stomach), occlusive dressing technique (topical route administration which is then covered by a dressing which occludes the area), ophthalmic (to the external eye), oropharyngeal (directly to the mouth and pharynx), parenteral, percutaneous, periarticular, peridural, perineural, periodontal, rectal, respiratory (within the respiratory tract by inhaling orally or nasally for local or systemic effect), retrobulbar (behind the pons or behind the eyeball), soft tissue, subarachnoid, subconjunctival, submucosal, topical, transplacental (through or across the placenta), transtracheal (through the wall of the trachea), transtympanic (across or through the tympanic cavity), ureteral (to the ureter), urethral (to the urethra), vaginal, caudal block, diagnostic, nerve block, biliary perfusion, cardiac perfusion, photopheresis or spinal. In specific cases, compositions may be administered in a way which allows them cross the blood-brain barrier, vascular barrier, or other epithelial barrier.In one case, a formulation for a route of administration may include at least one inactive ingredient. Non-limiting examples of routes of administration and inactive ingredients which may be included in formulations for the specific route of administration is shown in Table 9 of co-pending International Publication No. WO2015038892.

Non-limiting routes of administration for the chimeric polynucleotides of the present disclosure are described below.

### Parenteral and Injectable Administration

Liquid dosage forms for parenteral administration include, but are not limited to, pharmaceutically acceptable emulsions, microemulsions, solutions, suspensions, syrups, and/or elixirs. In addition to active ingredients, liquid dosage forms may comprise inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, oral compositions can include adjuvants such as wetting agents, emulsifying and suspending agents, sweetening, flavoring, and/or perfuming agents. In certain cases for parenteral administration, compositions are mixed with solubilizing agents such as CREMOPHOR^{®}, alcohols, oils, modified oils, glycols, polysorbates, cyclodextrins, polymers, and/or combinations thereof.

A pharmaceutical composition for parenteral administration may comprise at least one inactive ingredient. Any or none of the inactive ingredients used may have been approved by the US Food and Drug Administration (FDA). A non-exhaustive list of inactive ingredients for use in pharmaceutical compositions for parenteral administration includes hydrochloric acid, mannitol, nitrogen, sodium acetate, sodium chloride and sodium hydroxide.

Injectable preparations, for example, sterile injectable aqueous or oleaginous suspensions may be formulated according to the known art using suitable dispersing agents, wetting agents, and/or suspending agents. Sterile injectable preparations may be sterile injectable solutions, suspensions, and/or emulsions in nontoxic parenterally acceptable diluents and/or solvents, for example, as a solution in 1,3-butanediol. Among the acceptable vehicles and solvents that may be employed are water, Ringer's solution, U.S.P., and isotonic sodium chloride solution. Sterile, fixed oils are conventionally employed as a solvent or suspending medium. For this purpose any bland fixed oil can be employed including synthetic mono- or diglycerides. Fatty acids such as oleic acid can be used in the preparation of injectables. The sterile formulation may also comprise adjuvants such as local anesthetics, preservatives and buffering agents.

Injectable formulations can be sterilized, for example, by filtration through a bacterial-retaining filter, and/or by incorporating sterilizing agents in the form of sterile solid compositions which can be dissolved or dispersed in sterile water or other sterile injectable medium prior to use.

In order to prolong the effect of an active ingredient, it is often desirable to slow the absorption of the active ingredient from subcutaneous or intramuscular injection. This may be accomplished by the use of a liquid suspension of crystalline or amorphous material with poor water solubility. The rate of absorption of the drug then depends upon its rate of dissolution which, in turn, may depend upon crystal size and crystalline form. Alternatively, delayed absorption of a parenterally administered drug form is accomplished by dissolving or suspending the drug in an oil vehicle. Injectable depot forms are made by forming microencapsule matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides). Depot injectable formulations are prepared by entrapping the drug in liposomes or microemulsions which are compatible with body tissues.

### Rectal and Vaginal Administration

Rectal and vaginal administration and corresponding dosage forms are described in co-pending International Patent Publication No. WO2015038892 such as, but not limited to, in paragraphs [000856] - [000859].

### Oral Administration

Oral administration and corresponding dosage forms (e.g., liquid dosage forms) are described in co-pending International Patent Publication No. WO2015038892, such as, but not limited to, in paragraphs [000860] - [000869].

### Topical or Transdermal Administration

As described herein, compositions containing the chimeric polynucleotides may be formulated for administration topically and/or transdermally. The skin may be an ideal target site for delivery as it is readily accessible. Gene expression may be restricted not only to the skin, potentially avoiding nonspecific toxicity, but also to specific layers and cell types within the skin.

The site of cutaneous expression of the delivered compositions will depend on the route of nucleic acid delivery. Three routes are commonly considered to deliver chimeric polynucleotides to the skin: (i) topical application (e.g. for local/regional treatment and/or cosmetic applications); (ii) intradermal injection (e.g. for local/regional treatment and/or cosmetic applications); and (iii) systemic delivery (e.g. for treatment of dermatologic diseases that affect both cutaneous and extracutaneous regions). Chimeric polynucleotides can be delivered to the skin by several different approaches known in the art. Most topical delivery approaches have been shown to work for delivery of DNA, such as but not limited to, topical application of non-cationic liposome-DNA complex, cationic liposome-DNA complex, particle-mediated (gene gun), puncture-mediated gene transfections, and viral delivery approaches. After delivery of the nucleic acid, gene products have been detected in a number of different skin cell types, including, but not limited to, basal keratinocytes, sebaceous gland cells, dermal fibroblasts and dermal macrophages.

Ointments, creams and gels for topical administration, can, for example, can be formulated with an aqueous or oily base with the addition of suitable thickening and/or gelling agent and/or solvents. Non limiting examples of such bases can thus, for example, include water and/or an oil such as liquid paraffin or a vegetable oil such as arachis oil or castor oil, or a solvent such as polyethylene glycol. Various thickening agents and gelling agents can be used depending on the nature of the base. Non-limiting examples of such agents include soft paraffin, aluminum stearate, cetostearyl alcohol, polyethylene glycols, woolfat, beeswax, carboxypolymethylene and cellulose derivatives, and/or glyceryl monostearate and/or non-ionic emulsifying agents.

Lotions for topical administration may be formulated with an aqueous or oily base and will in general also contain one or more emulsifying agents, stabilizing agents, dispersing agents, suspending agents or thickening agents.

Also described herein is a variety of dressings (e.g., wound dressings) or bandages (e.g., adhesive bandages) for conveniently and/or effectively carrying out methods of the present disclosure. Typically dressing or bandages may comprise sufficient amounts of pharmaceutical compositions and/or chimeric polynucleotides described herein to allow a user to perform multiple treatments of a subject(s).

Further described herein is the chimeric polynucleotides compositions to be delivered in more than one injection.

In one case, before topical and/or transdermal administration at least one area of tissue, such as skin, may be subjected to a device and/or solution which may increase permeability. In one case, the tissue may be subjected to an abrasion device to increase the permeability of the skin (see U.S. Patent Publication No. 20080275468). In another case, the tissue may be subjected to an ultrasound enhancement device. An ultrasound enhancement device may include, but is not limited to, the devices described in U.S. Publication No. 20040236268 and U.S. Patent Nos. 6,491,657 and 6,234,990.

Methods of enhancing the permeability of tissue are described in U.S. Publication Nos. 20040171980 and 20040236268 and U.S. Pat. No. 6,190,315.

In one case, a device may be used to increase permeability of tissue before delivering formulations of modified mRNA described herein. The permeability of skin may be measured by methods known in the art and/or described in U.S. Patent No. 6,190,315. As a non-limiting example, a modified mRNA formulation may be delivered by the drug delivery methods described in U.S. Patent No. 6,190,315.

In another non-limiting example tissue may be treated with a eutectic mixture of local anesthetics (EMLA) cream before, during and/or after the tissue may be subjected to a device which may increase permeability. Katz et al. (Anesth Analg (2004); 98:371-76; showed that using the EMLA cream in combination with a low energy, an onset of superficial cutaneous analgesia was seen as fast as 5 minutes after a pretreatment with a low energy ultrasound.

In one case, enhancers may be applied to the tissue before, during, and/or after the tissue has been treated to increase permeability. Enhancers include, but are not limited to, transport enhancers, physical enhancers, and cavitation enhancers. Non-limiting examples of enhancers are described in U.S. Patent No. 6,190,315.

In one case, a device may be used to increase permeability of tissue before delivering formulations of modified mRNA described herein, which may further contain a substance that invokes an immune response. In another non-limiting example, a formulation containing a substance to invoke an immune response may be delivered by the methods described in U.S. Publication Nos. 20040171980 and 20040236268.

Dosage forms for topical and/or transdermal administration of a composition may include ointments, pastes, creams, lotions, gels, powders, solutions, sprays, inhalants and/or patches. Generally, an active ingredient is admixed under sterile conditions with a pharmaceutically acceptable excipient and/or any needed preservatives and/or buffers as may be required.

Additionally, the present disclosure contemplates the use of transdermal patches, which often have the added advantage of providing controlled delivery of a compound to the body. Such dosage forms may be prepared, for example, by dissolving and/or dispensing the compound in the proper medium. Alternatively or additionally, rate may be controlled by either providing a rate controlling membrane and/or by dispersing the compound in a polymer matrix and/or gel.

Formulations suitable for topical administration include, but are not limited to, liquid and/or semi liquid preparations such as liniments, lotions, oil in water and/or water in oil emulsions such as creams, ointments and/or pastes, and/or solutions and/or suspensions.

Topically-administrable formulations may, for example, comprise from about 0.1% to about 10% (w/w) active ingredient, although the concentration of active ingredient may be as high as the solubility limit of the active ingredient in the solvent. Formulations for topical administration may further comprise one or more of the additional ingredients described herein.

Topical, transdermal and transcutaneous administration and corresponding dosage forms are described in co-pending International Patent Publication No. WO2015038892, such as, but not limited to, in paragraphs [000870] - [000888].

### Depot Administration

As described herein, in some cases, the composition is formulated in depots for extended release. Generally, a specific organ or tissue (a "target tissue") is targeted for administration.

In some cases of the disclosure, the chimeric polynucleotides are spatially retained within or proximal to a target tissue. Described herein are method of providing a composition to a target tissue of a mammalian subject by contacting the target tissue (which contains one or more target cells) with the composition under conditions such that the composition, in particular the nucleic acid component(s) of the composition, is substantially retained in the target tissue, meaning that at least 10, 20, 30, 40, 50, 60, 70, 80, 85, 90, 95, 96, 97, 98, 99, 99.9, 99.99 or greater than 99.99% of the composition is retained in the target tissue. Advantageously, retention is determined by measuring the amount of the nucleic acid present in the composition that enters one or more target cells. For example, at least 1, 5, 10, 20, 30, 40, 50, 60, 70, 80, 85, 90, 95, 96, 97, 98, 99, 99.9, 99.99 or greater than 99.99% of the nucleic acids administered to the subject are present intracellularly at a period of time following administration. For example, intramuscular injection to a mammalian subject is performed using an aqueous composition containing a ribonucleic acid and a transfection reagent, and retention of the composition is determined by measuring the amount of the ribonucleic acid present in the muscle cells.

Some cases of the disclosure are directed to methods of describing a composition to a target tissue of a mammalian subject, by contacting the target tissue (containing one or more target cells) with the composition under conditions such that the composition is substantially retained in the target tissue. The composition contains an effective amount of a chimeric polynucleotides such that the polypeptide of interest is produced in at least one target cell. The compositions generally contain a cell penetration agent, although "naked" nucleic acid (such as nucleic acids without a cell penetration agent or other agent) is also contemplated, and a pharmaceutically acceptable carrier.

In some circumstances, the amount of a protein produced by cells in a tissue is desirably increased. Preferably, this increase in protein production is spatially restricted to cells within the target tissue. Thus, described are methods of increasing production of a protein of interest in a tissue of a mammalian subject. A composition is described that contains chimeric polynucleotides characterized in that a unit quantity of composition has been determined to produce the polypeptide of interest in a substantial percentage of cells contained within a predetermined volume of the target tissue.

In some cases, the composition includes a plurality of different chimeric polynucleotides, where one or more than one of the chimeric polynucleotides encodes a polypeptide of interest. Optionally, the composition also contains a cell penetration agent to assist in the intracellular delivery of the composition. A determination is made of the dose of the composition required to produce the polypeptide of interest in a substantial percentage of cells contained within the predetermined volume of the target tissue (generally, without inducing significant production of the polypeptide of interest in tissue adjacent to the predetermined volume, or distally to the target tissue). Subsequent to this determination, the determined dose is introduced directly into the tissue of the mammalian subject.

Described herein is the chimeric polynucleotides to be delivered in more than one injection or by split dose injections.

In one case, the disclosure may be retained near target tissue using a small disposable drug reservoir, patch pump or osmotic pump. Non-limiting examples of patch pumps include those manufactured and/or sold by BD^{®} (Franklin Lakes, NJ), Insulet Corporation (Bedford, MA), SteadyMed Therapeutics (San Francisco, CA), Medtronic (Minneapolis, MN) (e.g., MiniMed), UniLife (York, PA), Valeritas (Bridgewater, NJ), and SpringLeaf Therapeutics (Boston, MA). A non-limiting example of an osmotic pump include those manufactured by DURECT^{®} (Cupertino, CA) (e.g., DUROS^{®} and ALZET ^{®}).

### Pulmonary Administration

A pharmaceutical composition may be prepared, packaged, and/or sold in a formulation suitable for pulmonary administration via the buccal cavity. Pulmonary administration and corresponding dosage forms are described in co-pending International Patent Publication No. WO2015038892, such as, but not limited to, in paragraphs [000896] - [000901

### Intranasal, nasal and buccal Administration

Formulations described herein as being useful for pulmonary delivery are useful for intranasal delivery of a pharmaceutical composition. Another formulation suitable for intranasal administration is a coarse powder comprising the active ingredient and having an average particle from about 0.2 µm to 500 µm. Such a formulation is administered in the manner in which snuff is taken, *i.e.* by rapid inhalation through the nasal passage from a container of the powder held close to the nose. Intranasal, nasal and buccal administration and corresponding dosage forms are described in co-pending International Patent Publication No. WO2015038892, such as, but not limited to, in paragraphs [000902] - [000905].

### Ophthalmic and Auricular (Otic) Administration

A pharmaceutical composition may be prepared, packaged, and/or sold in a formulation suitable for delivery to and/or around the eye and/or delivery to the ear (e.g., auricular (otic) administration). Non-limiting examples of route of administration for delivery to and/or around the eye include retrobulbar, conjuctival, intracorneal, intraocular, intravitreal, ophthalmic and subconjuctiva. Ophthalmic and auricular administration and corresponding dosage forms are described in co-pending International Patent Publication No. WO2015038892, such as, but not limited to, in paragraphs [000906] - [000912].

### Payload Administration: Detectable Agents and Therapeutic Agents

The chimeric polynucleotides described herein can be used in a number of different scenarios in which delivery of a substance (the "payload") to a biological target is desired, for example delivery of detectable substances for detection of the target, or delivery of a therapeutic agent. Detection methods can include, but are not limited to, both imaging *in vitro* and *in vivo* imaging methods, *e.g.,* immunohistochemistry, bioluminescence imaging (BLI), Magnetic Resonance Imaging (MRI), positron emission tomography (PET), electron microscopy, X-ray computed tomography, Raman imaging, optical coherence tomography, absorption imaging, thermal imaging, fluorescence reflectance imaging, fluorescence microscopy, fluorescence molecular tomographic imaging, nuclear magnetic resonance imaging, X-ray imaging, ultrasound imaging, photoacoustic imaging, lab assays, or in any situation where tagging/staining/imaging is required.

The chimeric polynucleotides can be designed to include both a linker and a payload in any useful orientation. For example, a linker having two ends is used to attach one end to the payload and the other end to the nucleobase, such as at the C-7 or C-8 positions of the deaza-adenosine or deaza-guanosine or to the N-3 or C-5 positions of cytosine or uracil. The polynucleotide can include more than one payload (e.g., a label and a transcription inhibitor), as well as a cleavable linker. In one case, the modified nucleotide is a modified 7-deaza-adenosine triphosphate, where one end of a cleavable linker is attached to the C7 position of 7-deaza-adenine, the other end of the linker is attached to an inhibitor (e.g., to the C5 position of the nucleobase on a cytidine), and a label (e.g., Cy5) is attached to the center of the linker (see, e.g., compound 1 of A^{∗}pCp C5 Parg Capless in Fig. 5 and columns 9 and 10 of U.S. Pat. No. 7,994,304). Upon incorporation of the modified 7-deaza-adenosine triphosphate to an encoding region, the resulting polynucleotide having a cleavable linker attached to a label and an inhibitor (e.g., a polymerase inhibitor). Upon cleavage of the linker (e.g., with reductive conditions to reduce a linker having a cleavable disulfide moiety), the label and inhibitor are released. Additional linkers and payloads (e.g., therapeutic agents, detectable labels, and cell penetrating payloads) are described herein and in International Application PCT/US2013/30062 filed March 9, 2013 (Attorney Docket Number M300).

For example, the chimeric polynucleotides described herein can be used in reprogramming induced pluripotent stem cells (iPS cells), which can directly track cells that are transfected compared to total cells in the cluster. In another example, a drug that may be attached to the chimeric polynucleotides via a linker and may be fluorescently labeled can be used to track the drug *in vivo, e.g.* intracellularly. Other examples include, but are not limited to, the use of a chimeric polynucleotides in reversible drug delivery into cells.

The chimeric polynucleotides described herein can be used in intracellular targeting of a payload, *e*.*g*., detectable or therapeutic agent, to specific organelle. Exemplary intracellular targets can include, but are not limited to, the nuclear localization for advanced mRNA processing, or a nuclear localization sequence (NLS) linked to the mRNA containing an inhibitor.

In addition, the chimeric polynucleotides described herein can be used to deliver therapeutic agents to cells or tissues, *e*.*g*., in living animals. For example, the chimeric polynucleotides described herein can be used to deliver highly polar chemotherapeutics agents to kill cancer cells. The chimeric polynucleotides attached to the therapeutic agent through a linker can facilitate member permeation allowing the therapeutic agent to travel into a cell to reach an intracellular target.

In one example, the linker is attached at the 2'-position of the ribose ring and/or at the 3' and/or 5' position of the chimeric polynucleotides (See e.g., International Pub. No. WO2012030683).

The linker may be any linker disclosed herein, known in the art and/or disclosed in International Pub. No. WO2012030683.

In another example, the chimeric polynucleotides can be attached to the chimeric polynucleotides a viral inhibitory peptide (VIP) through a cleavable linker. The cleavable linker can release the VIP and dye into the cell. In another example, the chimeric polynucleotides can be attached through the linker to an ADP-ribosylate, which is responsible for the actions of some bacterial toxins, such as cholera toxin, diphtheria toxin, and pertussis toxin. These toxin proteins are ADP-ribosyltransferases that modify target proteins in human cells. For example, cholera toxin ADP-ribosylates G proteins modifies human cells by causing massive fluid secretion from the lining of the small intestine, which results in life-threatening diarrhea.

In some cases, the payload may be a therapeutic agent such as a cytotoxin, radioactive ion, chemotherapeutic, or other therapeutic agent. A cytotoxin or cytotoxic agent includes any agent that may be detrimental to cells. Examples include, but are not limited to, taxol, cytochalasin B, gramicidin D, ethidium bromide, emetine, mitomycin, etoposide, teniposide, vincristine, vinblastine, colchicine, doxorubicin, daunorubicin, dihydroxyanthracinedione, mitoxantrone, mithramycin, actinomycin D, 1-dehydrotestosterone, glucocorticoids, procaine, tetracaine, lidocaine, propranolol, puromycin, maytansinoids, e.g., maytansinol (see U.S. Pat. No. 5,208,020 incorporated herein in its entirety), rachelmycin (CC-1065, see U.S. Pat. Nos. 5,475,092, 5,585,499, and 5,846,545),
and analogs or homologs thereof. Radioactive ions include, but are not limited to iodine (*e*.*g*., iodine 125 or iodine 131), strontium 89, phosphorous, palladium, cesium, iridium, phosphate, cobalt, yttrium 90, samarium 153, and praseodymium. Other therapeutic agents include, but are not limited to, antimetabolites (e.g., methotrexate, 6-mercaptopurine, 6-thioguanine, cytarabine, 5-fluorouracil decarbazine), alkylating agents (e.g., mechlorethamine, thiotepa chlorambucil, rachelmycin (CC-1065), melphalan, carmustine (BSNU), lomustine (CCNU), cyclophosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine, vinblastine, taxol and maytansinoids).

In some cases, the payload may be a detectable agent, such as various organic small molecules, inorganic compounds, nanoparticles, enzymes or enzyme substrates, fluorescent materials, luminescent materials (e.g., luminol), bioluminescent materials (e.g., luciferase, luciferin, and aequorin), chemiluminescent materials, radioactive materials (e.g., ¹⁸F, ⁶⁷Ga, ^{81m}Kr, ⁸²Rb, ¹¹¹In, ¹²³I, ¹³³Xe, ²⁰¹Tl, ¹²⁵I, ³⁵S, ¹⁴C, ³H, or ^{99m}Tc (e.g., as pertechnetate (technetate(VII), TcO₄⁻)), and contrast agents (e.g., gold *(e.g.,* gold nanoparticles), gadolinium (e.g., chelated Gd), iron oxides (e.g., superparamagnetic iron oxide (SPIO), monocrystalline iron oxide nanoparticles (MIONs), and ultrasmall superparamagnetic iron oxide (USPIO)), manganese chelates (e.g., Mn-DPDP), barium sulfate, iodinated contrast media (iohexol), microbubbles, or perfluorocarbons). Such optically-detectable labels include for example, without limitation, 4-acetamido-4'-isothiocyanatostilbene-2,2'disulfonic acid; acridine and derivatives (e.g., acridine and acridine isothiocyanate); 5-(2'-aminoethyl)aminonaphthalene-l-sulfonic acid (EDANS); 4-amino-N-[3-vinylsulfonyl)phenyl]naphthalimide-3,5 disulfonate; N-(4-anilino-1-naphthyl)maleimide; anthranilamide; BODIPY; Brilliant Yellow; coumarin and derivatives (e.g., coumarin, 7-amino-4-methylcoumarin (AMC, Coumarin 120), and 7-amino-4-trifluoromethylcoumarin (Coumarin 151)); cyanine dyes; cyanosine; 4',6-diaminidino-2-phenylindole (DAPI); 5' 5"-dibromopyrogallol-sulfonaphthalein (Bromopyrogallol Red); 7-diethylamino-3-(4'-isothiocyanatophenyl)-4-methylcoumarin; diethylenetriamine pentaacetate; 4,4'-diisothiocyanatodihydro-stilbene-2,2'-disulfonic acid; 4,4'-diisothiocyanatostilbene-2,2'-disulfonic acid; 5-[dimethylamino]-naphthalene-1-sulfonyl chloride (DNS, dansylchloride); 4-dimethylaminophenylazophenyl-4'-isothiocyanate (DABITC); eosin and derivatives (e.g., eosin and eosin isothiocyanate); erythrosin and derivatives (e.g., erythrosin B and erythrosin isothiocyanate); ethidium; fluorescein and derivatives (e.g., 5-carboxyfluorescein (FAM), 5-(4,6-dichlorotriazin-2-yl)aminofluorescein (DTAF), 2',7'-dimethoxy-4Y-dichloro-6-carboxyfluorescein, fluorescein, fluorescein isothiocyanate, X-rhodamine-5-(and-6)-isothiocyanate (QFITC or XRITC), and fluorescamine); 2-[2-[3-[[1,3-dihydro-1,1-dimethyl-3-(3-sulfopropyl)-2H-benz[e]indol-2-ylidene]ethylidene]-2-[4-(ethoxycarbonyl)-1-piperazinyl]-1-cyclopenten-1-yl]ethenyl]-1,1-dimethyl-3-(3-sulforpropyl)-1H-benz[e]indolium hydroxide, inner salt, compound with n,n-diethylethanamine(1:1) (IR144); 5-chloro-2-[2-[3-[(5-chloro-3-ethyl-2(3H)-benzothiazol- ylidene)ethylidene]-2-(diphenylamino)-1-cyclopenten-1-yl] ethenyl]-3-ethyl benzothiazolium perchlorate (IR140); Malachite Green isothiocyanate; 4-methylumbelliferone orthocresolphthalein; nitrotyrosine; pararosaniline; Phenol Red; B-phycoerythrin; o-phthaldialdehyde; pyrene and derivatives(e.g., pyrene, pyrene butyrate, and succinimidyl 1-pyrene); butyrate quantum dots; Reactive Red 4 (CIBACRON^{™} Brilliant Red 3B-A); rhodamine and derivatives (e.g., 6-carboxy-X-rhodamine (ROX), 6-carboxyrhodamine (R6G), lissamine rhodamine B sulfonyl chloride rhodarnine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'tetramethyl-6-carboxyrhodamine (TAMRA) tetramethyl rhodamine, and tetramethyl rhodamine isothiocyanate (TRITC)); riboflavin; rosolic acid; terbium chelate derivatives; Cyanine-3 (Cy3); Cyanine-5 (Cy5); cyanine-5.5 (Cy5.5), Cyanine-7 (Cy7); IRD 700; IRD 800; Alexa 647; La Jolta Blue; phthalo cyanine; and naphthalo cyanine.

In some cases, the detectable agent may be a non-detectable precursor that becomes detectable upon activation (e.g., fluorogenic tetrazine-fluorophore constructs (e.g., tetrazine-BODIPY FL, tetrazine-Oregon Green 488, or tetrazine-BODIPY TMR-X) or enzyme activatable fluorogenic agents (e.g., PROSENSE^{®} (VisEn Medical))). In vitro assays in which the enzyme labeled compositions can be used include, but are not limited to, enzyme linked immunosorbent assays (ELISAs), immunoprecipitation assays, immunofluorescence, enzyme immunoassays (EIA), radioimmunoassays (RIA), and Western blot analysis.

### Combinations

The chimeric polynucleotides may be used in combination with one or more other therapeutic, prophylactic, diagnostic, or imaging agents. By "in combination with," it is not intended to imply that the agents must be administered at the same time and/or formulated for delivery together, although these methods of delivery are within the scope of the present disclosure. Compositions can be administered concurrently with, prior to, or subsequent to, one or more other desired therapeutics or medical procedures. In general, each agent will be administered at a dose and/or on a time schedule determined for that agent. In some cases, the present disclosure encompasses the delivery of pharmaceutical, prophylactic, diagnostic, or imaging compositions in combination with agents that may improve their bioavailability, reduce and/or modify their metabolism, inhibit their excretion, and/or modify their distribution within the body. As a non-limiting example, the chimeric polynucleotides may be used in combination with a pharmaceutical agent for the treatment of cancer or to control hyperproliferative cells. In U.S. Pat. No. 7,964,571, a combination therapy for the treatment of solid primary or metastasized tumor is described using a pharmaceutical composition including a DNA plasmid encoding for interleukin-12 with a lipopolymer and also administering at least one anticancer agent or chemotherapeutic. Further, the chimeric polynucleotides of the present disclosure that encodes anti-proliferative molecules may be in a pharmaceutical composition with a lipopolymer (see e.g., U.S. Pub. No. 20110218231,
claiming a pharmaceutical composition comprising a DNA plasmid encoding an anti-proliferative molecule and a lipopolymer) which may be administered with at least one chemotherapeutic or anticancer agent (See e.g., the "Combination" Section in US Patent No. 8,518,907 and International Patent Publication No. WO201218754).

The chimeric polynucleotides and pharmaceutical formulations thereof may be administered to a subject alone or used in combination with or include one or more other therapeutic agents, for example, anticancer agents. Thus, combinations of chimeric polynucleotides with other anti-cancer or chemotherapeutic agents are within the scope of the disclosure.

Examples of such agents can be found in Cancer Principles and Practice of Oncology by V. T. Devita and S. Hellman (editors), 6thedition (Feb. 15, 2001), Lippincott Williams & Wilkins Publishers. A person of ordinary skill in the art would be able to discern which combinations of agents would be useful based on the particular characteristics of the drugs and the cancer involved. Such anti-cancer agents include, but are not limited to, the following: estrogen receptor modulators, androgen receptor modulators, retinoid receptor modulators, cytotoxic/cytostatic agents, antiproliferative agents, prenyl-protein transferase inhibitors, HMG-CoA reductase inhibitors and other angiogenesis inhibitors, inhibitors of cell proliferation and survival signaling, apoptosis inducing agents and agents that interfere with cell cycle checkpoints. The chimeric polynucleotides may also be useful in combination with any therapeutic agent used in the treatment of HCC, for example, but not limitation sorafenib. Chimeric polynucleotides may be particularly useful when co-administered with radiation therapy.

In certain cases, the chimeric polynucleotides may be useful in combination with known anti-cancer agents including the following: estrogen receptor modulators, androgen receptor modulators, retinoid receptor modulators, cytotoxic agents, antiproliferative agents, prenyl-protein transferase inhibitors, HMG-CoA reductase inhibitors, HIV protease inhibitors, reverse transcriptase inhibitors, and other angiogenesis inhibitors.

Examples of estrogen receptor modulators, androgen receptor modulators, retinoid receptor modulators, cytotoxic agents, a hypoxia activatable, proteasome inhibitors, microtubule inhibitors/microtubule-stabilising agents, topoisomerase inhibitors, inhibitors of mitotic kinesins, histone deacetylase inhibitors, inhibitors of kinases involved in mitotic progression, antiproliferative agents, monoclonal antibody targeted therapeutic agents, HMG-CoA reductase inhibitors, prenyl-protein transferase inhibitors, angiogenesis inhibitors, therapeutic agents that modulate or inhibit angiogenesis, agents that interfere with cell cycle checkpoints, agents that interfere with receptor tyrosine kinases (RTKs), inhibitors of cell proliferation and survival signaling pathway, apoptosis inducing agents, NSAIDs that are selective COX-2 inhibitors, inhibitors of COX-2, compounds that have been described as specific inhibitors of COX-2, angiogenesis inhibitors, tyrosine kinase inhibitors, compounds other than anti-cancer compounds, inhibitor of inherent multidrug resistance (MDR), anti-emetic agents to treat nausea or emesis, and neurokinin-1 receptor antagonists, are described in co-pending International Patent Publication No. WO2015038892, such as, but not limited to, in pargraphs [000925] - [000957].

Another case of the disclosure is the use of the chimeric polynucleotides in combination with gene therapy for the treatment of cancer. For an overview of genetic strategies to treating cancer see Hall et al. (Am J Hum Genet 61:785-789 (1997)) and Kufe et al. (Cancer Medicine, 5th Ed, pp 876-889, BC Decker, Hamilton, 2000). Gene therapy can be used to deliver any tumor suppressing gene. Examples of such genes include, but are not limited to, p53, which can be delivered via recombinant virus-mediated gene transfer (see U.S. Pat. No. 6,069,134, for example), a uPA/uPAR antagonist ("Adenovirus-Mediated Delivery of a uPA/uPAR Antagonist Suppresses Angiogenesis-Dependent Tumor Growth and Dissemination in Mice," Gene Therapy, August 5(8):1105-13 (1998)), and interferon gamma (J Immunol 164:217-222 (2000)).

Chimeric polynucleotides may also be useful for treating or preventing cancer, including bone cancer, in combination with bisphosphonates (understood to include bisphosphonates, diphosphonates, bisphosphonic acids and diphosphonic acids). Examples of bisphosphonates include but are not limited to: etidronate (Didronel), pamidronate (Aredia), alendronate (Fosamax), risedronate (Actonel), zoledronate (Zometa), ibandronate (Boniva), incadronate or cimadronate, clodronate, EB-1053, minodronate, neridronate, piridronate and tiludronate including any and all pharmaceutically acceptable salts, derivatives, hydrates and mixtures thereof.

Chimeric polynucleotides may also be administered with an agent useful in the treatment of anemia. Such an anemia treatment agent is, for example, a continuous eythropoiesis receptor activator (such as epoetin alfa).

Chimeric polynucleotides may also be administered with an agent useful in the treatment of neutropenia. Such a neutropenia treatment agent is, for example, a hematopoietic growth factor which regulates the production and function of neutrophils such as a human granulocyte colony stimulating factor, (G-CSF). Examples of a G-CSF include filgrastim and PEG-filgrastim.

Chimeric polynucleotides may also be administered with an immunologic-enhancing drug, such as levamisole, isoprinosine and Zadaxin.

Chimeric polynucleotides may also be useful for treating or preventing breast cancer in combination with aromatase inhibitors. Examples of aromatase inhibitors include but are not limited to: anastrozole, letrozole and exemestane.

Chimeric polynucleotides may also be useful for treating or preventing cancer in combination with other nucleic acid therapeutics.

Chimeric polynucleotides may also be administered in combination with γ-secretase inhibitors and/or inhibitors of NOTCH signaling. Such inhibitors include compounds described in co-pending International Patent Publication No. WO2015038892, such as, but not limited to, in paragraph [000964].

Chimeric polynucleotides may also be useful for treating or preventing cancer in combination with PARP inhibitors.

Chimeric polynucleotides may also be useful for treating cancer in combination with the therapeutic agents described in co-pending International Patent Publication No. WO2015038892, such as, but not limited to, in paragraph [000966].

The combinations referred to above can conveniently be presented for use in the form of a pharmaceutical formulation and thus pharmaceutical compositions comprising a combination as defined above together with a pharmaceutically acceptable diluent or carrier represent a further aspect of the disclosure.

The individual compounds of such combinations can be administered either sequentially or simultaneously in separate or combined pharmaceutical formulations. In one case, the individual compounds will be administered simultaneously in a combined pharmaceutical formulation.

It will further be appreciated that therapeutically, prophylactically, diagnostically, or imaging active agents utilized in combination may be administered together in a single composition or administered separately in different compositions. In general, it is expected that agents utilized in combination with be utilized at levels that do not exceed the levels at which they are utilized individually. In some cases, the levels utilized in combination will be lower than those utilized individually. In one case, the combinations, each or together may be administered according to the split dosing regimens described herein.

### Dosing

The present disclosure describes methods comprising administering modified mRNAs and their encoded proteins or complexes in accordance with the disclosure to a subject in need thereof. Nucleic acids, proteins or complexes, or pharmaceutical, imaging, diagnostic, or prophylactic compositions thereof, may be administered to a subject using any amount and any route of administration effective for preventing, treating, diagnosing, or imaging a disease, disorder, and/or condition (*e.g.,* a disease, disorder, and/or condition relating to working memory deficits). The exact amount required will vary from subject to subject, depending on the species, age, and general condition of the subject, the severity of the disease, the particular composition, its mode of administration, its mode of activity, and the like. Compositions in accordance with the disclosure are typically formulated in dosage unit form for ease of administration and uniformity of dosage. It will be understood, however, that the total daily usage of the compositions may be decided by the attending physician within the scope of sound medical judgment. The specific therapeutically effective, prophylactically effective, or appropriate imaging dose level for any particular patient will depend upon a variety of factors including the disorder being treated and the severity of the disorder; the activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts.

In certain cases, compositions may be administered at dosage levels sufficient to deliver from about 0.0001 mg/kg to about 100 mg/kg, from about 0.001 mg/kg to about 0.05 mg/kg, from about 0.005 mg/kg to about 0.05 mg/kg, from about 0.001 mg/kg to about 0.005 mg/kg, from about 0.05 mg/kg to about 0.5 mg/kg, from about 0.01 mg/kg to about 50 mg/kg, from about 0.1 mg/kg to about 40 mg/kg, from about 0.5 mg/kg to about 30 mg/kg, from about 0.01 mg/kg to about 10 mg/kg, from about 0.1 mg/kg to about 10 mg/kg, or from about 1 mg/kg to about 25 mg/kg, of subject body weight per day, one or more times a day, to obtain the desired therapeutic, diagnostic, prophylactic, or imaging effect (see e.g., the range of unit doses described in International Publication No WO2013078199). The desired dosage may be delivered three times a day, two times a day, once a day, every other day, every third day, every week, every two weeks, every three weeks, or every four weeks. In certain cases, the desired dosage may be delivered using multiple administrations (*e*.*g*., two, three, four, five, six, seven, eight, nine, ten, eleven, twelve, thirteen, fourteen, or more administrations). When multiple administrations are employed, split dosing regimens such as those described herein may be used.

According to the present disclosure it has been discovered that administration of chimeric polynucleotides in split-dose regimens produce higher levels of proteins in mammalian subjects. As used herein, a "split dose" is the division of single unit dose or total daily dose into two or more doses, e.g., two or more administrations of the single unit dose. As used herein, a "single unit dose" is a dose of any therapeutic administered in one dose/at one time/single route/single point of contact, i.e., single administration event. As used herein, a "total daily dose" is an amount given or prescribed in 24 hr period. It may be administered as a single unit dose. In one case, the chimeric polynucleotides of the present disclosure are administered to a subject in split doses. The chimeric polynucleotides may be formulated in buffer only or in a formulation described herein.

### Dosage Forms

A pharmaceutical composition described herein can be formulated into a dosage form described herein, such as a topical, intranasal, intratracheal, or injectable (e.g., intravenous, intraocular, intravitreal, intramuscular, intracardiac, intraperitoneal, and subcutaneous).

### Liquid dosage forms

Liquid dosage forms for parenteral administration are described in co-pending International Patent Publication No. WO2015038892, such as, but not limited to, in paragraph [0001037].

### Injectable

Injectable preparations, for example, sterile injectable aqueous or oleaginous suspensions may be formulated according to the known art and may include suitable dispersing agents, wetting agents, and/or suspending agents. Sterile injectable preparations may be sterile injectable solutions, suspensions, and/or emulsions in nontoxic parenterally acceptable diluents and/or solvents, for example, a solution in 1,3-butanediol. Among the acceptable vehicles and solvents that may be employed include, but are not limited to, water, Ringer's solution, U.S.P., and isotonic sodium chloride solution. Sterile, fixed oils are conventionally employed as a solvent or suspending medium. For this purpose any bland fixed oil can be employed including synthetic mono- or diglycerides. Fatty acids such as oleic acid can be used in the preparation of injectables.

Injectable formulations can be sterilized, for example, by filtration through a bacterial-retaining filter, and/or by incorporating sterilizing agents in the form of sterile solid compositions which can be dissolved or dispersed in sterile water or other sterile injectable medium prior to use.

In order to prolong the effect of an active ingredient, it may be desirable to slow the absorption of the active ingredient from subcutaneous or intramuscular injection. This may be accomplished by the use of a liquid suspension of crystalline or amorphous material with poor water solubility. The rate of absorption of the chimeric polynucleotides then depends upon its rate of dissolution which, in turn, may depend upon crystal size and crystalline form. Alternatively, delayed absorption of a parenterally administered chimeric polynucleotides may be accomplished by dissolving or suspending the chimeric polynucleotides in an oil vehicle. Injectable depot forms are made by forming microencapsule matrices of the chimeric polynucleotides in biodegradable polymers such as polylactide-polyglycolide. Depending upon the ratio of chimeric polynucleotides to polymer and the nature of the particular polymer employed, the rate of chimeric polynucleotides release can be controlled. Examples of other biodegradable polymers include, but are not limited to, poly(orthoesters) and poly(anhydrides). Depot injectable formulations may be prepared by entrapping the chimeric polynucleotides in liposomes or microemulsions which are compatible with body tissues.

### Pulmonary

Pulmonary and intranasal formulations for delivery and administration are described in co-pending International Patent Publication No. WO2013151666, such as, but not limited to, in paragraphs [000766] - [000781].

### Coatings or Shells

Solid dosage forms of tablets, dragees, capsules, pills, and granules can be prepared with coatings and shells such as enteric coatings and other coatings well known in the pharmaceutical formulating art. They may optionally comprise opacifying agents and can be of a composition that they release the active ingredient(s) only, or preferentially, in a certain part of the intestinal tract, optionally, in a delayed manner. Examples of embedding compositions which can be used include polymeric substances and waxes. Solid compositions of a similar type may be employed as fillers in soft and hard-filled gelatin capsules using such excipients as lactose or milk sugar as well as high molecular weight polyethylene glycols and the like.

### Multi-dose and repeat-dose administration

In some cases, compounds and/or compositions may be administered in two or more doses (referred to herein as "multi-dose administration"). Such doses may comprise the same components or may comprise components not included in a previous dose. Such doses may comprise the same mass and/or volume of components or an altered mass and/or volume of components in comparison to a previous dose. In some cases, multi-dose administration may comprise repeat-dose administration. As used herein, the term "repeat-dose administration" refers to two or more doses administered consecutively or within a regimen of repeat doses comprising substantially the same components provided at substantially the same mass and/or volume. In some cases, subjects may display a repeat-dose response. As used herein, the term "repeat-dose response" refers to a response in a subject to a repeat-dose that differs from that of another dose administered within a repeat-dose administration regimen. In some cases, such a response may be the expression of a protein in response to a repeat-dose comprising mRNA. In such cases, protein expression may be elevated in comparison to another dose administered within a repeat-dose administration regimen or protein expression may be reduced in comparison to another dose administered within a repeat-dose administration regimen. Alteration of protein expression may be from about 1% to about 20%, from about 5% to about 50% from about 10% to about 60%, from about 25% to about 75%, from about 40% to about 100% and/or at least 100%. A reduction in expression of mRNA administered as part of a repeat-dose regimen, wherein the level of protein translated from the administered RNA is reduced by more than 40% in comparison to another dose within the repeat-dose regimen is referred to herein as "repeat-dose resistance."

### Properties of the Pharmaceutical Compositions

The pharmaceutical compositions described herein can be characterized by one or more of the following properties:

### Bioavailability

The chimeric polynucleotides, when formulated into a composition with a delivery agent as described herein, can exhibit an increase in bioavailability as compared to a composition lacking a delivery agent as described herein. As used herein, the term "bioavailability" refers to the systemic availability of a given amount of chimeric polynucleotides administered to a mammal. Bioavailability can be assessed by measuring the area under the curve (AUC) or the maximum serum or plasma concentration (Cₘₐₓ) of the unchanged form of a compound following administration of the compound to a mammal. AUC is a determination of the area under the curve plotting the serum or plasma concentration of a compound along the ordinate (Y-axis) against time along the abscissa (X-axis). Generally, the AUC for a particular compound can be calculated using methods known to those of ordinary skill in the art and as described in G. S. Banker, Modern Pharmaceutics, Drugs and the Pharmaceutical Sciences, v. 72, Marcel Dekker, New York, Inc., 1996.

The Cₘₐₓ value is the maximum concentration of the compound achieved in the serum or plasma of a mammal following administration of the compound to the mammal. The Cₘₐₓ value of a particular compound can be measured using methods known to those of ordinary skill in the art. The phrases "increasing bioavailability" or "improving the pharmacokinetics," as used herein mean that the systemic availability of a first chimeric polynucleotides, measured as AUC, Cₘₐₓ, or Cₘᵢₙ in a mammal is greater, when co-administered with a delivery agent as described herein, than when such co-administration does not take place. In some cases, the bioavailability of the chimeric polynucleotides can increase by at least about 2%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%.

In some cases, liquid formulations of chimeric polynucleotides may have varying in vivo half-life, requiring modulation of doses to yield a therapeutic effect. To address this, in some cases, chimeric polynucleotides formulations may be designed to improve bioavailability and/or therapeutic effect during repeat administrations. Such formulations may enable sustained release of chimeric polynucleotides and/or reduce chimeric polynucleotide degradation rates by nucleases. In some cases, suspension formulations are described comprising chimeric polynucleotides, water immiscible oil depots, surfactants and/or co-surfactants and/or co-solvents. Combinations of oils and surfactants may enable suspension formulation with chimeric polynucleotides. Delivery of chimeric polynucleotides in a water immiscible depot may be used to improve bioavailability through sustained release of chimeric polynucleotides from the depot to the surrounding physiologic environment and/or prevent chimeric polynucleotide degradation by nucleases.

In some cases, cationic nanoparticles comprising combinations of divalent and monovalent cations may be formulated with chimeric polynucleotides. Such nanoparticles may form spontaneously in solution over a given period (e.g. hours, days, etc.). Such nanoparticles do not form in the presence of divalent cations alone or in the presence of monovalent cations alone. The delivery of chimeric polynucleotides in cationic nanoparticles or in one or more depot comprising cationic nanoparticles may improve chimeric polynucleotide bioavailability by acting as a long-acting depot and/or reducing the rate of degradation by nucleases.

### Therapeutic Window

The chimeric polynucleotides, when formulated into a composition with a delivery agent as described herein, can exhibit an increase in the therapeutic window of the administered chimeric polynucleotides composition as compared to the therapeutic window of the administered chimeric polynucleotides composition lacking a delivery agent as described herein. As used herein "therapeutic window" refers to the range of plasma concentrations, or the range of levels of therapeutically active substance at the site of action, with a high probability of eliciting a therapeutic effect. In some cases, the therapeutic window of the chimeric polynucleotides when co-administered with a delivery agent as described herein can increase by at least about 2%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%, at least about 75%, at least about 80%, at least about 85%, at least about 90%, at least about 95%, or about 100%.

### Volume of Distribution

The chimeric polynucleotides, when formulated into a composition with a delivery agent as described herein, can exhibit an improved volume of distribution (V_{dist}), e.g., reduced or targeted, relative to a composition lacking a delivery agent as described herein. The volume of distribution (Vdist) relates the amount of the drug in the body to the concentration of the drug in the blood or plasma. As used herein, the term "volume of distribution" refers to the fluid volume that would be required to contain the total amount of the drug in the body at the same concentration as in the blood or plasma: Vdist equals the amount of drug in the body/concentration of drug in blood or plasma. For example, for a 10 mg dose and a plasma concentration of 10 mg/L, the volume of distribution would be 1 liter. The volume of distribution reflects the extent to which the drug is present in the extravascular tissue. A large volume of distribution reflects the tendency of a compound to bind to the tissue components compared with plasma protein binding. In a clinical setting, Vdist can be used to determine a loading dose to achieve a steady state concentration. In some cases, the volume of distribution of the chimeric polynucleotides when co-administered with a delivery agent as described herein can decrease at least about 2%, at least about 5%, at least about 10%, at least about 15%, at least about 20%, at least about 25%, at least about 30%, at least about 35%, at least about 40%, at least about 45%, at least about 50%, at least about 55%, at least about 60%, at least about 65%, at least about 70%.

### Biological Effect

In one case, the biological effect of the modified mRNA delivered to the animals may be categorized by analyzing the protein expression in the animals. The protein expression may be determined from analyzing a biological sample collected from a mammal administered the modified mRNA of the present disclosure. In one case, the expression protein encoded by the modified mRNA administered to the mammal of at least 50 pg/ml may be preferred. For example, a protein expression of 50-200 pg/ml for the protein encoded by the modified mRNA delivered to the mammal may be seen as a therapeutically effective amount of protein in the mammal.

### Detection of Chimeric Polynucleotides Acids by Mass Spectrometry

Mass spectrometry (MS) is an analytical technique that can provide structural and molecular mass/concentration information on molecules after their conversion to ions. The molecules are first ionized to acquire positive or negative charges and then they travel through the mass analyzer to arrive at different areas of the detector according to their mass/charge (m/z) ratio. Methods of detecting polynucleotides are described in co-pending International Patent Publication No. WO2015038892, such as, but not limited to, in paragraphs [0001055] - [0001067].

### V. Uses of Chimeric polynucleotides of the Disclosure

The chimeric polynucleotides of the present disclosure are designed, in preferred cases, to provide for avoidance or evasion of deleterious bio-responses such as the immune response and/or degradation pathways, overcoming the threshold of expression and/or improving protein production capacity, improved expression rates or translation efficiency, improved drug or protein half-life and/or protein concentrations, optimized protein localization, to improve one or more of the stability and/or clearance in tissues, receptor uptake and/or kinetics, cellular access by the compositions, engagement with translational machinery, secretion efficiency (when applicable), accessibility to circulation, and/or modulation of a cell's status, function and/or activity.

### Therapeutics

### Therapeutic Agents

The chimeric polynucleotides of the present disclosure, such as modified nucleic acids and modified RNAs, and the proteins translated from them described herein can be used as therapeutic or prophylactic agents. They are disclosed for use in medicine. For example, a chimeric polynucleotide described herein can be administered to a subject, wherein the chimeric polynucleotides is translated *in vivo* to produce a therapeutic or prophylactic polypeptide in the subject. Disclosed are compositions, methods, kits, and reagents for diagnosis, treatment or prevention of a disease or condition in humans and other mammals. The active therapeutic agents of the disclosure include chimeric polynucleotides, cells containing chimeric polynucleotides or polypeptides translated from the chimeric polynucleotides.

In certain cases, described herein are combination therapeutics containing one or more chimeric polynucleotides containing translatable regions that encode for a protein or proteins that boost a mammalian subject's immunity along with a protein that induces antibody-dependent cellular toxicity. For example, described herein are therapeutics containing one or more nucleic acids that encode trastuzumab and granulocyte-colony stimulating factor (G-CSF). In particular, such combination therapeutics are useful in Her2+ breast cancer patients who develop induced resistance to trastuzumab. (See, e.g., Albrecht, Immunotherapy. 2(6):795-8 (2010)).

Disclosed herein are methods of inducing translation of a recombinant polypeptide in a cell population using the chimeric polynucleotides described herein. Such translation can be *in vivo, ex vivo, in culture,* or *in vitro.* The cell population is contacted with an effective amount of a composition containing a nucleic acid that has at least one nucleoside modification, and a translatable region encoding the recombinant polypeptide. The population is contacted under conditions such that the nucleic acid is localized into one or more cells of the cell population and the recombinant polypeptide is translated in the cell from the nucleic acid.

An "effective amount" of the composition is described based, at least in part, on the target tissue, target cell type, means of administration, physical characteristics of the nucleic acid (e.g., size, and extent of modified nucleosides), and other determinants. In general, an effective amount of the composition provides efficient protein production in the cell, preferably more efficient than a composition containing a corresponding unmodified nucleic acid. Increased efficiency may be demonstrated by increased cell transfection (i.e., the percentage of cells transfected with the nucleic acid), increased protein translation from the nucleic acid, decreased nucleic acid degradation (as demonstrated, e.g., by increased duration of protein translation from a modified nucleic acid), or reduced innate immune response of the host cell.

Some cases of the disclosure described herein are methods of inducing *in vivo* translation of a recombinant polypeptide in a mammalian subject in need thereof. Therein, an effective amount of a composition containing a nucleic acid that has at least one structural or chemical modification and a translatable region encoding the recombinant polypeptide is administered to the subject using the delivery methods described herein. The nucleic acid is provided in an amount and under other conditions such that the nucleic acid is localized into a cell of the subject and the recombinant polypeptide is translated in the cell from the nucleic acid. The cell in which the nucleic acid is localized, or the tissue in which the cell is present, may be targeted with one or more than one rounds of nucleic acid administration.

In certain cases, the administered chimeric polynucleotides directs production of one or more recombinant polypeptides that provide a functional activity which is substantially absent in the cell, tissue or organism in which the recombinant polypeptide is translated. For example, the missing functional activity may be enzymatic, structural, or gene regulatory in nature. In related cases, the administered chimeric polynucleotides directs production of one or more recombinant polypeptides that increases (e.g., synergistically) a functional activity which is present but substantially deficient in the cell in which the recombinant polypeptide is translated.

In other cases, the administered chimeric polynucleotides directs production of one or more recombinant polypeptides that replace a polypeptide (or multiple polypeptides) that is substantially absent in the cell in which the recombinant polypeptide is translated. Such absence may be due to genetic mutation of the encoding gene or regulatory pathway thereof. In some cases, the recombinant polypeptide increases the level of an endogenous protein in the cell to a desirable level; such an increase may bring the level of the endogenous protein from a subnormal level to a normal level or from a normal level to a super-normal level.

Alternatively, the recombinant polypeptide functions to antagonize the activity of an endogenous protein present in, on the surface of, or secreted from the cell. Usually, the activity of the endogenous protein is deleterious to the subject; for example, due to mutation of the endogenous protein resulting in altered activity or localization. Additionally, the recombinant polypeptide antagonizes, directly or indirectly, the activity of a biological moiety present in, on the surface of, or secreted from the cell. Examples of antagonized biological moieties include lipids (e.g., cholesterol), a lipoprotein (e.g., low density lipoprotein), a nucleic acid, a carbohydrate, a protein toxin such as shiga and tetanus toxins, or a small molecule toxin such as botulinum, cholera, and diphtheria toxins. Additionally, the antagonized biological molecule may be an endogenous protein that exhibits an undesirable activity, such as a cytotoxic or cytostatic activity.

The recombinant proteins described herein may be engineered for localization within the cell, potentially within a specific compartment such as the nucleus, or are engineered for secretion from the cell or translocation to the plasma membrane of the cell.

In some cases, modified mRNAs and their encoded polypeptides may be used for treatment of any of a variety of diseases, disorders, and/or conditions, including but not limited to one or more of the following: autoimmune disorders (*e*.*g*. diabetes, lupus, multiple sclerosis, psoriasis, rheumatoid arthritis); inflammatory disorders (*e*.*g*. arthritis, pelvic inflammatory disease); infectious diseases (*e.g.* viral infections (*e.g.,* HIV, HCV, RSV), bacterial infections, fungal infections, sepsis); neurological disorders (*e*.*g*. Alzheimer's disease, Huntington's disease; autism; Duchenne muscular dystrophy); cardiovascular disorders (*e*.*g*. atherosclerosis, hypercholesterolemia, thrombosis, clotting disorders, angiogenic disorders such as macular degeneration); proliferative disorders (*e.g.* cancer, benign neoplasms); respiratory disorders (*e.g.* chronic obstructive pulmonary disease); digestive disorders (*e*.*g*. inflammatory bowel disease, ulcers); musculoskeletal disorders (*e*.*g*. fibromyalgia, arthritis); endocrine, metabolic, and nutritional disorders (*e.g.* diabetes, osteoporosis); urological disorders (*e.g.* renal disease); psychological disorders (*e.g.* depression, schizophrenia); skin disorders (*e.g.* wounds, eczema); blood and lymphatic disorders (*e.g.* anemia, hemophilia); *etc.*

Diseases characterized by dysfunctional or aberrant protein activity include cystic fibrosis, sickle cell anemia, epidermolysis bullosa, amyotrophic lateral sclerosis, and glucose-6-phosphate dehydrogenase deficiency. Described herein is a method for treating such conditions or diseases in a subject by introducing nucleic acid or cell-based therapeutics containing the chimeric polynucleotides disclosed herein, wherein the chimeric polynucleotides encode for a protein that antagonizes or otherwise overcomes the aberrant protein activity present in the cell of the subject. Specific examples of a dysfunctional protein are the missense mutation variants of the cystic fibrosis transmembrane conductance regulator (CFTR) gene, which produce a dysfunctional protein variant of CFTR protein, which causes cystic fibrosis.

Diseases characterized by missing (or substantially diminished such that proper (normal or physiological protein function does not occur) protein activity include cystic fibrosis, Niemann-Pick type C, β thalassemia major, Duchenne muscular dystrophy, Hurler Syndrome, Hunter Syndrome, and Hemophilia A. Such proteins may not be present, or are essentially non-functional. Described herein is a method for treating such conditions or diseases in a subject by introducing nucleic acid or cell-based therapeutics containing the chimeric polynucleotides disclosed herein, wherein the chimeric polynucleotides encode for a protein that replaces the protein activity missing from the target cells of the subject. Specific examples of a dysfunctional protein are the nonsense mutation variants of the cystic fibrosis transmembrane conductance regulator (CFTR) gene, which produce a nonfunctional protein variant of CFTR protein, which causes cystic fibrosis.

Thus, disclosed are methods of treating cystic fibrosis in a mammalian subject by contacting a cell of the subject with a chimeric polynucleotide having a translatable region that encodes a functional CFTR polypeptide, under conditions such that an effective amount of the CTFR polypeptide is present in the cell. Preferred target cells are epithelial, endothelial and mesothelial cells, such as the lung, and methods of administration are determined in view of the target tissue; i.e., for lung delivery, the RNA molecules are formulated for administration by inhalation.

In another case, described herein is a method for treating hyperlipidemia in a subject, by introducing into a cell population of the subject with a modified mRNA molecule encoding Sortilin, a protein recently characterized by genomic studies, thereby ameliorating the hyperlipidemia in a subject. The *SORT1* gene encodes a trans-Golgi network (TGN) transmembrane protein called Sortilin. Genetic studies have shown that one of five individuals has a single nucleotide polymorphism, rs12740374, in the 1p13 locus of the SORT1 gene that predisposes them to having low levels of low-density lipoprotein (LDL) and very-low-density lipoprotein (VLDL). Each copy of the minor allele, present in about 30% of people, alters LDL cholesterol by 8 mg/dL, while two copies of the minor allele, present in about 5% of the population, lowers LDL cholesterol 16 mg/dL. Carriers of the minor allele have also been shown to have a 40% decreased risk of myocardial infarction. Functional *in vivo* studies in mice describes that overexpression of *SORT1* in mouse liver tissue led to significantly lower LDL-cholesterol levels, as much as 80% lower, and that silencing SORT1 increased LDL cholesterol approximately 200% (Musunuru K et al. From noncoding variant to phenotype via SORT1 at the 1p13 cholesterol locus. Nature 2010; 466: 714-721).

In another case, described herein is a method for treating hematopoietic disorders, cardiovascular disease, oncology, diabetes, cystic fibrosis, neurological diseases, inborn errors of metabolism, skin and systemic disorders, and blindness. The identity of molecular targets to treat these specific diseases has been described (Templeton ed., Gene and Cell Therapy: Therapeutic Mechanisms and Strategies, 3rd Edition, Bota Raton, FL:CRC Press).

Described herein, are methods to prevent infection and/or sepsis in a subject at risk of developing infection and/or sepsis, the method comprising administering to a subject in need of such prevention a composition comprising a chimeric polynucleotide precursor encoding an anti-microbial polypeptide (e.g., an anti-bacterial polypeptide), or a partially or fully processed form thereof in an amount sufficient to prevent infection and/or sepsis. In certain cases, the subject at risk of developing infection and/or sepsis may be a cancer patient. In certain cases, the cancer patient may have undergone a conditioning regimen. In some cases, the conditioning regiment may include, but is not limited to, chemotherapy, radiation therapy, or both. As a non-limiting example, a chimeric polynucleotide can encode Protein C, its zymogen or prepro-protein, the activated form of Protein C (APC) or variants of Protein C which are known in the art. The chimeric polynucleotides may be chemically modified and delivered to cells. Non-limiting examples of polypeptides which may be encoded within the chemically modified mRNAs of the present disclosure include those taught in US Patents 7,226,999; 7,498,305; 6,630,138 These patents teach Protein C like molecules, variants and derivatives, any of which may be encoded within the chemically modified molecules of the present disclosure.

Further disclosed herein, are methods to treat infection and/or sepsis in a subject, the method comprising administering to a subject in need of such treatment a composition comprising a chimeric polynucleotide precursor encoding an anti-microbial polypeptide (*e.g.,* an anti-bacterial polypeptide), *e.g.,* an anti-microbial polypeptide described herein, or a partially or fully processed form thereof in an amount sufficient to treat an infection and/or sepsis. In certain cases, the subject in need of treatment is a cancer patient. In certain cases, the cancer patient has undergone a conditioning regimen. In some cases, the conditioning regiment may include, but is not limited to, chemotherapy, radiation therapy, or both.

In certain cases, the subject may exhibits acute or chronic microbial infections (*e*.*g*., bacterial infections). In certain cases, the subject may have received or may be receiving a therapy. In certain cases, the therapy may include, but is not limited to, radiotherapy, chemotherapy, steroids, ultraviolet radiation, or a combination thereof. In certain cases, the patient may suffer from a microvascular disorder. In some cases, the microvascular disorder may be diabetes. In certain cases, the patient may have a wound. In some cases, the wound may be an ulcer. In a specific case, the wound may be a diabetic foot ulcer. In certain cases, the subject may have one or more burn wounds. In certain cases, the administration may be local or systemic. In certain cases, the administration may be subcutaneous. In certain cases, the administration may be intravenous. In certain cases,

the administration may be oral. In certain cases, the administration may be topical. In certain cases, the administration may be by inhalation. In certain cases, the administration may be rectal. In certain cases, the administration may be vaginal.

Other aspects of the present disclosure relate to transplantation of cells containing chimeric polynucleotides to a mammalian subject. Administration of cells to mammalian subjects is known to those of ordinary skill in the art, and include, but is not limited to, local implantation (e.g., topical or subcutaneous administration), organ delivery or systemic injection (e.g., intravenous injection or inhalation), and the formulation of cells in pharmaceutically acceptable carrier. Such compositions containing chimeric polynucleotides can be formulated for administration intramuscularly, transarterially, intraperitoneally, intravenously, intranasally, subcutaneously, endoscopically, transdermally, or intrathecally. In some cases, the composition may be formulated for extended release.

The subject to whom the therapeutic agent may be administered suffers from or may be at risk of developing a disease, disorder, or deleterious condition. Described herein are methods of identifying, diagnosing, and classifying subjects on these bases, which may include clinical diagnosis, biomarker levels, genome-wide association studies (GWAS), and other methods known in the art.

### Wound Management

The chimeric polynucleotides may be used for wound treatment, *e*.*g*. of wounds exhibiting delayed healing. Disclosed herein are methods comprising the administration of chimeric polynucleotides in order to manage the treatment of wounds are described in co-pending International Patent Publication No. WO2015038892, such as, but not limited to, in paragraphs [0001089] - [0001092].

### Production of Antibodies

In one case, the chimeric polynucleotides may encode antibodies and fragments of such antibodies such as those described in co-pending International Patent Publication No. WO2015038892, such as, but not limited to, in paragraphs [0001093] - [0001095].

### Managing Infection

In one case, described herein are methods for treating or preventing a microbial infection (*e*.*g*., a bacterial infection) and/or a disease, disorder, or condition associated with a microbial or viral infection, or a symptom thereof, in a subject, by administering a chimeric polynucleotide encoding an anti-microbial polypeptide. The administration may be in combination with an anti-microbial agent (*e*.*g*., an anti-bacterial agent), *e*.*g*., an anti-microbial polypeptide or a small molecule anti-microbial compound described herein. The anti-microbial agents include, but are not limited to, anti-bacterial agents, anti-viral agents, anti-fungal agents, anti-protozoal agents, antiparasitic agents, and anti-prion agents as well as compositions, delivery and methods of use of the polynucleotides herein are described in co-pending International Patent Publication No. WO2015038892, such as, but not limited to, in paragraphs [0001096] - [0001116].

### Modulation of the Immune Response

### Avoidance of the immune response

As described herein, a useful feature of the chimeric polynucleotides of the disclosure is the capacity to reduce, evade or avoid the innate immune response of a cell. Also described herein are chimeric polynucleotides encoding a polypeptide of interest which when delivered to cells, results in a reduced immune response from the host as compared to the response triggered by a reference compound, e.g. an unmodified polynucleotide corresponding to a chimeric polynucleotide of the disclosure, or a different chimeric polynucleotides of the disclosure. As used herein, a "reference compound" is any molecule or substance which when administered to a mammal, results in an innate immune response having a known degree, level or amount of immune stimulation. A reference compound need not be a nucleic acid molecule and it need not be any of the chimeric polynucleotides of the disclosure. Hence, the measure of a chimeric polynucleotides avoidance, evasion or failure to trigger an immune response can be expressed in terms relative to any compound or substance which is known to trigger such a response.

The term "innate immune response" includes a cellular response to exogenous single stranded nucleic acids, generally of viral or bacterial origin, which involves the induction of cytokine expression and release, particularly the interferons, and cell death. As used herein, the innate immune response or interferon response operates at the single cell level causing cytokine expression, cytokine release, global inhibition of protein synthesis, global destruction of cellular RNA, upregulation of major histocompatibility molecules, and/or induction of apoptotic death, induction of gene transcription of genes involved in apoptosis, anti-growth, and innate and adaptive immune cell activation. Some of the genes induced by type I IFNs include PKR, ADAR (adenosine deaminase acting on RNA), OAS (2',5'-oligoadenylate synthetase), RNase L, and Mx proteins. PKR and ADAR lead to inhibition of translation initiation and RNA editing, respectively. OAS is a dsRNA-dependent synthetase that activates the endoribonuclease RNase L to degrade ssRNA.

In some cases, the innate immune response comprises expression of a Type I or Type II interferon, and the expression of the Type I or Type II interferon is not increased more than two-fold compared to a reference from a cell which has not been contacted with a chimeric polynucleotide of the disclosure.

In some cases, the innate immune response comprises expression of one or more IFN signature genes and where the expression of the one of more IFN signature genes is not increased more than three-fold compared to a reference from a cell which has not been contacted with the chimeric polynucleotides of the disclosure.

While in some circumstances, it might be advantageous to eliminate the innate immune response in a cell, described herein are chimeric that upon administration result in a substantially reduced (significantly less) the immune response, including interferon signaling, without entirely eliminating such a response.

In some cases, the immune response is lower by 10%, 20%, 30%, 40%, 50%, 60%, 70%, 80%, 90%, 95%, 99%, 99.9%, or greater than 99.9% as compared to the immune response induced by a reference compound. The immune response itself may be measured by determining the expression or activity level of Type 1 interferons or the expression of interferon-regulated genes such as the toll-like receptors (e.g., TLR7 and TLR8). Reduction of innate immune response can also be measured by measuring the level of decreased cell death following one or more administrations to a cell population; e.g., cell death is 10%, 25%, 50%, 75%, 85%, 90%, 95%, or over 95% less than the cell death frequency observed with a reference compound. Moreover, cell death may affect fewer than 50%, 40%, 30%, 20%, 10%, 5%, 1%, 0.1%, 0.01% or fewer than 0.01% of cells contacted with the chimeric polynucleotides.

In another case, the chimeric polynucleotides of the present disclosure is significantly less immunogenic than an unmodified in vitro-synthesized chimeric polynucleotide with the same sequence or a reference compound. As used herein, "significantly less immunogenic" refers to a detectable decrease in immunogenicity. In another case, the term refers to a fold decrease in immunogenicity. In another case, the term refers to a decrease such that an effective amount of the chimeric polynucleotides can be administered without triggering a detectable immune response. In another case, the term refers to a decrease such that the chimeric polynucleotides can be repeatedly administered without eliciting an immune response sufficient to detectably reduce expression of the recombinant protein. In another case, the decrease is such that the chimeric polynucleotides can be repeatedly administered without eliciting an immune response sufficient to eliminate detectable expression of the recombinant protein.

In another case, the chimeric polynucleotides is 2-fold less immunogenic than its unmodified counterpart or reference compound. In another case, immunogenicity is reduced by a 3-fold factor. In another case, immunogenicity is reduced by a 5-fold factor. In another case, immunogenicity is reduced by a 7-fold factor. In another case, immunogenicity is reduced by a 10-fold factor. In another case, immunogenicity is reduced by a 15-fold factor. In another case, immunogenicity is reduced by a fold factor. In another case, immunogenicity is reduced by a 50-fold factor. In another case, immunogenicity is reduced by a 100-fold factor. In another case, immunogenicity is reduced by a 200-fold factor. In another immunogenicity is reduced by a 500-fold factor. In another case, immunogenicity is reduced by a 1000-fold factor. In another case, immunogenicity is reduced by a 2000-fold factor. In another case, immunogenicity is reduced by another fold difference.

Methods of determining immunogenicity are well known in the art, and include, e.g. measuring secretion of cytokines (e.g. IL-12, IFNalpha, TNF-alpha, RANTES, MIP-1alpha or beta, IL-6, IFN-beta, or IL-8), measuring expression of DC activation markers (e.g. CD83, HLA-DR, CD80 and CD86), or measuring ability to act as an adjuvant for an adaptive immune response.

The chimeric polynucleotides, including the combination of modifications taught herein may have superior properties making them more suitable as therapeutic modalities.

It has been determined that the "all or none" model in the art is sorely insufficient to describe the biological phenomena associated with the therapeutic utility of modified mRNA. The present inventors have determined that to improve protein production, one may consider the nature of the modification, or combination of modifications, the percent modification and survey more than one cytokine or metric to determine the efficacy and risk profile of a particular modified mRNA.

In one case, methods of determining the effectiveness of a modified mRNA as compared to unmodified involves the measure and analysis of one or more cytokines whose expression is triggered by the administration of the exogenous nucleic acid of the disclosure. These values are compared to administration of an unmodified nucleic acid or to a standard metric such as cytokine response, PolyIC, R-848 or other standard known in the art.

One example of a standard metric developed herein is the measure of the ratio of the level or amount of encoded polypeptide (protein) produced in the cell, tissue or organism to the level or amount of one or more (or a panel) of cytokines whose expression is triggered in the cell, tissue or organism as a result of administration or contact with the modified nucleic acid. Such ratios are referred to herein as the Protein:Cytokine Ratio or "PC" Ratio. The higher the PC ratio, the more efficacious the modified nucleic acid (polynucleotide encoding the protein measured). Preferred PC Ratios, by cytokine, of the present disclosure may be greater than 1, greater than 10, greater than 100, greater than 1000, greater than 10,000 or more. Modified nucleic acids having higher PC Ratios than a modified nucleic acid of a different or unmodified construct are preferred.

The PC ratio may be further qualified by the percent modification present in the polynucleotide. For example, normalized to a 100% modified nucleic acid, the protein production as a function of cytokine (or risk) or cytokine profile can be determined.

In one case, described herein is a method for determining, across chemistries, cytokines or percent modification, the relative efficacy of any particular modified the chimeric polynucleotides by comparing the PC Ratio of the modified nucleic acid (chimeric polynucleotides).

Chimeric polynucleotides containing varying levels of nucleobase substitutions could be produced that maintain increased protein production and decreased immunostimulatory potential. The relative percentage of any modified nucleotide to its naturally occurring nucleotide counterpart can be varied during the IVT reaction (for instance, 100, 50, 25, 10, 5, 2.5, 1, 0.1, 0.01% 5 methyl cytidine usage versus cytidine; 100, 50, 25, 10, 5, 2.5, 1, 0.1, 0.01% pseudouridine or N1-methyl-pseudouridine usage versus uridine). Chimeric polynucleotides can also be made that utilize different ratios using 2 or more different nucleotides to the same base (for instance, different ratios of pseudouridine and N1-methyl-pseudouridine). Chimeric polynucleotides can also be made with mixed ratios at more than 1 "base" position, such as ratios of 5 methyl cytidine/cytidine and pseudouridine/N1-methyl-pseudouridine/uridine at the same time. Use of modified mRNA with altered ratios of modified nucleotides can be beneficial in reducing potential exposure to chemically modified nucleotides. Lastly, positional introduction of modified nucleotides into the chimeric polynucleotides which modulate either protein production or immunostimulatory potential or both is also possible. The ability of such chimeric polynucleotides to demonstrate these improved properties can be assessed in vitro (using assays such as the PBMC assay described herein), and can also be assessed in vivo through measurement of both chimeric polynucleotides-encoded protein production and mediators of innate immune recognition such as cytokines.

In another case, the relative immunogenicity of the chimeric polynucleotides and its unmodified counterpart are determined by determining the quantity of the chimeric polynucleotides required to elicit one of the above responses to the same degree as a given quantity of the unmodified nucleotide or reference compound. For example, if twice as much chimeric polynucleotides is required to elicit the same response, than the chimeric polynucleotides is two-fold less immunogenic than the unmodified nucleotide or the reference compound.

In another case, the relative immunogenicity of the chimeric polynucleotides and its unmodified counterpart are determined by determining the quantity of cytokine (e.g. IL-12, IFNalpha, TNF-alpha, RANTES, MIP-1alpha or beta, IL-6, IFN-beta, or IL-8) secreted in response to administration of the chimeric polynucleotides, relative to the same quantity of the unmodified nucleotide or reference compound. For example, if one-half as much cytokine is secreted, than the chimeric polynucleotides is two-fold less immunogenic than the unmodified nucleotide. In another case, background levels of stimulation are subtracted before calculating the immunogenicity in the above methods.

Described herein are also methods for performing the titration, reduction or elimination of the immune response in a cell or a population of cells. In some cases, the cell is contacted with varied doses of the same chimeric polynucleotides and dose response is evaluated. In some cases, a cell is contacted with a number of different chimeric polynucleotides at the same or different doses to determine the optimal composition for producing the desired effect. Regarding the immune response, the desired effect may be to avoid, evade or reduce the immune response of the cell. The desired effect may also be to alter the efficiency of protein production.

The chimeric polynucleotides may be used to reduce the immune response using the method described in International Publication No. WO2013003475

### Activation of the immune response: Vaccines

The chimeric polynucleotides disclosed herein, may encode one or more vaccines. As used herein, a "vaccine" is a biological preparation that improves immunity to a particular disease or infectious agent. A vaccine introduces an antigen into the tissues or cells of a subject and elicits an immune response, thereby protecting the subject from a particular disease or pathogen infection. The chimeric polynucleotides may encode an antigen and when the chimeric polynucleotides are expressed in cells, a desired immune response is achieved.

The use of RNA as a vaccine overcomes the disadvantages of conventional genetic vaccination involving incorporating DNA into cells in terms of safeness, feasibility, applicability, and effectiveness to generate immune responses. RNA molecules are considered to be significantly safer than DNA vaccines, as RNAs are more easily degraded. They are cleared quickly out of the organism and cannot integrate into the genome and influence the cell's gene expression in an uncontrollable manner. It is also less likely for RNA vaccines to cause severe side effects like the generation of autoimmune disease or anti-DNA antibodies (Bringmann A. et al., Journal of Biomedicine and Biotechnology (2010), vol. 2010, article ID623687). Transfection with RNA requires only insertion into the cell's cytoplasm, which is easier to achieve than into the nucleus. However, RNA is susceptible to RNase degradation and other natural decomposition in the cytoplasm of cells. Various attempts to increase the stability and shelf life of RNA vaccines. US 2005/0032730 to Von Der Mulbe et al. discloses improving the stability of mRNA vaccine compositions by increasing G(guanosine)/C(cytosine) content of the mRNA molecules. US 5580859 to Felgner et al. teaches incorporating polynucleotide sequences coding for regulatory proteins that binds to and regulates the stabilities of mRNA. While not wishing to be bound by theory, it is believed that the chimeric polynucleotides vaccines of the disclosure will result in improved stability and therapeutic efficacy due at least in part to the specificity, purity and selectivity of the construct designs.

Additionally, certain modified nucleosides, or combinations thereof, when introduced into the chimeric polynucleotides of the disclosure will activate the innate immune response. Such activating molecules are useful as adjuvants when combined with polypeptides and/or other vaccines. In certain cases, the activating molecules contain a translatable region which encodes for a polypeptide sequence useful as a vaccine, thus providing the ability to be a self-adjuvant.

In one case, the chimeric polynucleotides may be used in the prevention, treatment and diagnosis of diseases and physical disturbances caused by antigens or infectious agents. The chimeric polynucleotide of may encode at least one polypeptide of interest (e.g. antibody or antigen) and may be provided to an individual in order to stimulate the immune system to protect against the disease-causing agents. As a non-limiting example, the biological activity and/or effect from an antigen or infectious agent may be inhibited and/or abolished by providing one or more chimeric polynucleotides which have the ability to bind and neutralize the antigen and/or infectious agent.

In one case, the chimeric polynucleotides may encode an immunogen. The delivery of the chimeric polynucleotides encoding an immunogen may activate the immune response. As a non-limiting example, the chimeric polynucleotides encoding an immunogen may be delivered to cells to trigger multiple innate response pathways (see International Pub. No. WO2012006377 and US Patent Publication No. US20130177639). As another non-limiting example, the chimeric polynucleotides encoding an immunogen may be delivered to a vertebrate in a dose amount large enough to be immunogenic to the vertebrate (see International Pub. No. WO2012006372 and WO2012006369 and US Publication No. US20130149375 and US20130177640). A non-limiting list of infectious disease that the chimeric polynucleotide vaccines may treat includes, viral infectious diseases such as AIDS (HIV), hepatitis A, B or C, herpes, herpes zoster (chicken pox), German measles (rubella virus), yellow fever, dengue fever etc. (flavi viruses), flu (influenza viruses), haemorrhagic infectious diseases (Marburg or Ebola viruses), bacterial infectious diseases such as Legionnaires' disease (Legionella), gastric ulcer (Helicobacter), cholera (Vibrio), E. coli infections, staphylococcal infections, salmonella infections or streptococcal infections, tetanus (Clostridium tetani), or protozoan infectious diseases (malaria, sleeping sickness, leishmaniasis, toxoplasmosis, i.e. infections caused by plasmodium, trypanosomes, leishmania and toxoplasma).

In one case, the chimeric polynucleotides may encode a tumor antigen to treat cancer. A non-limiting list of tumor antigens includes, 707-AP, AFP, ART-4, BAGE, .beta.-catenin/m, Bcr-abl, CAMEL, CAP-1, CASP-8, CDC27/m, CDK4/m, CEA, CT, Cyp-B, DAM, ELF2M, ETV6-AML1, G250, GAGE, GnT-V, Gp100, HAGE, HER-2/neu, HLA-A^{∗}0201-R170I, HPV-E7, HSP70-2M, HAST-2, hTERT (or hTRT), iCE, KIAA0205, LAGE, LDLR/FUT, MAGE, MART-1/melan-A, MC1R, myosin/m, MUC1, MUM-1, -2, -3, NA88-A, NY-ESO-1, p190 minor bcr-abl, Pml/RAR.alpha., PRAME, PSA, PSM, RAGE, RU1 or RU2, SAGE, SART-1 or SART-3, TEUAML1, TPI/m, TRP-1, TRP-2, TRP-2/INT2 and WT1.

The chimeric polynucleotides may encode a polypeptide sequence for a vaccine and may further comprise an inhibitor. The inhibitor may impair antigen presentation and/or inhibit various pathways known in the art. As a non-limiting example, the chimeric polynucleotides may be used for a vaccine in combination with an inhibitor which can impair antigen presentation (see International Pub. No. WO2012089225 and WO2012089338).

In one case, the chimeric polynucleotides may be self-replicating RNA. Self-replicating RNA molecules can enhance efficiency of RNA delivery and expression of the enclosed gene product. In one case, the chimeric polynucleotides may comprise at least one modification described herein and/or known in the art. In one case, the self-replicating RNA can be designed so that the self-replicating RNA does not induce production of infectious viral particles. As a non-limiting example the self-replicating RNA may be designed by the methods described in US Pub. No. US20110300205 and International Pub. No. WO2011005799 and WO2013055905.

In one case, the self-replicating chimeric polynucleotides may encode a protein which may raise the immune response. As a non-limiting example, the chimeric polynucleotides may be self-replicating mRNA may encode at least one antigen (see US Pub. No. US20110300205, US20130171241, US20130177640 and US20130177639 and International Pub. Nos. WO2011005799, WO2012006372, WO2012006377, WO2013006838, WO2013006842, WO2012006369 and WO2013055905). In one case, the self-replicating RNA may be administered to mammals at a large enough dose to raise the immune response in a large mammal (see e.g., International Publication No. WO2012006369).

In one case, the self-replicating chimeric polynucleotides may be formulated using methods described herein or known in the art. As a non-limiting example, the self-replicating RNA may be formulated for delivery by the methods described in Geall et al (Nonviral delivery of self-amplifying RNA vaccines, PNAS 2012; PMID: 22908294).

As another non-limiting example, the chimeric polynucleotides (e.g., nucleic acid molecules encoding an immunogen such as self-replicating RNA) may be substantially encapsulated within a PEGylated liposome (see International Patent Application No. WO2013033563). In yet another non-limiting example, the self-replicating RNA may be formulated as described in International Application No. WO201305590. In one non-limiting example, the self-replicating RNA may be formulated using biodegradable polymer particles as described in International Publication No WO2012006359 or US Patent Publication No. US20130183355.

In one case, the self-replicating RNA may be formulated in virion-like particles. As a non-limiting example, the self-replicating RNA is formulated in virion-like particles as described in International Publication No WO2012006376.

In another case, the self-replicating RNA may be formulated in a liposome. As a non-limiting example, the self-replicating RNA may be formulated in liposomes as described in International Publication No. WO20120067378.

In one case, the liposomes may comprise lipids which have a pKa value which may be advantageous for delivery of chimeric polynucleotides such as, but not limited to, mRNA. In another case, the liposomes may have an essentially neutral surface charge at physiological pH and may therefore be effective for immunization (see e.g., the liposomes described in International Publication No. WO20120067378).

In yet another case, the self-replicating RNA may be formulated in a cationic oil-in-water emulsion. As a non-limiting example, the self-replicating RNA may be formulated in the cationic oil-in-water emulsion described in International Publication No. WO2012006380. The cationic oil-in-water emulsions which may be used with the self-replicating RNA described herein (e.g., chimeric polynucleotides) may be made by the methods described in International Publication No. WO2012006380.

In one case, the chimeric polynucleotides may encode amphipathic and/or immunogenic amphipathic peptides.

In on case, a formulation of the chimeric polynucleotides may further comprise an amphipathic and/or immunogenic amphipathic peptide. As a non-limiting example, the chimeric polynucleotides comprising an amphipathic and/or immunogenic amphipathic peptide may be formulated as described in US. Pub. No. US20110250237 and International Pub. Nos. WO2010009277 and WO2010009065;

In one case, the chimeric polynucleotides may be immunostimulatory. As a non-limiting example, the chimeric polynucleotides may encode all or a part of a positive-sense or a negative-sense stranded RNA virus genome (see International Pub No. WO2012092569 and US Pub No. US20120177701 In another non-limiting example, the immunostimulatory chimeric polynucleotides may be formulated with an excipient for administration as described herein and/or known in the art (see International Pub No. WO2012068295 and US Pub No. US20120213812, The chimeric polynucleotides may further comprise a sequence region encoding a cytokine that promotes the immune response, such as a monokine, lymphokine, interleukin or chemokine, such as IL-1, IL-2, IL-3, IL-4, IL-5, IL-6, IL-7, IL-8, IL-9, IL-10, IL-12, INF-α, INF-γ, GM-CFS, LT-α, or growth factors such as hGH.

In one case, the response of the vaccine formulated by the methods described herein may be enhanced by the addition of various compounds to induce the therapeutic effect. As a non-limiting example, the vaccine formulation may include a MHC II binding peptide or a peptide having a similar sequence to a MHC II binding peptide (see International Pub Nos. WO2012027365, WO2011031298 and US Pub No. US20120070493, US20110110965). As another example, the vaccine formulations may comprise modified nicotinic compounds which may generate an antibody response to nicotine residue in a subject (see International Pub No. WO2012061717 and US Pub No. US2012011467).

In one case, the chimeric polynucleotides may encode at least one antibody or a fragment or portion thereof. The antibodies may be broadly neutralizing antibodies which may inhibit and protect against a broad range of infectious agents. As a non-limiting example, the chimeric polynucleotides encoding at least one antibody or fragment or portion thereof are provided to protect a subject against an infection disease and/or treat the disease. As another non-limiting example, the chimeric polynucleotides encoding two or more antibodies or fragments or portions thereof which are able to neutralize a wide spectrum of infectious agents are provided to protect a subject against an infection disease and/or treat the disease.

In one case, the chimeric polynucleotide may encode an antibody heavy chain or an antibody light chain. The optimal ratio of chimeric polynucleotide encoding antibody heavy chain and antibody light chain may be evaluated to determine the ratio that produces the maximal amount of a functional antibody and/or desired response. The chimeric polynucleotide may also encode a single svFv chain of an antibody.

The chimeric polynucleotides which encode one or more broadly neutralizing antibodies may be administrated to a subject prior to exposure to infectious viruses.

In one case, the effective amount of the chimeric polynucleotides provided to a cell, a tissue or a subject may be enough for immune prophylaxis.

In some case, the chimeric polynucleotide encoding cancer cell specific proteins may be formulated as a cancer vaccines. As a non-limiting example, the cancer vaccines comprising at least one chimeric polynucleotide may be used prophylactically to prevent cancer. The vaccine may comprise an adjuvant and/or a preservative. As a non-limiting example, the adjuvant may be squalene. As another non-limiting example, the preservative may be thimerosal.

In one case, described herein are immunogenic compositions containing chimeric polynucleotides which encode one or more antibodies, and/or other anti-infection reagents. These immunogenic compositions may comprise an adjuvant and/or a preservative. As a non-limiting example, the antibodies may be broadly neutralizing antibodies.

In another instance, described herein are antibody therapeutics containing the chimeric polynucleotides which encode one or more antibodies, and/or other anti-infectious reagents.

In one case, the chimeric polynucleotide compositions may be administrated with other prophylactic or therapeutic compounds. As a non-limiting example, the prophylactic or therapeutic compound may be an adjuvant or a booster. As used herein, when referring to a prophylactic composition, such as a vaccine, the term "booster" refers to an extra administration of the prophylactic ophalytic composition. A booster (or booster vaccine) may be given after an earlier administration of the prophylactic composition. The time of administration between the initial administration of the prophylactic composition and the booster may be, but is not limited to, 1 minute, 2 minutes, 3 minutes, 4 minutes, 5 minutes, 6 minutes, 7 minutes, 8 minutes, 9 minutes, 10 minutes, 15 minutes, 20 minutes 35 minutes, 40 minutes, 45 minutes, 50 minutes, 55 minutes, 1 hour, 2 hours, 3 hours, 4 hours, 5 hours, 6 hours, 7 hours, 8 hours, 9 hours, 10 hours, 11 hours, 12 hours, 13 hours, 14 hours, 15 hours, 16 hours, 17 hours, 18 hours, 19 hours, 20 hours, 21 hours, 22 hours, 23 hours, 1 day, 36 hours, 2 days, 3 days, 4 days, 5 days, 6 days, 1 week, 10 days, 2 weeks, 3 weeks, 1 month, 2 months, 3 months, 4 months, 5 months, 6 months, 7 months, 8 months, 9 months, 10 months, 11 months, 1 year, 18 months, 2 years, 3 years, 4 years, 5 years, 6 years, 7 years, 8 years, 9 years, 10 years, 11 years, 12 years, 13 years, 14 years, 15 years, 16 years, 17 years, 18 years, 19 years, 20 years, 25 years, 30 years, 35 years, 40 years, 45 years, 50 years, 55 years, 60 years, 65 years, 70 years, 75 years, 80 years, 85 years, 90 years, 95 years or more than 99 years.

In one case, the chimeric polynucleotide may be administered intranasally similar to the administration of live vaccines. In another case the chimeric polynucleotide may be administered intramuscularly or intradermally similarly to the administration of inactivated vaccines known in the art.

In one case, the chimeric polynucleotides may be used to protect against and/or prevent the transmission of an emerging or engineered threat which may be known or unknown.

In another case, the chimeric polynucleotides may be formulated by the methods described herein. The formulations may comprise chimeric polynucleotides for more than one antibody or vaccine. In one case, the formulation may comprise chimeric polynucleotide which can have a therapeutic and/or prophylactic effect on more than one disease, disorder or condition. As a non-limiting example, the formulation may comprise chimeric polynucleotides encoding an antigen, antibody or viral protein.

In addition, the antibodies may be used for research in many applications, such as, but not limited to, identifying and locating intracellular and extracellular proteins, protein interaction, signal pathways and cell biology.

In another case, the chimeric polynucleotide may be used in a vaccine such as, but not limited to, the modular vaccines described in International Publication No. WO2013093629,

As a non-limiting example, the chimeric polynucleotide encode at least one antigen, at least one subcellular localization element and at least one CD4 helper element. In one case, the subcellular localization element may be a signal peptide of protein sequence that results in the exportation of the antigen from the cytosol. In another case the CD4 helper element may be, but is not limited to, P30, NEF, P23TT, P32TT, P21TT, PfT3, P2TT, HBVnc, HA, HBsAg and MT (International Publication No. WO2013093629,

In one case, the chimeric polynucleotide may be used in the prevention or treatment of RSV infection or reducing the risk of RSV infection. Vaishnaw et al. in US Patent Publication No. US20131065499, describe using a composition comprising a siRNA to treat and/or prevent a RSV infection. As a non-limiting example, the chimeric polynucleotide may be formulated for intranasal administration for the prevention and/or treatment of RSV (see e.g., US Patent Publication No. US20130165499 ).

In another case, the chimeric polynucleotide may be used in to reduce the risk or inhibit the infection of influenza viruses such as, but not limited to, the highly pathogenic avian influenza virus (such as, but not limited to, H5N1 subtype) infection and human influenza virus (such as, but not limited to, H1N1 subtype and H3N2 subtype) infection. The chimeric polynucleotide described herein which may encode any of the protein sequences described in US Patent No. 8470771, may be used in the treatment or to reduce the risk of an influenza infection.

In one case, the chimeric polynucleotide may be used to as a vaccine or modulating the immune response against a protein produced by a parasite. Bergmann-Leitner et al. in US Patent No. 8470560, , describe a DNA vaccine against the circumsporozoite protein (CSP) of malaria parasites. As a non-limiting example, the chimeric polynucleotide may encode the CR2 binding motif of C3d and may be used a vaccine or therapeutic to modulate the immune system against the CSP of malaria parasites.

In one case, the chimeric polynucleotide may be used to produce a virus which may be labeled with alkyne-modified biomolecules such as, but not limited to, those described in International Patent Publication No. WO2013112778 and WO201311278

The labeled viruses may increase the infectivity of the virus and thus may be beneficial in making vaccines. The labeled viruses may be produced by various methods including those described in International Patent Publication No. WO2013112778 and WO2013112780,

In one case, the chimeric polynucleotide may be used as a vaccine and may further comprise an adjuvant which may enable the vaccine to elicit a higher immune response. As a non-limiting example, the adjuvant could be a sub-micron oil-in-water emulsion which can elicit a higher immune response in human pediatric populations (see e.g., the adjuvanted vaccines described in US Patent Publication No. US20120027813 and US Patent No. US8506966 ).

In another case, the chimeric polynucleotide may be used to as a vaccine and may also comprise 5' cap analogs to improve the stability and increase the expression of the vaccine. Non-limiting examples of 5'cap analogs are described in US Patent Publication No. US20120195917

### Naturally Occurring Mutants

In another case, the chimeric polynucleotides can be utilized to express variants of naturally occurring proteins that have an improved disease modifying activity, including increased biological activity, improved patient outcomes, or a protective function, etc., as described in co-pending International Patent Publication No. WO2015038892 , such as, but not limited to, in paragraphs [0001174] - [0001175].

### Targeting of pathogenic organisms or diseased cells

Disclosed herein are methods for targeting pathogenic microorganisms, such as bacteria, yeast, protozoa, helminthes and the like, or diseased cells such as cancer cells using chimeric polynucleotides that encode cytostatic or cytotoxic polypeptides. Preferably the mRNA introduced contains modified nucleosides or other nucleic acid sequence modifications that are translated exclusively, or preferentially, in the target pathogenic organism, to reduce possible off-target effects of the therapeutic. Such methods are useful for removing pathogenic organisms or killing diseased cells found in any biological material, including blood, semen, eggs, and transplant materials including embryos, tissues, and organs.

### Bioprocessing

The methods described herein may be useful for enhancing protein product yield in a cell culture process as described in co-pending International Patent Publication No. WO2015038892 such as, but not limited to, in paragraphs [0001176] - [0001187].

### Cells

In one case, the cells are selected from the group consisting of mammalian cells, bacterial cells, plant, microbial, algal and fungal cells. In some cases, the cells are mammalian cells, such as, but not limited to, human, mouse, rat, goat, horse, rabbit, hamster or cow cells. In a further case, the cells may be from an established cell line, including, but not limited to, HeLa, NS0, SP2/0, KEK 293T, Vero, Caco, Caco-2, MDCK, COS-1, COS-7, K562, Jurkat, CHO-K1, DG44, CHOK1SV, CHO-S, Huvec, CV-1, Huh-7, NIH3T3, HEK293, 293, A549, HepG2, IMR-90, MCF-7, U-20S, Per.C6, SF9, SF21 or Chinese Hamster Ovary (CHO) cells.

In certain cases, the cells are fungal cells, such as, but not limited to, Chrysosporium cells, Aspergillus cells, Trichoderma cells, Dictyostelium cells, Candida cells, Saccharomyces cells, Schizosaccharomyces cells, and Penicillium cells.

In certain cases, the cells are bacterial cells such as, but not limited to, E. coli, B. subtilis, or BL21 cells. Primary and secondary cells to be transfected by the methods of the disclosure can be obtained from a variety of tissues and include, but are not limited to, all cell types which can be maintained in culture. For examples, primary and secondary cells which can be transfected by the methods of the disclosure include, but are not limited to, fibroblasts, keratinocytes, epithelial cells (e.g., mammary epithelial cells, intestinal epithelial cells), endothelial cells, glial cells, neural cells, formed elements of the blood (e.g., lymphocytes, bone marrow cells), muscle cells and precursors of these somatic cell types. Primary cells may also be

obtained from a donor of the same species or from another species (e.g., mouse, rat, rabbit, cat, dog, pig, cow, bird, sheep, goat, horse).

### Purification and Isolation

Those of ordinary skill in the art should be able to make a determination of the methods to use to purify or isolate of a protein of interest from cultured cells. Generally, this is done through a capture method using affinity binding or non-affinity purification. If the protein of interest is not secreted by the cultured cells, then a lysis of the cultured cells should be performed prior to purification or isolation. One may use unclarified cell culture fluid containing the protein of interest along with cell culture media components as well as cell culture additives, such as anti-foam compounds and other nutrients and supplements, cells, cellular debris, host cell proteins, DNA, viruses and the like in the present disclosure. The process may be conducted in the bioreactor itself. The fluid may either be preconditioned to a desired stimulus such as pH, temperature or other stimulus characteristic or the fluid can be conditioned upon the addition of polymer(s) or the polymer(s) can be added to a carrier liquid that is properly conditioned to the required parameter for the stimulus condition required for that polymer to be solubilized in the fluid. The polymer may be allowed to circulate thoroughly with the fluid and then the stimulus may be applied (change in pH, temperature, salt concentration, etc.) and the desired protein and polymer(s) precipitate can out of the solution. The polymer and the desired protein(s) can be separated from the rest of the fluid and optionally washed one or more times to remove any trapped or loosely bound contaminants. The desired protein may then be recovered from the polymer(s) by, for example, elution and the like. Preferably, the elution may be done under a set of conditions such that the polymer remains in its precipitated form and retains any impurities to it during the selected elution of the desired protein. The polymer and protein as well as any impurities may be solubilized in a new fluid such as water or a buffered solution and the protein may be recovered by a means such as affinity, ion exchanged, hydrophobic, or some other type of chromatography that has a preference and selectivity for the protein over that of the polymer or impurities. The eluted protein may then be recovered and may be subjected to additional processing steps, either batch like steps or continuous flow through steps if appropriate.

In another case, it may be useful to optimize the expression of a specific polypeptide in a cell line or collection of cell lines of potential interest, particularly a polypeptide of interest such as a protein variant of a reference protein having a known activity. In one case, described herein is a method of optimizing expression of a polypeptide of interest in a target cell, by providing a plurality of target cell types, and independently contacting with each of the plurality of target cell types a modified mRNA encoding a polypeptide. Additionally, culture conditions may be altered to increase protein production efficiency. Subsequently, the presence and/or level of the polypeptide of interest in the plurality of target cell types is detected and/or quantitated, allowing for the optimization of a polypeptide of interest's expression by selection of an efficient target cell and cell culture conditions relating thereto. Such methods may be useful when the polypeptide of interest contains one or more post-translational modifications or has substantial tertiary structure, which often complicate efficient protein production.

### Protein recovery

The protein of interest may be preferably recovered from the culture medium as a secreted polypeptide, or it can be recovered from host cell lysates if expressed without a secretory signal. It may be necessary to purify the protein of interest from other recombinant proteins and host cell proteins in a way that substantially homogenous preparations of the protein of interest are obtained. The cells and/or particulate cell debris may be removed from the culture medium or lysate. The product of interest may then be purified from contaminant soluble proteins, polypeptides and nucleic acids by, for example, fractionation on immunoaffinity or ion-exchange columns, ethanol precipitation, reverse phase HPLC (RP-HPLC), SEPHADEX^{®} chromatography, chromatography on silica or on a cation exchange resin such as DEAE. Methods of purifying a protein heterologous expressed by a host cell are well known in the art.

Methods and compositions described herein may be used to produce proteins which are capable of attenuating or blocking the endogenous agonist biological response and/or antagonizing a receptor or signaling molecule in a mammalian subject. For example, IL-12 and IL-23 receptor signaling may be enhanced in chronic autoimmune disorders such as multiple sclerosis and inflammatory diseases such as rheumatoid arthritis, psoriasis, lupus erythematosus, ankylosing spondylitis and Chron's disease (Kikly K, Liu L, Na S, Sedgwich JD (2006) Cur. Opin. Immunol. 18(6): 670-5). In another case, a nucleic acid encodes an antagonist for chemokine receptors. Chemokine receptors CXCR-4 and CCR-5 are required for HIV entry into host cells (Arenzana-Seisdedos F et al, (1996) Nature. Oct 3; 383 (6599):400).

### Gene Silencing

The chimeric polynucleotides described herein are useful to silence (i.e., prevent or substantially reduce) expression of one or more target genes in a cell population. A chimeric polynucleotide encoding a polypeptide of interest capable of directing sequence-specific histone H3 methylation is introduced into the cells in the population under conditions such that the polypeptide is translated and reduces gene transcription of a target gene via histone H3 methylation and subsequent heterochromatin formation. In some cases, the silencing mechanism is performed on a cell population present in a mammalian subject. By way of non-limiting example, a useful target gene is a mutated Janus Kinase-2 family member, wherein the mammalian subject expresses the mutant target gene suffers from a myeloproliferative disease resulting from aberrant kinase activity.

Co-administration of chimeric polynucleotides and RNAi agents are also disclosed herein.

### Modulation of Biological Pathways

The rapid translation chimeric polynucleotides introduced into cells provides a desirable mechanism of modulating target biological pathways. Such modulation includes antagonism or agonism of a given pathway. In one case, described herein is a method for antagonizing a biological pathway in a cell by contacting the cell with an effective amount of a composition comprising a chimeric polynucleotide encoding a polypeptide of interest, under conditions such that the chimeric polynucleotides is localized into the cell and the polypeptide is capable of being translated in the cell from the chimeric polynucleotides, wherein the polypeptide inhibits the activity of a polypeptide functional in the biological pathway. Exemplary biological pathways are those defective in an autoimmune or inflammatory disorder such as multiple sclerosis, rheumatoid arthritis, psoriasis, lupus erythematosus, ankylosing spondylitis colitis, or Crohn's disease; in particular, antagonism of the IL-12 and IL-23 signaling pathways are of particular utility. (See Kikly K, Liu L, Na S, Sedgwick JD (2006) Curr. Opin. Immunol. 18 (6): 670-5).

Further, disclosed are chimeric polynucleotides encoding an antagonist for chemokine receptors; chemokine receptors CXCR-4 and CCR-5 are required for, e.g., HIV entry into host cells (Arenzana-Seisdedos F et al, (1996) Nature. Oct 3;383(6599):400).

Alternatively, described are methods of agonizing a biological pathway in a cell by contacting the cell with an effective amount of a chimeric polynucleotide encoding a recombinant polypeptide under conditions such that the nucleic acid is localized into the cell and the recombinant polypeptide is capable of being translated in the cell from the nucleic acid, and the recombinant polypeptide induces the activity of a polypeptide functional in the biological pathway. Exemplary agonized biological pathways include pathways that modulate cell fate determination. Such agonization is reversible or, alternatively, irreversible.

### Expression of Ligand or Receptor on Cell Surface

In some cases, described herein, the chimeric polynucleotides described herein can be used to express a ligand or ligand receptor on the surface of a cell *(e.g.,* a homing moiety). A ligand or ligand receptor moiety attached to a cell surface can permit the cell to have a desired biological interaction with a tissue or an agent *in vivo.* A ligand can be an antibody, an antibody fragment, an aptamer, a peptide, a vitamin, a carbohydrate, a protein or polypeptide, a receptor, *e.g.,* cell-surface receptor, an adhesion molecule, a glycoprotein, a sugar residue, a therapeutic agent, a drug, a glycosaminoglycan, or any combination thereof. For example, a ligand can be an antibody that recognizes a cancer-cell specific antigen, rendering the cell capable of preferentially interacting with tumor cells to permit tumor-specific localization of a modified cell. A ligand can confer the ability of a cell composition to accumulate in a tissue to be treated, since a preferred ligand may be capable of interacting with a target molecule on the external face of a tissue to be treated. Ligands having limited cross-reactivity to other tissues are generally preferred.

In some cases, a ligand can act as a homing moiety which permits the cell to target to a specific tissue or interact with a specific ligand. Such homing moieties can include, but are not limited to, any member of a specific binding pair, antibodies, monoclonal antibodies, or derivatives or analogs thereof, including without limitation: Fv fragments, single chain Fv (scFv) fragments, Fab' fragments, F(ab')2 fragments, single domain antibodies, camelized antibodies and antibody fragments, humanized antibodies and antibody fragments, and multivalent versions of the foregoing; multivalent binding reagents including without limitation: monospecific or bispecific antibodies, such as disulfide stabilized Fv fragments, scFv tandems ((SCFV)2 fragments), diabodies, tribodies or tetrabodies, which typically are covalently linked or otherwise stabilized *(i.e.,* leucine zipper or helix stabilized) scFv fragments; and other homing moieties include for example, aptamers, receptors, and fusion proteins.

In some cases, the homing moiety may be a surface-bound antibody, which can permit tuning of cell targeting specificity. This is especially useful since highly specific antibodies can be raised against an epitope of interest for the desired targeting site. In one case, multiple antibodies are expressed on the surface of a cell, and each antibody can have a different specificity for a desired target. Such approaches can increase the avidity and specificity of homing interactions.

A skilled artisan can select any homing moiety based on the desired localization or function of the cell, for example an estrogen receptor ligand, such as tamoxifen, can target cells to estrogen-dependent breast cancer cells that have an increased number of estrogen receptors on the cell surface. Other non-limiting examples of ligand/receptor interactions include CCRI *(e.g.,* for treatment of inflamed joint tissues or brain in rheumatoid arthritis, and/or multiple sclerosis), CCR7, CCR8 *(e.g.,* targeting to lymph node tissue), CCR6, CCR9,CCR10 (*e.g.,* to target to intestinal tissue), CCR4, CCR10 (*e.g.,* for targeting to skin), CXCR4 (*e.g.,* for general enhanced transmigration), HCELL (*e.g*., for treatment of inflammation and inflammatory disorders, bone marrow), Alpha4beta7 (*e.g*., for intestinal mucosa targeting), VLA-4/VCAM-1 (*e.g*., targeting to endothelium). In general, any receptor involved in targeting (*e.g*., cancer metastasis) can be harnessed for use in the methods and compositions described herein.

### Modulation of Cell Lineage

Described are methods of inducing an alteration in cell fate in a target mammalian cell. The target mammalian cell may be a precursor cell and the alteration may involve driving differentiation into a lineage, or blocking such differentiation. Alternatively, the target mammalian cell may be a differentiated cell, and the cell fate alteration includes driving de-differentiation into a pluripotent precursor cell, or blocking such de-differentiation, such as the dedifferentiation of cancer cells into cancer stem cells. In situations where a change in cell fate is desired, effective amounts of mRNAs encoding a cell fate inductive polypeptide is introduced into a target cell under conditions such that an alteration in cell fate is induced. In some cases, the modified mRNAs are useful to reprogram a subpopulation of cells from a first phenotype to a second phenotype. Such a reprogramming may be temporary or permanent. Optionally, the reprogramming induces a target cell to adopt an intermediate phenotype.

Additionally, the methods of the present disclosure are particularly useful to generate induced pluripotent stem cells (iPS cells) because of the high efficiency of transfection, the ability to re-transfect cells, and the tenability of the amount of recombinant polypeptides produced in the target cells. Further, the use of iPS cells generated using the methods described herein is expected to have a reduced incidence of teratoma formation.

Also described herein are methods of reducing cellular differentiation in a target cell population. For example, a target cell population containing one or more precursor cell types is contacted with a composition having an effective amount of a chimeric polynucleotides encoding a polypeptide, under conditions such that the polypeptide is translated and reduces the differentiation of the precursor cell. In nonlimiting cases, the target cell population contains injured tissue in a mammalian subject or tissue affected by a surgical procedure. The precursor cell is, e.g., a stromal precursor cell, a neural precursor cell, or a mesenchymal precursor cell.

In a specific case, described herein are chimeric polynucleotides that encode one or more differentiation factors Gata4, Mef2c and Tbx4. These mRNA-generated factors are introduced into fibroblasts and drive the reprogramming into cardiomyocytes. Such a reprogramming can be performed *in vivo,* by contacting an mRNA-containing patch or other material to damaged cardiac tissue to facilitate cardiac regeneration. Such a process promotes cardiomyocyte genesis as opposed to fibrosis.

### Mediation of cell death

In one case, chimeric polynucleotides compositions can be used to induce apoptosis in a cell *(e.g.,* a cancer cell) by increasing the expression of a death receptor, a death receptor ligand or a combination thereof. This method can be used to induce cell death in any desired cell and has particular usefulness in the treatment of cancer where cells escape natural apoptotic signals.

Apoptosis can be induced by multiple independent signaling pathways that converge upon a final effector mechanism consisting of multiple interactions between several "death receptors" and their ligands, which belong to the tumor necrosis factor

(TNF) receptor/ligand superfamily. The best-characterized death receptors are CD95 ("Fas"), TNFRI (p55), death receptor 3 (DR3 or Apo3/TRAMO), DR4 and DR5 (apo2-TRAIL-R2). The final effector mechanism of apoptosis may be the activation of a series of proteinases designated as caspases. The activation of these caspases results in the cleavage of a series of vital cellular proteins and cell death. The molecular mechanism of death receptors/ligands-induced apoptosis is well known in the art. For example, Fas/FasL-mediated apoptosis is induced by binding of three FasL molecules which induces trimerization of Fas receptor via C-terminus death domains (DDs), which in turn recruits an adapter protein FADD (Fas-associated protein with death domain) and Caspase-8. The oligomerization of this trimolecular complex, Fas/FAIDD/caspase-8, results in proteolytic cleavage of proenzyme caspase-8 into active caspase-8 that, in turn, initiates the apoptosis process by activating other downstream caspases through proteolysis, including caspase-3. Death ligands in general are apoptotic when formed into trimers or higher order of structures. As monomers, they may serve as antiapoptotic agents by competing with the trimers for binding to the death receptors.

In one case, the chimeric polynucleotides composition encodes for a death receptor *(e.g.,* Fas, TRAIL, TRAMO, TNFR, TLR etc.). Cells made to express a death receptor by transfection of chimeric polynucleotides become susceptible to death induced by the ligand that activates that receptor. Similarly, cells made to express a death ligand, *e.g*., on their surface, will induce death of cells with the receptor when the transfected cell contacts the target cell. In another case, the chimeric polynucleotides composition encodes for a death receptor ligand *(e.g.,* FasL, TNF, etc.). In another case, the chimeric polynucleotides composition encodes a caspase *(e.g.,* caspase 3, caspase 8, caspase 9 etc.). Where cancer cells often exhibit a failure to properly differentiate to a non-proliferative or controlled proliferative form, in another case, the synthetic, chimeric polynucleotides composition encodes for both a death receptor and its appropriate activating ligand. In another case, the synthetic, chimeric polynucleotides composition encodes for a differentiation factor that when expressed in the cancer cell, such as a cancer stem cell, will induce the cell to differentiate to a non-pathogenic or nonself-renewing phenotype *(e.g.,* reduced cell growth rate, reduced cell division etc.) or to induce the cell to enter a dormant cell phase *(e.g.,* G₀ resting phase).

One of skill in the art will appreciate that the use of apoptosis-inducing techniques may require that the chimeric polynucleotides are appropriately targeted to *e.g.,* tumor cells to prevent unwanted wide-spread cell death. Thus, one can use a delivery mechanism *(e.g.,* attached ligand or antibody, targeted liposome etc.) that recognizes a cancer antigen such that the chimeric polynucleotides are expressed only in cancer cells.

### Cosmetic Applications

In one case, the chimeric polynucleotides may be used in the treatment, amelioration or prophylaxis of cosmetic conditions. Such conditions include acne, rosacea, scarring, wrinkles, eczema, shingles, psoriasis, age spots, birth marks, dry skin, calluses, rash (e.g., diaper, heat), scabies, hives, warts, insect bites, vitiligo, dandruff, freckles, and general signs of aging.

### VI. Kits and Devices

### Kits

Described herein are a variety of kits for conveniently and/or effectively carrying out methods of the present disclosure. Typically kits will comprise sufficient amounts and/or numbers of components to allow a user to perform multiple treatments of a subject(s) and/or to perform multiple experiments.

In one case, desribed herein are kits comprising the molecules (chimeric polynucleotides) of the disclosure. In one case, the kit comprises one or more functional antibodies or function fragments thereof.

The kits can be for protein production, comprising a first chimeric polynucleotides comprising a translatable region. The kit may further comprise packaging and instructions and/or a delivery agent to form a formulation composition. The delivery agent may comprise a saline, a buffered solution, a lipidoid or any delivery agent disclosed herein.

In one case, the buffer solution may include sodium chloride, calcium chloride, phosphate and/or EDTA. In another case, the buffer solution may include, but is not limited to, saline, saline with 2mM calcium, 5% sucrose, 5% sucrose with 2mM calcium, 5% Mannitol, 5% Mannitol with 2mM calcium, Ringer's lactate, sodium chloride, sodium chloride with 2mM calcium and mannose (See e.g., U.S. Pub. No. 20120258046; ). In a further case, the buffer solutions may be precipitated or it may be lyophilized. The amount of each component may be varied to enable consistent, reproducible higher concentration saline or simple buffer formulations. The components may also be varied in order to increase the stability of modified RNA in the buffer solution over a period of time and/or under a variety of conditions. In one case, described herein are kits for protein production, comprising: a chimeric polynucleotide comprising a translatable region, provided in an amount effective to produce a desired amount of a protein encoded by the translatable region when introduced into a target cell; a second polynucleotide comprising an inhibitory nucleic acid, provided in an amount effective to substantially inhibit the innate immune response of the cell; and packaging and instructions.

In one case, described herein are kits for protein production, comprising a chimeric polynucleotide comprising a translatable region, wherein the polynucleotide exhibits reduced degradation by a cellular nuclease, and packaging and instructions.

In one case, described herein are kits for protein production, comprising a chimeric polynucleotide comprising a translatable region, wherein the polynucleotide exhibits reduced degradation by a cellular nuclease, and a mammalian cell suitable for translation of the translatable region of the first nucleic acid.

### Devices

Also described herein are devices which may incorporate chimeric polynucleotides that encode polypeptides of interest. These devices contain in a stable formulation the reagents to synthesize a polynucleotide in a formulation available to be immediately delivered to a subject in need thereof, such as a human patient

Devices for administration may be employed to deliver the chimeric polynucleotides of the present disclosure according to single, multi- or split-dosing regimens taught herein. Such devices are taught in, for example, International Application PCT/US2013/30062 filed March 9, 2013 (Attorney Docket Number M300)

Method and devices known in the art for multi-administration to cells, organs and tissues are contemplated for use in conjunction with the methods and compositions disclosed herein These include, for example, those methods and devices having multiple needles, hybrid devices employing for example lumens or catheters as well as devices utilizing heat, electric current or radiation driven mechanisms.

These multi-administration devices may be utilized to deliver the single, multi- or split doses contemplated herein. Such devices are taught for example in, International Application PCT/US2013/30062 filed March 9, 2013 (Attorney Docket Number M300).

In one case, the polynucleotide is administered subcutaneously or intramuscularly via at least 3 needles to three different, optionally adjacent, sites simultaneously, or within a 60 minutes period (e.g., administration to 4 ,5, 6, 7, 8, 9, or 10 sites simultaneously or within a 60 minute period).

Methods of delivering therapeutic agents using solid biodegradable microneedles are described by O'hagan et al. in US Patent Publication No. US20130287832, entirety. The microneedles are fabricated from the therapeutic agent (e.g., influenza vaccine) in combination with at least one solid excipient. After penetrating the skin, the microneedles dissolve in situ and release the therapeutic agent to the subject. As a non-limiting example, the therapeutic agents used in the fabrication of the microneedles are the polynucleotides described herein.

A microneedle assembly for transdermal drug delivery is described by Ross et al. in US Patent No. US8636696, The assembly has a first surface and a second surface where the microneedles project outwardly from the second surface of the support. The assembly may include a channel and aperture to form a junction which allows fluids (e.g., therapeutic agents or drugs) to pass.

### Methods and Devices utilizing catheters and/or lumens

Methods and devices using catheters and lumens may be employed to administer the chimeric polynucleotides of the present disclosure on a single, multi- or split dosing schedule. Such methods and devices are described in International Application PCT/US2013/30062 filed March 9, 2013 (Attorney Docket Number M300)

### Methods and Devices utilizing electrical current

Methods and devices utilizing electric current may be employed to deliver the chimeric polynucleotides of the present disclosure according to the single, multi-or split dosing regimens taught herein. Such methods and devices are described in International Application PCT/US2013/30062 filed March 9, 2013 (Attorney Docket Number M300).

### VII. Definitions

At various places in the present specification, substituents of compounds of the present disclosure are disclosed in groups or in ranges. It is specifically intended that the present disclosure include each and every individual subcombination of the members of such groups and ranges. For example, the term "C₁₋₆ alkyl" is specifically intended to individually disclose methyl, ethyl, C₃ alkyl, C₄ alkyl, C₅ alkyl, and C₆ alkyl. Herein a phrase of the form "optionally substituted X" (*e.g.,* optionally substituted alkyl) is intended to be equivalent to "X, wherein X is optionally substituted" (e.g., "alkyl, wherein said alkyl is optionally substituted"). It is not intended to mean that the feature "X" (*e.g.* alkyl) *per se* is optional.

*About:* As used herein, the term "about" means +/- 10% of the recited value.

*Administered in combination:* As used herein, the term "administered in combination" or "combined administration" means that two or more agents are administered to a subject at the same time or within an interval such that there may be an overlap of an effect of each agent on the patient. In some cases, they are administered within about 60, 30, 15, 10, 5, or 1 minute of one another. In some cases, the administrations of the agents are spaced sufficiently closely together such that a combinatorial *(e.g.,* a synergistic) effect is achieved.

*Adjuvant:* As used herein, the term "adjuvant" means a substance that enhances a subject's immune response to an antigen.

*Animal:* As used herein, the term "animal" refers to any member of the animal kingdom. In some cases, "animal" refers to humans at any stage of development. In some cases, "animal" refers to non-human animals at any stage of development. In certain cases, the non-human animal is a mammal (*e.g.,* a rodent, a mouse, a rat, a rabbit, a monkey, a dog, a cat, a sheep, cattle, a primate, or a pig). In some cases, animals include, but are not limited to, mammals, birds, reptiles, amphibians, fish, and worms. In some cases, the animal is a transgenic animal, genetically-engineered animal, or a clone.

*Antibody Fragment:* As used herein, the term "antibody fragment" comprises a portion of an intact antibody, preferably the antigen binding and/or the variable region of the intact antibody. Examples of antibody fragments include Fab, Fab', F(ab')₂ and Fv fragments; diabodies; linear antibodies; nanobodies; single-chain antibody molecules and multispecific antibodies formed from antibody fragments.

*Antigen:* As used herein, the term "antigen" refers to the substance that binds specifically to the respective antibody. An antigen may originate either from the body, such as cancer antigen used herein, or from the external environment, for instance, from infectious agents.

*Antigens of interest or desired antigens:* As used herein, the terms "antigens of interest" or "desired antigens" include those proteins and other biomolecules disclosed herein that are immunospecifically bound by the antibodies and fragments, mutants, variants, and alterations thereof described herein. Examples of antigens of interest include, but are not limited to, insulin, insulin-like growth factor, hGH, tPA, cytokines, such as interleukins (IL), e.g., IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, interferon (IFN) alpha, IFN beta, IFN gamma, IFN omega or IFN tau, tumor necrosis factor (TNF), such as TNF alpha and TNF beta, TNF gamma, TRAIL; G-CSF, GM-CSF, M-CSF, MCP-1 and VEGF.

*Approximately:* As used herein, the term "approximately" or "about," as applied to one or more values of interest, refers to a value that is similar to a stated reference value. In certain cases, the term "approximately" or "about" refers to a range of values that fall within 25%, 20%, 19%, 18%, 17%, 16%, 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

*Associated with:* As used herein, the terms "associated with," "conjugated," "linked," "attached," and "tethered," when used with respect to two or more moieties, means that the moieties are physically associated or connected with one another, either directly or via one or more additional moieties that serves as a linking agent, to form a structure that is sufficiently stable so that the moieties remain physically associated under the conditions in which the structure is used, *e.g.,* physiological conditions. An "association" need not be strictly through direct covalent chemical bonding. It may also suggest ionic or hydrogen bonding or a hybridization based connectivity sufficiently stable such that the "associated" entities remain physically associated.

*Bifunctional:* As used herein, the term "bifunctional" refers to any substance, molecule or moiety which is capable of or maintains at least two functions. The functions may effect the same outcome or a different outcome. The structure that produces the function may be the same or different. For example, bifunctional modified RNAs disclosure may encode a cytotoxic peptide (a first function) while those nucleosides which comprise the encoding RNA are, in and of themselves, cytotoxic (second function). In this example, delivery of the bifunctional modified RNA to a cancer cell would produce not only a peptide or protein molecule which may ameliorate or treat the cancer but would also deliver a cytotoxic payload of nucleosides to the cell should degradation, instead of translation of the modified RNA, occur.

*Biocompatible:* As used herein, the term "biocompatible" means compatible with living cells, tissues, organs or systems posing little to no risk of injury, toxicity or rejection by the immune system.

*Biodegradable:* As used herein, the term "biodegradable" means capable of being broken down into innocuous products by the action of living things.

*Biologically active:* As used herein, the phrase "biologically active" refers to a characteristic of any substance that has activity in a biological system and/or organism. For instance, a substance that, when administered to an organism, has a biological effect on that organism, is considered to be biologically active. In particular cases, a chimeric polynucleotide may be considered biologically active if even a portion of the chimeric polynucleotides is biologically active or mimics an activity considered biologically relevant.

*Cancer stem cells:* As used herein, "cancer stem cells" are cells that can undergo self-renewal and/or abnormal proliferation and differentiation to form a tumor.

*Chemical terms:* The following provides the definition of various chemical terms from "acyl" to "thiol."

The term "acyl," as used herein, represents a hydrogen or an alkyl group (e.g., a haloalkyl group), as defined herein, that is attached to the parent molecular group through a carbonyl group, as defined herein, and is exemplified by formyl (i.e., a carboxyaldehyde group), acetyl, trifluoroacetyl, propionyl, butanoyl and the like. Exemplary unsubstituted acyl groups include from 1 to 7, from 1 to 11, or from 1 to 21 carbons. In some cases, the alkyl group is further substituted with 1, 2, 3, or 4 substituents as described herein.

Non-limiting examples of optionally substituted acyl groups include, alkoxycarbonyl, alkoxycarbonylacyl, arylalkoxycarbonyl, aryloyl, carbamoyl, carboxyaldehyde, (heterocyclyl) imino, and (heterocyclyl)oyl:

The "alkoxycarbonyl" group, which as used herein, represents an alkoxy, as defined herein, attached to the parent molecular group through a carbonyl atom (e.g., -C(O)-OR, where R is H or an optionally substituted C₁₋₆, C₁₋₁₀, or C₁₋₂₀ alkyl group). Exemplary unsubstituted alkoxycarbonyl include from 1 to 21 carbons (e.g., from 1 to 11 or from 1 to 7 carbons). In some cases, the alkoxy group is further substituted with 1, 2, 3, or 4 substituents as described herein.

The "alkoxycarbonylacyl" group, which as used herein, represents an acyl group, as defined herein, that is substituted with an alkoxycarbonyl group, as defined herein (e.g., -C(O) -alkyl-C(O)-OR, where R is an optionally substituted C₁₋₆, C₁₋₁₀, or C₁₋₂₀ alkyl group). Exemplary unsubstituted alkoxycarbonylacyl include from 3 to 41 carbons (e.g., from 3 to 10, from 3 to 13, from 3 to 17, from 3 to 21, or from 3 to 31 carbons, such as C₁₋₆ alkoxycarbonyl-C₁₋₆ acyl, C₁₋₁₀ alkoxycarbonyl-C₁₋₁₀ acyl, or C₁₋₂₀ alkoxycarbonyl-C₁₋₂₀ acyl). In some cases, each alkoxy and alkyl group is further independently substituted with 1, 2, 3, or 4 substituents, as described herein (e.g., a hydroxy group) for each group.

The "arylalkoxycarbonyl" group, which as used herein, represents an arylalkoxy group, as defined herein, attached to the parent molecular group through a carbonyl (e.g., -C(O)-O-alkyl-aryl). Exemplary unsubstituted arylalkoxy groups include from 8 to 31 carbons (e.g., from 8 to 17 or from 8 to 21 carbons, such as C₆₋₁₀ aryl-C₁₋₆ alkoxy-carbonyl, C₆₋₁₀ aryl-C₁₋₁₀ alkoxy-carbonyl, or C₆₋₁₀ aryl-C₁₋₂₀ alkoxy-carbonyl). In some cases, the arylalkoxycarbonyl group can be substituted with 1, 2, 3, or 4 substituents as defined herein.

The "aryloyl" group, which as used herein, represents an aryl group, as defined herein, that is attached to the parent molecular group through a carbonyl group. Exemplary unsubstituted aryloyl groups are of 7 to 11 carbons. In some cases, the aryl group can be substituted with 1, 2, 3, or 4 substituents as defined herein.

The "carbamoyl" group, which as used herein, represents -C(O)-N(R^{N1})₂, where the meaning of each R^{N1} is found in the definition of "amino" described herein.

The "carboxyaldehyde" group, which as used herein, represents an acyl group having the structure -CHO.

The "(heterocyclyl) imino" group, which as used herein, represents a heterocyclyl group, as defined herein, attached to the parent molecular group through an imino group. In some cases, the heterocyclyl group can be substituted with 1, 2, 3, or 4 substituent groups as defined herein.

The "(heterocyclyl)oyl" group, which as used herein, represents a heterocyclyl group, as defined herein, attached to the parent molecular group through a carbonyl group. In some cases, the heterocyclyl group can be substituted with 1, 2, 3, or 4 substituent groups as defined herein.

The term "alkyl," as used herein, is inclusive of both straight chain and branched chain saturated groups from 1 to 20 carbons (e.g., from 1 to 10 or from 1 to 6), unless otherwise specified. Alkyl groups are exemplified by methyl, ethyl, n- and iso-propyl, n-, sec-, iso- and tert-butyl, neopentyl, and the like, and may be optionally substituted with one, two, three, or, in the case of alkyl groups of two carbons or more, four substituents independently selected from the group consisting of: (1) C₁₋₆ alkoxy; (2) C₁₋₆ alkylsulfinyl; (3) amino, as defined herein (e.g., unsubstituted amino (i.e., -NH₂) or a substituted amino (i.e., -N(R^{N1})₂, where R^{N1} is as defined for amino); (4) C₆₋₁₀ aryl-C₁₋₆ alkoxy; (5) azido; (6) halo; (7) (C₂₋₉ heterocyclyl)oxy; (8) hydroxy, optionally substituted with an *O*-protecting group; (9) nitro; (10) oxo (e.g., carboxyaldehyde or acyl); (11) C₁₋₇ spirocyclyl; (12) thioalkoxy; (13) thiol; (14) - CO₂R^{A'}, optionally substituted with an *O*-protecting group and where R^{A'} is selected from the group consisting of (a) C₁₋₂₀ alkyl (e.g., C₁₋₆ alkyl), (b) C₂₋₂₀ alkenyl (e.g., C₂₋₆ alkenyl), (c) C₆₋₁₀ aryl, (d) hydrogen, (e) C₁₋₆ alk-C₆₋₁₀ aryl, (f) amino-C₁₋₂₀ alkyl, (g) polyethylene glycol of -(CH₂)ₛ₂(OCH₂CH₂)ₛ₁(CH₂)ₛ₃OR', wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and R' is H or C₁₋₂₀ alkyl, and (h) amino-polyethylene glycol of - NR^{N1}(CH₂)ₛ₂(CH₂CH₂O)ₛ₁(CH₂)ₛ₃NR^{N1}, wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and each R^{N1} is, independently, hydrogen or optionally substituted C₁₋₆ alkyl; (15) -C(O)NR^{B'}R^{C'}, where each of R^{B'} and R^{C'} is, independently, selected from the group consisting of (a) hydrogen, (b) C₁₋₆ alkyl, (c) C₆₋₁₀ aryl, and (d) C₁₋₆ alk-C₆₋₁₀ aryl; (16) -SO₂R^{D'}, where R^{D'} is selected from the group consisting of (a) C₁₋₆ alkyl, (b) C₆₋₁₀ aryl, (c) C₁₋₆ alk-C₆₋₁₀ aryl, and (d) hydroxy; (17) -SO₂NR^{E'}R^{F'}, where each of R^{E'} and R^{F'} is, independently, selected from the group consisting of (a) hydrogen, (b) C₁₋₆ alkyl, (c) C₆₋₁₀ aryl and (d) C₁₋₆ alk-C₆₋₁₀ aryl; (18) -C(O)R^{G'}, where R^{G'} is selected from the group consisting of (a) C₁₋₂₀ alkyl (e.g., C₁₋₆ alkyl), (b) C₂₋₂₀ alkenyl (e.g., C₂₋₆ alkenyl), (c) C₆₋₁₀ aryl, (d) hydrogen, (e) C₁₋₆ alk-C₆₋₁₀ aryl, (f) amino-C₁₋₂₀ alkyl, (g) polyethylene glycol of -(CH₂)ₛ₂(OCH₂CH₂)ₛ₁(CH₂)ₛ₃OR', wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and R' is H or C₁₋₂₀ alkyl, and (h) amino-polyethylene glycol of -Ne^{N1}(CH₂)ₛ₂(CH₂CH₂O)ₛ₁(CH₂)ₛ₃NR^{N1}, wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and each R^{N1} is, independently, hydrogen or optionally substituted C₁₋₆ alkyl; (19) - NR^{H'}C(O)R^{I'}, wherein R^{H'} is selected from the group consisting of (a1) hydrogen and (b1) C₁₋₆ alkyl, and R^{I'} is selected from the group consisting of (a2) C₁₋₂₀ alkyl (e.g., C₁₋₆ alkyl), (b2) C₂₋₂₀ alkenyl (e.g., C₂₋₆ alkenyl), (c2) C₆₋₁₀ aryl, (d2) hydrogen, (e2) C₁₋₆ alk-C₆₋₁₀ aryl, (f2) amino-C₁₋₂₀ alkyl, (g2) polyethylene glycol of - (CH₂)ₛ₂(OCH₂CH₂)ₛ₁(CH₂)ₛ₃OR', wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and R' is H or C₁₋₂₀ alkyl, and (h2) amino-polyethylene glycol of -NR^{N1}(CH₂)ₛ₂(CH₂CH₂O)ₛ₁(CH₂)ₛ₃NR^{N1}, wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and each R^{N1} is, independently, hydrogen or optionally substituted C₁₋₆ alkyl; (20) -NR^{J'}C(O)OR^{K'}, wherein R^{J'} is selected from the group consisting of (a1) hydrogen and (b1) C₁₋₆ alkyl, and R^{K'} is selected from the group consisting of (a2) C₁₋₂₀ alkyl (e.g., C₁₋₆ alkyl), (b2) C₂₋₂₀ alkenyl (e.g., C₂₋₆ alkenyl), (c2) C₆₋₁₀ aryl, (d2) hydrogen, (e2) C₁₋₆ alk-C₆₋₁₀ aryl, (f2) amino-C₁₋₂₀ alkyl, (g2) polyethylene glycol of -(CH₂)ₛ₂(OCH₂CH₂)ₛ₁(CH₂)ₛ₃OR', wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1

to 10), and R' is H or C₁₋₂₀ alkyl, and (h2) amino-polyethylene glycol of - NR^{N1}(CH₂)ₛ₂(CH₂CH₂O)ₛ₁(CH₂)ₛ₃NR^{N1}, wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and each R^{N1} is, independently, hydrogen or optionally substituted C₁₋₆ alkyl; and (21) amidine. In some cases, each of these groups can be further substituted as described herein. For example, the alkylene group of a C₁-alkaryl can be further substituted with an oxo group to afford the respective aryloyl substituent.

The term "alkylene," as used herein, represent a saturated divalent hydrocarbon group derived from a straight or branched chain saturated hydrocarbon by the removal of two hydrogen atoms, and is exemplified by methylene, ethylene, isopropylene, and the like. The term "C_{x-y} alkylene" and the prefix "C_{x-y} alk-" represent alkylene groups having between x and y carbons. Exemplary values for x are 1, 2, 3, 4, 5, and 6, and exemplary values for y are 2, 3, 4, 5, 6, 7, 8, 9, 10, 12, 14, 16, 18, or 20 (e.g., C₁₋₆, C₁₋₁₀, C₂₋₂₀, C₂₋₆, C₂₋₁₀, or C₂₋₂₀ alkylene). In some cases, the alkylene can be further substituted with 1, 2, 3, or 4 substituent groups as defined herein for an alkyl group. Similarly, the suffix "-ene" appended to any group indicates that the group is a divalent group.

Non-limiting examples of optionally substituted alkyl and alkylene groups include acylaminoalkyl, acyloxyalkyl, alkoxyalkyl, alkoxycarbonylalkyl, alkylsulfinyl, alkylsulfinylalkyl, aminoalkyl, carbamoylalkyl, carboxyalkyl, carboxyaminoalkyl, haloalkyl, hydroxyalkyl, perfluoroalkyl, and sulfoalkyl:

The "acylaminoalkyl" group, which as used herein, represents an acyl group, as defined herein, attached to an amino group that is in turn attached to the parent molecular group through an alkylene group, as defined herein (i.e., -alkyl-N(R^{N1})-C(O)-R, where R is H or an optionally substituted C₁₋₆, C₁₋₁₀, or C₁₋₂₀ alkyl group (e.g., haloalkyl) and R^{N1} is as defined herein). Exemplary unsubstituted acylaminoalkyl groups include from 1 to 41 carbons (e.g., from 1 to 7, from 1 to 13, from 1 to 21, from 2 to 7, from 2 to 13, from 2 to 21, or from 2 to 41 carbons). In some cases, the alkylene group is further substituted with 1, 2, 3, or 4 substituents as described herein, and/or the amino group is -NH₂ or -NHR^{N1}, wherein R^{N1} is, independently, OH, NO₂, NH₂, NR^{N2}₂, SO₂OR^{N2}, SO₂R^{N2}, SOR^{N2}, alkyl, aryl, acyl (e.g., acetyl, trifluoroacetyl, or others described herein), or alkoxycarbonylalkyl, and each R^{N2} can be H, alkyl, or aryl.

The "acyloxyalkyl" group, which as used herein, represents an acyl group, as defined herein, attached to an oxygen atom that in turn is attached to the parent molecular group though an alkylene group (i.e., -alkyl-O-C(O)-R, where R is H or an optionally substituted C₁₋₆, C₁₋₁₀, or C₁₋₂₀ alkyl group). Exemplary unsubstituted acyloxyalkyl groups include from 1 to 21 carbons (e.g., from 1 to 7 or from 1 to 11 carbons). In some cases, the alkylene group is, independently, further substituted with 1, 2, 3, or 4 substituents as described herein.

The "alkoxyalkyl" group, which as used herein, represents an alkyl group that is substituted with an alkoxy group. Exemplary unsubstituted alkoxyalkyl groups include between 2 to 40 carbons (e.g., from 2 to 12 or from 2 to 20 carbons, such as C₁₋₆ alkoxy-C₁₋₆ alkyl, C₁₋₁₀ alkoxy-C₁₋₁₀ alkyl, or C₁₋₂₀ alkoxy-C₁₋₂₀ alkyl). In some cases, the alkyl and the alkoxy each can be further substituted with 1, 2, 3, or 4 substituent groups as defined herein for the respective group.

The "alkoxycarbonylalkyl" group, which as used herein, represents an alkyl group, as defined herein, that is substituted with an alkoxycarbonyl group, as defined herein (e.g., -alkyl-C(O)-OR, where R is an optionally substituted C₁₋₂₀, C₁₋₁₀, or C₁₋₆ alkyl group). Exemplary unsubstituted alkoxycarbonylalkyl include from 3 to 41 carbons (e.g., from 3 to 10, from 3 to 13, from 3 to 17, from 3 to 21, or from 3 to 31 carbons, such as C₁₋₆ alkoxycarbonyl-C₁₋₆ alkyl, C₁₋₁₀ alkoxycarbonyl-C₁₋₁₀ alkyl, or C₁₋₂₀ alkoxycarbonyl-C₁₋₂₀ alkyl). In some cases, each alkyl and alkoxy group is further independently substituted with 1, 2, 3, or 4 substituents as described herein (e.g., a hydroxy group).

The "alkylsulfinylalkyl" group, which as used herein, represents an alkyl group, as defined herein, substituted with an alkylsulfinyl group. Exemplary unsubstituted alkylsulfinylalkyl groups are from 2 to 12, from 2 to 20, or from 2 to 40 carbons. In some cases, each alkyl group can be further substituted with 1, 2, 3, or 4 substituent groups as defined herein.

The "aminoalkyl" group, which as used herein, represents an alkyl group, as defined herein, substituted with an amino group, as defined herein. The alkyl and amino each can be further substituted with 1, 2, 3, or 4 substituent groups as described herein for the respective group (e.g., CO₂R^{A'}, where R^{A'} is selected from the group consisting of (a) C₁₋₆ alkyl, (b) C₆₋₁₀ aryl, (c) hydrogen, and (d) C₁₋₆ alk-C₆₋₁₀ aryl, e.g., carboxy, and/or an *N*-protecting group).

The "carbamoylalkyl" group, which as used herein, represents an alkyl group, as defined herein, substituted with a carbamoyl group, as defined herein. The alkyl group can be further substituted with 1, 2, 3, or 4 substituent groups as described herein.

The "carboxyalkyl" group, which as used herein, represents an alkyl group, as defined herein, substituted with a carboxy group, as defined herein. The alkyl group can be further substituted with 1, 2, 3, or 4 substituent groups as described herein, and the carboxy group can be optionally substituted with one or more *O-*protecting groups.

The "carboxyaminoalkyl" group, which as used herein, represents an aminoalkyl group, as defined herein, substituted with a carboxy, as defined herein. The carboxy, alkyl, and amino each can be further substituted with 1, 2, 3, or 4 substituent groups as described herein for the respective group (e.g., CO₂R^{A'}, where R^{A'} is selected from the group consisting of (a) C₁₋₆ alkyl, (b) C₆₋₁₀ aryl, (c) hydrogen, and (d) C₁₋₆ alk-C₆₋₁₀ aryl, e.g., carboxy, and/or an *N*-protecting group, and/or an *O-*protecting group).

The "haloalkyl" group, which as used herein, represents an alkyl group, as defined herein, substituted with a halogen group (i.e., F, Cl, Br, or I). A haloalkyl may be substituted with one, two, three, or, in the case of alkyl groups of two carbons or more, four halogens. Haloalkyl groups include perfluoroalkyls (e.g., -CF₃), -CHF₂, -CH₂F, -CCl₃, -CH₂CH₂Br, -CH₂CH(CH₂CH₂Br)CH₃, and -CHICH₃. In some cases, the haloalkyl group can be further substituted with 1, 2, 3, or 4 substituent groups as described herein for alkyl groups.

The "hydroxyalkyl" group, which as used herein, represents an alkyl group, as defined herein, substituted with one to three hydroxy groups, with the proviso that no more than one hydroxy group may be attached to a single carbon atom of the alkyl group, and is exemplified by hydroxymethyl, dihydroxypropyl, and the like. In some cases, the hydroxyalkyl group can be substituted with 1, 2, 3, or 4 substituent groups (e.g., *O*-protecting groups) as defined herein for an alkyl.

The "perfluoroalkyl" group, which as used herein, represents an alkyl group, as defined herein, where each hydrogen radical bound to the alkyl group has been replaced by a fluoride radical. Perfluoroalkyl groups are exemplified by trifluoromethyl, pentafluoroethyl, and the like.

The "sulfoalkyl" group, which as used herein, represents an alkyl group, as cases defined herein, substituted with a sulfo group of -SO₃H. In some cases, the alkyl group can be further substituted with 1, 2, 3, or 4 substituent groups as described herein, and the sulfo group can be further substituted with one or more *O*-protecting groups (e.g., as described herein).

The term "alkenyl," as used herein, represents monovalent straight or branched chain groups of, unless otherwise specified, from 2 to 20 carbons (e.g., from 2 to 6 or from 2 to 10 carbons) containing one or more carbon-carbon double bonds and is exemplified by ethenyl, 1-propenyl, 2-propenyl, 2-methyl-1-propenyl, 1-butenyl, 2-butenyl, and the like. Alkenyls include both cis and trans isomers. Alkenyl groups may be optionally substituted with 1, 2, 3, or 4 substituent groups that are selected, independently, from amino, aryl, cycloalkyl, or heterocyclyl (e.g., heteroaryl), as defined herein, or any of the exemplary alkyl substituent groups described herein.

Non-limiting examples of optionally substituted alkenyl groups include, alkoxycarbonylalkenyl, aminoalkenyl, and hydroxyalkenyl:

The "alkoxycarbonylalkenyl" group, which as used herein, represents an alkenyl group, as defined herein, that is substituted with an alkoxycarbonyl group, as defined herein (e.g., -alkenyl-C(O)-OR, where R is an optionally substituted C₁₋₂₀, C₁₋₁₀, or C₁₋₆ alkyl group). Exemplary unsubstituted alkoxycarbonylalkenyl include from 4 to 41 carbons (e.g., from 4 to 10, from 4 to 13, from 4 to 17, from 4 to 21, or from 4 to 31 carbons, such as C₁₋₆ alkoxycarbonyl-C₂₋₆ alkenyl, C₁₋₁₀ alkoxycarbonyl-C₂₋₁₀ alkenyl, or C₁₋₂₀ alkoxycarbonyl-C₂₋₂₀ alkenyl). In some cases, each alkyl, alkenyl, and alkoxy group is further independently substituted with 1, 2, 3, or 4 substituents as described herein (e.g., a hydroxy group).

The "aminoalkenyl" group, which as used herein, represents an alkenyl group, as defined herein, substituted with an amino group, as defined herein. The alkenyl and amino each can be further substituted with 1, 2, 3, or 4 substituent groups as described herein for the respective group (e.g., CO₂R^{A'}, where R^{A'} is selected from the group consisting of (a) C₁₋₆ alkyl, (b) C₆₋₁₀ aryl, (c) hydrogen, and (d) C₁₋₆ alk-C₆₋₁₀ aryl, e.g., carboxy, and/or an *N*-protecting group).

The "hydroxyalkenyl" group, which as used herein, represents an alkenyl group, as defined herein, substituted with one to three hydroxy groups, with the proviso that no more than one hydroxy group may be attached to a single carbon atom of the alkyl group, and is exemplified by dihydroxypropenyl, hydroxyisopentenyl, and the like. In some cases, the hydroxyalkenyl group can be substituted with 1, 2, 3, or 4 substituent groups (e.g., *O*-protecting groups) as defined herein for an alkyl.

The term "alkynyl," as used herein, represents monovalent straight or branched chain groups from 2 to 20 carbon atoms (e.g., from 2 to 4, from 2 to 6, or from 2 to 10 carbons) containing a carbon-carbon triple bond and is exemplified by ethynyl, 1-propynyl, and the like. Alkynyl groups may be optionally substituted with 1, 2, 3, or 4 substituent groups that are selected, independently, from aryl, cycloalkyl, or heterocyclyl (e.g., heteroaryl), as defined herein, or any of the exemplary alkyl substituent groups described herein.

Non-limiting examples of optionally substituted alkynyl groups include alkoxycarbonylalkynyl, aminoalkynyl, and hydroxyalkynyl:

The "alkoxycarbonylalkynyl" group, which as used herein, represents an alkynyl group, as defined herein, that is substituted with an alkoxycarbonyl group, as defined herein (e.g., -alkynyl-C(O)-OR, where R is an optionally substituted C₁₋₂₀, C₁₋₁₀, or C₁₋₆ alkyl group). Exemplary unsubstituted alkoxycarbonylalkynyl include from 4 to 41 carbons (e.g., from 4 to 10, from 4 to 13, from 4 to 17, from 4 to 21, or from 4 to 31 carbons, such as C₁₋₆ alkoxycarbonyl-C₂₋₆ alkynyl, C₁₋₁₀ alkoxycarbonyl-C₂₋₁₀ alkynyl, or C₁₋₂₀ alkoxycarbonyl-C₂₋₂₀ alkynyl). In some cases, each alkyl, alkynyl, and alkoxy group is further independently substituted with 1, 2, 3, or 4 substituents as described herein (e.g., a hydroxy group).

The "aminoalkynyl" group, which as used herein, represents an alkynyl group, as defined herein, substituted with an amino group, as defined herein. The alkynyl and amino each can be further substituted with 1, 2, 3, or 4 substituent groups as described herein for the respective group (e.g., CO₂R^{A'}, where R^{A'} is selected from the group consisting of (a) C₁₋₆ alkyl, (b) C₆₋₁₀ aryl, (c) hydrogen, and (d) C₁₋₆ alk-C₆₋₁₀ aryl, e.g., carboxy, and/or an *N*-protecting group).

The "hydroxyalkynyl" group, which as used herein, represents an alkynyl group, as defined herein, substituted with one to three hydroxy groups, with the proviso that no more than one hydroxy group may be attached to a single carbon atom of the alkyl group. In some cases, the hydroxyalkynyl group can be substituted with 1, 2, 3, or 4 substituent groups (e.g., *O*-protecting groups) as defined herein for an alkyl.

The term "amino," as used herein, represents -N(R^{N1})₂, wherein each R^{N1} is, independently, H, OH, NO₂, N(R^{N2})₂, SO₂OR^{N2}, SO₂R^{N2}, SOR^{N2}, an *N*-protecting group, alkyl, alkenyl, alkynyl, alkoxy, aryl, alkaryl, cycloalkyl, alkcycloalkyl, carboxyalkyl (e.g., optionally substituted with an *O*-protecting group, such as optionally substituted arylalkoxycarbonyl groups or any described herein), sulfoalkyl, acyl (e.g., acetyl, trifluoroacetyl, or others described herein), alkoxycarbonylalkyl (e.g., optionally substituted with an *O*-protecting group, such as optionally substituted arylalkoxycarbonyl groups or any described herein), heterocyclyl (e.g., heteroaryl), or alkheterocyclyl (e.g., alkheteroaryl), wherein each of these recited R^{N1} groups can be optionally substituted, as defined herein for each group; or two R^{N1} combine to form a heterocyclyl or an *N*-protecting group, and wherein each R^{N2} is, independently, H, alkyl, or aryl. The amino groups disclosure can be an unsubstituted amino (i.e., -NH₂) or a substituted amino (i.e., -N(RN¹)₂). In a preferred case, amino is - NH₂ or -NHR^{N1}, wherein R^{N1} is, independently, OH, NO₂, NH₂, NR^{N2}₂, SO₂OR^{N2}, SO₂R^{N2}, SOR^{N2}, alkyl, carboxyalkyl, sulfoalkyl, acyl (e.g., acetyl, trifluoroacetyl, or others described herein), alkoxycarbonylalkyl (e.g., t-butoxycarbonylalkyl) or aryl, and each R^{N2} can be H, C₁₋₂₀ alkyl (e.g., C₁₋₆ alkyl), or C₆₋₁₀ aryl.

Non-limiting examples of optionally substituted amino groups include acylamino and carbamyl:

The "acylamino" group, which as used herein, represents an acyl group, as defined herein, attached to the parent molecular group though an amino group, as defined herein (i.e., -N(R^{N1})-C(O)-R, where R is H or an optionally substituted C₁₋₆, C₁₋₁₀, or C₁₋₂₀ alkyl group (e.g., haloalkyl) and R^{N1} is as defined herein). Exemplary unsubstituted acylamino groups include from 1 to 41 carbons (e.g., from 1 to 7, from 1 to 13, from 1 to 21, from 2 to 7, from 2 to 13, from 2 to 21, or from 2 to 41 carbons). In some cases, the alkyl group is further substituted with 1, 2, 3, or 4 substituents as described herein, and/or the amino group is -NH₂ or -NHR^{N1}, wherein R^{N1} is, independently, OH, NO₂, NH₂, NR^{N2}₂, SO₂OR^{N2}, SO₂R^{N2}, SOR^{N2}, alkyl, aryl, acyl (e.g., acetyl, trifluoroacetyl, or others described herein), or alkoxycarbonylalkyl, and each R^{N2} can be H, alkyl, or aryl.

The "carbamyl" group, which as used herein, refers to a carbamate group having the structure -NR^{N1}C(=O)OR or -OC(=O)N(R^{N1})₂, where the meaning of each R^{N1} is found in the definition of "amino" described herein, and R is alkyl, cycloalkyl, alkcycloalkyl, aryl, alkaryl, heterocyclyl (e.g., heteroaryl), or alkheterocyclyl (e.g., alkheteroaryl), as defined herein.

The term "amino acid," as described herein, refers to a molecule having a side chain, an amino group, and an acid group (e.g., a carboxy group of -CO₂H or a sulfo group of -SO₃H), wherein the amino acid is attached to the parent molecular group by the side chain, amino group, or acid group (e.g., the side chain). In some cases, the amino acid is attached to the parent molecular group by a carbonyl group, where the side chain or amino group is attached to the carbonyl group. Exemplary side chains include an optionally substituted alkyl, aryl, heterocyclyl, alkaryl, alkheterocyclyl, aminoalkyl, carbamoylalkyl, and carboxyalkyl. Exemplary amino acids include alanine, arginine, asparagine, aspartic acid, cysteine, glutamic acid, glutamine, glycine, histidine, hydroxynorvaline, isoleucine, leucine, lysine, methionine, norvaline, ornithine, phenylalanine, proline, pyrrolysine, selenocysteine, serine, taurine, threonine, tryptophan, tyrosine, and valine. Amino acid groups may be optionally substituted with one, two, three, or, in the case of amino acid groups of two carbons or more, four substituents independently selected from the group consisting of: (1) C₁₋₆ alkoxy; (2) C₁₋₆ alkylsulfinyl; (3) amino, as defined herein (e.g., unsubstituted amino (i.e., -NH₂) or a substituted amino (i.e., -N(R^{N1})₂, where R^{N1} is as defined for amino); (4) C₆₋₁₀ aryl-C₁₋₆ alkoxy; (5) azido; (6) halo; (7) (C₂₋₉ heterocyclyl)oxy; (8) hydroxy; (9) nitro; (10) oxo (e.g., carboxyaldehyde or acyl); (11) C₁₋₇ spirocyclyl; (12) thioalkoxy; (13) thiol; (14) -CO₂R^{A'}, where R^{A'} is selected from the group consisting of (a) C₁₋₂₀ alkyl (e.g., C₁₋₆ alkyl), (b) C₂₋₂₀ alkenyl (e.g., C₂₋₆ alkenyl), (c) C₆₋₁₀ aryl, (d) hydrogen, (e) C₁₋₆ alk-C₆₋₁₀ aryl, (f) amino-C₁₋₂₀ alkyl, (g) polyethylene glycol of -(CH₂)ₛ₂(OCH₂CH₂)ₛ₁(CH₂)ₛ₃OR', wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and R' is H or C₁₋₂₀ alkyl, and (h) amino-polyethylene glycol of - NR^{N1}(CH₂)ₛ₂(CH₂CH₂O)ₛ₁(CH₂)ₛ₃NR^{N1}, wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and each R^{N1} is, independently, hydrogen or optionally substituted C₁₋₆ alkyl; (15) -C(O)NR^{B'}R^{C'}, where each of R^{B'} and R^{C'} is, independently, selected from the group consisting of (a) hydrogen, (b) C₁₋₆ alkyl, (c) C₆₋₁₀ aryl, and (d) C₁₋₆ alk-C₆₋₁₀ aryl; (16) -SO₂R^{D'}, where R^{D'} is selected from the group consisting of (a) C₁₋₆ alkyl, (b) C₆₋₁₀ aryl, (c) C₁₋₆ alk-C₆₋₁₀ aryl, and (d) hydroxy; (17) -SO₂NR^{E'}R^{F'}, where each of R^{E'} and R^{F'} is, independently, selected from the group consisting of (a) hydrogen, (b) C₁₋₆ alkyl, (c) C₆₋₁₀ aryl and (d) C₁₋₆ alk-C₆₋₁₀ aryl; (18) -C(O)R^{G'}, where R^{G'} is selected from the group consisting of (a) C₁₋₂₀ alkyl (e.g., C₁₋₆ alkyl), (b) C₂₋₂₀ alkenyl (e.g., C₂₋₆ alkenyl), (c) C₆₋₁₀ aryl, (d) hydrogen, (e) C₁₋₆ alk-C₆₋₁₀ aryl, (f) amino-C₁₋₂₀ alkyl, (g) polyethylene glycol of -(CH₂)ₛ₂(OCH₂CH₂)ₛ₁(CH₂)ₛ₃OR', wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and R' is H or C₁₋₂₀ alkyl, and (h) amino-polyethylene glycol of -NR^{N1}(CH₂)ₛ₂(CH₂CH₂O)ₛ₁(CH₂)ₛ₃NR^{N1}, wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and each R^{N1} is, independently, hydrogen or optionally substituted C₁₋₆ alkyl; (19) - NR^{H'}C(O)R^{I'}, wherein R^{H'} is selected from the group consisting of (a1) hydrogen and (b1) C₁₋₆ alkyl, and R^{I'} is selected from the group consisting of (a2) C₁₋₂₀ alkyl (e.g., C₁₋₆ alkyl), (b2) C₂₋₂₀ alkenyl (e.g., C₂₋₆ alkenyl), (c2) C₆₋₁₀ aryl, (d2) hydrogen, (e2) C₁₋₆ alk-C₆₋₁₀ aryl, (f2) amino-C₁₋₂₀ alkyl, (g2) polyethylene glycol of - (CH₂)ₛ₂(OCH₂CH₂)ₛ₁(CH₂)ₛ₃OR', wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and R' is H or C₁₋₂₀ alkyl, and (h2) amino-polyethylene glycol of -NR^{N1}(CH₂)ₛ₂(CH₂CH₂O)ₛ₁(CH₂)ₛ₃NR^{N1}, wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and each R^{N1} is, independently, hydrogen or optionally substituted C₁₋₆ alkyl; (20) -NR^{J'}C(O)OR^{K'}, wherein R^{J'} is selected from the group consisting of (a1) hydrogen and (b1) C₁₋₆ alkyl, and R^{K'} is selected from the group consisting of (a2) C₁₋₂₀ alkyl (e.g., C₁₋₆ alkyl), (b2) C₂₋₂₀ alkenyl (e.g., C₂₋₆ alkenyl), (c2) C₆₋₁₀ aryl, (d2) hydrogen, (e2) C₁₋₆ alk-C₆₋₁₀ aryl, (f2) amino-C₁₋₂₀ alkyl, (g2) polyethylene glycol of -(CH₂)ₛ₂(OCH₂CH₂)ₛ₁(CH₂)ₛ₃OR', wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and R' is H or C₁₋₂₀ alkyl, and (h2) amino-polyethylene glycol of - NR^{N1}(CH₂)ₛ₂(CH₂CH₂O)ₛ₁(CH₂)ₛ₃NR^{N1}, wherein s1 is an integer from 1 to 10 (e.g., from 1 to 6 or from 1 to 4), each of s2 and s3, independently, is an integer from 0 to 10 (e.g., from 0 to 4, from 0 to 6, from 1 to 4, from 1 to 6, or from 1 to 10), and each R^{N1} is, independently, hydrogen or optionally substituted C₁₋₆ alkyl; and (21) amidine. In some cases, each of these groups can be further substituted as described herein.

The term "aryl," as used herein, represents a mono-, bicyclic, or multicyclic carbocyclic ring system having one or two aromatic rings and is exemplified by phenyl, naphthyl, 1,2-dihydronaphthyl, 1,2,3,4-tetrahydronaphthyl, anthracenyl, phenanthrenyl, fluorenyl, indanyl, indenyl, and the like, and may be optionally substituted with 1, 2, 3, 4, or 5 substituents independently selected from the group consisting of: (1) C₁₋₇ acyl (e.g., carboxyaldehyde); (2) C₁₋₂₀ alkyl (e.g., C₁₋₆ alkyl, C₁₋₆ alkoxy-C₁₋₆ alkyl, C₁₋₆ alkylsulfinyl-C₁₋₆ alkyl, amino-C₁₋₆ alkyl, azido-C₁₋₆ alkyl, (carboxyaldehyde)-C₁₋₆ alkyl, halo-C₁₋₆ alkyl (e.g., perfluoroalkyl), hydroxy-Ci-₆ alkyl, nitro-C₁₋₆ alkyl, or C₁₋₆ thioalkoxy-C₁₋₆ alkyl); (3) C₁₋₂₀ alkoxy (e.g., C₁₋₆ alkoxy, such as perfluoroalkoxy); (4) C₁₋₆ alkylsulfinyl; (5) C₆₋₁₀ aryl; (6) amino; (7) C₁₋₆ alk-C₆₋₁₀ aryl; (8) azido; (9) C₃₋₈ cycloalkyl; (10) C₁₋₆ alk-C₃₋₈ cycloalkyl; (11) halo; (12) C₁₋₁₂ heterocyclyl (e.g., C₁₋₁₂ heteroaryl); (13) (C₁₋₁₂ heterocyclyl)oxy; (14) hydroxy; (15) nitro; (16) C₁₋₂₀ thioalkoxy (e.g., C₁₋₆ thioalkoxy); (17) -(CH₂)_{q}CO₂R^{A'}, where q is an integer from zero to four, and R^{A'} is selected from the group consisting of (a) C₁₋₆ alkyl, (b) C₆₋₁₀ aryl, (c) hydrogen, and (d) C₁₋₆ alk-C₆₋₁₀ aryl; (18) - (CH₂)_{q}CONR^{B'}R^{C'}, where q is an integer from zero to four and where R^{B'} and R^{C'} are independently selected from the group consisting of (a) hydrogen, (b) C₁₋₆ alkyl, (c) C₆₋₁₀ aryl, and (d) C₁₋₆ alk-C₆₋₁₀ aryl; (19) -(CH₂)_{q}SO₂R^{D'}, where q is an integer from zero to four and where R^{D'} is selected from the group consisting of (a) alkyl, (b) C₆₋₁₀ aryl, and (c) alk-C₆₋₁₀ aryl; (20) -(CH₂)_{q}SO₂NR^{E'}R^{F'}, where q is an integer from zero to four and where each of R^{E'} and R^{F'} is, independently, selected from the group consisting of (a) hydrogen, (b) C₁₋₆ alkyl, (c) C₆₋₁₀ aryl, and (d) C₁₋₆ alk-C₆₋₁₀ aryl; (21) thiol; (22) C₆₋₁₀ aryloxy; (23) C₃₋₈ cycloalkoxy; (24) C₆₋₁₀ aryl-C₁₋₆ alkoxy; (25) C₁₋₆ alk-C₁₋₁₂ heterocyclyl (e.g., C₁₋₆ alk-C₁₋₁₂ heteroaryl); (26) C₂₋₂₀ alkenyl; and (27) C₂₋₂₀ alkynyl. In some cases, each of these groups can be further substituted as described herein. For example, the alkylene group of a C₁-alkaryl or a C₁-alkheterocyclyl can be further substituted with an oxo group to afford the respective aryloyl and (heterocyclyl)oyl substituent group.

The "arylalkyl" group, which as used herein, represents an aryl group, as defined herein, attached to the parent molecular group through an alkylene group, as defined herein. Exemplary unsubstituted arylalkyl groups are from 7 to 30 carbons (e.g., from 7 to 16 or from 7 to 20 carbons, such as C₁₋₆ alk-C₆₋₁₀ aryl, C₁₋₁₀ alk-C₆₋₁₀ aryl, or C₁₋₂₀ alk-C₆₋₁₀ aryl). In some cases, the alkylene and the aryl each can be further substituted with 1, 2, 3, or 4 substituent groups as defined herein for the respective groups. Other groups preceded by the prefix "alk-" are defined in the same manner, where "alk" refers to a C₁₋₆ alkylene, unless otherwise noted, and the attached chemical structure is as defined herein.

The term "azido" represents an -N₃ group, which can also be represented as -N=N=N.

The term "bicyclic," as used herein, refer to a structure having two rings, which may be aromatic or non-aromatic. Bicyclic structures include spirocyclyl groups, as defined herein, and two rings that share one or more bridges, where such bridges can include one atom or a chain including two, three, or more atoms. Exemplary bicyclic groups include a bicyclic carbocyclyl group, where the first and second rings are carbocyclyl groups, as defined herein; a bicyclic aryl groups, where the first and second rings are aryl groups, as defined herein; bicyclic heterocyclyl groups, where the first ring is a heterocyclyl group and the second ring is a carbocyclyl (e.g., aryl) or heterocyclyl (e.g., heteroaryl) group; and bicyclic heteroaryl groups, where the first ring is a heteroaryl group and the second ring is a carbocyclyl (e.g., aryl) or heterocyclyl (e.g., heteroaryl) group. In some cases, the bicyclic group can be substituted with 1, 2, 3, or 4 substituents as defined herein for cycloalkyl, heterocyclyl, and aryl groups.

The term "boranyl," as used herein, represents -B(R^{B1})₃, where each R^{B1} is, independently, selected from the group consisting of H and optionally substituted alkyl. In some cases, the boranyl group can be substituted with 1, 2, 3, or 4 substituents as defined herein for alkyl.

The terms "carbocyclic" and "carbocyclyl," as used herein, refer to an optionally substituted C₃₋₁₂ monocyclic, bicyclic, or tricyclic structure in which the rings, which may be aromatic or non-aromatic, are formed by carbon atoms. Carbocyclic structures include cycloalkyl, cycloalkenyl, cycloalkynyl, and aryl groups.

The term "carbonyl," as used herein, represents a C(O) group, which can also be represented as C=O.

The term "carboxy," as used herein, means -CO₂H.

The term "cyano," as used herein, represents an -CN group.

The term "cycloalkyl," as used herein represents a monovalent saturated or unsaturated non-aromatic cyclic hydrocarbon group from three to eight carbons, unless otherwise specified, and is exemplified by cyclopropyl, cyclobutyl, cyclopentyl, cyclohexyl, cycloheptyl, bicycle heptyl, and the like. When what would otherwise be a cycloalkyl group includes one or more carbon-carbon double bonds, the group is referred to as a "cycloalkenyl" group. For the purposes of this disclosure, cycloalkenyl excludes aryl groups. When what would otherwise be a cycloalkyl group includes one or more carbon-carbon triple bonds, the group is referred to as a "cycloalkynyl" group. Exemplary cycloalkenyl groups include cyclopentenyl, cyclohexenyl, and the like. The cycloalkyl groups can be optionally substituted with: (1) C₁₋₇ acyl (e.g., carboxyaldehyde); (2) C₁₋₂₀ alkyl (e.g., C₁₋₆ alkyl, C₁₋₆ alkoxy-C₁₋₆ alkyl, C₁₋₆ alkylsulfinyl-C₁₋₆ alkyl, amino-C₁₋₆ alkyl, azido-C₁₋₆ alkyl, (carboxyaldehyde)-C₁₋₆ alkyl, halo-C₁₋₆ alkyl (e.g., perfluoroalkyl), hydroxy-C₁₋₆ alkyl, nitro-C₁₋₆ alkyl, or C₁₋₆ thioalkoxy-C₁₋₆ alkyl); (3) C₁₋₂₀ alkoxy (e.g., C₁₋₆ alkoxy, such as perfluoroalkoxy); (4) C₁₋₆ alkylsulfinyl; (5) C₆₋₁₀ aryl; (6) amino; (7) C₁₋₆ alk-C₆₋₁₀ aryl; (8) azido; (9) C₃₋₈ cycloalkyl; (10) C₁₋₆ alk-C₃₋₈ cycloalkyl; (11) halo; (12) C₁₋₁₂ heterocyclyl (e.g., C₁₋₁₂ heteroaryl); (13) (C₁₋₁₂ heterocyclyl)oxy; (14) hydroxy; (15) nitro; (16) C₁₋₂₀ thioalkoxy (e.g., C₁₋₆ thioalkoxy); (17) -(CH₂)_{q}CO₂R^{A'}, where q is an integer from zero to four, and R^{A'} is selected from the group consisting of (a) C₁₋₆ alkyl, (b) C₆₋₁₀ aryl, (c) hydrogen, and (d) C₁₋₆ alk-C₆₋₁₀ aryl; (18) - (CH₂)_{q}CONR^{B'}R^{C'}, where q is an integer from zero to four and where R^{B'} and R^{C'} are independently selected from the group consisting of (a) hydrogen, (b) C₆₋₁₀ alkyl, (c) C₆₋₁₀ aryl, and (d) C₁₋₆ alk-C₆₋₁₀ aryl; (19) -(CH₂)_{q}SO₂R^{D'}, where q is an integer from zero to four and where R^{D'} is selected from the group consisting of (a) C₆₋₁₀ alkyl, (b) C₆₋₁₀ aryl, and (c) C₁₋₆ alk-C₆₋₁₀ aryl; (20) -(CH₂)_{q}SO₂NR^{E'}R^{F'}, where q is an integer from zero to four and where each of R^{E'} and R^{F'} is, independently, selected from the group consisting of (a) hydrogen, (b) C₆₋₁₀ alkyl, (c) C₆₋₁₀ aryl, and (d) C₁₋₆ alk-C₆₋₁₀ aryl; (21) thiol; (22) C₆₋₁₀ aryloxy; (23) C₃₋₈ cycloalkoxy; (24) C₆₋₁₀ aryl-C₁₋₆ alkoxy; (25) C₁₋₆ alk-C₁₋₁₂ heterocyclyl (e.g., C₁₋₆ alk-C₁₋₁₂ heteroaryl); (26) oxo; (27) C₂₋₂₀ alkenyl; and (28) C₂₋₂₀ alkynyl. In some cases, each of these groups can be further substituted as described herein. For example, the alkylene group of a C₁-alkaryl or a C₁-alkheterocyclyl can be further substituted with an oxo group to afford the respective aryloyl and (heterocyclyl)oyl substituent group.

The "cycloalkylalkyl" group, which as used herein, represents a cycloalkyl group, as defined herein, attached to the parent molecular group through an alkylene group, as defined herein (e.g., an alkylene group of from 1 to 4, from 1 to 6, from 1 to 10, or form 1 to 20 carbons). In some cases, the alkylene and the cycloalkyl each can be further substituted with 1, 2, 3, or 4 substituent groups as defined herein for the respective group.

The term "diastereomer," as used herein means stereoisomers that are not mirror images of one another and are non-superimposable on one another.

The term "enantiomer," as used herein, means each individual optically active form of a compound of the disclosure, having an optical purity or enantiomeric excess (as determined by methods standard in the art) of at least 80% (i.e., at least 90% of one enantiomer and at most 10% of the other enantiomer), preferably at least 90% and more preferably at least 98%.

The term "halo," as used herein, represents a halogen selected from bromine, chlorine, iodine, or fluorine.

The term "heteroalkyl," as used herein, refers to an alkyl group, as defined herein, in which one or two of the constituent carbon atoms have each been replaced by nitrogen, oxygen, or sulfur. In some cases, the heteroalkyl group can be further substituted with 1, 2, 3, or 4 substituent groups as described herein for alkyl groups. The terms "heteroalkenyl" and heteroalkynyl," as used herein refer to alkenyl and alkynyl groups, as defined herein, respectively, in which one or two of the constituent carbon atoms have each been replaced by nitrogen, oxygen, or sulfur. In some cases, the heteroalkenyl and heteroalkynyl groups can be further substituted with 1, 2, 3, or 4 substituent groups as described herein for alkyl groups.

Non-limiting examples of optionally substituted heteroalkyl, heteroalkenyl, and heteroalkynyl groups include acyloxy, alkenyloxy, alkoxy, alkoxyalkoxy, alkoxycarbonylalkoxy, alkynyloxy, aminoalkoxy, arylalkoxy, carboxyalkoxy, cycloalkoxy, haloalkoxy, (heterocyclyl)oxy, perfluoroalkoxy, thioalkoxy, and thioheterocyclylalkyl:

The "acyloxy" group, which as used herein, represents an acyl group, as defined herein, attached to the parent molecular group though an oxygen atom (i.e., - O-C(O)-R, where R is H or an optionally substituted C₁₋₆, C₁₋₁₀, or C₁₋₂₀ alkyl group). Exemplary unsubstituted acyloxy groups include from 1 to 21 carbons (e.g., from 1 to 7 or from 1 to 11 carbons). In some cases, the alkyl group is further substituted with 1, 2, 3, or 4 substituents as described herein.

The "alkenyloxy" group, which as used here, represents a chemical substituent of formula -OR, where R is a C₂₋₂₀ alkenyl group (e.g., C₂₋₆ or C₂₋₁₀ alkenyl), unless otherwise specified. Exemplary alkenyloxy groups include ethenyloxy, propenyloxy, and the like. In some cases, the alkenyl group can be further substituted with 1, 2, 3, or 4 substituent groups as defined herein (e.g., a hydroxy group).

The "alkoxy" group, which as used herein, represents a chemical substituent of formula -OR, where R is a C₁₋₂₀ alkyl group (e.g., C₁₋₆ or C₁₋₁₀ alkyl), unless otherwise specified. Exemplary alkoxy groups include methoxy, ethoxy, propoxy (e.g., n-propoxy and isopropoxy), t-butoxy, and the like. In some cases, the alkyl group can be further substituted with 1, 2, 3, or 4 substituent groups as defined herein (e.g., hydroxy or alkoxy).

The "alkoxyalkoxy" group, which as used herein, represents an alkoxy group that is substituted with an alkoxy group. Exemplary unsubstituted alkoxyalkoxy groups include between 2 to 40 carbons (e.g., from 2 to 12 or from 2 to 20 carbons, such as C₁₋₆ alkoxy-C₁₋₆ alkoxy, C₁₋₁₀ alkoxy-C₁₋₁₀ alkoxy, or C₁₋₂₀ alkoxy-C₁₋₂₀ alkoxy). In some cases, the each alkoxy group can be further substituted with 1, 2, 3, or 4 substituent groups as defined herein.

The "alkoxycarbonylalkoxy" group, which as used herein, represents an alkoxy group, as defined herein, that is substituted with an alkoxycarbonyl group, as defined herein (e.g., -O-alkyl-C(O)-OR, where R is an optionally substituted C₁₋₆, C₁₋₁₀, or C₁₋₂₀ alkyl group). Exemplary unsubstituted alkoxycarbonylalkoxy include from 3 to 41 carbons (e.g., from 3 to 10, from 3 to 13, from 3 to 17, from 3 to 21, or from 3 to 31 carbons, such as C₁₋₆ alkoxycarbonyl-C₁₋₆ alkoxy, C₁₋₁₀ alkoxycarbonyl-C₁₋₁₀ alkoxy, or C₁₋₂₀ alkoxycarbonyl-C₁₋₂₀ alkoxy). In some cases, each alkoxy group is further independently substituted with 1, 2, 3, or 4 substituents, as described herein (e.g., a hydroxy group).

The "alkynyloxy" group, which as used herein, represents a chemical substituent of formula -OR, where R is a C₂₋₂₀ alkynyl group (e.g., C₂₋₆ or C₂₋₁₀ alkynyl), unless otherwise specified. Exemplary alkynyloxy groups include ethynyloxy, propynyloxy, and the like. In some cases, the alkynyl group can be further substituted with 1, 2, 3, or 4 substituent groups as defined herein (e.g., a hydroxy group).

The "aminoalkoxy" group, which as used herein, represents an alkoxy group, as defined herein, substituted with an amino group, as defined herein. The alkyl and amino each can be further substituted with 1, 2, 3, or 4 substituent groups as described herein for the respective group (e.g., CO₂R^{A'}, where R^{A'} is selected from the group consisting of (a) C₁₋₆ alkyl, (b) C₆₋₁₀ aryl, (c) hydrogen, and (d) C₁₋₆ alk-C₆₋₁₀ aryl, e.g., carboxy).

The "arylalkoxy" group, which as used herein, represents an alkaryl group, as defined herein, attached to the parent molecular group through an oxygen atom. Exemplary unsubstituted arylalkoxy groups include from 7 to 30 carbons (e.g., from 7 to 16 or from 7 to 20 carbons, such as C₆₋₁₀ aryl-C₁₋₆ alkoxy, C₆₋₁₀ aryl-C₁₋₁₀ alkoxy, or C₆₋₁₀ aryl-C₁₋₂₀ alkoxy). In some cases, the arylalkoxy group can be substituted with 1, 2, 3, or 4 substituents as defined herein.

The "aryloxy" group, which as used herein, represents a chemical substituent of formula -OR', where R' is an aryl group of 6 to 18 carbons, unless otherwise specified. In some cases, the aryl group can be substituted with 1, 2, 3, or 4 substituents as defined herein.

The "carboxyalkoxy" group, which as used herein, represents an alkoxy group, as defined herein, substituted with a carboxy group, as defined herein. The alkoxy group can be further substituted with 1, 2, 3, or 4 substituent groups as described herein for the alkyl group, and the carboxy group can be optionally substituted with one or more *O*-protecting groups.

The "cycloalkoxy" group, which as used herein, represents a chemical substituent of formula -OR, where R is a C₃₋₈ cycloalkyl group, as defined herein, unless otherwise specified. The cycloalkyl group can be further substituted with 1, 2, 3, or 4 substituent groups as described herein. Exemplary unsubstituted cycloalkoxy groups are from 3 to 8 carbons. In some case, the cycloalkyl group can be further substituted with 1, 2, 3, or 4 substituent groups as described herein.

The "haloalkoxy" group, which as used herein, represents an alkoxy group, as defined herein, substituted with a halogen group (i.e., F, Cl, Br, or I). A haloalkoxy may be substituted with one, two, three, or, in the case of alkyl groups of two carbons or more, four halogens. Haloalkoxy groups include perfluoroalkoxys (e.g., -OCF₃), -OCHF₂, -OCH₂F, -OCCl₃, -OCH₂CH₂Br, -OCH₂CH(CH₂CH₂Br)CH₃, and -OCHICH₃. In some cases, the haloalkoxy group can be further substituted with 1, 2, 3, or 4 substituent groups as described herein for alkyl groups.

The "(heterocyclyl)oxy" group, which as used herein, represents a heterocyclyl group, as defined herein, attached to the parent molecular group through an oxygen atom. In some cases, the heterocyclyl group can be substituted with 1, 2, 3, or 4 substituent groups as defined herein.

The "perfluoroalkoxy" group, which as used herein, represents an alkoxy group, as defined herein, where each hydrogen radical bound to the alkoxy group has been replaced by a fluoride radical. Perfluoroalkoxy groups are exemplified by trifluoromethoxy, pentafluoroethoxy, and the like.

The "alkylsulfinyl" group, which as used herein, represents an alkyl group attached to the parent molecular group through an -S(O)- group. Exemplary unsubstituted alkylsulfinyl groups are from 1 to 6, from 1 to 10, or from 1 to 20 carbons. In some cases, the alkyl group can be further substituted with 1, 2, 3, or 4 substituent groups as defined herein.

The "thioarylalkyl" group, which as used herein, represents a chemical substituent of formula -SR, where R is an arylalkyl group. In some cases, the arylalkyl group can be further substituted with 1, 2, 3, or 4 substituent groups as described herein.

The "thioalkoxy" group as used herein, represents a chemical substituent of formula -SR, where R is an alkyl group, as defined herein. In some cases, the alkyl group can be further substituted with 1, 2, 3, or 4 substituent groups as described herein.

The "thioheterocyclylalkyl" group, which as used herein, represents a chemical substituent of formula -SR, where R is an heterocyclylalkyl group. In some cases, the heterocyclylalkyl group can be further substituted with 1, 2, 3, or 4 substituent groups as described herein.

The term "heteroaryl," as used herein, represents that subset of heterocyclyls, as defined herein, which are aromatic: i.e., they contain 4*n*+2 pi electrons within the mono- or multicyclic ring system. Exemplary unsubstituted heteroaryl groups are of 1 to 12 (e.g., 1 to 11, 1 to 10, 1 to 9, 2 to 12, 2 to 11, 2 to 10, or 2 to 9) carbons. In some case, the heteroaryl is substituted with 1, 2, 3, or 4 substituents groups as defined for a heterocyclyl group.

The term "heteroarylalkyl" refers to a heteroaryl group, as defined herein, attached to the parent molecular group through an alkylene group, as defined herein. Exemplary unsubstituted heteroarylalkyl groups are from 2 to 32 carbons (e.g., from 2 to 22, from 2 to 18, from 2 to 17, from 2 to 16, from 3 to 15, from 2 to 14, from 2 to 13, or from 2 to 12 carbons, such as C₁₋₆ alk-C₁₋₁₂ heteroaryl, C₁₋₁₀ alk-C₁₋₁₂ heteroaryl, or C₁₋₂₀ alk-C₁₋₁₂ heteroaryl). In some cases, the alkylene and the
heteroaryl each can be further substituted with 1, 2, 3, or 4 substituent groups as defined herein for the respective group. Heteroarylalkyl groups are a subset of heterocyclylalkyl groups.

The term "heterocyclyl," as used herein represents a 5-, 6- or 7-membered ring, unless otherwise specified, containing one, two, three, or four heteroatoms independently selected from the group consisting of nitrogen, oxygen, and sulfur. The 5-membered ring has zero to two double bonds, and the 6- and 7-membered rings have zero to three double bonds. Exemplary unsubstituted heterocyclyl groups are of 1 to 12 (e.g., 1 to 11, 1 to 10, 1 to 9, 2 to 12, 2 to 11, 2 to 10, or 2 to 9) carbons. The term "heterocyclyl" also represents a heterocyclic compound having a bridged multicyclic structure in which one or more carbons and/or heteroatoms bridges two non-adjacent members of a monocyclic ring, e.g., a quinuclidinyl group. The term "heterocyclyl" includes bicyclic, tricyclic, and tetracyclic groups in which any of the above heterocyclic rings is fused to one, two, or three carbocyclic rings, e.g., an aryl ring, a cyclohexane ring, a cyclohexene ring, a cyclopentane ring, a cyclopentene ring, or another monocyclic heterocyclic ring, such as indolyl, quinolyl, isoquinolyl, tetrahydroquinolyl, benzofuryl, benzothienyl and the like. Examples of fused heterocyclyls include tropanes and 1,2,3,5,8,8a-hexahydroindolizine. Heterocyclics include pyrrolyl, pyrrolinyl, pyrrolidinyl, pyrazolyl, pyrazolinyl, pyrazolidinyl, imidazolyl, imidazolinyl, imidazolidinyl, pyridyl, piperidinyl, homopiperidinyl, pyrazinyl, piperazinyl, pyrimidinyl, pyridazinyl, oxazolyl, oxazolidinyl, isoxazolyl, isoxazolidiniyl, morpholinyl, thiomorpholinyl, thiazolyl, thiazolidinyl, isothiazolyl, isothiazolidinyl, indolyl, indazolyl, quinolyl, isoquinolyl, quinoxalinyl, dihydroquinoxalinyl, quinazolinyl, cinnolinyl, phthalazinyl, benzimidazolyl, benzothiazolyl, benzoxazolyl, benzothiadiazolyl, furyl, thienyl, thiazolidinyl, isothiazolyl, triazolyl, tetrazolyl, oxadiazolyl (e.g., 1,2,3-oxadiazolyl), purinyl, thiadiazolyl (e.g., 1,2,3-thiadiazolyl), tetrahydrofuranyl, dihydrofuranyl, tetrahydrothienyl, dihydrothienyl, dihydroindolyl, dihydroquinolyl, tetrahydroquinolyl, tetrahydroisoquinolyl, dihydroisoquinolyl, pyranyl, dihydropyranyl, dithiazolyl, benzofuranyl, isobenzofuranyl, benzothienyl, and the like, including dihydro and tetrahydro forms thereof, where one or more double bonds are reduced and replaced with hydrogens. Still other exemplary heterocyclyls include: 2,3,4,5-tetrahydro-2-oxo-oxazolyl; 2,3-dihydro-2-oxo-1H-imidazolyl; 2,3,4,5-tetrahydro-5-oxo-1H-pyrazolyl (e.g., 2,3,4,5-tetrahydro-2-phenyl-5-oxo-1H-pyrazolyl); 2,3,4,5-tetrahydro-2,4-dioxo-1H-imidazolyl (e.g., 2,3,4,5-tetrahydro-2,4-dioxo-5-methyl-5-phenyl-1H-imidazolyl); 2,3-dihydro-2-thioxo-1,3,4-oxadiazolyl (e.g., 2,3-dihydro-2-thioxo-5-phenyl-1,3,4-oxadiazolyl); 4,5-dihydro-5-oxo-1*H-*triazolyl (e.g., 4,5-dihydro-3-methyl-4-amino 5-oxo-1*H*-triazolyl); 1,2,3,4-tetrahydro-2,4-dioxopyridinyl (e.g., 1,2,3,4-tetrahydro-2,4-dioxo-3,3-diethylpyridinyl); 2,6-dioxo-piperidinyl (e.g., 2,6-dioxo-3-ethyl-3-phenylpiperidinyl); 1,6-dihydro-6-oxopyridiminyl; 1,6-dihydro-4-oxopyrimidinyl (e.g., 2-(methylthio)-1,6-dihydro-4-oxo-5-methylpyrimidin-1-yl); 1,2,3,4-tetrahydro-2,4-dioxopyrimidinyl (e.g., 1,2,3,4-tetrahydro-2,4-dioxo-3-ethylpyrimidinyl); 1,6-dihydro-6-oxo-pyridazinyl (e.g., 1,6-dihydro-6-oxo-3-ethylpyridazinyl); 1,6-dihydro-6-oxo-1,2,4-triazinyl (e.g., 1,6-dihydro-5-isopropyl-6-oxo-1,2,4-triazinyl); 2,3-dihydro-2-oxo-1*H*-indolyl (e.g., 3,3-dimethyl-2,3-dihydro-2-oxo-1*H*-indolyl and 2,3-dihydro-2-oxo-3,3'-spiropropane-1*H-*indol-1-yl); 1,3-dihydro-1-oxo-2*H*-iso-indolyl; 1,3-dihydro-1,3-dioxo-2*H*-iso-indolyl; 1*H*-benzopyrazolyl (e.g., 1-(ethoxycarbonyl)- 1*H*-benzopyrazolyl); 2,3-dihydro-2-oxo-1*H*-benzimidazolyl (e.g., 3-ethyl-2,3-dihydro-2-oxo-1*H*-benzimidazolyl); 2,3-dihydro-2-oxo-benzoxazolyl (e.g., 5-chloro-2,3-dihydro-2-oxo-benzoxazolyl); 2,3-dihydro-2-oxo-benzoxazolyl; 2-oxo-2H-benzopyranyl; 1,4-benzodioxanyl; 1,3-benzodioxanyl; 2,3-dihydro-3-oxo,4*H*-1,3-benzothiazinyl; 3,4-dihydro-4-oxo-3*H-*quinazolinyl (e.g., 2-methyl-3,4-dihydro-4-oxo-3*H*-quinazolinyl); 1,2,3,4-tetrahydro-2,4-dioxo-3*H*-quinazolyl (e.g., 1-ethyl-1,2,3,4-tetrahydro-2,4-dioxo-3*H*-quinazolyl); 1,2,3,6-tetrahydro-2,6-dioxo-7*H*-purinyl (e.g., 1,2,3,6-tetrahydro-1,3-dimethyl-2,6-dioxo-7 H -purinyl); 1,2,3,6-tetrahydro-2,6-dioxo-1 *H* -purinyl (e.g., 1,2,3,6-tetrahydro-3,7-dimethyl-2,6-dioxo-1 *H* -purinyl); 2-oxobenz[*c*,*d*]indolyl; 1,1-dioxo-2H-naphth[1,8-*c*,*d*]isothiazolyl; and 1,8-naphthylenedicarboxamido. Additional heterocyclics include 3,3a,4,5,6,6a-hexahydro-pyrrolo[3,4-b]pyrrol-(2H)-yl, and 2,5-diazabicyclo[2.2.1]heptan-2-yl, homopiperazinyl (or diazepanyl), tetrahydropyranyl, dithiazolyl, benzofuranyl, benzothienyl, oxepanyl, thiepanyl, azocanyl, oxecanyl, and thiocanyl. Heterocyclic groups also include groups of the formula 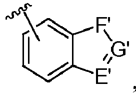 where

E' is selected from the group consisting of -N- and -CH-; F' is selected from the group consisting of -N=CH-, -NH-CH₂-, -NH-C(O)-, -NH-, -CH=N-, -CH₂-NH-, -C(O)-NH-, -CH=CH-, -CH₂-, -CH₂CH₂-, -CH₂O-, -OCH₂-, -O-, and -S-; and G' is selected from the group consisting of -CH- and -N-. Any of the heterocyclyl groups mentioned herein may be optionally substituted with one, two, three, four or five substituents independently selected from the group consisting of: (1) C₁₋₇ acyl (e.g., carboxyaldehyde); (2) C₁₋₂₀ alkyl (e.g., C₁₋₆ alkyl, C₁₋₆ alkoxy-C₁₋₆ alkyl, C₁₋₆ alkylsulfinyl-C₁₋₆ alkyl, amino-C₁₋₆ alkyl, azido-C₁₋₆ alkyl, (carboxyaldehyde)-C₁₋₆ alkyl, halo-C₁₋₆ alkyl (e.g., perfluoroalkyl), hydroxy-C₁₋₆ alkyl, nitro-C₁₋₆ alkyl, or C₁₋₆ thioalkoxy-C₁₋₆ alkyl); (3) C₁₋₂₀ alkoxy (e.g., C₁₋₆ alkoxy, such as perfluoroalkoxy); (4) C₁₋₆ alkylsulfinyl; (5) C₆₋₁₀ aryl; (6) amino; (7) C₁₋₆ alk-C₆₋₁₀ aryl; (8) azido; (9) C₃₋₈ cycloalkyl; (10) C₁₋₆ alk-C₃₋₈ cycloalkyl; (11) halo; (12) C₁₋₁₂ heterocyclyl (e.g., C₂₋₁₂ heteroaryl); (13) (C₁₋₁₂ heterocyclyl)oxy; (14) hydroxy; (15) nitro; (16) C₁₋₂₀ thioalkoxy (e.g., C₁₋₆ thioalkoxy); (17) -(CH₂)_{q}CO₂R^{A'}, where q is an integer from zero to four, and R^{A'} is selected from the group consisting of (a) C₁₋₆ alkyl, (b) C₆₋₁₀ aryl, (c) hydrogen, and (d) C₁₋₆ alk-C₆₋₁₀ aryl; (18) -(CH₂)_{q}CONR^{B'}R^{C'}, where q is an integer from zero to four and where R^{B'} and R^{C'} are independently selected from the group consisting of (a) hydrogen, (b) C₁₋₆ alkyl, (c) C₆₋₁₀ aryl, and (d) C₁₋₆ alk-C₆₋₁₀ aryl; (19) -(CH₂)_{q}SO₂R^{D'}, where q is an integer from zero to four and where R^{D'} is selected from the group consisting of (a) C₁₋₆ alkyl, (b) C₆₋₁₀ aryl, and (c) C₁₋₆ alk-C₆₋₁₀ aryl; (20) -(CH₂)_{q}SO₂NR^{E'}R^{F'}, where q is an integer from zero to four and where each of R^{E'} and R^{F'} is, independently, selected from the group consisting of (a) hydrogen, (b) C₁₋₆ alkyl, (c) C₆₋₁₀ aryl, and (d) C₁₋₆ alk-C₆₋₁₀ aryl; (21) thiol; (22) C₆₋₁₀ aryloxy; (23) C₃₋₈ cycloalkoxy; (24) arylalkoxy; (25) C₁₋₆ alk-C₁₋₁₂ heterocyclyl (e.g., C₁₋₆ alk-C₁₋₁₂ heteroaryl); (26) oxo; (27) (C₁₋₁₂ heterocyclyl)imino; (28) C₂₋₂₀ alkenyl; and (29) C₂₋₂₀ alkynyl. In some cases, each of these groups can be further substituted as described herein. For example, the alkylene group of a C₁-alkaryl or a C₁-alkheterocyclyl can be further substituted with an oxo group to afford the respective aryloyl and (heterocyclyl)oyl substituent group.

The "heterocyclylalkyl" group, which as used herein, represents a heterocyclyl group, as defined herein, attached to the parent molecular group through an alkylene group, as defined herein. Exemplary unsubstituted heterocyclylalkyl groups are from 2 to 32 carbons (e.g., from 2 to 22, from 2 to 18, from 2 to 17, from 2 to 16, from 3 to 15, from 2 to 14, from 2 to 13, or from 2 to 12 carbons, such as C₁₋₆ alk-C₁₋₁₂ heterocyclyl, C₁₋₁₀ alk-C₁₋₁₂ heterocyclyl, or C₁₋₂₀ alk-C₁₋₁₂ heterocyclyl). In some cases, the alkylene and the heterocyclyl each can be further substituted with 1, 2, 3, or 4 substituent groups as defined herein for the respective group.

The term "hydrocarbon," as used herein, represents a group consisting only of carbon and hydrogen atoms.

The term "hydroxy," as used herein, represents an -OH group.

The term "isomer," as used herein, means any tautomer, stereoisomer, enantiomer, or diastereomer of any compound of the disclosure. It is recognized that the compounds of the disclosure can have one or more chiral centers and/or double bonds and, therefore, exist as stereoisomers, such as double-bond isomers (i.e., geometric E/Z isomers) or diastereomers (e.g., enantiomers (i.e., (+) or (-)) or cis/trans isomers). According to the disclosure, the chemical structures depicted herein, and therefore the compounds of the disclosure, encompass all of the corresponding stereoisomers, that is, both the stereomerically pure form (e.g., geometrically pure, enantiomerically pure, or diastereomerically pure) and enantiomeric and stereoisomeric mixtures, e.g., racemates. Enantiomeric and stereoisomeric mixtures of compounds can typically be resolved into their component enantiomers or stereoisomers by well-known methods, such as chiral-phase gas chromatography, chiral-phase high performance liquid chromatography, crystallizing the compound as a chiral salt complex, or crystallizing the compound in a chiral solvent. Enantiomers and stereoisomers can also be obtained from stereomerically or enantiomerically pure intermediates, reagents, and catalysts by well-known asymmetric synthetic methods.

The term "*N*-protected amino," as used herein, refers to an amino group, as defined herein, to which is attached one or two *N*-protecting groups, as defined herein.

The term "*N*-protecting group," as used herein, represents those groups intended to protect an amino group against undesirable reactions during synthetic procedures. Commonly used *N*-protecting groups are disclosed in Greene, "Protective Groups in Organic Synthesis," 3rd Edition (John Wiley & Sons, New York, 1999).

*N*-protecting groups include acyl, aryloyl, or carbamyl groups such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and chiral auxiliaries such as protected or unprotected D, L or D, L-amino acids such as alanine, leucine, phenylalanine, and the like; sulfonyl-containing groups such as benzenesulfonyl, p-toluenesulfonyl, and the like; carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 2-nitro-4,5-dimethoxybenzyloxycarbonyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxy carbonyl, t-butyloxycarbonyl, diisopropylmethoxycarbonyl, isopropyloxycarbonyl, ethoxycarbonyl, methoxycarbonyl, allyloxycarbonyl, 2,2,2,-trichloroethoxycarbonyl, phenoxycarbonyl, 4-nitrophenoxy carbonyl, fluorenyl-9-methoxycarbonyl, cyclopentyloxycarbonyl, adamantyloxycarbonyl, cyclohexyloxycarbonyl, phenylthiocarbonyl, and the like, alkaryl groups such as benzyl, triphenylmethyl, benzyloxymethyl, and the like and silyl groups, such as trimethylsilyl, and the like. Preferred *N*-protecting groups are formyl, acetyl, benzoyl, pivaloyl, t-butylacetyl, alanyl, phenylsulfonyl, benzyl, t-butyloxycarbonyl (Boc), and benzyloxycarbonyl (Cbz).

The term "nitro," as used herein, represents an -NO₂ group.

The term "*O*-protecting group," as used herein, represents those groups intended to protect an oxygen containing (e.g., phenol, hydroxyl, or carbonyl) group against undesirable reactions during synthetic procedures. Commonly used *O-*protecting groups are disclosed in Greene, "Protective Groups in Organic Synthesis," 3rd Edition (John Wiley & Sons, New York, 1999).

Exemplary *O*-protecting groups include acyl, aryloyl, or carbamyl groups, such as formyl, acetyl, propionyl, pivaloyl, t-butylacetyl, 2-chloroacetyl, 2-bromoacetyl, trifluoroacetyl, trichloroacetyl, phthalyl, o-nitrophenoxyacetyl, α-chlorobutyryl, benzoyl, 4-chlorobenzoyl, 4-bromobenzoyl, *t*-butyldimethylsilyl, tri-*iso*-propylsilyloxymethyl, 4,4'-dimethoxytrityl, isobutyryl, phenoxyacetyl, 4-isopropylpehenoxyacetyl, dimethylformamidino, and 4-nitrobenzoyl; alkylcarbonyl groups, such as acyl, acetyl, propionyl, pivaloyl, and the like; optionally substituted arylcarbonyl groups, such as benzoyl; silyl groups, such as trimethylsilyl (TMS), tertbutyldimethylsilyl (TBDMS), tri-iso-propylsilyloxymethyl (TOM), triisopropylsilyl (TIPS), and the like; ether-forming groups with the hydroxyl, such methyl, methoxymethyl, tetrahydropyranyl, benzyl, p-methoxybenzyl, trityl, and the like; alkoxycarbonyls, such as methoxycarbonyl, ethoxycarbonyl, isopropoxycarbonyl, n-isopropoxycarbonyl, n-butyloxycarbonyl, isobutyloxycarbonyl, sec-butyloxycarbonyl, t-butyloxycarbonyl, 2-ethylhexyloxycarbonyl, cyclohexyloxycarbonyl, methyloxycarbonyl, and the like; alkoxyalkoxycarbonyl groups, such as methoxymethoxycarbonyl, ethoxymethoxycarbonyl, 2-methoxyethoxycarbonyl, 2-ethoxyethoxycarbonyl, 2-butoxyethoxycarbonyl, 2-methoxyethoxymethoxycarbonyl, allyloxycarbonyl, propargyloxycarbonyl, 2-butenoxycarbonyl, 3-methyl-2-butenoxycarbonyl, and the like; haloalkoxycarbonyls, such as 2-chloroethoxycarbonyl, 2-chloroethoxycarbonyl, 2,2,2-trichloroethoxycarbonyl, and the like; optionally substituted arylalkoxycarbonyl groups, such as benzyloxycarbonyl, p-methylbenzyloxycarbonyl, p-methoxybenzyloxycarbonyl, p-nitrobenzyloxycarbonyl, 2,4-dinitrobenzyloxycarbonyl, 3,5-dimethylbenzyloxycarbonyl, p-chlorobenzyloxycarbonyl, p-bromobenzyloxycarbonyl, fluorenylmethyloxycarbonyl, and the like; and optionally substituted aryloxycarbonyl groups, such as phenoxycarbonyl, p-nitrophenoxycarbonyl, o-nitrophenoxycarbonyl, 2,4-dinitrophenoxycarbonyl, p-methyl-phenoxycarbonyl, m-methylphenoxycarbonyl, o-bromophenoxycarbonyl, 3,5-dimethylphenoxycarbonyl, p-chlorophenoxycarbonyl, 2-chloro-4-nitrophenoxy-carbonyl, and the like); substituted alkyl, aryl, and alkaryl ethers (e.g., trityl; methylthiomethyl; methoxymethyl; benzyloxymethyl; siloxymethyl; 2,2,2,-trichloroethoxymethyl; tetrahydropyranyl; tetrahydrofuranyl; ethoxyethyl; 1-[2-(trimethylsilyl)ethoxy]ethyl; 2-trimethylsilylethyl; t-butyl ether; p-chlorophenyl, p-methoxyphenyl, p-nitrophenyl, benzyl, p-methoxybenzyl, and nitrobenzyl); silyl ethers (e.g., trimethylsilyl; triethylsilyl; triisopropylsilyl; dimethylisopropylsilyl; t-butyldimethylsilyl; t-butyldiphenylsilyl; tribenzylsilyl; triphenylsilyl; and diphenymethylsilyl); carbonates (e.g., methyl, methoxymethyl, 9-fluorenylmethyl; ethyl; 2,2,2-trichloroethyl; 2-(trimethylsilyl)ethyl; vinyl, allyl, nitrophenyl; benzyl; methoxybenzyl; 3,4-dimethoxybenzyl; and nitrobenzyl); carbonyl-protecting groups (e.g., acetal and ketal groups, such as dimethyl acetal, 1,3-dioxolane, and the like; acylal groups; and dithiane groups, such as 1,3-dithianes, 1,3-dithiolane, and the like); carboxylic acid-protecting groups (e.g., ester groups, such as methyl ester, benzyl ester, t-butyl ester, orthoesters, and the like; and oxazoline groups.

The term "oxo" as used herein, represents =O.

The prefix "perfluoro," as used herein, represents anyl group, as defined herein, where each hydrogen radical bound to the alkyl group has been replaced by a fluoride radical. For example, perfluoroalkyl groups are exemplified by trifluoromethyl, pentafluoroethyl, and the like.

The term "phosphoryl," as used herein, refers to

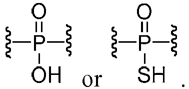

The term "protected hydroxyl," as used herein, refers to an oxygen atom bound to an *O*-protecting group.

The term "spirocyclyl," as used herein, represents a C₂₋₇ alkylene diradical, both ends of which are bonded to the same carbon atom of the parent group to form a spirocyclic group, and also a C₁₋₆ heteroalkylene diradical, both ends of which are bonded to the same atom. The heteroalkylene radical forming the spirocyclyl group can containing one, two, three, or four heteroatoms independently selected from the group consisting of nitrogen, oxygen, and sulfur. In some cases, the spirocyclyl group includes one to seven carbons, excluding the carbon atom to which the diradical is attached. The spirocyclyl groups may be optionally substituted with 1, 2, 3, or 4 substituents described herein as optional substituents for cycloalkyl and/or heterocyclyl groups.

The term "stereoisomer," as used herein, refers to all possible different isomeric as well as conformational forms which a compound may possess (e.g., a compound of any formula described herein), in particular all possible stereochemically and conformationally isomeric forms, all diastereomers, enantiomers and/or conformers of the basic molecular structure. Some compounds
may exist in different tautomeric forms, all of the latter being included within the scope of the present disclosure.

The term "sulfonyl," as used herein, represents an -S(O)₂- group.

The term "thiol," as used herein represents an -SH group.

*Compound:* As used herein, the term "compound," is meant to include all stereoisomers, geometric isomers, tautomers, and isotopes of the structures depicted.

The compounds described herein can be asymmetric (e.g., having one or more stereocenters). All stereoisomers, such as enantiomers and diastereomers, are intended unless otherwise indicated. Compounds of the present disclosure that contain asymmetrically substituted carbon atoms can be isolated in optically active or racemic forms. Methods on how to prepare optically active forms from optically active starting materials are known in the art, such as by resolution of racemic mixtures or by stereoselective synthesis. Many geometric isomers of olefins, C=N double bonds, and the like can also be present in the compounds described herein, and all such stable isomers are contemplated in the present disclosure. Cis and trans geometric isomers of the compounds of the present disclosure are described and may be isolated as a mixture of isomers or as separated isomeric forms.

Compounds of the present disclosure also include tautomeric forms. Tautomeric forms result from the swapping of a single bond with an adjacent double bond and the concomitant migration of a proton. Tautomeric forms include prototropic tautomers which are isomeric protonation states having the same empirical formula and total charge. Examples prototropic tautomers include ketone - enol pairs, amide - imidic acid pairs, lactam - lactim pairs, amide - imidic acid pairs, enamine - imine pairs, and annular forms where a proton can occupy two or more positions of a heterocyclic system, such as, 1H- and 3H-imidazole, 1H-, 2H- and 4H-1,2,4-triazole, 1H- and 2H- isoindole, and 1H- and 2H-pyrazole. Tautomeric forms can be in equilibrium or sterically locked into one form by appropriate substitution.

Compounds of the present disclosure also include all of the isotopes of the atoms occurring in the intermediate or final compounds. "Isotopes" refers to atoms having the same atomic number but different mass numbers resulting from a different number of neutrons in the nuclei. For example, isotopes of hydrogen include tritium and deuterium.

The compounds and salts of the present disclosure can be prepared in combination with solvent or water molecules to form solvates and hydrates by routine methods.

*Committed:* As used herein, the term "committed" means, when referring to a cell, when the cell is far enough into the differentiation pathway where, under normal circumstances, it will continue to differentiate into a specific cell type or subset of cell type instead of into a different cell type or reverting to a lesser differentiated cell type.

*Conserved:* As used herein, the term "conserved" refers to nucleotides or amino acid residues of a polynucleotide sequence or polypeptide sequence, respectively, that are those that occur unaltered in the same position of two or more sequences being compared. Nucleotides or amino acids that are relatively conserved are those that are conserved amongst more related sequences than nucleotides or amino acids appearing elsewhere in the sequences.

In some cases, two or more sequences are said to be "completely conserved" if they are 100% identical to one another. In some cases, two or more sequences are said to be "highly conserved" if they are at least 70% identical, at least 80% identical, at least 90% identical, or at least 95% identical to one another. In some cases, two or more sequences are said to be "highly conserved" if they are about 70% identical, about 80% identical, about 90% identical, about 95%, about 98%, or about 99% identical to one another. In some cases, two or more
sequences are said to be "conserved" if they are at least 30% identical, at least 40% identical, at least 50% identical, at least 60% identical, at least 70% identical, at least 80% identical, at least 90% identical, or at least 95% identical to one another. In some cases, two or more sequences are said to be "conserved" if they are about 30% identical, about 40% identical, about 50% identical, about 60% identical, about 70% identical, about 80% identical, about 90% identical, about 95% identical, about 98% identical, or about 99% identical to one another. Conservation of sequence may apply to the entire length of an polynucleotide or polypeptide or may apply to a portion, region or feature thereof.

*Controlled Release:* As used herein, the term "controlled release" refers to a pharmaceutical composition or compound release profile that conforms to a particular pattern of release to effect a therapeutic outcome.

*Cyclic or Cyclized:* As used herein, the term "cyclic" refers to the presence of a continuous loop. Cyclic molecules need not be circular, only joined to form an unbroken chain of subunits. Cyclic molecules such as the engineered RNA or mRNA may be single units or multimers or comprise one or more components of a complex or higher order structure.

*Cytostatic:* As used herein, "cytostatic" refers to inhibiting, reducing, suppressing the growth, division, or multiplication of a cell *(e.g.,* a mammalian cell *(e.g.,* a human cell)), bacterium, virus, fungus, protozoan, parasite, prion, or a combination thereof.

*Cytotoxic:* As used herein, "cytotoxic" refers to killing or causing injurious, toxic, or deadly effect on a cell *(e.g.,* a mammalian cell *(e.g.,* a human cell)), bacterium, virus, fungus, protozoan, parasite, prion, or a combination thereof.

*Delivery:* As used herein, "delivery" refers to the act or manner of delivering a compound, substance, entity, moiety, cargo or payload.

*Delivery Agent:* As used herein, "delivery agent" refers to any substance which facilitates, at least in part, the *in vivo* delivery of a chimeric polynucleotide to targeted cells.

*Destabilized:* As used herein, the term "destable," "destabilize," or "destabilizing region" means a region or molecule that is less stable than a starting, wild-type or native form of the same region or molecule.

*Detectable label:* As used herein, "detectable label" refers to one or more markers, signals, or moieties which are attached, incorporated or associated with another entity that is readily detected by methods known in the art including radiography, fluorescence, chemiluminescence, enzymatic activity, absorbance and the like. Detectable labels include radioisotopes, fluorophores, chromophores, enzymes, dyes, metal ions, ligands such as biotin, avidin, streptavidin and haptens, quantum dots, and the like. Detectable labels may be located at any position in the peptides or proteins disclosed herein. They may be within the amino acids, the peptides, or proteins, or located at the N- or C- termini.

*Diastereomer:* As used herein, the term "diastereomer," means stereoisomers that are not mirror images of one another and are non-superimposable on one another.

*Digest:* As used herein, the term "digest" means to break apart into smaller pieces or components. When referring to polypeptides or proteins, digestion results in the production of peptides.

*Differentiated cell:* As used herein, the term "differentiated cell" refers to any somatic cell that is not, in its native form, pluripotent. Differentiated cell also encompasses cells that are partially differentiated.

*Differentiation:* As used herein, the term "differentiation factor" refers to a developmental potential altering factor such as a protein, RNA or small molecule that can induce a cell to differentiate to a desired cell-type.

*Differentiate:* As used herein, "differentiate" refers to the process where an uncommitted or less committed cell acquires the features of a committed cell.

*Distal:* As used herein, the term "distal" means situated away from the center or away from a point or region of interest.

*Dosing regimen:* As used herein, a "dosing regimen" is a schedule of administration or physician determined regimen of treatment, prophylaxis, or palliative care.

*Dose splitting factor* (DSF)-ratio of PUD of dose split treatment divided by PUD of total daily dose or single unit dose. The value is derived from comparison of dosing regimens groups.

*Enantiomer:* As used herein, the term "enantiomer" means each individual optically active form of a compound of the disclosure, having an optical purity or enantiomeric excess (as determined by methods standard in the art) of at least 80% (i.e., at least 90% of one enantiomer and at most 10% of the other enantiomer), preferably at least 90% and more preferably at least 98%.

*Encapsulate:* As used herein, the term "encapsulate" means to enclose, surround or encase.

*Encoded protein cleavage signal:* As used herein, "encoded protein cleavage signal" refers to the nucleotide sequence which encodes a protein cleavage signal.

*Engineered:* As used herein, cases of the disclosure are "engineered" when they are designed to have a feature or property, whether structural or chemical, that varies from a starting point, wild type or native molecule.

*Effective Amount:* As used herein, the term "effective amount" of an agentis that amount sufficient to effect beneficial or desired results, for example, clinical results, and, as such, an "effective amount" depends upon the context in which it is being applied. For example, in the context of administering an agent that treats cancer, an effective amount of an agent is, for example, an amount sufficient to achieve treatment, as defined herein, of cancer, as compared to the response obtained without administration of the agent.

*Exosome:* As used herein, "exosome" is a vesicle secreted by mammalian cells or a complex involved in RNA degradation.

*Expression:* As used herein, "expression" of a nucleic acid sequence refers to one or more of the following events: (1) production of an RNA template from a DNA sequence *(e.g.,* by transcription); (2) processing of an RNA transcript *(e.g.,* by splicing, editing, 5' cap formation, and/or 3' end processing); (3) translation of an RNA into a polypeptide or protein; and (4) post-translational modification of a polypeptide or protein.

*Feature:* As used herein, a "feature" refers to a characteristic, a property, or a distinctive element.

*Formulation:* As used herein, a "formulation" includes at least a chimeric polynucleotide and a delivery agent.

*Fragment:* A "fragment," as used herein, refers to a portion. For example, fragments of proteins may comprise polypeptides obtained by digesting full-length protein isolated from cultured cells.

*Functional:* As used herein, a "functional" biological molecule is a biological molecule in a form in which it exhibits a property and/or activity by which it is characterized.

*Homology*: As used herein, the term "homology" refers to the overall relatedness between polymeric molecules, *e.g.* between nucleic acid molecules *(e.g.* DNA molecules and/or RNA molecules) and/or between polypeptide molecules. In some cases, polymeric molecules are considered to be "homologous" to one another if their sequences are at least 25%, 30%, 35%, 40%, 45%, 50%, 55%, 60%, 65%, 70%, 75%, 80%, 85%, 90%, 95%, or 99% identical or similar. The term "homologous" necessarily refers to a comparison between at least two sequences (polynucleotide or polypeptide sequences). In accordance with the disclosure, two polynucleotide sequences are considered to be homologous if the polypeptides they encode are at least about 50%, 60%, 70%, 80%, 90%, 95%, or even 99% for at least one stretch of at least about 20 amino acids. In some cases, homologous polynucleotide sequences are characterized by the ability to encode a stretch of at least 4-5 uniquely specified amino acids. For polynucleotide sequences less than 60 nucleotides in length, homology is determined by the ability to encode a stretch of at least 4-5 uniquely specified amino acids. In accordance with the disclosure, two protein sequences are considered to be homologous if the proteins are at least about 50%, 60%, 70%, 80%, or 90% identical for at least one stretch of at least about 20 amino acids.

*Identity:* As used herein, the term "identity" refers to the overall relatedness between polymeric molecules, e.g., between polynucleotide molecules (e.g. DNA molecules and/or RNA molecules) and/or between polypeptide molecules. Calculation of the percent identity of two polynucleotide sequences, for example, can be performed by aligning the two sequences for optimal comparison purposes (e.g., gaps can be introduced in one or both of a first and a second nucleic acid sequences for optimal alignment and non-identical sequences can be disregarded for comparison purposes). In certain cases, the length of a sequence aligned for comparison purposes is at least 30%, at least 40%, at least 50%, at least 60%, at least 70%, at least 80%, at least 90%, at least 95%, or 100% of the length of the reference sequence. The nucleotides at corresponding nucleotide positions are then compared. When a position in the first sequence is occupied by the same nucleotide as the corresponding position in the second sequence, then the molecules are identical at that position. The percent identity between the two sequences is a function of the number of identical positions shared by the sequences, taking into account the number of gaps, and the length of each gap, which needs to be introduced for optimal alignment of the two sequences. The comparison of sequences and determination of percent identity between two sequences can be accomplished using a mathematical algorithm. For example, the percent identity between two nucleotide sequences can be determined using methods such as those described in Computational Molecular Biology, Lesk, A. M., ed., Oxford University Press, New York, 1988; Biocomputing: Informatics and Genome Projects, Smith, D. W., ed., Academic Press, New York, 1993; Sequence Analysis in Molecular Biology, von Heinje, G., Academic Press, 1987; Computer Analysis of Sequence Data, Part I, Griffin, A. M., and Griffin, H. G., eds., Humana Press, New Jersey, 1994; and Sequence Analysis Primer, Gribskov, M. and Devereux, J., eds., M Stockton Press, New York, 1991. For example, the percent identity between two nucleotide sequences can be determined using the algorithm of Meyers and Miller (CABIOS, 1989, 4:11-17), which has been incorporated into the ALIGN program (version 2.0) using a PAM120 weight residue table, a gap length penalty of 12 and a gap penalty of 4. The percent identity between two nucleotide sequences can, alternatively, be determined using the GAP program in the GCG software package using an NWSgapdna.CMP matrix. Methods commonly employed to determine percent identity between sequences include, but are not limited to those disclosed in Carillo, H., and Lipman, D., SIAM J Applied Math., 48:1073 (1988). Techniques for determining identity are codified in publicly available computer programs. Exemplary computer software to determine homology between two sequences include, but are not limited to, GCG program package, Devereux, J., et al., Nucleic Acids Research, 12(1), 387 (1984)), BLASTP, BLASTN, and FASTA Altschul, S. F. et al., J. Molec. Biol., 215, 403 (1990)).

*Immunoglobin:* As used herein, the term "immunoglobin" (Ig) can be used interchangeably with "antibody."

*Infectious Agent:* As used herein, the phrase "infectious agent" means an agent capable of producing an infection.

*Inhibit expression of a gene:* As used herein, the phrase "inhibit expression of a gene" means to cause a reduction in the amount of an expression product of the gene. The expression product can be an RNA transcribed from the gene (e.g., an mRNA) or a polypeptide translated from an mRNA transcribed from the gene. Typically a reduction in the level of an mRNA results in a reduction in the level of a polypeptide translated therefrom. The level of expression may be determined using standard techniques for measuring mRNA or protein.

*Infectious agent:* As used herein, an "infectious agent" refers to any microorganism, virus, infectious substance, or biological product that may be engineered as a result of biotechnology, or any naturally occurring or bioengineered component of any such microorganism, virus, infectious substance, or biological product, can cause emerging and contagious disease, death or other biological malfunction in a human, an animal, a plant or another living organism.

*Influenza:* As used herein, "influenza" or "flu" is an infectious disease of birds and mammals caused by RNA viruses of the family Orthomyxoviridae, the influenza viruses.

*Isomer:* As used herein, the term "isomer" means any tautomer, stereoisomer, enantiomer, or diastereomer of any compound of the disclosure. It is recognized that the compounds of the disclosure can have one or more chiral centers and/or double bonds and, therefore, exist as stereoisomers, such as double-bond isomers (i.e., geometric E/Z isomers) or diastereomers (e.g., enantiomers (i.e., (+) or disclosure (-)) or cis/trans isomers). According to the disclosure, the chemical structures depicted herein, and therefore the compounds of the disclosure, encompass all of the corresponding stereoisomers, that is, both the stereomerically pure form (e.g., geometrically pure, enantiomerically pure, or diastereomerically pure) and enantiomeric and stereoisomeric mixtures, e.g., racemates. Enantiomeric and stereoisomeric mixtures of compounds of the disclosure can typically be resolved into their component enantiomers or stereoisomers by well-known methods, such as chiral-phase gas chromatography, chiral-phase high performance liquid chromatography, crystallizing the compound as a chiral salt complex, or crystallizing the compound in a chiral solvent. Enantiomers and stereoisomers can also be obtained from stereomerically or enantiomerically pure intermediates, reagents, and catalysts by well-known asymmetric synthetic methods.

*In vitro:* As used herein, the term *"in vitro"* refers to events that occur in an artificial environment, e.g., in a test tube or reaction vessel, in cell culture, in a Petri dish, *etc.,* rather than within an organism *(e.g.,* animal, plant, or microbe).

*In vivo:* As used herein, the term *"in vivo"* refers to events that occur within an organism (e.g., animal, plant, or microbe or cell or tissue thereof).

*Isolated:* As used herein, the term "isolated" refers to a substance or entity that has been separated from at least some of the components with which it was associated (whether in nature or in an experimental setting). Isolated substances may have varying levels of purity in reference to the substances from which they have been associated. Isolated substances and/or entities may be separated from at least about 10%, about 20%, about 30%, about 40%, about 50%, about 60%, about 70%, about 80%, about 90%, or more of the other components with which they were cases initially associated. In some cases, isolated agents are more than about 80%, about 85%, about 90%, about 91%, about 92%, about 93%, about 94%, about 95%, about 96%, about 97%, about 98%, about 99%, or more than about 99% pure. As used herein, a substance is "pure" if it is substantially free of other components. *Substantially isolated:* By "substantially isolated" is meant that the compound is substantially separated from the environment in which it was formed or detected. Partial separation can include, for example, a composition enriched in the compound of the present disclosure. Substantial separation can include compositions containing at least about 50%, at least about 60%, at least about 70%, at least about 80%, at least about 90%, at least about 95%, at least about 97%, or at least about 99% by weight of the compound of the present disclosure, or salt thereof. Methods for isolating compounds and their salts are routine in the art.

*Linker:* As used herein, a linker refers to a group of atoms, e.g., 10-1,000 atoms, and can be comprised of the atoms or groups such as, but not limited to, carbon, amino, alkylamino, oxygen, sulfur, sulfoxide, sulfonyl, carbonyl, and imine. The linker can be attached to a modified nucleoside or nucleotide on the nucleobase or sugar moiety at a first end, and to a payload, e.g., a detectable or therapeutic agent, at a second end. The linker may be of sufficient length as to not interfere with incorporation into a nucleic acid sequence. The linker can be used for any useful purpose, such as to form chimeric polynucleotide multimers (e.g., through linkage of two or more chimeric polynucleotides molecules) or chimeric polynucleotides conjugates, as well as to administer a payload, as described herein. Examples of chemical groups that can be incorporated into the linker include, but are not limited to, alkyl, alkenyl, alkynyl, amido, amino, ether, thioether, ester, alkylene, heteroalkylene, aryl, or heterocyclyl, each of which can be optionally substituted, as described herein. Examples of linkers include, but are not limited to, unsaturated alkanes, polyethylene glycols (e.g., ethylene or propylene glycol monomeric units, e.g., diethylene glycol, dipropylene glycol, triethylene glycol, tripropylene glycol, tetraethylene glycol, or tetraethylene glycol), and dextran polymers and derivatives thereof., Other examples include, but are not limited to, cleavable moieties within the linker, such as, for example, a disulfide bond (-S-S-) or an azo bond (-N=N-), which can be cleaved using a reducing agent or photolysis. Non-limiting examples of a selectively cleavable bond include an amido bond can be cleaved for example by the use of tris(2-carboxyethyl)phosphine (TCEP), or other reducing agents, and/or photolysis, as well as an ester bond can be cleaved for example by acidic or basic hydrolysis.

*MicroRNA (miRNA) binding site:* As used herein, a microRNA (miRNA) binding site represents a nucleotide location or region of a nucleic acid transcript to which at least the "seed" region of a miRNA binds.

*Modified:* As used herein "modified" refers to a changed state or structure of a molecule of the disclosure. Molecules may be modified in many ways including chemically, structurally, and functionally. In one case, the mRNA molecules of the present disclosure are modified by the introduction of non-natural nucleosides and/or nucleotides, e.g., as it relates to the natural ribonucleotides A, U, G, and C. Noncanonical nucleotides such as the cap structures are not considered "modified" although they differ from the chemical structure of the A, C, G, U ribonucleotides.

*Monoclonal Antibody*: As used herein the term "monoclonal antibody" refers to an antibody obtained from a population of substantially homogeneous antibodies, i.e., the individual antibodies comprising the population are identical except for possible naturally occurring mutations and/or post-translation modifications (e.g., isomerizations, amidations) that may be present in minor amounts. Monoclonal antibodies are highly specific, being directed against a single antigenic site.

*Mucus:* As used herein, "mucus" refers to the natural substance that is viscous and comprises mucin glycoproteins.

*Naturally occurring:* As used herein, "naturally occurring" means existing in nature without artificial aid.

*Neutralizing antibody*: As used herein, a "neutralizing antibody" refers to an antibody which binds to its antigen and defends a cell from an antigen or infectious agent by neutralizing or abolishing any biological activity it has.

*Non-human vertebrate:* As used herein, a "non human vertebrate" includes all vertebrates except *Homo sapiens,* including wild and domesticated species. Examples of non-human vertebrates include, but are not limited to, mammals, such as alpaca, banteng, bison, camel, cat, cattle, deer, dog, donkey, gayal, goat, guinea pig, horse, llama, mule, pig, rabbit, reindeer, sheep water buffalo, and yak.

*Off-target:* As used herein, "off target" refers to any unintended effect on any one or more target, gene, or cellular transcript.

*Open reading frame:* As used herein, "open reading frame" or "ORF" refers to a sequence which does not contain a stop codon in a given reading frame.

*Operably linked:* As used herein, the phrase "operably linked" refers to a functional connection between two or more molecules, constructs, transcripts, entities, moieties or the like.

*Optionally substituted:* Herein a phrase of the form "optionally substituted X" (e.g., optionally substituted alkyl) is intended to be equivalent to "X, wherein X is optionally substituted" (e.g., "alkyl, wherein the alkyl is optionally substituted"). It is not intended to mean that the feature "X" (*e.g.* alkyl) *per se* is optional.

*Peptide:* As used herein, "peptide" is less than or equal to 50 amino acids long, e.g., about 5, 10, 15, 20, 25, 30, 35, 40, 45, or 50 amino acids long.

*Paratope:* As used herein, a "paratope" refers to the antigen-binding site of an antibody.

*Patient:* As used herein, "patient" refers to a subject who may seek or be in need of treatment, requires treatment, is receiving treatment, will receive treatment, or a subject who is under care by a trained professional for a particular disease or condition.

*Pharmaceutically acceptable:* The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

*Pharmaceutically acceptable excipients:* The phrase "pharmaceutically acceptable excipient," as used herein, refers any ingredient other than the compounds described herein (for example, a vehicle capable of suspending or dissolving the active compound) and having the properties of being substantially nontoxic and noninflammatory in a patient. Excipients may include, for example: antiadherents, antioxidants, binders, coatings, compression aids, disintegrants, dyes (colors), emollients, emulsifiers, fillers (diluents), film formers or coatings, flavors, fragrances, glidants (flow enhancers), lubricants, preservatives, printing inks, sorbents, suspensing or dispersing agents, sweeteners, and waters of hydration. Exemplary excipients include, but are not limited to: butylated hydroxytoluene (BHT), calcium carbonate, calcium phosphate (dibasic), calcium stearate, croscarmellose, crosslinked polyvinyl pyrrolidone, citric acid, crospovidone, cysteine, ethylcellulose, gelatin, hydroxypropyl cellulose, hydroxypropyl methylcellulose, lactose, magnesium stearate, maltitol, mannitol, methionine, methylcellulose, methyl paraben, microcrystalline cellulose, polyethylene glycol, polyvinyl pyrrolidone, povidone, pregelatinized starch, propyl paraben, retinyl palmitate, shellac, silicon dioxide, sodium carboxymethyl cellulose, sodium citrate, sodium starch glycolate, sorbitol, starch (corn), stearic acid, sucrose, talc, titanium dioxide, vitamin A, vitamin E, vitamin C, and xylitol.

*Pharmaceutically acceptable salts:* The present disclosure also includes pharmaceutically acceptable salts of the compounds described herein. As used herein, "pharmaceutically acceptable salts" refers to derivatives of the disclosed compounds wherein the parent compound is modified by converting an existing acid or base moiety to its salt form (e.g., by reacting the free base group with a suitable organic acid). Examples of pharmaceutically acceptable salts include, but are not limited to, mineral or organic acid salts of basic residues such as amines; alkali or organic salts of acidic residues such as carboxylic acids; and the like. Representative acid addition salts include acetate, acetic acid, adipate, alginate, ascorbate, aspartate, benzenesulfonate, benzene sulfonic acid, benzoate, bisulfate, borate, butyrate, camphorate, camphorsulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like. The pharmaceutically acceptable salts of the present disclosure include the conventional non-toxic salts of the parent compound formed, for example, from non-toxic inorganic or organic acids. The pharmaceutically acceptable salts of the present disclosure can be synthesized from the parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, Pa., 1985, p. 1418, Pharmaceutical Salts: Properties, Selection, and Use, P.H. Stahl and C.G. Wermuth (eds.), Wiley-VCH, 2008, and Berge et al., Journal of Pharmaceutical Science, 66, 1-19 (1977).

*Pharmaceutically acceptable solvate:* The term "pharmaceutically acceptable solvate," as used herein, means a compound of the disclosure wherein molecules of a suitable solvent are incorporated in the crystal lattice. A suitable solvent is physiologically tolerable at the dosage administered. For example, solvates may be prepared by crystallization, recrystallization, or precipitation from a solution that includes organic solvents, water, or a mixture thereof. Examples of suitable solvents are ethanol, water (for example, mono-, di-, and tri-hydrates), N-methylpyrrolidinone (NMP), dimethyl sulfoxide (DMSO), *N,N*'-dimethylformamide (DMF), *N,N*'-dimethylacetamide (DMAC), 1,3-dimethyl-2-imidazolidinone (DMEU), 1,3-dimethyl-3,4,5,6-tetrahydro-2-(1H)-pyrimidinone (DMPU), acetonitrile (ACN), propylene glycol, ethyl acetate, benzyl alcohol, 2-pyrrolidone, benzyl benzoate, and the like. When water is the solvent, the solvate is referred to as a "hydrate."

*Pharmacokinetic:* As used herein, "pharmacokinetic" refers to any one or more properties of a molecule or compound as it relates to the determination of the fate of substances administered to a living organism. Pharmacokinetics is divided into several areas including the extent and rate of absorption, distribution, metabolism and excretion. This is commonly referred to as ADME where: (A) Absorption is the process of a substance entering the blood circulation; (D) Distribution is the dispersion or dissemination of substances throughout the fluids and tissues of the body; (M) Metabolism (or Biotransformation) is the irreversible transformation of parent compounds into daughter metabolites; and (E) Excretion (or Elimination) refers to the elimination of the substances from the body. In rare cases, some drugs irreversibly accumulate in body tissue.

*Physicochemical:* As used herein, "physicochemical" means of or relating to a physical and/or chemical property.

*Polypeptide per unit drug (PUD):* As used herein, a PUD or product per unit drug, is defined as a subdivided portion of total daily dose, usually 1 mg, pg, kg, etc., of a product (such as a polypeptide) as measured in body fluid or tissue, usually defined in concentration such as pmol/mL, mmol/mL, etc. divided by the measure in the body fluid.

*Preventing:* As used herein, the term "preventing" refers to partially or completely delaying onset of an infection, disease, disorder and/or condition; partially or completely delaying onset of one or more symptoms, features, or clinical manifestations of a particular infection, disease, disorder, and/or condition; partially or completely delaying onset of one or more symptoms, features, or manifestations of a particular infection, disease, disorder, and/or condition; partially or completely delaying progression from an infection, a particular disease, disorder and/or condition; and/or decreasing the risk of developing pathology associated with the infection, the disease, disorder, and/or condition.

*Prodrug:* The present disclosure also includes prodrugs of the compounds described herein. As used herein, "prodrugs" refer to any substance, molecule or entity which is in a form predicate for that substance, molecule or entity to act as a therapeutic upon chemical or physical alteration. Prodrugs may by covalently bonded or sequestered in some way and which release or are converted into the active drug moiety prior to, upon or after administered to a mammalian subject. Prodrugs can be prepared by modifying functional groups present in the compounds in such a way that the modifications are cleaved, either in routine manipulation or *in vivo,* to the parent compounds. Prodrugs include compounds wherein hydroxyl, amino, sulfhydryl, or carboxyl groups are bonded to any group that, when administered to a mammalian subject, cleaves to form a free hydroxyl, amino, sulfhydryl, or carboxyl group respectively. Preparation and use of prodrugs is discussed in T. Higuchi and V. Stella, "Pro-drugs as Novel Delivery Systems," Vol. 14 of the A.C.S. Symposium Series, and in Bioreversible Carriers in Drug Design, ed. Edward B. Roche, American Pharmaceutical Association and Pergamon Press, 1987.

*Proliferate:* As used herein, the term "proliferate" means to grow, expand or increase or cause to grow, expand or increase rapidly. "Proliferative" means having the ability to proliferate. "Anti-proliferative" means having properties counter to or inapposite to proliferative properties.

*Progenitor cell:* As used herein, the term "progenitor cell" refers to cells that have greater developmental potential relative to a cell which it can give rise to by differentiation.

*Prophylactic:* As used herein, "prophylactic" refers to a therapeutic or course of action used to prevent the spread of disease.

*Prophylaxis:* As used herein, a "prophylaxis" refers to a measure taken to maintain health and prevent the spread of disease. An "immune prophylaxis" refers to a measure to produce active or passive immunity to prevent the spread of disease.

*Protein cleavage site:* As used herein, "protein cleavage site" refers to a site where controlled cleavage of the amino acid chain can be accomplished by chemical, enzymatic or photochemical means.

*Protein cleavage signal:* As used herein "protein cleavage signal" refers to at least one amino acid that flags or marks a polypeptide for cleavage.

*Protein of interest:* As used herein, the terms "proteins of interest" or "desired proteins" include those described herein and fragments, mutants, variants, and alterations thereof.

*Proximal:* As used herein, the term "proximal" means situated nearer to the center or to a point or region of interest.

*Pseudouridine:* As used herein, pseudouridine refers to the C-glycoside isomer of the nucleoside uridine. A "pseudouridine analog" is any modification, variant, isoform or derivative of pseudouridine. For example, pseudouridine analogs include but are not limited to 1-carboxymethyl-pseudouridine, 1-propynyl-pseudouridine, 1-taurinomethyl-pseudouridine, 1-taurinomethyl-4-thio-pseudouridine, 1-methylpseudouridine (m¹ψ), 1-methyl-4-thio-pseudouridine (m¹s⁴ψ), 4-thio-1-methyl-pseudouridine, 3-methyl-pseudouridine (m³ψ), 2-thio-1-methyl-pseudouridine, 1-methyl-1-deaza-pseudouridine, 2-thio-1-methyl-1-deaza-pseudouridine, dihydropseudouridine, 2-thio-dihydropseudouridine, 2-methoxyuridine, 2-methoxy-4-thio-uridine, 4-methoxy-pseudouridine, 4-methoxy-2-thio-pseudouridine, N1-methyl-pseudouridine, 1-methyl-3-(3-amino-3-carboxypropyl)pseudouridine (acp³ ψ), and 2'-O-methyl-pseudouridine (ψm).

*Purified:* As used herein, "purify," "purified," "purification" means to make substantially pure or clear from unwanted components, material defilement, admixture or imperfection.

*Repeated transfection:* As used herein, the term "repeated transfection" refers to transfection of the same cell culture with a chimeric polynucleotide a plurality of times. The cell culture can be transfected at least twice, at least 3 times, at least 4 times, at least 5 times, at least 6 times, at least 7 times, at least 8 times, at least 9 times, at least 10 times, at least 11 times, at least 12 times, at least 13 times, at least 14 times, at least 15 times, at least 16 times, at least 17 times at least 18 times, at least 19 times, at least 20 times, at least 25 times, at least 30 times, at least 35 times, at least 40 times, at least 45 times, at least 50 times or more.

*Sample:* As used herein, the term "sample" or "biological sample" refers to a subset of its tissues, cells or component parts (e.g. body fluids, including but not limited to blood, mucus, lymphatic fluid, synovial fluid, cerebrospinal fluid, saliva, amniotic fluid, amniotic cord blood, urine, vaginal fluid and semen). A sample further may include a homogenate, lysate or extract prepared from a whole organism or a subset of its tissues, cells or component parts, or a fraction or portion thereof, including but not limited to, for example, plasma, serum, spinal fluid, lymph fluid, the external sections of the skin, respiratory, intestinal, and genitourinary tracts, tears, saliva, milk, blood cells, tumors, organs. A sample further refers to a medium, such as a nutrient broth or gel, which may contain cellular components, such as proteins or nucleic acid molecule.

*Signal Sequences:* As used herein, the phrase "signal sequences" refers to a sequence which can direct the transport or localization of a protein.

*Single unit dose:* As used herein, a "single unit dose" is a dose of any therapeutic administered in one dose/at one time/single route/single point of contact, i.e., single administration event.

*Similarity:* As used herein, the term "similarity" refers to the overall relatedness between polymeric molecules, e.g. between polynucleotide molecules (e.g. DNA molecules and/or RNA molecules) and/or between polypeptide molecules. Calculation of percent similarity of polymeric molecules to one another can be performed in the same manner as a calculation of percent identity, except that calculation of percent similarity takes into account conservative substitutions as is understood in the art.

*Split dose:* As used herein, a "split dose" is the division of single unit dose or total daily dose into two or more doses.

*Stable:* As used herein "stable" refers to a compound that is sufficiently robust to survive isolation to a useful degree of purity from a reaction mixture, and preferably capable of formulation into an efficacious therapeutic agent.

*Stabilized:* As used herein, the term "stabilize", "stabilized," "stabilized region" means to make or become stable.

*Stereoisomer:* As used herein, the term "stereoisomer" refers to all possible different isomeric as well as conformational forms which a compound may possess (e.g., a compound of any formula described herein), in particular all possible stereochemically and conformationally isomeric forms, all diastereomers, enantiomers and/or conformers of the basic molecular structure. Some compounds may exist in different tautomeric forms, all of the latter being included within the scope of the present disclosure.

*Subject:* As used herein, the term "subject" or "patient" refers to any organism to which a composition may be administered, e.g., for experimental, diagnostic, prophylactic, and/or therapeutic purposes. Typical subjects include animals (e.g., mammals such as mice, rats, rabbits, non-human primates, and humans) and/or plants.

*Substantially:* As used herein, the term "substantially" refers to the qualitative condition of exhibiting total or near-total extent or degree of a characteristic or property of interest. One of ordinary skill in the biological arts will understand that biological and chemical phenomena rarely, if ever, go to completion and/or proceed to completeness or achieve or avoid an absolute result. The term "substantially" is therefore used herein to capture the potential lack of completeness inherent in many biological and chemical phenomena.

*Substantially equal:* As used herein as it relates to time differences between doses, the term means plus/minus 2%.

*Substantially simultaneously:* As used herein and as it relates to plurality of doses, the term means within 2 seconds.

*Suffering from:* An individual who is "suffering from" a disease, disorder, and/or condition has been diagnosed with or displays one or more symptoms of a disease, disorder, and/or condition.

*Susceptible to:* An individual who is "susceptible to" a disease, disorder, and/or condition has not been diagnosed with and/or may not exhibit symptoms of the disease, disorder, and/or condition but harbors a propensity to develop a disease or its symptoms. In some cases, an individual who is susceptible to a disease, disorder, and/or condition (for example, cancer) may be characterized by one or more of the following: (1) a genetic mutation associated with development of the disease, disorder, and/or condition; (2) a genetic polymorphism associated with development of the disease, disorder, and/or condition; (3) increased and/or decreased expression and/or activity of a protein and/or nucleic acid associated with the disease, disorder, and/or condition; (4) habits and/or lifestyles associated with development of the disease, disorder, and/or condition; (5) a family history of the disease, disorder, and/or condition; and (6) exposure to and/or infection with a microbe associated with development of the disease, disorder, and/or condition. In some cases, an individual who is susceptible to a disease, disorder, and/or condition will develop the disease, disorder, and/or condition. In some cases, an individual who is susceptible to a disease, disorder, and/or condition will not develop the disease, disorder, and/or condition.

*Sustained release:* As used herein, the term "sustained release" refers to a pharmaceutical composition or compound release profile that conforms to a release rate over a specific period of time.

*Synthetic:* The term "synthetic" means produced, prepared, and/or manufactured by the hand of man. Synthesis of polynucleotides or polypeptides or other molecules may be chemical or enzymatic.

*Targeted Cells:* As used herein, "targeted cells" refers to any one or more cells of interest. The cells may be found *in vitro, in vivo, in situ* or in the tissue or organ of an organism. The organism may be an animal, preferably a mammal, more preferably a human and most preferably a patient.

*Therapeutic Agent:* The term "therapeutic agent" refers to any agent that, when administered to a subject, has a therapeutic, diagnostic, and/or prophylactic effect and/or elicits a desired biological and/or pharmacological effect.

*Therapeutically effective amount:* As used herein, the term "therapeutically effective amount" means an amount of an agent to be delivered (e.g., nucleic acid, drug, therapeutic agent, diagnostic agent, prophylactic agent, *etc.)* that is sufficient, when administered to a subject suffering from or susceptible to an infection, disease, disorder, and/or condition, to treat, improve symptoms of, diagnose, prevent, and/or delay the onset of the infection, disease, disorder, and/or condition.

*Therapeutically effective outcome:* As used herein, the term "therapeutically effective outcome" means an outcome that is sufficient in a subject suffering from or susceptible to an infection, disease, disorder, and/or condition, to treat, improve symptoms of, diagnose, prevent, and/or delay the onset of the infection, disease, disorder, and/or condition.

*Total daily dose:* As used herein, a "total daily dose" is an amount given or prescribed in 24 hr period. It may be administered as a single unit dose.

*Totipotency:* As used herein, "totipotency" refers to a cell with a developmental potential to make all of the cells found in the adult body as well as the extra-embryonic tissues, including the placenta.

*Transcription factor:* As used herein, the term "transcription factor" refers to a DNA-binding protein that regulates transcription of DNA into RNA, for example, by activation or repression of transcription. Some transcription factors effect regulation of transcription alone, while others act in concert with other proteins. Some transcription factor can both activate and repress transcription under certain conditions. In general, transcription factors bind a specific target sequence or sequences highly similar to a specific consensus sequence in a regulatory region of a target gene. Transcription factors may regulate transcription of a target gene alone or in a complex with other molecules.

*Transcription:* As used herein, the term "transcription" refers to methods to introduce exogenous nucleic acids into a cell. Methods of transfection include, but are not limited to, chemical methods, physical treatments and cationic lipids or mixtures.

*Transdifferentiation:* As used herein, "transdifferentiation" refers to the capacity of differentiated cells of one type to lose identifying characteristics and to change their phenotype to that of other fully differentiated cells.

*Treating:* As used herein, the term "treating" refers to partially or completely alleviating, ameliorating, improving, relieving, delaying onset of, inhibiting progression of, reducing severity of, and/or reducing incidence of one or more symptoms or features of a particular infection, disease, disorder, and/or condition. For example, "treating" cancer may refer to inhibiting survival, growth, and/or spread of a tumor. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition.

*Unmodified:* As used herein, "unmodified" refers to any substance, compound or molecule prior to being changed in any way. Unmodified may, but does not always, refer to the wild type or native form of a biomolecule. Molecules may undergo a series of modifications whereby each modified molecule may serve as the "unmodified" starting molecule for a subsequent modification.

*Unipotent:* As used herein, "unipotent" when referring to a cell means to give rise to a single cell lineage.

*Vaccine:* As used herein, the phrase "vaccine" refers to a biological preparation that improves immunity to a particular disease.

*Viral protein:* As used herein, the phrase "viral protein" means any protein originating from a virus.

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The disclosure includes cases in which exactly one member of the group is present in, employed in, or otherwise relevant to a given product or process. The disclosure includes cases in which more than one, or all of the group members are present in, employed in, or otherwise relevant to a given product or process.

It is also noted that the term "comprising" is intended to be open and permits but does not require the inclusion of additional elements or steps. When the term "comprising" is used herein, the term "consisting of' is thus also encompassed and disclosed.

Unless otherwise defined, all technical and scientific terms used herein have the same meaning as commonly understood by one of ordinary skill in the art to which this disclosure belongs. Methods and materials are described herein for use in the present disclosure; other, suitable methods and materials known in the art can also be used.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different cases of the disclosure, to the tenth of the unit of the lower limit of the range, unless the context clearly dictates otherwise.

Section and table headings are not intended to be limiting.

### EXAMPLES

### Example 1. Manufacture of chimeric polynucleotides

The manufacture of chimeric polynucleotides and or parts or regions thereof may be accomplished utilizing the methods taught in International Publication No. WO2014152027, entitled "Manufacturing Methods for Production of RNA Transcripts" (Attorney Docket number M500).

Purification methods may include those taught in International Publication No. WO2014152030, entitled "Methods of removing DNA fragments in mRNA production" (Attorney Docket number M501); International Publication No. WO2014152031, entitled "Ribonucleic acid purification" (Attorney Docket number M502).

Detection and characterization methods of the polynucleotides may be performed as taught in International Publication No. WO2014144039, entitled "Characterization of mRNA Molecules (Attorney Docket number M505).

Characterization of the chimeric polynucleotides may be accomplished using a procedure selected from the group consisting of polynucleotide mapping, reverse transcriptase sequencing, charge distribution analysis, and detection of RNA impurities, wherein characterizing comprises determining the RNA transcript sequence, determining the purity of the RNA transcript, or determining the charge heterogeneity of the RNA transcript. Such methods are taught in, for example, International Publication No. WO2014144039, entitled "Analysis of mRNA Heterogeneity and Stability" (Attorney Docket number M506) and International Publication Number WO2014144767 entitled "Ion Exchange Purification of mRNA" (Attorney Docket number M507).

### Example 2. Chimeric polynucleotide synthesis: triphosphate route Introduction

Two regions or parts of a chimeric polynucleotide may be joined or ligated using triphosphate chemistry.

According to this method, a first region or part of 100 nucleotides or less is chemically synthesized with a 5' monophosphate and terminal 3'desOH or blocked OH. If the region is longer than 80 nucleotides, it may be synthesized as two strands for ligation.

If the first region or part is synthesized as a non-positionally modified region or part using in vitro transcription (IVT), conversion the 5'monophosphate with subsequent capping of the 3' terminus may follow.

Monophosphate protecting groups may be selected from any of those known in the art.

The second region or part of the chimeric polynucleotide may be synthesized using either chemical synthesis or IVT methods. IVT methods may include an RNA polymerase that can utilize a primer with a modified cap. Alternatively, a cap of up to 130 nucleotides may be chemically synthesized and coupled to the IVT region or part.

It is noted that for ligation methods, ligation with DNA T4 ligase, followed by treatment with DNAse should readily avoid concatenation.

The entire chimeric polynucleotide need not be manufactured with a phosphate-sugar backbone. If one of the regions or parts encodes a polypeptide, then it is preferable that such region or part comprise a phosphate-sugar backbone.

Ligation is then performed using any known click chemistry, orthoclick chemistry, solulink, or other bioconjugate chemistries known to those in the art.

### Synthetic route

The chimeric polynucleotide is made using a series of starting segments. Such segments include:

(a) Capped and protected 5' segment comprising a normal 3'OH (SEG. 1)

(b) 5' triphosphate segment which may include the coding region of a polypeptide and comprising a normal 3'OH (SEG. 2)

(c) 5' monophosphate segment for the 3' end of the chimeric polynucleotide (e.g., the tail) comprising cordycepin or no 3'OH (SEG. 3)

After synthesis (chemical or IVT), segment 3 (SEG. 3) is treated with cordycepin and then with pyrophosphatase to create the 5 'monophosphate.

Segment 2 (SEG. 2) is then ligated to SEG. 3 using RNA ligase. The ligated polynucleotide is then purified and treated with pyrophosphatase to cleave the diphosphate. The treated SEG.2-SEG. 3 construct is then purified and SEG. 1 is ligated to the 5' terminus. A further purification step of the chimeric polynucleotide may be performed.

Where the chimeric polynucleotide encodes a polypeptide, the ligated or joined segments may be represented as: 5'UTR (SEG. 1), open reading frame or ORF (SEG. 2) and 3'UTR+PolyA (SEG. 3).

The yields of each step may be as much as 90-95%.

### Example 3: PCR for cDNA Production

PCR procedures for the preparation of cDNA are performed using 2x KAPA HIFI^{™} HotStart ReadyMix by Kapa Biosystems (Woburn, MA). This system includes 2x KAPA ReadyMix12.5 µl; Forward Primer (10 uM) 0.75 µl; Reverse Primer (10 uM) 0.75 µl; Template cDNA -100 ng; and dH₂0 diluted to 25.0 µl. The reaction conditions are at 95° C for 5 min. and 25 cycles of 98° C for 20 sec, then 58° C for 15 sec, then 72° C for 45 sec, then 72° C for 5 min. then 4° C to termination.

The reverse primer of the instant disclosure incorporates a poly-T₁₂₀ (SEQ ID NO: 24) for a poly-A₁₂₀ (SEQ ID NO: 21) in the mRNA. Other reverse primers with longer or shorter poly(T) tracts can be used to adjust the length of the poly(A) tail in the polynucleotide mRNA.

The reaction is cleaned up using Invitrogen's PURELINK^{™} PCR Micro Kit (Carlsbad, CA) per manufacturer's instructions (up to 5 µg). Larger reactions will require a cleanup using a product with a larger capacity. Following the cleanup, the cDNA is quantified using the NANODROP^{™} and analyzed by agarose gel electrophoresis to confirm the cDNA is the expected size. The cDNA is then submitted for sequencing analysis before proceeding to the *in vitro* transcription reaction.

### Example 4. In vitro Transcription (IVT)

The *in vitro* transcription reaction generates polynucleotides containing uniformly modified polynucleotides. Such uniformly modified polynucleotides may comprise a region or part of the chimeric polynucleotides of the disclosure. The input nucleotide triphosphate (NTP) mix is made in-house using natural and un-natural NTPs.

A typical *in vitro* transcription reaction includes the following:

| | | |
|---|---|---|
| 1 | Template cDNA | 1.0 µg |
| 2 | 10× transcription buffer (400 mM Tris-HCl pH 8.0, 190 mM MgCl₂, 50 mM DTT, 10 mM Spermidine) | 2.0 µl |
| 3 | Custom NTPs (25mM each) | 7.2 µl |
| 4 | RNase Inhibitor | 20 U |
| 5 | T7 RNA polymerase | 3000 U |
| 6 | dH₂0 | Up to 20.0 µl. and |
| 7 | Incubation at 37° C for 3 hr-5 hrs. | |

The crude IVT mix may be stored at 4° C overnight for cleanup the next day. 1 U of RNase-free DNase is then used to digest the original template. After 15 minutes of incubation at 37° C, the mRNA is purified using Ambion's MEGACLEAR^{™} Kit (Austin, TX) following the manufacturer's instructions. This kit can purify up to 500 µg of RNA. Following the cleanup, the RNA is quantified using the NanoDrop and analyzed by agarose gel electrophoresis to confirm the RNA is the proper size and that no degradation of the RNA has occurred.

### Example 5. Enzymatic Capping

Capping of a polynucleotide is performed as follows where the mixture includes: IVT RNA 60 µg-180µg and dH₂0 up to 72 µl. The mixture is incubated at 65° C for 5 minutes to denature RNA, and then is transferred immediately to ice.

The protocol then involves the mixing of 10× Capping Buffer (0.5 M Tris-HCl (pH 8.0), 60 mM KCl, 12.5 mM MgCl₂) (10.0 µl); 20 mM GTP (5.0 µl); 20 mM S-Adenosyl Methionine (2.5 µl); RNase Inhibitor (100 U); 2'-O-Methyltransferase (400U); Vaccinia capping enzyme (Guanylyl transferase) (40 U); dH₂0 (up to 28 µl); and incubation at 37° C for 30 minutes for 60 µg RNA or up to 2 hours for 180 µg of RNA.

The polynucleotide is then purified using Ambion's MEGACLEAR^{™} Kit (Austin, TX) following the manufacturer's instructions. Following the cleanup, the RNA is quantified using the NANODROP^{™} (ThermoFisher, Waltham, MA) and analyzed by agarose gel electrophoresis to confirm the RNA is the proper size and that no degradation of the RNA has occurred. The RNA product may also be sequenced by running a reverse-transcription-PCR to generate the cDNA for sequencing.

### Example 6. PolyA Tailing Reaction

Without a poly-T in the cDNA, a poly-A tailing reaction must be performed before cleaning the final product. This is done by mixing Capped IVT RNA (100 µl); RNase Inhibitor (20 U); 10× Tailing Buffer (0.5 M Tris-HCl (pH 8.0), 2.5 M NaCl, 100 mM MgCl₂)(12.0 µl); 20 mM ATP (6.0 µl); Poly-A Polymerase (20 U); dH₂0 up to 123.5 µl and incubation at 37° C for 30 min. If the poly-A tail is already in the transcript, then the tailing reaction may be skipped and proceed directly to cleanup with Ambion's MEGACLEAR^{™} kit (Austin, TX) (up to 500 µg). Poly-A Polymerase is preferably a recombinant enzyme expressed in yeast.

It should be understood that the processivity or integrity of the polyA tailing reaction may not always result in an exact size polyA tail. Hence polyA tails of approximately between 40-200 nucleotides, e.g., about 40, 50, 60, 70, 80, 90, 91, 92, 93, 94, 95, 96, 97, 98, 99, 100, 101, 102, 103, 104, 105, 106, 107, 108, 109, 110, 150-165, 155, 156, 157, 158, 159, 160, 161, 162, 163, 164 or 165 are within the scope of the disclosure.

### Example 7. Natural 5' Caps and 5' Cap Analogues

5'-capping of polynucleotides may be completed concomitantly during the *in vitro*-transcription reaction using the following chemical RNA cap analogs to generate the 5'-guanosine cap structure according to manufacturer protocols: 3'-O-Me-m7G(5')ppp(5') G [the ARCA cap];G(5')ppp(5')A; G(5')ppp(5')G; m7G(5')ppp(5')A; m7G(5')ppp(5')G (New England BioLabs, Ipswich, MA). 5'-capping of modified RNA may be completed post-transcriptionally using a Vaccinia Virus Capping Enzyme to generate the "Cap 0" structure: m7G(5')ppp(5')G (New England BioLabs, Ipswich, MA). Cap 1 structure may be generated using both Vaccinia Virus Capping Enzyme and a 2'-O methyl-transferase to generate: m7G(5')ppp(5')G-2'-O-methyl. Cap 2 structure may be generated from the Cap 1 structure followed by the 2'-O-methylation of the 5'-antepenultimate nucleotide using a 2'-O methyl-transferase. Cap 3 structure may be generated from the Cap 2 structure followed by the 2'-O-methylation of the 5'-preantepenultimate nucleotide using a 2'-O methyl-transferase. Enzymes are preferably derived from a recombinant source.

When transfected into mammalian cells, the modified mRNAs have a stability of between 12-18 hours or more than 18 hours, e.g., 24, 36, 48, 60, 72 or greater than 72 hours.

### Example 8. Capping Assays

### A. Protein Expression Assay

Chimeric polynucleotides encoding a polypeptide, containing any of the caps taught herein can be transfected into cells at equal concentrations. 6, 12, 24 and 36 hours post-transfection the amount of protein secreted into the culture medium can be assayed by ELISA. Synthetic chimeric polynucleotides that secrete higher levels of protein into the medium would correspond to a synthetic chimeric polynucleotide with a higher translationally-competent Cap structure.

### B. Purity Analysis Synthesis

Chimeric polynucleotides encoding a polypeptide, containing any of the caps taught herein can be compared for purity using denaturing Agarose-Urea gel electrophoresis or HPLC analysis. Chimeric polynucleotides with a single, consolidated band by electrophoresis correspond to the higher purity product compared to chimeric polynucleotides with multiple bands or streaking bands. Synthetic chimeric polynucleotides with a single HPLC peak would also correspond to a higher purity product. The capping reaction with a higher efficiency would provide a more pure chimeric polynucleotide population.

### C. Cytokine Analysis

Chimeric polynucleotides encoding a polypeptide, containing any of the caps taught herein can be transfected into cells at multiple concentrations. 6, 12, 24 and 36 hours post-transfection the amount of pro-inflammatory cytokines such as TNF-alpha and IFN-beta secreted into the culture medium can be assayed by ELISA. Chimeric polynucleotides resulting in the secretion of higher levels of pro-inflammatory cytokines into the medium would correspond to a chimeric polynucleotides containing an immune-activating cap structure.

### D. Capping Reaction Efficiency

Chimeric polynucleotides encoding a polypeptide, containing any of the caps taught herein can be analyzed for capping reaction efficiency by LC-MS after nuclease treatment. Nuclease treatment of capped chimeric polynucleotides would yield a mixture of free nucleotides and the capped 5'-5-triphosphate cap structure detectable by LC-MS. The amount of capped product on the LC-MS spectra can be expressed as a percent of total chimeric polynucleotide from the reaction and would correspond to capping reaction efficiency. The cap structure with higher capping reaction efficiency would have a higher amount of capped product by LC-MS.

### Example 9. Agarose Gel Electrophoresis of Modified RNA or RT PCR Products

Individual chimeric polynucleotides (200-400 ng in a 20 µl volume) or reverse transcribed PCR products (200-400 ng) are loaded into a well on a non-denaturing 1.2% Agarose E-Gel (Invitrogen, Carlsbad, CA) and run for 12-15 minutes according to the manufacturer protocol.

### Example 10. Nanodrop Modified RNA Quantification and UV Spectral Data

Modified chimeric polynucleotides in TE buffer (1 µl) are used for Nanodrop UV absorbance readings to quantitate the yield of each chimeric polynucleotide from a chemical synthesis or an *in vitro* transcription reaction.

### Example 11. Formulation of Modified mRNA Using Lipidoids

Chimeric polynucleotides are formulated for *in vitro* experiments by mixing the chimeric polynucleotides with the lipidoid at a set ratio prior to addition to cells. *In vivo* formulation may require the addition of extra ingredients to facilitate circulation throughout the body. To test the ability of these lipidoids to form particles suitable for *in vivo* work, a standard formulation process used for siRNA-lipidoid formulations may be used as a starting point. After formation of the particle, chimeric polynucleotide is added and allowed to integrate with the complex. The encapsulation efficiency is determined using a standard dye exclusion assays.

### Example 12. Method of Screening for Protein Expression

### A. Electrospray Ionization

A biological sample which may contain proteins encoded by a chimeric polynucleotide administered to the subject is prepared and analyzed according to the manufacturer protocol for electrospray ionization (ESI) using 1, 2, 3 or 4 mass analyzers. A biologic sample may also be analyzed using a tandem ESI mass spectrometry system.

Patterns of protein fragments, or whole proteins, are compared to known controls for a given protein and identity is determined by comparison.

### B. Matrix-Assisted Laser Desorption/Ionization

A biological sample which may contain proteins encoded by one or more chimeric polynucleotides administered to the subject is prepared and analyzed according to the manufacturer protocol for matrix-assisted laser desorption/ionization (MALDI).

Patterns of protein fragments, or whole proteins, are compared to known controls for a given protein and identity is determined by comparison.

### C. Liquid Chromatography-Mass spectrometry-Mass spectrometry

A biological sample, which may contain proteins encoded by one or more chimeric polynucleotides, may be treated with a trypsin enzyme to digest the proteins contained within. The resulting peptides are analyzed by liquid chromatography-mass spectrometry-mass spectrometry (LC/MS/MS). The peptides are fragmented in the mass spectrometer to yield diagnostic patterns that can be matched to protein sequence databases via computer algorithms. The digested sample may be diluted to achieve 1 ng or less starting material for a given protein. Biological samples containing a simple buffer background (e.g. water or volatile salts) are amenable to direct in-solution digest; more complex backgrounds (e.g. detergent, non-volatile salts, glycerol) require an additional clean-up step to facilitate the sample analysis.

Patterns of protein fragments, or whole proteins, are compared to known controls for a given protein and identity is determined by comparison.

### Example 13. Cyclization and/or concatemerization

A chimeric polynucleotide may be cyclized, or concatemerized, to generate a translation competent molecule to assist interactions between poly-A binding proteins and 5'-end binding proteins. The mechanism of cyclization or concatemerization may occur through at least 3 different routes: 1) chemical, 2) enzymatic, and 3) ribozyme catalyzed. The newly formed 5'-/3'-linkage may be intramolecular or intermolecular.

In the first route, the 5'-end and the 3'-end of the nucleic acid contain chemically reactive groups that, when close together, form a new covalent linkage between the 5'-end and the 3'-end of the molecule. The 5'-end may contain an NHS-ester reactive group and the 3'-end may contain a 3'-amino-terminated nucleotide such that in an organic solvent the 3'-amino-terminated nucleotide on the 3'-end of a synthetic mRNA molecule will undergo a nucleophilic attack on the 5'-NHS-ester moiety forming a new 5'-/3'-amide bond.

In the second route, T4 RNA ligase may be used to enzymatically link a 5'-phosphorylated nucleic acid molecule to the 3'-hydroxyl group of a nucleic acid forming a new phosphorodiester linkage. In an example reaction, 1µg of a nucleic acid molecule is incubated at 37°C for 1 hour with 1-10 units of T4 RNA ligase (New England Biolabs, Ipswich, MA) according to the manufacturer's protocol. The ligation reaction may occur in the presence of a split polynucleotide capable of basepairing with both the 5'- and 3'- region in juxtaposition to assist the enzymatic ligation reaction.

In the third route, either the 5'-or 3'-end of the cDNA template encodes a ligase ribozyme sequence such that during *in vitro* transcription, the resultant nucleic acid molecule can contain an active ribozyme sequence capable of ligating the 5'-end of a nucleic acid molecule to the 3'-end of a nucleic acid molecule. The ligase ribozyme may be derived from the Group I Intron, Group I Intron, Hepatitis Delta Virus, Hairpin ribozyme or may be selected by SELEX (systematic evolution of ligands by exponential enrichment). The ribozyme ligase reaction may take 1 to 24 hours at temperatures between 0 and 37°C.

### Example 14. Synthesis of mRNA Constructs

### Restriction Digest of Plasmid

DNA plasmid was digested by incubation at 37°C for 2 hr in a 50 µL reaction containing DNA plasmid (50 ng/µL), BSA (1X), IX NEBuffer 4 (50mM potassium acetate, 20mM Tris-acetate, 10mM magnesium acetate, ImM DTT, pH 7.9), and XbaI (400 U/mL) (New England Biolabs). The restriction digest was analyzed by 1% agarose gel and used directly for PCR.

### DNA Template Amplification

The desired DNA template was amplified by PCR in 100 uL reactions using linearized plasmid (20 ng), dNTPs (0.2 µM each), forward primer (0.2 µM), reverse primer (0.2 µM), IX Q5 reaction buffer, and Q5 high-fidelity DNA polymerase (20 U/mL) (New England Biolabs). All components were kept on ice until added to the thermocycler. The following thermocycler method shown in Table 7 was run.

**Table 7. Theromocycler Method**

| **STEP** | **TEMP** | **TIME** |
|---|---|---|
| Initial Denaturation | 98°C | 4 minutes |
| 30 Cycles | 98°C | 15 seconds |
| | 72°C | 45 seconds |
| | 72°C | 20 seconds per kb |
| Final Extension | 72°C | 5 minutes |
| Hold | 4°C | |

The DNA sequences of the linearized plasmids are listed in Table 8 and primer sequences are listed in Table 9.

**Table 8. DNA Sequences of Linearized Plasmids Used for PCR Amplification (5' to 3')**

| **mCherry** | **SEQ ID NO** |
|---|---|
| | 4 |
| **NanoLuc** | |
| | 5 |
| **GCSF** | |
| | 6 |
| | |

**Table 9. Primer Sequences Used for PCR Amplification (5' to 3')**

| Description | Sequence | SEQ ID NO |
|---|---|---|
| Forward Primer | TAATACGACTCACTATAGGG | 7 |
| Tailless Reverse Primer | | 8 |
| T80 Reverse Primer | | 9 |
| T140 Reverse Primer | | 10 |

The PCR product was analyzed by capillary electrophoresis (CE) (Agilent 2100 Bioanalyzer) and desalted by ultrafiltration (Amicon).

### In Vitro Transcription

In vitro transcription (IVT) reactions were performed in 50 uL containing template DNA (25 ng/µL), NTPs (7.6 mM each), IX T7 IVT buffer, RNase Inhibitor (1 U/µL), Pyrophosphatase (1 U/µL), and T7 RNA polymerase (7 U/µL) (NEB). In general, 24 50uL reactions per construct were used. Modified mRNA was generated using 5-methyl-CTP and 1-methyl-pseudoUTP. IVT reactions were incubated at 37 °C for 4 hr, after which 2.5 µL of DNase I (2000 U/mL) (NEB) was added, and the reaction allowed to incubated for another 45 min. The reactions were combined and purified using MEGAclear spin columns (Ambion) and eluted in 250 µL water. The IVT product was analyzed by CE (Agilent 2100 Bioanalyzer). Table 10 gives the sequences for the RNA transcripts. For mRNA containing a 5'-azide, transcriptions were performed using a 1:16 ratio of GTP: 5'-azido-5'-deoxyguanosine (Carbosynth, solubilized in DMSO) with final concentrations of 0.45 mM and 7.15 mM, respectively. Constructs of this nature were not subjected to enzymatic capping.

**Table 10. Sequences of mRNA Constructs 1-7 where U = 1-methylpseudouridine and C = 5-methyl-cytidine.**

| **Description** | **Sequence** | **SEQ ID No.** |
|---|---|---|
| RNA 1 (mCherry, 874 nt) , no poly(A) tail 5' UTR in bold, 3'UTR in italics | | 11 |
| RNA 2 (NanoLuc, 679 nt) , no poly(A) tail 5' UTR in bold, 3'UTR in italics | | 12 |
| RNA 3 (GCSF, 778 nt), no poly(A) tail, 5' UTR in | | 13 |
| bold, 3'UTR in italics | | |
| RNA 4 (mCherry, 80 nt poly(A) tail, 954 nt) , 5' UTR in bold, 3'UTR in italics | | 14 |
| RNA 5 (mCherry, 140 nt poly(A) tail, 1014 nt), 5' UTR in bold, 3'UTR in italics | | 15 |
| | | |
| RNA 6 (NanoLuc, 140 nt poly(A) tail, 819 nt), 5' UTR in bold, 3'UTR in italics | | 16 |
| RNA 7 (GCSF, 140 nt poly(A) tail, 918 nt), 5' UTR in bold, 3'UTR in italics | | 17 |
| | | |

### Capping

The RNA (220 µL) eluted from the last IVT step was denatured by heating to 65 °C for 15 minutes followed by cooling on ice for at least 2 min. The capping reaction was performed in 300 µL with the denatured RNA (220 µL), GTP (1mM), SAM (0.5 mM), RNase Inhibitor (1 U/µL), IX Capping buffer, and Vaccinia capping complex (0.4 U/µL) (NEB). These reactions were incubated at 37 °C for 2 hr on the thermomixer. The reactions were purified using MEGAclear spin columns (Ambion) and eluted in 250 µL water. The eluted mRNA was analyzed by CE (Agilent 2100 Bioanalyzer) and quantified by UV absorbance.

### Example 15. Incorporation of 3'-azido-2',3'-dideoxyadenosine-5'-triphosphate (3'-azido-ddATP)

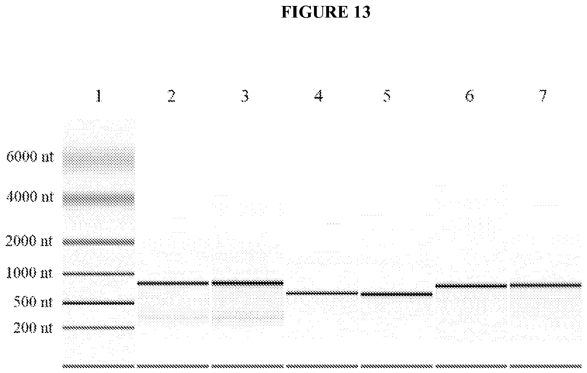

3'-azido-ddATP was incorporated into the 3'-end of tailless RNA 1-3 (Table 10) using yeast poly(A) polymerase as depicted in Scheme 1. In 100 µL reactions, RNA transcript (0.2 µM), 3'-azido-ddATP (500 µM), murine RNase inhibitor (NEB) (1 U/µL), 1× reaction buffer (20 mM Tris-HCl, pH 7.0, 0.6 mM MnCl₂, 20 µM EDTA, 0.2 mM DTT, 100 µg/mL acetylated BSA, 10% glycerol), and yeast poly(A) polymerase (2400 U, Affymetrix) were incubated at 37°C for 1 hr, followed by ethanol precipitation. The RNA was dissolved in 100 µL DEPC-treated H₂O and further purified by gel filtration using an illustra NICK column or illustra MicroSpin G-25 column (GE Healthcare). The RNA was concentrated, if necessary, by ultrafiltration using an Amicon Ultra-0.5 centrifugal device (100K NMWL), was quantified by UV absorbance, and analyzed by capillary electrophoresis (CE) (Agilent 2100 Bioanalyzer) (Figure 13). The RNA obtained at this point was a mixture of unmodified and 3 '-azido RNA which cannot be distinguished by CE, and this mixture was used without further purification in subsequent reactions.

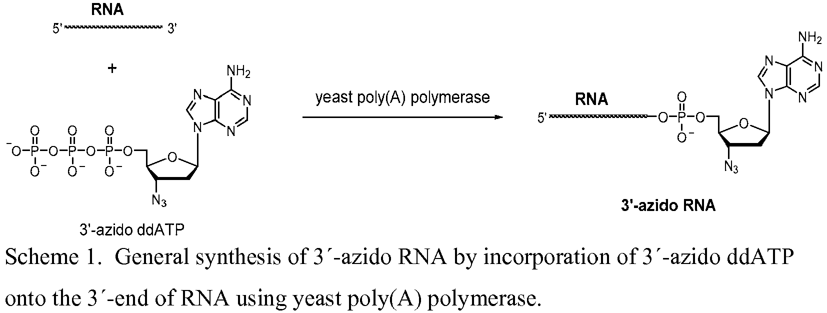

5'-bicyclo[6.1.0]nonyne (BCN) poly(A) tails 1-6 were synthesized for generating RNA-poly(A) tail conjugates using strain-promoted azide-alkyne cycloaddition (SPAAC) chemistry. While tails 1 and 4 could be synthesized directly by solid phase phosphoramidite oligomerization technology, tails 2, 3, 5, and 6 were first synthesized as the 5'-amino derivatives (tails 2a, 3a, 5a, and 6a) which were then coupled to the reactive BCN group via NHS chemistry (Scheme 2).

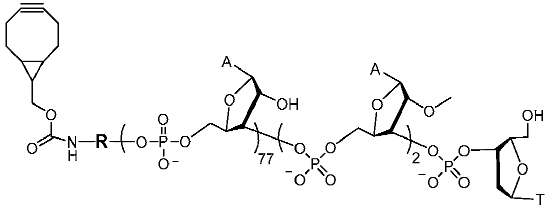

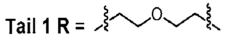

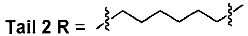

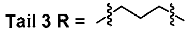

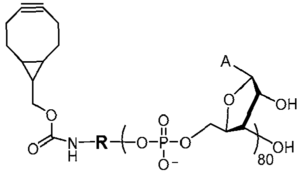

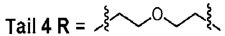

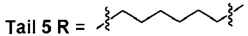

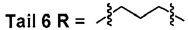

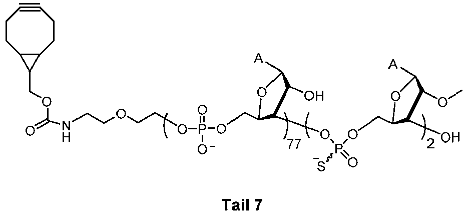

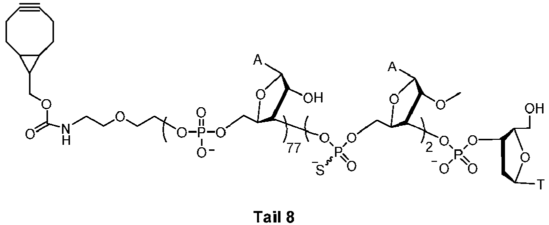

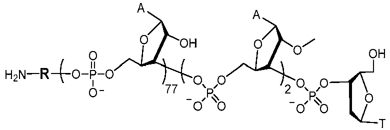

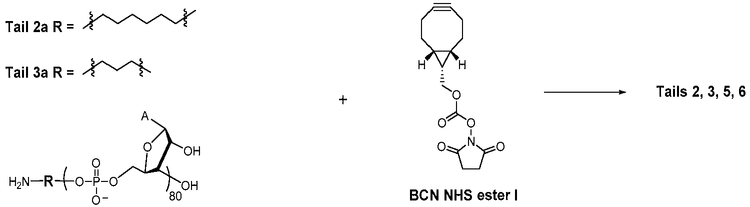

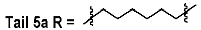

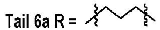

### Scheme 2. Synthesis of tails 2, 3, 5, and 6 was achieved by coupling the corresponding 5'-amino oligoribonucleotides to the BCN N-hydroxysuccinimide ester I.

Tails 1, 2a, 3a, 4, 5a, 6a, 7, and 8 were assembled on an Expedite 8909 DNA/RNA synthesizer (Perseptive) employing solid phase phosphoramidite oligomerization technology. Syntheses were initiated on a solid support made of controlled pore glass (CPG, 1000Ǻ) with either immobilized 3'-O-dimethoxytrityl-thymidine at a loading of 31 µmol/g (obtained from Prime Synthesis, Aston, PA, USA) generating a 3 '-3 '-linkage at the 3 '-end or immobilized 5'-O-dimethoxytrityl-adenosine loaded at 32 µmol/g (Chemgenes, Wilmington, MA; USA). For the synthesis of the intended sequences the following phosphoramidites were used: (5'-O-dimetboxytrityl-N6-(benzoyl)-2'-O-t-butyldimethylsilyl-adenosine-3'-O-(2-cyanoethyl-N,N-diisopropylamino) phosphoramidite, (5'-O-dimethoxytrityl-N6-(benzoyl)-2'-O-methyl-adenosine-3'-O-(2-cyanoethyl-N,N-diisopropylamino) phosphoramidite (SAFC Proligo, Hamburg, Germany) and 5'-Click-easy^{®} BCN CEP II (Berry & Associates, Inc., Dexter; MI, USA). In order to introduce an amino-linker at the 5 '-end either a trifluoracetyl (TFA)-protected aminohexyl phosphoramidite (SAFC Proligo, Hamburg, Germany) was used. 3'-Amino linkers were generated using a phthalimidyl protected aminohexyl derivatized CPG available from Prime Synthesis or a phthalimidyl protected aminopropyl derivatized CPG available from Glen Research (Sterling, Virginia, USA). All amidites were dissolved in anhydrous acetonitrile (100 mM) and molecular sieves (3Ǻ) were added. 5-Ethyl thiotetrazole (ETT, 500 mM in acetonitrile) was used as activator solution. Coupling times were 5 minutes for the nucleoside phosphoramidites and 12 minutes for the linker amidites. In order to introduce phosphorothioate linkages a 50 mM solution of 3-((Dimethylamino-methylidene)amino)-3H-1,2,4-dithiazole-3-thione (DDTT, obtained from Chemgenes) in anhydrous acetonitrile/pyridine (1:1 v/v) was employed. Ancillary reagents (Deblock, Oxidizer, Cap A and Cap B) for RNA synthesis were purchased from SAFC Proligo (Hamburg, Germany). After finalization of the solid phase synthesis, the dried solid support was transferred to a 15 mL polypropylene tube and the RNA was cleaved from the solid support and deprotected by methods known in the field (Wincott F., et al, Nucleic Acid Res., 1995, 23, 2677-84).

Crude oligomers were purified by RP HPLC using an XBridge C18 19x 50 mm column (Waters, Eschborn, Germany) on an AKTA Explorer system (GE Healthcare, Freiburg, Germany). Buffer A was 100 mM triethylammonium acetate (Biosolve, Valkenswaard, The Netherlands) and buffer B contained 95% acetonitrile in buffer A. A flow rate of 15 mL/min was employed. UV traces at 260 and 280 were recorded. A gradient of 5% B to 45% B within 57 column volumes was employed. Appropriate fractions were pooled and precipitated with 3M NaOAc, pH=5.2 and 70% ethanol. The pellet was isolated by centrifugation, dissolved in water and the concentration of the solution was determined by absorbance measurement at 260 nm in a UV photometer (Eppendorf, Germany). Alternatively, crude oligomers were purified by anion exchange HPLC using a Dionex DNA PAC PA200 column (9x 250 mm) on an AKTA Purifier equipped with autosampler A905 and Frac950 (GE Helthcare, Freiburg, Germany). The modified RNA sequences were eluted using a buffer system consisting of Buffer A which contained 10 mM NaClO₄, 20 mM Tris, 100 mM EDTA, 20% acetonitrile and Buffer B which contained 500 mM NaClO₄ in Buffer A. A flow rate of 5 mL/min and a gradient from 5% Buffer B to 95% Buffer B in 15 column volumes (CV) were employed. Elution was recorded at 280 nm, and appropriately sized fractions were taken. Fractions containing the desired sequence were pooled and precipitated with 3M NaOAc, pH=5.2 and 70% Ethanol. The pellet was isolated by centrifugation, washed with 85% ethanol, and dissolved in water, and the concentration of the solution was determined by absorbance measurement at 260 nm in a UV photometer (Eppendorf, Germany).

For the coupling step to produce tails 2, 3, 5, and 6 by NHS chemistry as depicted in Scheme 2, the respective amine-modified oligoribonucleotide was dissolved in 100 mM sodium borate/KCl buffer (pH 8.5) to yield a concentration of 500 µM. Click-easy^{®} BCN N-hydroxysuccinimide ester I (5 mg, Berry & Associates, Inc., Dexter; MI, USA) was dissolved in 50 µL DMSO. The reaction was initiated by addition of about 3 equivalents BCN derivative to the RNA solution. The progress of the reaction was monitored by the change of retention time on an anion exchange HPLC column (Dionex DNA Pac PA200, 4×250 mm, Dionex, Idstein, Germany). After completion of the reaction the oligoribonucleotide conjugate was precipitated using 3 M NaOAc (pH 5.2)/EtOH and purified on a C18 XBridge reversed phase HPLC column (Waters, Eschborn, Germany). Analysis of all oligoribonucleotides is shown in Table 11. In Table 11, "a" refers to purity determined by RP-HPLC and "b" refers to purity determined by AEX-HPLC.

**Table 11. ESI-MS and purity analysis of tails 1-8**

| | Mol weight (calculated) | Mol weight (observed) | Purity (%) by RP |
|---|---|---|---|
| **Tail 1** | 26951.2 | 26950.5 | 92.6^{a} |
| **Tail 4** | 26619.2 | 26618.7 | 97.0^{a} |
| **Tail 2** | 26961.1 | 26962.8 | 91.1^{a} |
| **Tail 3** | 26921.2 | 26920.4 | 97.1^{a} |
| **Tail 5** | 26631.1 | 26632.9 | 93.4^{a} |
| **Tail 6** | 26589.1 | 26588.2 | 98.3^{a} |
| **Tail 7** | 26618.2 | 26622.4 | 86.6^{b} |
| **Tail 8** | 26630.1 | 26631.2 | 85.4^{b} |

### Example 16. 5'-triphosphate, 3'-cyclooctyne oligo synthesis

The structure and sequences of TP oligo 1 and 2 with 5'-triphosphate and a 3'-cyclooctyne are given below. With slight modifications, 5'-triphosphates were synthesized pursuing the method published by Brownlee et al. (Nucleic Acid Res, 1995, 14, 2641-2647) which is adapted from the approach devised by Eckstein (J. Org. Chem, 1989, 54, 631-635). Briefly, the corresponding RNA sequence was synthesized at a 1 µmol scale on phthalimidyl protected aminopropyl derivatized controlled pore glass (CPG) with removal of the final DMT group ("DMT off' synthesis) according to the method described above. After completion of the automated synthesis, the synthesis column was washed with 5 mL acetonitrile and dried with an Argon flush. Subsequently using two syringes, the CPG bound RNA in the synthesis column was treated for 5 minutes at room temperature with 2 mL of a 1:1 mixture of 125 mM 2-chloro-4H-1,3,2-benzodioxaphosphorin-4-one (salicyl phosphorochloridite, obtained from TCI Europe, Eschborn, Germany) in acetonitrile and 50% 2,6-lutidine in acetonitrile. Next, the reagent mixture was removed, and the CPG thoroughly washed with acetonitrile (10 mL) followed by incubation with 2 mL 125 mM tributylammonium pyrophosphate in acetonitrile for 15 min at room temperature. The pyrophosphate salt was prepared according to a published procedure (Current Protocols in Nucleic Acid Chemistry 1.28.1-1.28.16). After wash out of this reagent with acetonitrile (10 mL), oxidation (5 min) was accomplished with the oxidizer solution employed in the solid phase RNA synthesis (100 mM iodine in water/ pyridine 10/90 v/v). Finally, the oxidizer solution was washed out again with acetonitrile, and the CPG was treated with 2 mL 100 mM triethylammonium bicarbaonate in 40% acetonitrile for 2 hours at room temperature. The CPG was washed with acetonitrile, dried for 15 min applying vacuum, and deprotected following the standard RNA deprotection described above.

Crude 5'-triphosphate RNA sequences were purified by anion exchange HPLC using a Dionex DNA PAC PA200 column (9x 250 mm) on an AKTA Purifier equipped with autosampler A905 and Frac950 (GE Helthcare, Freiburg, Germany). The modified RNA sequences were eluted using a buffer system consisting of Buffer A which contained 10 mM NaClO₄, 20 mM Tris, 100 mM EDTA, 20% acetonitrile and Buffer B which contained 500 mM NaClO₄ in Buffer A. A flow rate of 5 mL/min and a gradient from 5% Buffer B to 75% Buffer B in 15 column volumes (CV) was employed. Elution was recorded at 280 nm and appropriately sized fractions were taken. Fractions containing the desired sequence were pooled and precipitated with 3M NaOAc, pH=5.2 and 70% ethanol.

The pellet containing the 3'-aminopropyl derivatized triphosphate RNA was dissolved in 100 mM sodium borate/KCl buffer (pH 8.5) to yield a concentration of about 500 µM. To generate the sequences given in the table below the material was either reacted with CLICK-EASY^{®} BCN N-hydroxysuccinimide ester I or CLICK-EASY^{®} BCN N-hydroxysuccinimide ester II (both available from Berry & Associates, Inc., Dexter; MI, USA) according to the procedure given above. 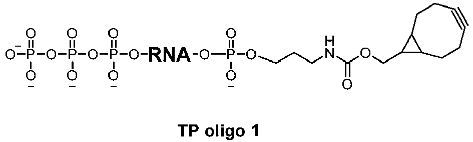 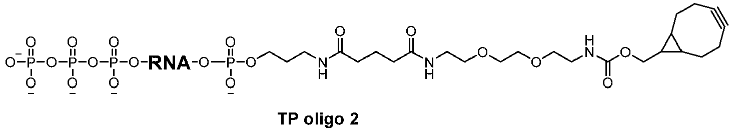 The sequence of TP oligo 1 and TP oligo 2 are shown in Table 12. In Table 12, A is for RNA-A; C is for RNA-C; G is for RNA-G; (C3NH) is aminopropyl; (BCN1) is CLICK-EASY^{®} BCN N-hydroxysuccinimide ester I; (COM-NHS) is CLICK-EASY^{®} BCN N-hydroxysuccinimide ester II; and (ppp) is for triphosphate.

**Table 12. TP Oligo Sequences**

| | **Sequence (5'-3')** | **SEQ ID NO** |
|---|---|---|
| **TP oligo 1** | | 18 |
| **TP oligo 2** | | 19 |

Table 13 shows the EMI-MS and purity analysis of TP oligo 1 and 2.

**Table 13. ESI-MS and purity analysis of TP oligo 1 and 2**

| | Purity (%AEX) | MW (calculated) | MW (measured) |
|---|---|---|---|
| **TP oligo 1** | 83.6 | 7292.5 | 7292.4 |
| **TP oligo 2** | 80.4 | 7458.6 | 7455.7 |

### Example 17. Polv(A) tail conjugation using strain-promoted azide-alkyne cycloaddition (SPAAC)

RNA transcripts modified on the 3 '-end with 3 '-azido-ddATP were ligated to 80 nt 5 '-BCN poly(A) tails using strain-promoted azide-alkyne cycloaddition (SPAAC) to give RNA-poly(A) tail conjugates of the general form shown in Scheme 3. 3 '-azido RNA 1 - 3 and tail 1 were mixed in at least a 1:50 molar ratio, respectively, in water and diluted with ethanol to a final concentration of 70% ethanol. Generally, the concentration of 3'-azido RNA was between 150-400 nM in the reaction mixture. The reactions were shaken at room temperature for 1 hr, diluted with water to 200 µL if necessary, ethanol precipitated, and dissolved in DEPC-treated water. Alternatively, the reactions were purified by MEGAclear kit (Ambion) and eluted in water. The RNA reaction mixture was analyzed by CE (Agilent 2100 Bioanalyzer) as shown in Figure 14. The shifted bands in lanes 3, 5, and 7 represent conjugates RNA 1-tail 1, RNA 2-tail 1, and RNA 3-tail 1, respectively, of the form depicted in Scheme 3. Conversion yields of RNA-tail 1 conjugates determined from CE were 71% for RNA 1, 80% for RNA 2, and 75% for RNA 3.

Conjugates were also made in this manner with RNA 4 and RNA 5, which already contained a poly(A) tail through transcription by T7 RNA polymerase, and tails 1 and 4. Conversion yields for these reactions (CE) were all approximately 95%. Denaturing ultrafiltration in the presence of 6M guanidine hydrochloride was used in order to remove excess unreacted 5 '-BCN tail. Salts were removed by UF buffer exchange with water, and removal of the tail was confirmed by CE.

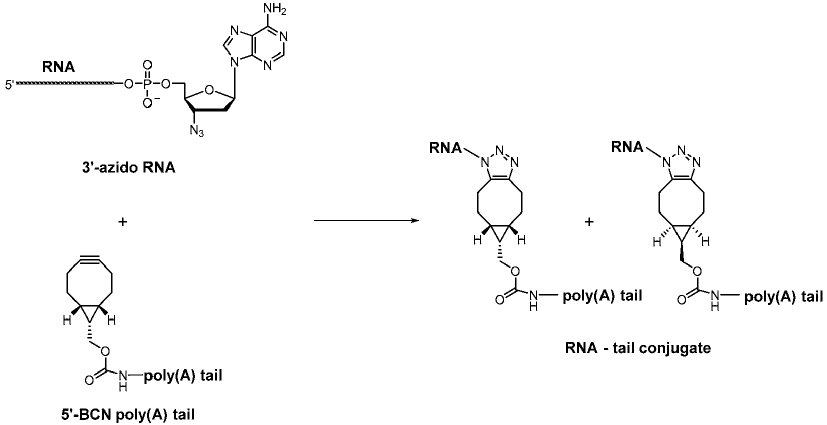

### Scheme 3. General synthesis of RNA-poly(A) tail conjugates by SPAAC with 3'-azido RNA and 5'-BCN poly(A) tail.

### Example 18. DNA splint-templated poly(A) tail conjugation using SPAAC

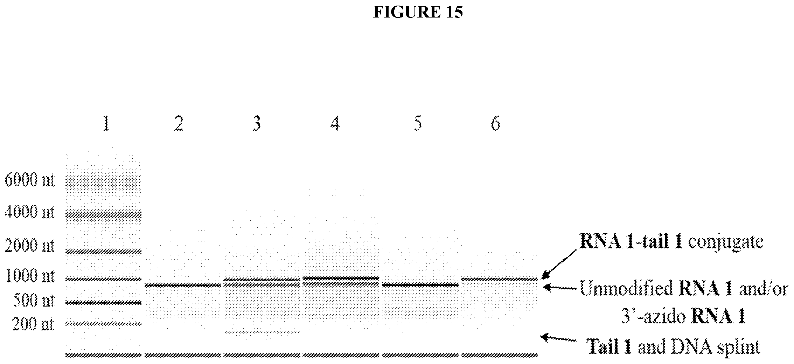

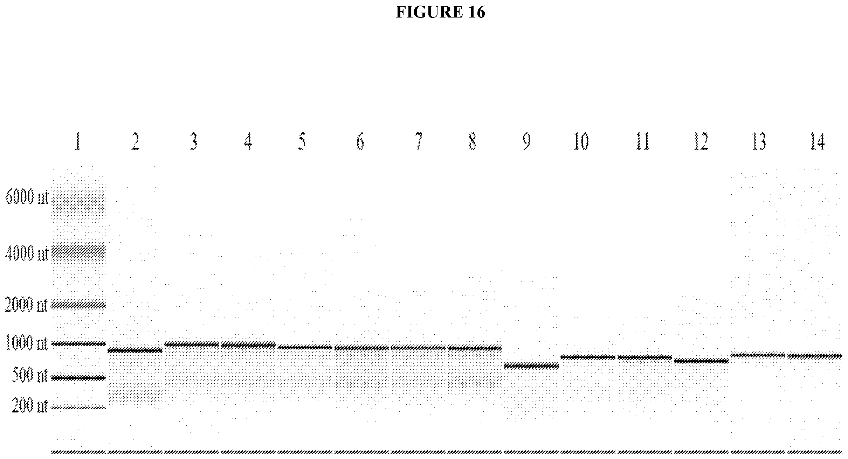

A DNA splint (5'-TTTTTTTTTTTTTTTTTTTTTTTTTTTTTTTTTTTGCCGCCCACTCAGACTTTA T-3'; SEQ ID NO: 20) complementary to the 3'-end of RNA 1-3 and to the poly(A) tail was used to template the SPAAC reaction. RNA-poly(A) tail conjugates were synthesized by mixing 3 '-azido RNA, 5 '-BCN poly(A) tail, and splint in a molar ratio of 1: 3: 3 with final concentrations of 100 nM: 300 nM: 300 nM, respectively, in a 100 µL reaction containing 1 M NaCl. The RNA and DNA splint mixture was heated to 70 °C for 5 min, cooled at 1 °C/min until reaching 25 °C, and maintained at 25 °C overnight. Salts were removed by ultrafiltration (Amicon Ultra-0.5 centrifugal device 100K NMWL). The DNA splint was removed by digestion with TURBO DNase (Ambion) in 50 µL reactions containing no more than 200 ng/µL of the reaction mixture, 1× reaction buffer, and TURBO DNase (2 U). These reactions were incubated for 30 min at 37°C and terminated by the addition of 2 µL of 0.5 M EDTA. The buffer components were again removed by ultrafiltration. The RNA-poly(A) tail conjugates were purified from unmodified and unreacted 3 '-azido RNA using oligo(T) Dynabeads (Ambion). The oligo(T) purification was performed as directed by the manufacturer's protocol, except the beads were washed and the RNA sample prepared in a high salt buffer containing 10 mM Tris-HCl, pH 7.4, 0.5 M NaCl, and 1 mM EDTA, the beads were washed after binding with a low salt buffer containing 10 mM Tris-HCl, pH 7.4, 0.1 M NaCl, and 1 mM EDTA, and the RNA-poly(A) tail conjugates were eluted in 10 mM Tris-HCl, pH 7.4, and 1 mM EDTA. All steps in the click reaction and purification were analyzed by CE (Agilent 2100 Bioanalyzer), an example of which is shown in Figure 15 with the reaction and purification of RNA 1 with tail 1 to give RNA 1-tail 1 conjugate. Figure 16 shows the conjugates after oligo(T) purification of RNA 1 with tails 1-6, and RNA 2 and RNA 3 with tails 1 and 4. The percent yield and purity of these conjugates are given in Table 14.

**Table 14. Yield and Purity of RNA-tail Conjugates after Oligo(T) Purification**

| | **% Yield** | **% Purity (CE)** |
|---|---|---|
| **RNA 1-tail 1** | 36 | 78 |
| **RNA 1-tail 2** | 32 | 76 |
| **RNA 1-tail 3** | 33 | 75 |
| **RNA 1-tail 4** | 38 | 80 |
| **RNA 1-tail 5** | 30 | 76 |
| **RNA 1-tail 6** | 36 | 70 |
| **RNA 1-tail 7** | 39 | 66 |
| **RNA 1-tail 8** | 36 | 67 |
| **RNA 2-tail 1** | 45 | 92 |
| **RNA 2-tail 4** | 46 | 90 |
| **RNA 3-tail 1** | 43 | 95 |
| **RNA 3-tail 4** | 44 | 97 |

### Example 19. DNA splint-templated 5'-oligo conjugation using SPAAC

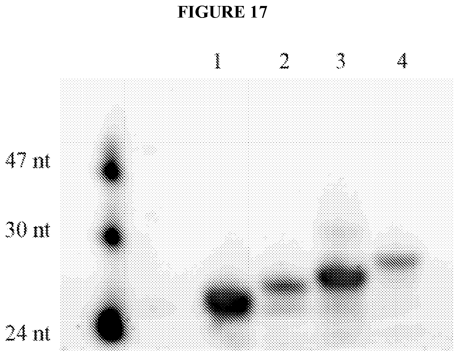

TP oligos 1 and 2 were capped enzymatically using the Vaccinia capping complex (NEB). Prior to the reaction, the RNA oligos were denatured by heating at 65°C for 10 min, followed by cooling on ice. The capping reaction was performed in 100 µL with denatured RNA (15 µM), GTP (1 mM), SAM (0.5 mM), RNase inhibitor (1 U/µL), 1x capping buffer, and Vaccinia capping complex (0.4 U/µL). Reactions were heated at 37°C for an hour on the thermomixer, extracted with phenol/chloroform/isoamyl alcohol (25:24:1) two times, and ethanol precipitated. The capped oligos were analyzed by denaturing PAGE, as shown in Figure 17. In Figure 17, lane 1 is TP oligo 1, lane 2 is capped TP oligo 1, lane 3 is TP oligo 2, lane 4 is capped TP oligo 2.

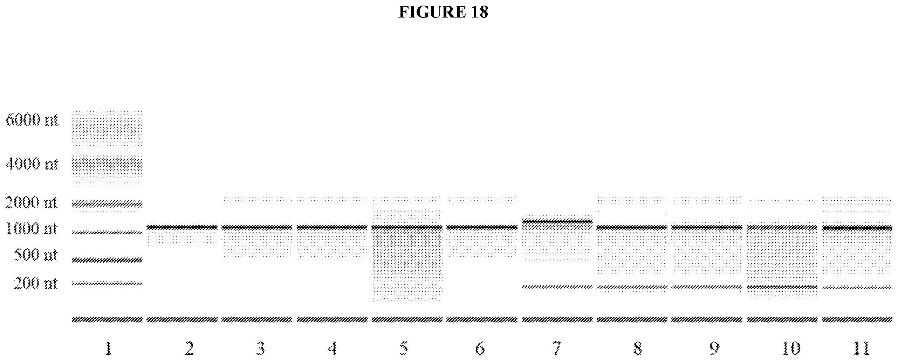

The DNA-splint templated cycloaddition was performed with capped TP oligo 1 and 2, and 5'-azido RNA 5 as described above, except using a splint with the sequence 5'-ACTCTTCTTTTCTCTCTTATTTCCCTTGTTGTTGTTGTTGTTGTCC-3' (SEQ ID NO: 22). Click reactions with uncapped TP oligo 1 and 2 were also performed as controls. 5'-azido RNA 5 was generated as described in the *Synthesis of mRNA Constructs* section. 5-azido RNA 5 and capped TP oligo-RNA 5 conjugates were analyzed by performing SPAAC reactions with a 5'-BCN tail in 70% ethanol as described previously. By determining the ratio of clicked to unclicked 5'-azido RNA 5 and assuming complete reaction of the 5'-azide, the incorporation of 5-azido G into RNA 5 was found to be 75%, as shown in Figure 18. In Figure 18, lane 1 is a ladder, lane 2 is 5'-azido RNA 5, lane 3 is uncapped TP oligo 1-RNA 5 conjugate, lane 4 is capped TP oligo 1-RNA 5 conjugate, lane 5 is uncapped TP oligo 2-RNA 5 conjugate, lane 6 is capped TP oligo 1-RNA 5 conjugate. Lanes 7-11 are those aforementioned samples after the SPAAC reaction with 5'-BCN tail.

However, the capped TP oligo-RNA 5 conjugates showed no reaction with 5'-BCN tail, indicating all the 5'-azido RNA 5 had reacted. Although approximately 25% of the capped TP oligo-RNA 5 conjugate reaction mixtures contain 5'-triphosphate RNA 5, these conjugates were carried forward to the transfection experiments without further purification.

### Example 20. Analysis of 3'-azido-ddATP incorporation

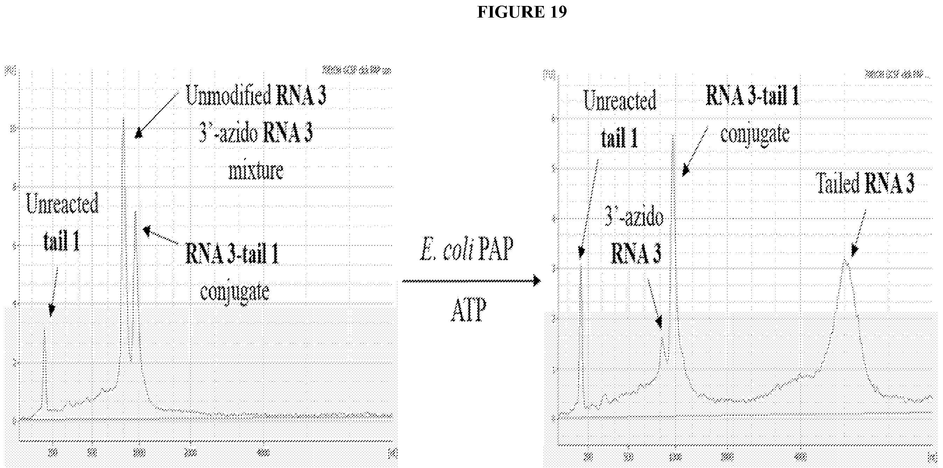

After the SPAAC reactions in 70% ethanol, a mixture of RNA species is produced which presumably includes unmodified RNA, unreacted 3 '-azido RNA, and the desired RNA-tail 1 conjugate. However, this only corresponds to two distinct peaks in the CE electropherogram, as unmodified RNA and 3 '-azido RNA are indistinguishable. Since 3 '-azido RNA and RNA-tail 1 conjugates are blocked on the 3 '-end for poly(A) extension by poly(A) polymerase, only the unmodified RNA is a substrate for enzymatic tailing. The percentage of unmodified RNA, and therefore 3'-azido RNA, can be determined by calculating the % difference in the area of the peak corresponding to the unmodified RNA and 3 '-azido RNA mixture after removal of the unmodified RNA and normalization to the area of the RNA-tail 1 conjugate peak, as depicted in Figure 19. In many cases, the click reaction goes to completion under the conditions described, allowing for a determination of azide incorporation simply by determining the % yield of the RNA-tail 1 conjugate.

In 10 µL, the RNA mixture after the SPAAC reaction in 70% ethanol was treated with E. coli poly(A) polymerase (NEB) (5 U) in a reaction containing the RNA reaction mixture (300 - 400 ng/µL), ATP (1 mM), and 1x reaction buffer (50 mM Tris-HCl, pH 7.9, 250 mM NaCl, 10 mM MgCl₂). Reactions containing no enzyme were also used for comparative controls. Controls where unmodified RNA was mixed with tail 1 and treated with poly(A) polymerase were also performed to ensure that all unmodified RNA would become tailed. Salts were removed from the reactions by ultrafiltration, and the reactions were analyzed by CE. Figure 20 shows the CE gel image for RNA 1, 2, and 3 analyzed in this manner. In the control reactions, all unmodified RNA was lengthened by treatment with PAP. In all these cases, after the SPAAC reaction and treatment with PAP, no RNA is left in the peak representing the putative mixture of unmodified RNA and 3 '-azido RNA, indicating the click reactions went to completion and azide incorporation could be determined from % yield of the RNA-tail conjugate. For these examples, azide incorporation was 60% for RNA 1, 60% for RNA 2, and 75% for RNA 3.

### Example 21. Total Area Under the Curve of mCherry Fluorescence

Indicated mRNA (50 ng) was transfected using Lipofectamine2000^{™} into HeLa cells. The cells were placed in the Incucyte kinetic imaging system (Essen Bioscience) where mCherry fluorescence was measured every 2hrs for 142hrs. Each transfection was performed in triplicate. The total area under the curve was integrated using GraphPad Prism. Tables 15 and 16 give the AUC for mCherry fluorescence of RNA 1-tail conjugates and appropriate controls, where RNA 4 and RNA 5 are T7 RNA polymerase-transcribed constructs containing 80-mer and 140-mer poly(A) tails, respectively. Table 17 gives the AUC for RNA 4-tail and RNA 5-tail conjugates. Table 18 gives the AUC for capped TP oligo-RNA 5 conjugates.

**Table 15. AUC for mCherry fluorescence for RNA 1-tail conjugates**

| | **Average AUC (fluorescence*hr)** | **Std Deviation** |
|---|---|---|
| **Tailless RNA 1** | 2.4E+04 | 1.2E+04 |
| **RNA 4** | 2.0E+07 | 0.0E+00 |
| **RNA 5** | 1.3E+07 | 6.1E+06 |
| **RNA 1-tail 1** | 5.7E+07 | 1.2E+07 |
| **RNA 1-tail 4** | 9.3E+06 | 1.2E+06 |
| **RNA 1-tail 7** | 4.0E+07 | 0.0E+00 |
| **RNA 1-tail 8** | 4.3E+07 | 1.2E+07 |

**Table 16. AUC for mCherry fluorescence for RNA 1-tail conjugates**

| | **Average AUC (fluorescence *hr)** | **Std Deviation** |
|---|---|---|
| **Tailless RNA 1** | 1.5E+06 | 2.9E+05 |
| **RNA 4** | 7.4E+07 | 1.8E+07 |
| **RNA 5** | 1.4E+08 | 2.8E+07 |
| **RNA 1-tail 2** | 8.3E+07 | 7.8E+06 |
| **RNA 1-tail 3** | 8.4E+07 | 1.5E+07 |
| **RNA 1-tail 5** | 2.8E+07 | 2.9E+06 |
| **RNA 1-tail 6** | 3.7E+07 | 4.3E+06 |

**Table 17. AUC for mCherry fluorescence for RNA 4-tail and RNA 5-tail conjugates**

| | **Average AUC (fluorescence*hr)** | **Std Deviation** |
|---|---|---|
| **RNA 4** | 2.7E+08 | 5.8E+07 |
| **RNA 5** | 3.0E+08 | 1.0E+08 |
| **RNA 4-tail 1** | 3.3E+08 | 1.5E+08 |
| **RNA 4-tail 4** | 3.3E+08 | 5.8E+07 |
| **RNA 5-tail 1** | 2.7E+08 | 5.8E+07 |
| **RNA 5-tail 4** | 3.0E+08 | 0.0E+00 |

**Table 18. AUC for mCherry fluorescence for capped TP oligo-RNA 5 conjugates**

| | **Average AUC (fluorescence*hr)** | **Std Deviation** |
|---|---|---|
| **5'-azido RNA 5** | 1.0E+06 | 0.0E+00 |
| **RNA 5** | 6.7E+07 | 3.3E+07 |
| **uncapped TP oligo 1-RNA 5** | 4.3E+06 | 2.2E+06 |
| **capped TP oligo 1-RNA 5** | 8.7E+07 | 2.0E+07 |
| **uncapped TP oligo 2-RNA 5** | 2.3E+06 | 1.3E+06 |
| **capped TP oligo 2-RNA 5** | 5.7E+07 | 2.1E+07 |

### Example 22. NanoLuciferase activity in HeLa cells

Indicated mRNA (25 ng) was transfected in triplicate using Lipofectamine2000^{™} into HeLa cells. After incubation overnight, the cells were lysed in GLO lysis buffer (Promega). NanoGlo substrate was added and luminescent signal was quantified using Synergy MicroPlate Reader (BioTek). Table 19 gives the nanoLuciferase activity for RNA 2-tail conjugates and appropriate controls, where RNA 6 is a T7 RNA polymerase-transcribed construct containing a 140-mer poly(A) tail.

**Table 19. NanoLuciferase activity for RNA 2-tail conjugates**

| | **Average (RLU)** | **Std Deviation** |
|---|---|---|
| **Tailless RNA 2** | 7.0E+05 | 2.4E+05 |
| **RNA 6** | 6.7E+06 | 6.2E+05 |
| **RNA 2-tail 1** | 2.9E+07 | 4.6E+06 |
| **RNA 2-tail 4** | 2.2E+07 | 2.1E+06 |

### Example 23. Human GCSF expression in HeLa cells

Indicated mRNA (250 ng) was transfected in triplicate using Lipofectamine2000^{™} into HeLa cells. After incubation overnight, the supernatant was collected and used to measure the levels of human GCSF (R&D Systems). Table 20 gives the expression levels of GCSF for RNA 3-tail conjugates and appropriate controls, where RNA 7 is a T7 RNA polymerase-transcribed construct containing a 140-mer poly(A) tail.

**Table 20. Human GCSF expression for RNA 3-tail conjugates**

| | **Average (pg/mL)** | **Std Deviation** |
|---|---|---|
| **Tailless RNA 3** | 6.8E+03 | 2.2E+02 |
| **RNA 7** | 4.0E+05 | 9.5E+04 |
| **RNA 3-tail 1** | 1.9E+05 | 2.8E+04 |
| **RNA 3-tail 4** | 2.1E+05 | 2.2E+04 |

### Example 24. IFNβ levels in supernatant of BJ fibroblasts transfected with mRNA

Indicated mRNA (500 ng) was transfected in triplicate using Lipofectamine2000^{™} into BJ fibroblasts. After incubation for 48hrs, the supernatant was collected and used to measure the levels of human Interferon-β (R&D Systems). Table 21 gives the amount of detected IFNβ for RNA 2-tail conjugates and appropriate controls, where RNA 6 is a T7 RNA polymerase-transcribed construct containing a 140-mer poly(A) tail and wild type RNA 6 is transcribed with no modified nucleotides.

**Table 21. INFβ induced expression for RNA 2-tail conjugates**

| | **Average (pg/mL)** | **Std Deviation** |
|---|---|---|
| **Tailless RNA 2** | 450 | 29 |
| **wild type RNA 6** | 2700 | 50.0 |
| **RNA 6** | 13 | 0.3 |
| **RNA 2-tail 1** | 67 | 4.2 |
| **RNA 2-tail 4** | 140 | 11 |

## Claims

1. A chimeric polynucleotide encoding a polypeptide, wherein said chimeric polynucleotide has a sequence comprising Formula II:
[Aₙ]-L¹-[Bₒ] **Formula II**
wherein each A and B independently comprises any nucleoside;
n and o are, independently 10 to 1000, wherein [Aₙ] or [Bₒ] independently comprise an mRNA, said mRNA comprising a 5' untranslated region (UTR), 3'UTR and a coding region;
and
L¹ comprises the structure 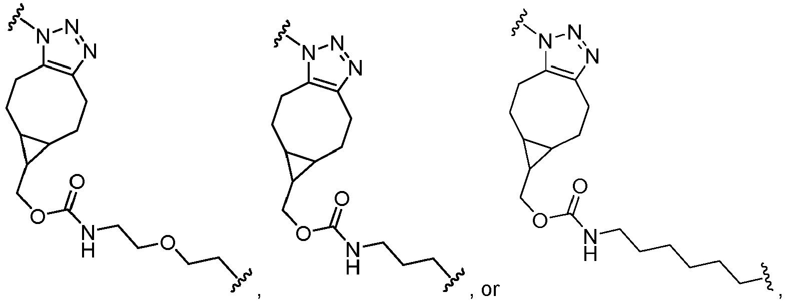
wherein L¹ is attached to [Aₙ] at the 3' or 4' position of the sugar of one of the nucleosides and to [Bₒ] at the 5' or 6' position of the sugar of one of the nucleosides.

2. The chimeric polynucleotide of claim 1, further comprising at least one 5' cap structure and/or further comprising a poly-A tail.

3. The chimeric polynucleotide of claims 1 or 2, wherein one of the coding region, the 5' UTR, the 3' UTR, the 5' cap structure, or the poly-A tail comprises [Aₙ]-L¹-[Bₒ], more preferably, wherein one of the coding region, the 5' UTR, the 3' UTR, the 5' cap structure, or the poly-A tail comprises [Aₙ] and another of the coding region, the 5' UTR, the 3' UTR, the 5' cap structure, or the poly-A tail comprises [Bₒ].

4. The chimeric polynucleotide of any one of claims 1 to 3 wherein the chimeric polynucleotide comprises at least one modified nucleoside.

5. The chimeric polynucleotide of any one of claims 2 to 4, wherein the poly-A tail terminates in the structure of Formula XXI: 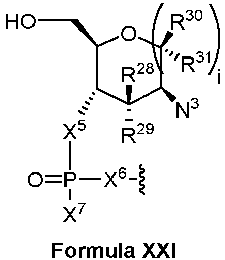 wherein N³ is a nucleobase
each of R²⁸, R²⁹, R³⁰, and R³¹ is, independently, H, halo, hydroxy, thiol, optionally substituted C₁-C₆ alkyl, optionally substituted C₁-C₆ heteroalkyl, optionally substituted C₂-C₆ heteroalkenyl, optionally substituted C₂-C₆ heteroalkynyl, optionally substituted amino, azido, or optionally substituted C₆-C₁₀ aryl;
i is 0 or 1;
X⁵ is O, NH, or S; and
X⁶ is O or S; and
X⁷ is OH or SH, or a salt thereof.

6. The chimeric polynucleotide of claim 5, wherein the structure of Formula XXI is:

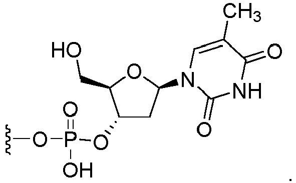

7. The chimeric polynucleotide of claim 6, wherein the poly-A tail has 40 to 80 nucleosides.

8. The chimeric polynucleotide of any one of claims 5 to 7, wherein said structure of Formula XXI is attached to two to four 2'-methoxy-adenosines and/or 2'-fluoro-adenosines.

9. The chimeric polynucleotide of any one of claims 5 to 8, wherein the poly-A tail terminates in the structure:

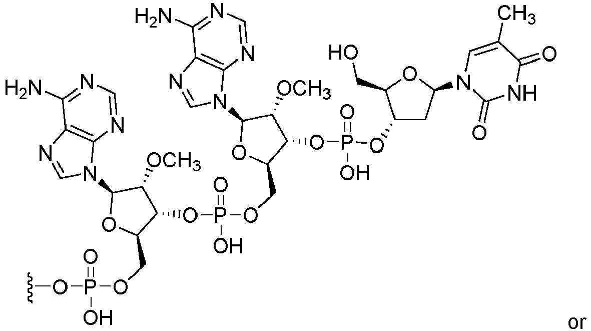

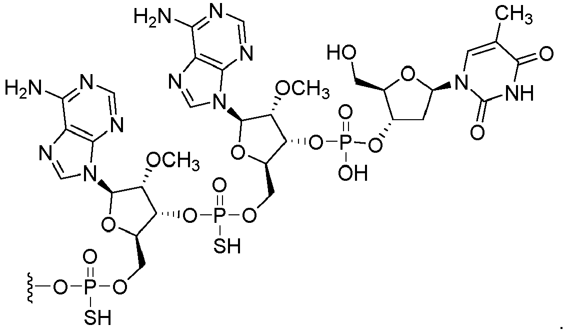

10. The chimeric polynucleotide of any one of claims 2 to 9, wherein the poly-A tail comprises the structure:

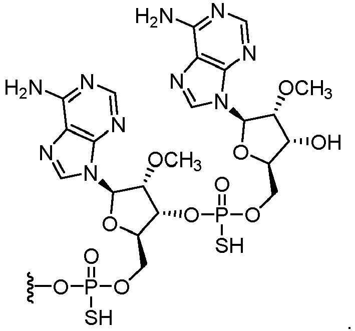

## Patentansprüche

1. Chimäres Polynukleotid, welches ein Polypeptid kodiert, wobei das chimäre Polynukleotid eine Sequenz hat, die die Formel II umfasst:
[Aₙ]-L¹-[Bₒ] **Formel II**
wobei jedes A und B unabhängig voneinander ein beliebiges Nukleosid umfassen;
n und o unabhängig voneinander 10 bis 1000 sind, wobei [Aₙ] oder [Bₒ] unabhängig voneinander eine mRNA umfassen, wobei die mRNA eine 5'-untranslatierte Region (UTR), 3'-UTR und eine kodierende Region umfasst; und
L¹ die Struktur 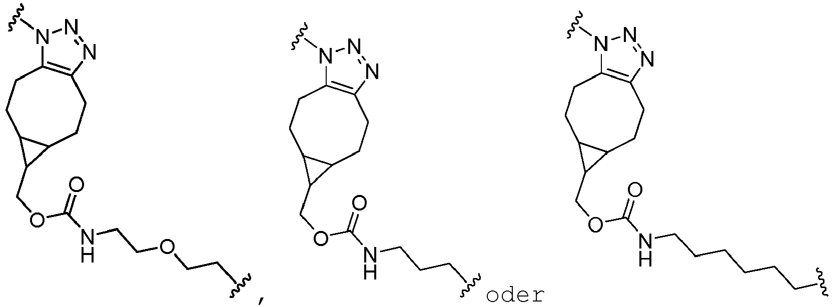
umfasst,
wobei L¹ an [Aₙ] an der 3'- oder 4'-Position des Zuckers eines der Nukleoside und an [Bₒ] an der 5'- oder 6'-Position des Zuckers eines der Nukleoside gebunden (attached) ist.

2. Chimäres Polynukleotid gemäß Anspruch 1, welches weitergehend mindestens eine 5'-Cap-Struktur und/oder weitergehend einen Poly-A-Schwanz umfasst.

3. Chimäres Polynukleotid gemäß Anspruch 1 oder 2, wobei eine der kodierenden Region, der 5'-UTR, der 3'-UTR, der 5'-Cap-Struktur oder des Poly-A-Schwanzes [Aₙ]-L¹-[Bₒ] umfasst, bevorzugter wobei eine der kodierenden Region, der 5'-UTR, der 3'-UTR, der 5'-Cap-Struktur oder des Poly-A-Schwanzes [Aₙ] umfasst und ein anderer der kodierenden Region, der 5'-UTR, der 3'-UTR, der 5'-Cap-Struktur oder des Poly-A-Schwanzes [Bₒ] umfasst.

4. Chimäres Polynukleotid gemäß irgendeinem der Ansprüche 1 bis 3, wobei das chimäre Polynukleotid mindestens ein modifiziertes Nukleosid umfasst.

5. Chimäres Polynukleotid gemäß irgendeinem der Ansprüche 2 bis 4, wobei der Poly-A-Schwanz in der Struktur der Formel XXI endet: 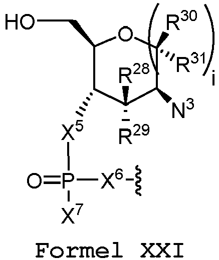 wobei N³ eine Nukleobase ist
jedes von R²⁸, R²⁹, R³⁰ und R³¹ unabhängig voneinander H, Halogen, Hydroxy, Thiol, optional substituiertes C₁-C₆-Alkyl, optional substituiertes C₁-C₆-Heteroalkyl, optional substituiertes C₂-C₆-Heteroalkenyl, optional substituiertes C₂-C₆-Heteroalkinyl, optional substituiertes Amino, Azido oder optional substituiertes C₆-C₁₀-Aryl ist;
i 0 oder 1 ist;
X⁵ O, NH oder S ist; und
X⁶ O oder S ist; und
X⁷ OH oder SH ist, oder ein Salz davon.

6. Chimäres Polynukleotid gemäß Anspruch 5, wobei die Struktur der Formel XXI: 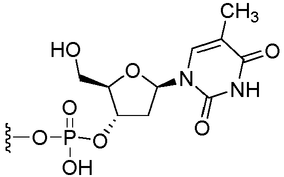 ist.

7. Chimäres Polynukleotid gemäß Anspruch 6, wobei der Poly-A-Schwanz 40 bis 80 Nukleoside aufweist.

8. Chimäres Polynukleotid gemäß irgendeinem der Ansprüche 5 bis 7, wobei die Struktur der Formel XXI an zwei bis vier 2'-Methoxyadenosine und/oder 2'-Fluoradenosine gebunden ist.

9. Chimäres Polynukleotid gemäß irgendeinem der Ansprüche 5 bis 8, wobei der Poly-A-Schwanz in der Struktur: 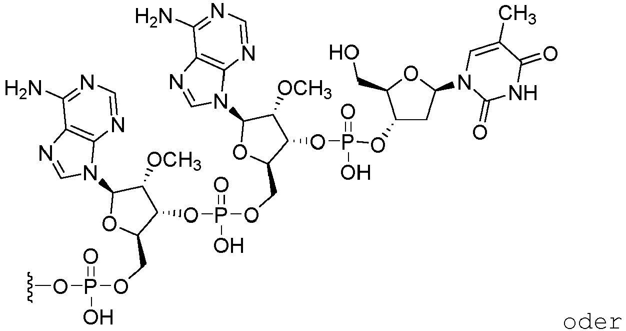 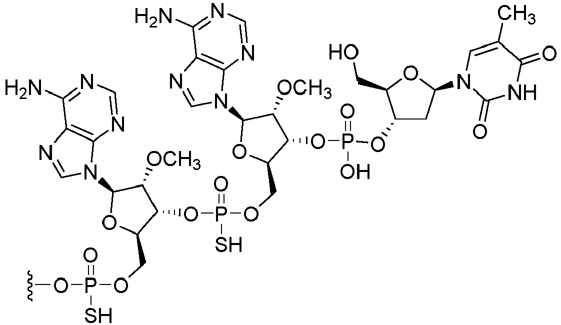 endet.

10. Chimäres Polynukleotid gemäß irgendeinem der Ansprüche 2 bis 9, wobei der Poly-A-Schwanz die Struktur: 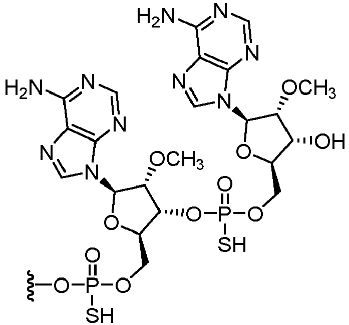 umfasst.

## Revendications

1. Polynucléotide chimérique codant pour un polypeptide, dans lequel ledit polynucléotide chimérique présente une séquence comprenant la formule II :
[Aₙ]-L¹-[Bₒ] Formule II
dans lequel chaque A et B comprennent indépendamment un quelconque nucléoside ;
n et o valent indépendamment 10 à 1000, dans lequel [Aₙ] ou [Bₒ] comprennent indépendamment un ARNm, ledit ARNm comprenant une région non traduite en 5'(UTR), une UTR en 3' et une région codante ; et L¹ comprend la structure 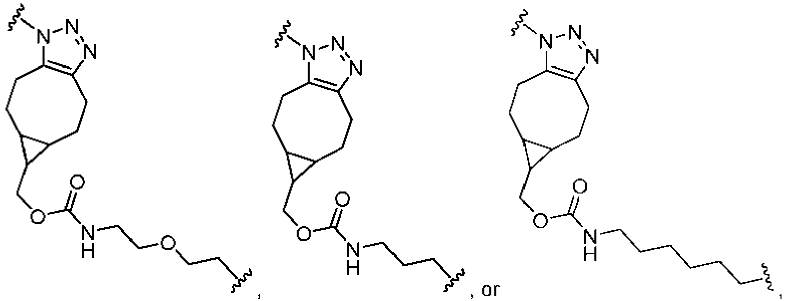
dans lequel L¹ est attaché à [Aₙ] en position 3' ou 4' du sucre d'un des nucléosides et à [Bₒ] en position 5' ou 6' du sucre d'un des nucléosides.

2. Polynucléotide chimérique selon la revendication 1, comprenant en outre au moins une structure de coiffe en 5' et/ou comprenant en outre une queue poly-A.

3. Polynucléotide chimérique selon les revendications 1 ou 2, dans lequel une parmi la région codante, l'UTR en 5', l'UTR en 3', la structure de coiffe en 5' ou la queue poly-A comprend [Aₙ]-L¹-[B₀], plus préférentiellement, dans lequel une parmi la région codante, l'UTR en 5', l'UTR en 3', la structure de coiffe en 5' ou la queue poly-A comprend [Aₙ] et une autre parmi la région codante, l'UTR en 5', l'UTR en 3', la structure de coiffe en 5' ou la queue poly-A comprend [Bₒ],

4. Polynucléotide chimérique selon l'une quelconque des revendications 1 à 3, dans lequel le polynucléotide chimérique comprend au moins un nucléoside modifié.

5. Polynucléotide chimérique selon l'une quelconque des revendications 2 à 4, dans lequel la queue poly-A se termine en la structure de formule XXI : 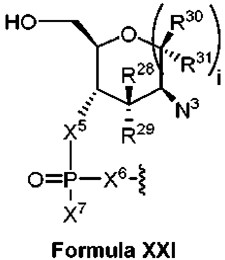
dans lequel N³ est une base nucléique
chacun de R²⁸, R²⁹, R³⁰ et R³¹ est, indépendamment, H, halo, hydroxy, thiol, alkyle en C₁-C₆ acultativement substitué, hétéroalkyle en C₁-C₆ facultativement substitué, hétéroalcényle en C₂-C₆ facultativement substitué, hétéroalcynyle en C₂-C₆ facultativement substitué, amino facultativement substitué, azido ou aryle en C₆-C₁₀ facultativement substitué ;
i est 0 ou 1 ;
X⁵ est O, NH, ou S; et
X⁶ est O ou S ; et
X⁷ est OH ou SH, ou un sel de celui-ci.

6. Polynucléotide chimérique selon la revendication 5, dans lequel la structure de formule XXI est :

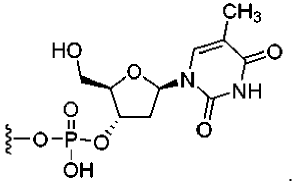

7. Polynucléotide chimérique selon la revendication 6, dans lequel la queue poly-A présente 40 à 80 nucléosides.

8. Polynucléotide chimérique selon l'une quelconque des revendications 5 à 7, dans lequel ladite structure de formule XXI est attachée à deux à quatre 2'-méthoxy-adénosines et/ou 2'-fluoro-adénosines.

9. Polynucléotide chimérique selon l'une quelconque des revendications 5 à 8, dans lequel la queue poly-A se termine en la structure :

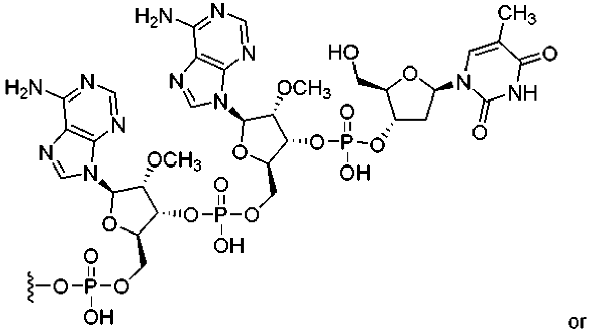

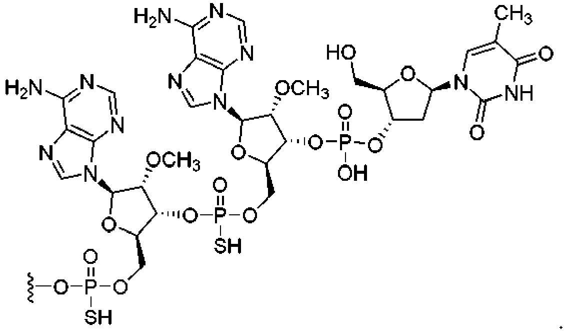

10. Polynucléotide chimérique selon l'une quelconque des revendications 2 à 9, dans lequel la queue poly-A comprend la structure :


### SEQUENCE LISTING

<110> MODERNA THERAPEUTICS, INC.
<120> CHIMERIC POLYNUCLEOTIDES
<130> 2030.1137PCT/M137.20
<140>
   <141>
<150> 62/045,359 <151> 2014-09-03
<150> 62/025,399 <151> 2014-07-16
<160> 24
<170> PatentIn version 3.5
<210> 1
   <211> 22
   <212> PRT
   <213> Artificial Sequence
<220>
   <223> Description of Artificial Sequence: Synthetic peptide
<400> 1
   Gly Ser Gly Ala Thr Asn Phe Ser Leu Leu Lys Gln Ala Gly Asp Val
   1               5                   10                  15
   Glu Glu Asn Pro Gly Pro
               20
<210> 2
   <211> 66
   <212> DNA
   <213> Artificial Sequence
<220>
   <223> Description of Artificial Sequence: Synthetic oligonucleotide
<400> 2
   ggaagcggag ctactaactt cagcctgctg aagcaggctg gagacgtgga ggagaaccct       60
   ggacct                                                                  66
<210> 3
   <211> 18
   <212> DNA
   <213> Artificial Sequence
<220>
   <223> Description of Artificial Sequence: Synthetic oligonucleotide
<400> 3
   attgggcacc cgtaaggg 18
<210> 4
   <211> 920
   <212> DNA
   <213> Artificial Sequence
<220>
   <223> Description of Artificial Sequence: Synthetic polynucleotide
<400> 4
   tcaagctttt ggaccctcgt acagaagcta atacgactca ctatagggaa ataagagaga 60
   aaagaagagt aagaagaaat ataagagcca ccatggtatc caagggggag gaggacaaca 120
   tggcgatcat caaggagttc atgcgattca aggtgcacat ggaaggttcg gtcaacggac 180
   acgaatttga aatcgaagga gagggtgaag gaaggcccta tgaagggaca cagaccgcga 240
   aactcaaggt cacgaaaggg ggaccacttc ctttcgcctg ggacattctt tcgccccagt 300
   ttatgtacgg gtccaaagca tatgtgaagc atcccgccga tattcctgac tatctgaaac 360
   tcagctttcc cgagggattc aagtgggagc gggtcatgaa ctttgaggac gggggtgtag 420
   tcaccgtaac ccaagactca agcctccaag acggcgagtt catctacaag gtcaaactgc 480
   gggggactaa ctttccgtcg gatgggccgg tgatgcagaa gaaaacgatg ggatgggaag 540
   cgtcatcgga gaggatgtac ccagaagatg gtgcattgaa gggggagatc aagcagagac       600
   tgaagttgaa agatggggga cattatgatg ccgaggtgaa aacgacatac aaagcgaaaa       660
   agccggtgca gcttcccgga gcgtataatg tgaatatcaa gttggatatt acttcacaca 720
   atgaggacta cacaattgtc gaacagtacg aacgcgctga gggtagacac tcgacgggag 780
   gcatggacga gttgtacaaa tgataatagg ctggagcctc ggtggccatg cttcttgccc 840
   cttgggcctc cccccagccc ctcctcccct tcctgcaccc gtacccccgt ggtctttgaa 900
   taaagtctga gtgggcggct 920
<210> 5
   <211> 725
   <212> **DNA**
   <213> Artificial Sequence
<220>
   <223> Description of Artificial Sequence: Synthetic polynucleotide
<400> 5
   tcaagctttt ggaccctcgt acagaagcta atacgactca ctatagggaa ataagagaga 60
   aaagaagagt aagaagaaat ataagagcca ccatggtttt taccctcgaa gattttgtcg 120
   gagattggag acagactgcc ggatacaacc ttgaccaagt cctcgagcaa ggcggtgtgt 180
   cgtcactctt ccaaaacctg ggtgtgtccg tgactcccat ccagcgcatc gtcctgagcg       240
   gcgaaaatgg gttgaagatc gacatccatg tgatcattcc atacgaggga ctgtccgggg       300
   accagatggg tcagatcgaa aagattttca aagtggtgta cccggtcgac gatcatcact       360
   tcaaggtgat cctgcactac ggaacgctgg tgatcgatgg ggtgaccccg aacatgattg       420
   actatttcgg acggccttac gagggcatcg cagtgttcga cggaaagaag atcaccgtga       480
   ccggcactct gtggaatgga aacaaaatca tcgacgaacg cctgatcaat ccggatggct       540
   cgctgttgtt ccgggtgacc attaacggag tcactggatg gaggctctgc gagcgcatcc       600
   ttgcgtgata ataggctgga gcctcggtgg ccatgcttct tgccccttgg gcctcccccc       660
   agcccctcct ccccttcctg cacccgtacc cccgtggtct ttgaataaag tctgagtggg       720
   cggct                                                                   725
<210> 6
   <211> 824
   <212> DNA
   <213> Artificial Sequence
<220>
   <223> Description of Artificial Sequence: Synthetic polynucleotide
<400> 6
   tcaagctttt ggaccctcgt acagaagcta atacgactca ctatagggaa ataagagaga        60
   aaagaagagt aagaagaaat ataagagcca ccatggccgg tcccgcgacc caaagcccca       120
   tgaaacttat ggccctgcag ttgctgcttt ggcactcggc cctctggaca gtccaagaag       180
   cgactcctct cggacctgcc tcatcgttgc cgcagtcatt ccttttgaag tgtctggagc       240
   aggtgcgaaa gattcagggc gatggagccg cactccaaga gaagctctgc gcgacataca       300
   aactttgcca tcccgaggag ctcgtactgc tcgggcacag cttggggatt ccctgggctc       360
   ctctctcgtc ctgtccgtcg caggctttgc agttggcagg gtgcctttcc cagctccact       420
   ccggtttgtt cttgtatcag ggactgctgc aagcccttga gggaatctcg ccagaattgg       480
   gcccgacgct ggacacgttg cagctcgacg tggcggattt cgcaacaacc atctggcagc       540
   agatggagga actggggatg gcacccgcgc tgcagcccac gcagggggca atgccggcct       600
   ttgcgtccgc gtttcagcgc agggcgggtg gagtcctcgt agcgagccac cttcaatcat       660
   ttttggaagt ctcgtaccgg gtgctgagac atcttgcgca gccgtgataa taggctggag       720
   cctcggtggc catgcttctt gccccttggg cctcccccca gcccctcctc cccttcctgc       780
   acccgtaccc ccgtggtctt tgaataaagt ctgagtgggc ggct                        824
<210> 7
   <211> 20
   <212> **DNA**
   <213> Artificial Sequence
<220>
   <223> Description of Artificial Sequence: Synthetic primer
<400> 7
   taatacgact cactataggg 20
<210> 8
   <211> 31
   <212> DNA
   <213> Artificial Sequence
<220>
   <223> Description of Artificial Sequence: Synthetic primer
<400> 8
   gccgcccact cagactttat tcaaagacca c 31
<210> 9
   <211> 111
   <212> DNA
   <213> Artificial Sequence
<220>
   <223> Description of Artificial Sequence: Synthetic primer
<400> 9
   tttttttttt tttttttttt tttttttttt tttttttttt tttttttttt tttttttttt 60
   tttttttttt tttttttttt gccgcccact cagactttat tcaaagacca c 111
<210> 10
   <211> 171
   <212> DNA
   <213> Artificial Sequence
<220>
   <223> Description of Artificial Sequence: Synthetic primer
<400> 10
   tttttttttt tttttttttt tttttttttt tttttttttt tttttttttt tttttttttt 60
   tttttttttt tttttttttt tttttttttt tttttttttt tttttttttt tttttttttt 120
   tttttttttt tttttttttt gccgcccact cagactttat tcaaagacca c 171
<210> 11
   <211> 874
   <212> RNA
   <213> Artificial Sequence
<220>
   <223> Description of Artificial Sequence: Synthetic polynucleotide
<400> 11
   gggaaauaag agagaaaaga agaguaagaa gaaauauaag agccaccaug guauccaagg      60
   gggaggagga caacauggcg aucaucaagg aguucaugcg auucaaggug cacauggaag     120
   guucggucaa cggacacgaa uuugaaaucg aaggagaggg ugaaggaagg cccuaugaag     180
   ggacacagac cgcgaaacuc aaggucacga aagggggacc acuuccuuuc gccugggaca     240
   uucuuucgcc ccaguuuaug uacgggucca aagcauaugu gaagcauccc gccgauauuc     300
   cugacuaucu gaaacucagc uuucccgagg gauucaagug ggagcggguc augaacuuug     360
   aggacggggg uguagucacc guaacccaag acucaagccu ccaagacggc gaguucaucu     420
   acaaggucaa acugcggggg acuaacuuuc cgucggaugg gccggugaug cagaagaaaa     480
   cgaugggaug ggaagcguca ucggagagga uguacccaga agauggugca uugaaggggg     540
   agaucaagca gagacugaag uugaaagaug ggggacauua ugaugccgag gugaaaacga     600
   cauacaaagc gaaaaagccg gugcagcuuc ccggagcgua uaaugugaau aucaaguugg     660
   auauuacuuc acacaaugag gacuacacaa uugucgaaca guacgaacgc gcugagggua     720
   gacacucgac gggaggcaug gacgaguugu acaaaugaua auaggcugga gccucggugg     780
   ccaugcuucu ugccccuugg gccucccccc agccccuccu ccccuuccug cacccguacc     840
   cccguggucu uugaauaaag ucugaguggg cggc                                 874
<210> 12
   <211> 679
   <212> RNA
   <213> Artificial Sequence
<220>
   <223> Description of Artificial Sequence: Synthetic polynucleotide
<400> 12
   gggaaauaag agagaaaaga agaguaagaa gaaauauaag agccaccaug guuuuuaccc      60
   ucgaagauuu ugucggagau uggagacaga cugccggaua caaccuugac caaguccucg     120
   agcaaggcgg ugugucguca cucuuccaaa accugggugu guccgugacu cccauccagc     180
   gcaucguccu gagcggcgaa aauggguuga agaucgacau ccaugugauc auuccauacg     240
   agggacuguc cggggaccag augggucaga ucgaaaagau uuucaaagug guguacccgg     300
   ucgacgauca ucacuucaag gugauccugc acuacggaac gcuggugauc gaugggguga     360
   ccccgaacau gauugacuau uucggacggc cuuacgaggg caucgcagug uucgacggaa     420
   agaagaucac cgugaccggc acucugugga auggaaacaa aaucaucgac gaacgccuga     480
   ucaauccgga uggcucgcug uuguuccggg ugaccauuaa cggagucacu ggauggaggc     540
   ucugcgagcg cauccuugcg ugauaauagg cuggagccuc gguggccaug cuucuugccc     600
   cuugggccuc cccccagccc cuccuccccu uccugcaccc guacccccgu ggucuuugaa     660
   uaaagucuga gugggcggc                                                  679
<210> 13
   <211> 778
   <212> RNA
   <213> Artificial Sequence
<220>
   <223> Description of Artificial Sequence: Synthetic polynucleotide
<400> 13
   gggaaauaag agagaaaaga agaguaagaa gaaauauaag agccaccaug gccggucccg      60
   cgacccaaag ccccaugaaa cuuauggccc ugcaguugcu gcuuuggcac ucggcccucu     120
   ggacagucca agaagcgacu ccucucggac cugccucauc guugccgcag ucauuccuuu     180
   ugaagugucu ggagcaggug cgaaagauuc agggcgaugg agccgcacuc caagagaagc     240
   ucugcgcgac auacaaacuu ugccaucccg aggagcucgu acugcucggg cacagcuugg     300
   ggauucccug ggcuccucuc ucguccuguc cgucgcaggc uuugcaguug gcagggugcc     360
   uuucccagcu ccacuccggu uuguucuugu aucagggacu gcugcaagcc cuugagggaa     420
   ucucgccaga auugggcccg acgcuggaca cguugcagcu cgacguggcg gauuucgcaa     480
   caaccaucug gcagcagaug gaggaacugg ggauggcacc cgcgcugcag cccacgcagg     540
   gggcaaugcc ggccuuugcg uccgcguuuc agcgcagggc ggguggaguc cucguagcga     600
   gccaccuuca aucauuuuug gaagucucgu accgggugcu gagacaucuu gcgcagccgu     660
   gauaauaggc uggagccucg guggccaugc uucuugcccc uugggccucc ccccagcccc     720
   uccuccccuu ccugcacccg uacccccgug gucuuugaau aaagucugag ugggcggc       778
<210> 14
   <211> 954
   <212> RNA
   <213> Artificial Sequence
<220>
   <223> Description of Artificial Sequence: Synthetic polynucleotide
<400> 14
   gggaaauaag agagaaaaga agaguaagaa gaaauauaag agccaccaug guauccaagg        60
   gggaggagga caacauggcg aucaucaagg aguucaugcg auucaaggug cacauggaag       120
   guucggucaa cggacacgaa uuugaaaucg aaggagaggg ugaaggaagg cccuaugaag       180
   ggacacagac cgcgaaacuc aaggucacga aagggggacc acuuccuuuc gccugggaca      240
   uucuuucgcc ccaguuuaug uacgggucca aagcauaugu gaagcauccc gccgauauuc       300
   cugacuaucu gaaacucagc uuucccgagg gauucaagug ggagcggguc augaacuuug       360
   aggacggggg uguagucacc guaacccaag acucaagccu ccaagacggc gaguucaucu       420
   acaaggucaa acugcggggg acuaacuuuc cgucggaugg gccggugaug cagaagaaaa       480
   cgaugggaug ggaagcguca ucggagagga uguacccaga agauggugca uugaaggggg       540
   agaucaagca gagacugaag uugaaagaug ggggacauua ugaugccgag gugaaaacga       600
   cauacaaagc gaaaaagccg gugcagcuuc ccggagcgua uaaugugaau aucaaguugg       660
   auauuacuuc acacaaugag gacuacacaa uugucgaaca guacgaacgc gcugagggua       720
   gacacucgac gggaggcaug gacgaguugu acaaaugaua auaggcugga gccucggugg       780
   ccaugcuucu ugccccuugg gccucccccc agccccuccu ccccuuccug cacccguacc       840
   cccguggucu uugaauaaag ucugaguggg cggcaaaaaa aaaaaaaaaa aaaaaaaaaa      900
   aaaaaaaaaa aaaaaaaaaa aaaaaaaaaa aaaaaaaaaa aaaaaaaaaa aaaa             954
<210> 15
   <211> 1014
   <212> RNA
   <213> Artificial Sequence
<220>
   <223> Description of Artificial Sequence: Synthetic polynucleotide
<400> 15
   gggaaauaag agagaaaaga agaguaagaa gaaauauaag agccaccaug guauccaagg        60
   gggaggagga caacauggcg aucaucaagg aguucaugcg auucaaggug cacauggaag       120
   guucggucaa cggacacgaa uuugaaaucg aaggagaggg ugaaggaagg cccuaugaag       180
   ggacacagac cgcgaaacuc aaggucacga aagggggacc acuuccuuuc gccugggaca      240
   uucuuucgcc ccaguuuaug uacgggucca aagcauaugu gaagcauccc gccgauauuc       300
   cugacuaucu gaaacucagc uuucccgagg gauucaagug ggagcggguc augaacuuug       360
   aggacggggg uguagucacc guaacccaag acucaagccu ccaagacggc gaguucaucu       420
   acaaggucaa acugcggggg acuaacuuuc cgucggaugg gccggugaug cagaagaaaa       480
   cgaugggaug ggaagcguca ucggagagga uguacccaga agauggugca uugaaggggg       540
   agaucaagca gagacugaag uugaaagaug ggggacauua ugaugccgag gugaaaacga        600
   cauacaaagc gaaaaagccg gugcagcuuc ccggagcgua uaaugugaau aucaaguugg       660
   auauuacuuc acacaaugag gacuacacaa uugucgaaca guacgaacgc gcugagggua       720
   gacacucgac gggaggcaug gacgaguugu acaaaugaua auaggcugga gccucggugg       780
   ccaugcuucu ugccccuugg gccucccccc agccccuccu ccccuuccug cacccguacc       840
   cccguggucu uugaauaaag ucugaguggg cggcaaaaaa aaaaaaaaaa aaaaaaaaaa       900
   aaaaaaaaaa aaaaaaaaaa aaaaaaaaaa aaaaaaaaaa aaaaaaaaaa aaaaaaaaaa       960
   aaaaaaaaaa aaaaaaaaaa aaaaaaaaaa aaaaaaaaaa aaaaaaaaaa aaaa            1014
<210> 16
   <211> 819
   <212> RNA
   <213> Artificial Sequence
<220>
   <223> Description of Artificial Sequence: Synthetic polynucleotide
<400> 16
   gggaaauaag agagaaaaga agaguaagaa gaaauauaag agccaccaug guuuuuaccc     60
   ucgaagauuu ugucggagau uggagacaga cugccggaua caaccuugac caaguccucg    120
   agcaaggcgg ugugucguca cucuuccaaa accugggugu guccgugacu cccauccagc    180
   gcaucguccu gagcggcgaa aauggguuga agaucgacau ccaugugauc auuccauacg    240
   agggacuguc cggggaccag augggucaga ucgaaaagau uuucaaagug guguacccgg    300
   ucgacgauca ucacuucaag gugauccugc acuacggaac gcuggugauc gaugggguga    360
   ccccgaacau gauugacuau uucggacggc cuuacgaggg caucgcagug uucgacggaa    420
   agaagaucac cgugaccggc acucugugga auggaaacaa aaucaucgac gaacgccuga    480
   ucaauccgga uggcucgcug uuguuccggg ugaccauuaa cggagucacu ggauggaggc    540
   ucugcgagcg cauccuugcg ugauaauagg cuggagccuc gguggccaug cuucuugccc    600
   cuugggccuc cccccagccc cuccuccccu uccugcaccc guacccccgu ggucuuugaa    660
   uaaagucuga gugggcggca aaaaaaaaaa aaaaaaaaaa aaaaaaaaaa aaaaaaaaaa    720
   aaaaaaaaaa aaaaaaaaaa aaaaaaaaaa aaaaaaaaaa aaaaaaaaaa aaaaaaaaaa    780
   aaaaaaaaaa aaaaaaaaaa aaaaaaaaaa aaaaaaaaa                           819
<210> 17
   <211> 918
   <212> RNA
   <213> Artificial Sequence
<220>
   <223> Description of Artificial Sequence: Synthetic polynucleotide
<400> 17
   gggaaauaag agagaaaaga agaguaagaa gaaauauaag agccaccaug gccggucccg     60
   cgacccaaag ccccaugaaa cuuauggccc ugcaguugcu gcuuuggcac ucggcccucu    120
   ggacagucca agaagcgacu ccucucggac cugccucauc guugccgcag ucauuccuuu    180
   ugaagugucu ggagcaggug cgaaagauuc agggcgaugg agccgcacuc caagagaagc    240
   ucugcgcgac auacaaacuu ugccaucccg aggagcucgu acugcucggg cacagcuugg    300
   ggauucccug ggcuccucuc ucguccuguc cgucgcaggc uuugcaguug gcagggugcc     360
   uuucccagcu ccacuccggu uuguucuugu aucagggacu gcugcaagcc cuugagggaa     420
   ucucgccaga auugggcccg acgcuggaca cguugcagcu cgacguggcg gauuucgcaa     480
   caaccaucug gcagcagaug gaggaacugg ggauggcacc cgcgcugcag cccacgcagg     540
   gggcaaugcc ggccuuugcg uccgcguuuc agcgcagggc ggguggaguc cucguagcga     600
   gccaccuuca aucauuuuug gaagucucgu accgggugcu gagacaucuu gcgcagccgu     660
   gauaauaggc uggagccucg guggccaugc uucuugcccc uugggccucc ccccagcccc     720
   uccuccccuu ccugcacccg uacccccgug gucuuugaau aaagucugag ugggcggcaa     780
   aaaaaaaaaa aaaaaaaaaa aaaaaaaaaa aaaaaaaaaa aaaaaaaaaa aaaaaaaaaa     840
   aaaaaaaaaa aaaaaaaaaa aaaaaaaaaa aaaaaaaaaa aaaaaaaaaa aaaaaaaaaa     900
   aaaaaaaaaa aaaaaaaa                                                   918
<210> 18
   <211> 21
   <212> DNA
   <213> Artificial Sequence
<220>
   <223> Description of Artificial Sequence: Synthetic oligonucleotide
<400> 18
   ggacaacaac aacaacaaca a 21
<210> 19
   <211> 21
   <212> DNA
   <213> Artificial Sequence
<220>
   <223> Description of Artificial Sequence: Synthetic oligonucleotide
<400> 19
   ggacaacaac aacaacaaca a 21
<210> 20
   <211> 55
   <212> DNA
   <213> Artificial Sequence
<220>
   <223> Description of Artificial Sequence: Synthetic oligonucleotide
<400> 20
   tttttttttt tttttttttt tttttttttt tttttgccgc ccactcagac tttat 55
<210> 21
   <211> 120
   <212> DNA
   <213> Artificial Sequence
<220>
   <223> Description of Artificial Sequence: Synthetic polynucleotide
<400> 21
   aaaaaaaaaa aaaaaaaaaa aaaaaaaaaa aaaaaaaaaa aaaaaaaaaa aaaaaaaaaa 60
   aaaaaaaaaa aaaaaaaaaa aaaaaaaaaa aaaaaaaaaa aaaaaaaaaa aaaaaaaaaa 120
<210> 22
   <211> 46
   <212> DNA
   <213> Artificial Sequence
<220>
   <223> Description of Artificial Sequence: Synthetic oligonucleotide
<400> 22
   actcttcttt tctctcttat ttcccttgtt gttgttgttg ttgtcc 46
<210> 23
   <211> 80
   <212> DNA
   <213> Artificial Sequence
<220>
   <223> Description of Artificial Sequence: Synthetic oligonucleotide
<220>
   <221> modified_base
   <222> (41)..(80)
   <223> May or may not be present
<400> 23
   aaaaaaaaaa aaaaaaaaaa aaaaaaaaaa aaaaaaaaaa aaaaaaaaaa aaaaaaaaaa 60
   aaaaaaaaaa aaaaaaaaaa 80
<210> 24
   <211> 120
   <212> DNA
   <213> Artificial Sequence
<220>
   <223> Description of Artificial Sequence: Synthetic polynucleotide
<400> 24
   tttttttttt tttttttttt tttttttttt tttttttttt tttttttttt tttttttttt 60
   tttttttttt tttttttttt tttttttttt tttttttttt tttttttttt tttttttttt 120